# EUROPÄISCHE PATENTSCHRIFT

(11) **EP 1 599 582 B1**
(45) Veröffentlichungstag und Bekanntmachung des Hinweises auf die Patenterteilung: **27.07.2011**
(21) Anmeldenummer: 04706146.0
(22) Anmeldetag: 29.01.2004
(51) Int. Cl.: C12N 9/10, C12N 15/82, C12P 7/64

(54) **VERFAHREN ZUR HERSTELLUNG MEHRFACH UNGESÄTTIGTER FETTSÄUREN**
METHOD FOR THE PRODUCTION OF POLYUNSATURATED FATTY ACIDS
PROCEDE DE PRODUCTION D'ACIDES GRAS POLYINSATURES

(30) Priorität: 27.02.2003 DE 10308836
(43) Veröffentlichungstag der Anmeldung: 30.11.2005
(73) Patentinhaber: BASF Plant Science GmbH, 67056 Ludwigshafen (DE)
(72) Erfinder: RENZ, Andreas, 67117 Limburgerhof (DE); HEINZ, Ernst, 22609 Hamburg (DE); ABBADI, Amine, 37136 Ebergötzen (DE); DOMERGUE, Frederic, 22761 Hamburg (DE); ZANK, Thorsten, 68165 Mannheim (DE)
(74) Vertreter: Popp, Andreas
(86) Internationale Anmeldenummer: PCT/EP2004/000771
(87) Internationale Veröffentlichungsnummer: WO 2004/076617

(56) Entgegenhaltungen:
- WO-A-01/59128
- WO-A-02/072742
- LOPEZ ALONSO D ET AL: "Plants as 'chemical factories' for the production of polyunsaturated fatty acids" BIOTECHNOLOGY ADVANCES, ELSEVIER PUBLISHING, BARKING, GB, Bd. 18, Nr. 6, Oktober 2000 (2000-10), Seiten 481-497, XP002208312 ISSN: 0734-9750
- DATABASE UNIPRIROT [Online] 15. Dezember 1998 (1998-12-15), WILKINSON: "Putative 1-acyl-sn-glycerol-3-phosphate acyltransferase T06E8.1 (EC 2.3.1.51) (1-AGP acyltransferase) (1-AGPAT) (Lysophosphatidic acid acyltransferase) (LPAAT)." XP002290711 gefunden im EBI Database accession no. Q22267 & DATABASE EMBL [Online] Caenorhabditis elegans cosmid T06E8 9. Juni 1996 (1996-06-09), WILKINSON: "Genome sequence of the nematode C.elegans: A platform for investigating biology" gefunden im EBI Database accession no. Z73975

## Beschreibung

Die vorliegende Erfindung betrifft ein Verfahren zur Herstellung von mehrfach ungesättigten Fettsäuren in einem Organismus indem Nukleinsäuren in den Organismus eingebracht werden, die für Polypeptide mit Acyl-CoA:Lysophospholipid-Acyltransferaseaktivität codieren. Vorteilhaft können diese Nukleinsäuresequenzen gegebenenfalls zusammen mit weiteren Nukleinsäuresequenzen, die für Polypeptide der Biosynthese des Fettsäure- oder Lipidstoffwechels codieren, in dem transgenen Organismus exprimiert werden.

Fettsäuren und Triglyceride haben eine Vielzahl von Anwendungen in der Lebensmittelindustrie, der Tierernährung, der Kosmetik und im Pharmabereich. Je nachdem ob es sich um freie gesättigte oder ungesättigte Fettsäuren oder um Triglyceride mit einem erhöhten Gehalt an gesättigten oder ungesättigten Fettsäuren handelt, sind sie für die unterschiedlichsten Anwendungen geeignet, so werden beispielsweise mehrfach ungesättigte Fettsäuren Babynahrung zur Erhöhung des Nährwertes zugesetzt.. Mehrfach ungesättigte ω-3-Fettsäuren und ω-6-Fettsäuren stellen dabei einen wichtigen Bestandteil der tierischen und menschlichen Nahrung dar. Aufgrund der heute üblichen Zusammensetzung der menschlichen Nahrung ist ein Zusatz von mehrfach ungesättigten ω-3-Fettsäuren, die bevorzugt in Fischölen vorkommen, zur Nahrung besonders wichtig. So werden beispielsweise mehrfach ungesättigte Fettsäuren wie Docosahexaensäure (= DHA, C22:6^{Δ4,7,10,13,16,19}) oder Eisosapentaensäure (= EPA, C20:5^{Δ5,8,11,14,17}) Babynahrung zur Erhöhung des Nährwertes zugesetzt. Der ungesättigten Fettsäure DHA wird dabei ein positiver Effekt auf die Entwicklung des Gehirns zugeschrieben.

Im folgenden werden mehrfach ungesättigte Fettsäuren als PUFA, PUFAs, LCPUFA oder LCPUFAs bezeichnet (**p**oly **u**nsaturated **f**atty **a**cids, **PUFA**, **l**ong **c**hain **p**oly **u**nsaturated **f**atty acids, **LCPUFA**).

Hauptsächlich werden die verschiedenen Fettsäuren und Triglyceride aus Mikroorganismen wie Mortierella oder Schizochytrium oder aus Öl-produzierenden Pflanzen wie Soja, Raps, Algen wie Crypthecodinium oder Phaeodactylum und weiteren gewonnen, wobei sie in der Regel in Form ihrer Triacylglyceride (= Triglyceride = Triglycerole) anfallen. Sie können aber auch aus Tieren wie z.B. Fischen gewonnen werden. Die freien Fettsäuren werden vorteilhaft durch Verseifung hergestellt. Höhere mehrfach ungesättigte Fettsäuren wie DHA, EPA, Arachidonsäure (= ARA, C20:4^{Δ5,8,11,14}), Dihomo-γlinolensäure (C20:3^{Δ8,11,14}) oder Docosapentaensäure (DPA, C22:5^{Δ7,10,13,16,19}) lassen sich nicht aus Ölfruchtpflanzen wie Raps, Soja, Sonnenblume, Färberdistel oder anderen isolieren. Übliche natürliche Quellen für diese Fettsäuren sind Fische wie Hering, Lachs, Sardine, Goldbarsch, Aal, Karpfen, Forelle, Heilbutt, Makrele, Zander oder Thunfisch oder Algen.

Je nach Anwendungszweck sind Öle mit gesättigten oder ungesättigten Fettsäuren bevorzugt, so sind zB. in der humanen Ernährung Lipide mit ungesättigten Fettsäuren speziell mehrfach ungesättigten Fettsäuren bevorzugt. Den mehrfach ungesättigten ω-3-Fettsäuren wir dabei ein positiver Effekt auf den Cholesterinspiegel im Blut und damit auf die Möglichkeit der Prävention einer Herzerkrankung zugeschrieben. Durch Zugabe dieser ω-3-Fettsäuren zu Nahrung kann das Risiko einer Herzerkrankung, eines Schlaganfalls oder von Bluthochdruck deutlich verringert werden. Auch entzündliche speziell chronisch entzündliche Prozesse im Rahmen immunologischer Erkrankungen wie rheumatroider Arthritis lassen sich durch w-3-Fettsäuren positiv beeinflussen. Sie werden deshalb Lebensmitteln speziell diätischen Lebensmitteln zugegeben oder finden in Medikamenten Anwendung. ω-6-Fettsäuren wie Arachidonsäure haben bei diesen rheumatischen Erkrankungen aufgrund unserer üblichen Nahrungsmittelzusammensetzung eher einen negativen Effekt auf diese Krankheiten.

ω-3- und ω-6-Fettsäuren sind Vorläufer von Gewebshormonen, den sogenannten Eicosanoiden wie den Prostaglandinen, die sich von der Dihomo-γ-linolensäure, der Arachidonsäure und der Eicosapentaensäure ableiten, den Thromoxanen und Leukotrienen, die sich von der Arachidonsäure und der Eicosapentaensäure ableiten. Eicosanoide (sog. PG₂-Serie), die aus ω-6-Fettsäuren gebildet werden fördern in der Regel Entzündungsreaktionen, während Eicosanoide (sog. PG₃-Serie) aus ω-3-Fettsäuren geringe oder keine entzündungsfördernde Wirkung haben.

Aufgrund ihrer positiven Eigenschaften hat es in der Vergangenheit nicht an Ansätzen gefehlt, Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine Δ-15-Desaturase in WO 94/11516 wird eine Δ-12-Desaturase beansprucht. Weitere Desaturasen werden beispielsweise in EP-A-0 550162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 oder Huang et al., Lipids 34, 1999: 649-659 beschrieben. Die biochemische Charakterisierung der verschiedenen Desaturasen ist jedoch bisher nur unzureichend erfolgt, da die Enzyme als membrangebundene Proteine nur sehr schwer zu isolieren und zu charakterisieren sind (Mckeon et al., Methods in Enzymol. 71, 1981: 12141-12147, Wang et al., Plant Physiol. Biochem., 26, 1988: 777-792). In der Regel erfolgt die Charakterisierung membrangebundener Desaturasen durch Einbringung in einen geeigneten Organismus, der anschließend auf Enzymaktivität mittels Edukt- und Produktanalyse untersucht wird. Δ-6-Desaturasen werden in WO 93/06712, US 5,614,393, US5614393, WO 96/21022, WO00/21557 und WO 99/27111 beschrieben und auch die Anwendung zur Produktion in transgenen Organismen beschrieben wie in WO98/46763 WO98/46764, WO9846765. Dabei wird auch die Expression verschiedener Desaturasen wie in WO99/64616 oder WO98/46776 und Bildung polyungesättigter Fettsäuren beschrieben und beansprucht. Bzgl. der Effektivität der Expression von Desaturasen und ihren Einfluss auf die Bildung polyungesättigter Fettsäuren ist anzumerken, dass durch Expression einer einzelnen Desaturase wie bisher beschrieben lediglich geringe Gehalte an ungesättigten Fettsäuren/Lipiden wie z.B. γ-Linolensäure und Stearidonsäure erreicht wurden. Weiterhin wurde in der Regel ein Gemisch aus ω-3- und ω-6-Fettsäuren erhalten.

Besonders geeignete Mikroorganismen zur Herstellung von PUFAs sind Mikroorganismen wie Thraustochytrien oder Schizochytrien-Stämme, Algen wie Phaeodactylum tricornutum oder Crypthecodinium-Arten, Ciliaten, wie Stylonychia oder Colpidium, Pilze, wie Mortierella, Entomophthora oder Mucor. Durch Stammselektion ist eine Anzahl von Mutantenstämmen der entsprechenden Mikroorganismen entwickelt worden, die eine Reihe wünschenswerter Verbindungen, einschließlich PUFAs, produzieren. Die Mutation und Selektion von Stämmen mit verbesserter Produktion eines bestimmten Moleküls wie den mehrfach ungesättigten Fettsäuren ist jedoch ein zeitraubendes und schwieriges Verfahren. Deshalb werden, wenn immer möglich wie oben beschrieben gentechnologische Verfahren bevorzugt. Mit Hilfe der vorgenannten Mikroorganismen lassen sich jedoch nur begrenzte Mengen der gewünschten mehrfach ungesättigten Fettsäuren wie DPA, EPA oder ARA herstellen. Wobei diese in der Regel je nach verwendeten Mikroorganismus als Fettsäuregemische aus beispielsweise EPA, DPA und DHA anfallen.

Alternativ kann die Produktion von Feinchemikalien im großen Maßstab vorteilhaft über die Produktion in Pflanzen durchgeführt werden, die so entwickelt werden, dass sie die vorstehend genannten PUFAs herstellen. Besonders gut für diesen Zweck geeignete Pflanzen sind Ölfruchtpflanzen, die große Mengen an Upidverbindungen enthalten wie Raps, Canola, Lein, Soja, Sonnenblumen, Borretsch und Nachtkerze. Aber auch andere Nutzpflanzen, die Öle oder Lipide und Fettsäuren enthalten, sind gut geeignet, wie in der eingehenden Beschreibung dieser Erfindung erwähnt. Mittels herkömmlicher Züchtung ist eine Reihe von Mutantenpflanzen entwickelt worden, die ein Spektrum an wünschenswerten lipiden und Fettsäuren, Cofaktoren und Enzymen produzieren. Die Selektion neuer Pflanzensorten mit verbesserter Produktion eines bestimmten Moleküls ist jedoch ein zeitaufwändiges und schwieriges Verfahren oder sogar unmöglich, wenn die Verbindung in der entsprechenden Pflanze nicht natürlich vorkommt, wie im Fall von mehrfach ungesättigten C₁₈-, C₂₀-Fettsäuren und C₂₂-Fettsäuren und solchen mit längeren Kohlenstoffketten.

Aufgrund der positiven Eigenschaften ungesättigter Fettsäuren hat es in der Vergangenheit nicht an Ansätzen gefehlt, diese Gene, die an der Synthese von Fettsäuren bzw. Triglyceriden beteiligt sind, für die Herstellung von Ölen in verschiedenen Pflanzen mit einem geändertem Gehalt an mehrfach ungesättigten Fettsäuren verfügbar zu machen. Bisher konnten jedoch längerkettige mehrfach ungesättigte C₂₀- und/oder C₂₂-Fettsäuren wie EPA oder ARA nicht in Pflanzen hergestellt werden.

Aber auch in anderen Organismen wie Mikroorganismen wie Algen oder Pilzen führten die gentechnischen Veränderungen des Fettsäurestoffwechselweges über das Einbringen und die Expression beispielsweise von Desaturasen nur zu relativ geringen Steigerungen der Produktivität in diesen Organismen. Ein Grund hierfür mag in dem sehr komplexen Fettsäurestoffwechsel liegen. So ist der Einbau von mehrfach ungesättigten Fettsäuren in Membranlipide und/oder in Triacylglyceride und deren Ab- und Umbau sehr komplex und bis heute biochemisch und speziell genetisch noch nicht vollständig aufgeklärt und verstanden.

Die Biosynthese von LCPUFAs und der Einbau von LCPUFAs in Membranen oder Triacylglyceride erfolgt über verschiedene Stoffwechselwege (Abbadi et al. (2001) European Journal of Lipid Science & Technology 103:106-113). In Bakterien wie Vibrio und Mikroalgen wie Schizochytrium wird Malonyl-CoA über eine LCPUFAproduzierende Polyketidsynthase zu LCPUFAs umgesetzt (Metz et al. (2001) Science 293: 290-293; WO 00/42195; WO 98/27203; WO 98/55625). In Mikroalgen wie Phaeodactylum und Moosen wie Physcomitrella werden ungesättigte Fettsäuren wie Linolsäure oder Linolensäure in Form ihrer Acyl-CoAs in mehreren Desaturierungs- und Elongationsschritten zu LCPUFAs umgesetzt (Zank et al. (2000) Biochemical Society Transactions 28: 654-658). Bei Säugetieren beinhaltet die Biosynthese von DHA zusätzlich zu Desaturierungs- und Elongationsschritten eine Kettenverkürzung über β-Oxidation.

LCPUFAs liegen in Mikroorganismen und niederen Pflanzen entweder ausschließlich in Form von Membranlipiden vor, wie bei Physcomitrella und Phaeodactylum oder sie sind in Membranlipiden und Triacylglyceriden vorhanden, wie bei Schizochytrium und Mortierella. Der Einbau von LCPUFAs in Lipide und Öle wird durch verschiedene - Acyttransferasen und Transacylasen katalysiert. Diese Enzyme sind bereits bekannt für den Einbau von gesättigten und ungesättigten Fettsäuren [Slabas (2001) J. Plant Physiology 158: 505-513; Frentzen (1998) Fett/Lipid 100: 161-166); Cases et al. (1998) Proc. Nat. Acad. Sci. USA 95: 13018-13023]. Bei den Acyltransferasen handelt sich um Enzyme des sogenannten Kennedy-Pathways, die an der cytoplasmatischen Seite des Membransystems des Endoplasmatischen Reticulums, nachfolgend als ,ER' bezeichnet, lokalisiert sind. Experimentell können Membranen des ER als sogenannte ,mikrosomale Fraktionen' aus verschiedenen Organismen isoliert werden (Knutzon et al. (1995) Plant Physiology 109: 999-1006; Mishra & Kamisaka (2001) Biochemistry 355: 315-322; US 5968791). Diese ER-gebundenen Acyltransferasen in der mikrosomalen Fraktion verwenden Acyl-CoA als aktivierte Form der Fettsäuren. Glycerin-3-phosphat Acyltransferase, im folgenden GPAT genannt, katalysiert den Einbau von Acylgruppen an der sn-1 Position von Glycerin-3-phosphat. 1-Acylglycerin-3-phosphat Acyltransferase (EC. 2.3.1.51), auch Lysophosphatidsäure Acyltransferase, im folgenden LPAAT genannt, katalysiert den Einbau von Acylgruppen an der sn-2 Position von Lysophosphatidsäure, nachfolgend als LPA abgekürzt. Nach Dephosphorylierung von Phosphatidsäure durch Phosphatidsäure Phosphatase katalysiert Diacylglycerin Acyltransferase, im folgenden DAGAT genannt, den Einbau von Acylgruppen an der sn-3 Position von Diacylglycerins. Neben diesen Kennedy Pathway Enzymen sind weitere Enzyme am Einbau von Fettsäuren in Triacylglyceride beteiligt, die Acylgruppen aus Membranlipiden in Triacylglyceride einbauen können. Phospholipid Diacylglycerin Acyltransferase, nachfolgend PDAT genannt, und Lysophosphatidylcholin Acyltransferase, nachfolgend LPCAT genannt

Die enzymatische Aktivität einer LPCAT wurde erstmals in Ratten beschrieben [Land (1960) Journal of Biological Chemistry 235: 2233-2237]. In Pflanzen existiert eine plastidäre Isoform der LPCAT [Akermoun et al. (2000) Biochemical Society Transactions 28: 713-715] sowie eine ER gebundene Isoform [Tumaney und Rajasekharan (1999) Biochimica et Biophysica Acta 1439: 47-56; Fraser und Stobart, Biochemical Society Transactions (2000) 28: 715-7718]. LPCAT ist in Tieren wie auch in Pflanzen an der Biosynthese und der Transacylierung von mehrfach ungesättigten Fettsäuren beteiligt [Stymne und Stobart (1984) Biochem. J. 223: 305-314; Stymne und Stobart (1987) in 'The Biochemistry of Plants: a Comprehensive Treatise', Vol. 9 (Stumpf, P.K. ed.) pp. 175-214, Academic Press, New York]. Eine wichtige Funktion der LPCAT oder allgemeiner gesagt einer Acyl-CoA:Lysophospholipid Acyltransferase, nachfolgend LPLAT genannt, bei der ATP-unabhängigen Synthese von Acyl-CoA aus Phospholipiden wurde von Yamashita et al. (2001; Journal of Biological Chemistry 276: 26745-26752) beschrieben.

Trotz vieler biochemischer Daten konnten bisher keine Gene kodierend für LPCAT identifiziert werden. Gene anderer verschiedener pflanzlicher Acyltransferasen konnten isoliert werden und werden in WO 00/18889 (Novel Plant Acyltransferases) beschrieben.

Höhere Pflanzen enthalten mehrfach ungesättigte Fettsäuren wie Linolsäure (C18:2) und Linolensäure (C18:3). Arachidonsäure (ARA), Eicosapentaensäure (EPA) und Docosahexaensäure (DHA) kommen wie oben beschrieben im Samenöl höherer Pflanzen gar nicht oder nur in Spuren vor (E. Ucciani: Nouveau Dictionnaire des Huiles Végétales. Technique & Documentation - Lavoisier, 1995. ISBN: 2-7430-0009-0). Es ist vorteilhaft, in höheren Pflanzen, bevorzugt in Ölsaaten wie Raps, Lein, Sonnenblume und Soja, LCPUFAs herzustellen, da auf diese Weise große Mengen qualitativ hochwertiger LCPUFAs für die Lebensmittelindustrie, die Tieremährung und für pharmazeutische Zwecke kostengünstig gewonnen werden können. Hierzu werden vorteilhaft über gentechnische Methoden Gene kodierend für Enzyme der Biosynthese von LCPUFAs in Ölsaaten eingeführt und exprimiert werden. Dies sind beispielsweise Gene kodierend für Δ-6-Desaturase, Δ-6-Elongase, Δ-5-Desaturase, Δ-5-Elongase und Δ-4-Desaturase. Diese Gene können vorteilhaft aus Mikroorganismen, Tieren und niederen Pflanzen isoliert werden, die LCPUFAs herstellen und in den Membranen oder Triacylglyceriden einbauen. So konnten bereits Δ-6-Desaturase-Gene aus dem Moos Physcomitrella patens und Δ-6-Elongase-Gene aus P. patens und dem Nematoden C. elegans isoliert.

Erste transgene Pflanzen, die Gene kodierend für Enzyme der LCPUFA-Biosynthese enthalten und exprimieren und LCPUFAs produzieren wurden beispielsweise in DE 102 19 203 (Verfahren zur Herstellung mehrfach ungesättigter Fettsäuren in Pflanzen) erstmals beschrieben. Diese Pflanzen produzieren allerdings LCPUFAs in Mengen, die für eine Aufarbeitung der in den Pflanzen enthaltenen Öle noch weiter optimiert werden müssen.

Um eine Anreicherung der Nahrung und des Futters mit diesen mehrfach ungesättigten Fettsäuren zu ermöglichen, besteht daher ein großer Bedarf an einem einfachen, kostengünstigen Verfahren zur Herstellung dieser mehrfach ungesättigten Fettsäuren speziell in eukaryontischen Systemen.

Es bestand daher die Aufgabe ein Verfahren zur Herstellung von mehrfach ungesättigten Fettsäuren in einem eukaryontischen Organismus zu entwickeln. Diese Aufgabe wurde durch das erfindungsgemäße Verfahren zur Herstellung mehrfach ungesättigter Fettsäuren in einem Organismus, dadurch gekennzeichnet, dass das Verfahren - folgende Schritte umfasst:
a) Einbringen mindestens einer Nukleinsäuresequenz in den Organismus mit der in SEQ ID NO: 1, SEQ ID NO: 3, SEQ ID NO: 5 oder SEQ ID NO: 7 dargestellten Sequenz, die für ein Polypeptid mit einer Acyl-CoA:Lysophospholipid-Acyltransferaseaktivität codiert; oder '
b) Einbringen mindestens einer Nukleinsäuresequenz in den Organismus, die sich als Ergebnis des degenerierten genetischen Codes von der in SEQ ID NO:1, SEQ ID NO: 3, SEQ ID NO: 5 oder SEQ ID NO: 7 enthaltenden codierenden Sequenz ableiten lässt, oder
c) Einbringen mindestens eines Derivates der in SEQ ID NO: 1, SEQ ID NO: 3, SEQ ID NO: 5 oder SEQ ID NO: 7 dargestellten Nukleinsäuresequenz in den Organismus, die für Polypeptide mit der in SEQ ID NO: 2, SEQ ID NO: 4, SEQ ID NO: 6 oder SEQ ID NO: 8 dargestellten Aminosäuresequenz codieren und mindestens 90 % Homologie auf Aminosäureebene mit SEQ ID NO: 2, SEQ ID NO: 4, SEQ ID NO: 6 oder SEQ ID NO: 8 aufweisen und cyl-CoA:Lysophospholipid-Acyltransferaseaktivität aufweisen, und
d) kultivieren und ernten des Organismus.

Vorteilhaft enthalten die im erfindungsgemäßen Verfahren hergestellten mehrfach ungesättigten Fettsäuren mindestens zwei vorteilhaft drei Doppelbindungen. Besonders vorteilhaft enthalten die Fettsäuren vier oder fünf Doppelbindungen. Im Verfahren hergestellte Fettsäuren haben vorteilhaft 16-, 18-, 20- oder 22 C-Atome in der Fettsäurekette. Diese hergestellten Fettsäuren können als einziges Produkt im Verfahren hergestellt werden oder in einem Fettsäuregemisch vorliegen.

Bei den im erfindungsgemäßen Verfahren verwendeten Nukleinsäuresequenzen handelt es sich um isolierte Nukleinsäuresequenzen, die für Polypeptide mit Acyl-CoA:Lysophospholipid-Acyltransferaseaktivität codieren.

Die im Verfahren hergestellten mehrfach ungesättigten Fettsäuren sind vorteilhaft in Membranlipiden und/oder Triacylglyceriden gebunden, können aber auch als frei Fettsäuren oder aber gebunden in Form anderer Fettsäureester in den Organismen vorkommen. Dabei können sie wie gesagt als "Reinprodukte" oder aber vorteilhaft in Form von Mischungen verschiedener Fettsäuren oder Mischungen unterschiedlicher Glyceride vorliegen. Dabei lassen sich die in den Triacylglyceriden gebundenen verschieden Fettsäuren von kurzkettigen Fettsäuren mit 4 bis 6 C-Atomen, mittelkettigen Fettsäuren mit 8 bis 12 C-Atomen oder langkettigen Fettsäuren mit 14 bis 24 C-Atomen ableiten, bevorzugt sind die langkettigen Fettsäuren besonders bevorzugt sind die langkettigen Fettsäuren LCPUFAs von C₁₈-, C₂₀- und/oder C₂₂-Fettsäuren.

Im erfindungsgemäßen Verfahren werden vorteilhaft Fettsäureester mit mehrfach ungesättigten C₁₆-, C₁₈-, C₂₀- und/oder C₂₂-Fettsäuremolekülen mit mindestens zwei Doppelbindungen im Fettsäureester hergestellt. Bevorzugt enthalten diese Fettsäuremoleküle drei, vier oder fünf Doppelbindungen und führen vorteilhaft zur Synthese von Hexadecadiensäure (C16:2^{Δ9,12}), γ-Linolensäure (= GLA, C18:3^{Δ6,9,12}), Stearidonsäure (= SDA, C18:4^{Δ6,9,12,15}), Dihomo-γ-Linolensäure (= DGLA, 20:3^{Δ8,11,14}), Eicosatetraensäure (= ETA, C20:4^{Δ5,8,11,14}), Arachidonsäure (ARA), Eicosapentaensäure (EPA) oder deren Mischungen, bevorzugt EPA und/oder ARA.

Die Fettsäureester mit mehrfach ungesättigten C₁₆-, C₁₈-, C₂₀- und/oder C₂₂-Fettsäuremolekülen können aus den Organismen, die für die Herstellung der Fettsäureester verwendet wurden, in Form eines Öls oder Lipids beispielsweise in Form von Verbindungen wie Sphingolipide, Phosphoglyceride, Lipide, Glycolipide wie Glycosphingolipid, Phospholipide wie Phosphatidylethanolamin, Phosphatidylchotin, Phosphatidylserin, Phosphatidylglycerol, Phosphatidylinositol oder Diphosphatidylglycerol, Monoacylglyceride, Diacylglyceride, Triacylglyceride oder sonstige Fettsäureester wie die AcetylCoenzymA-Ester, die die mehrfach ungesättigten Fettsäuren mit mindestens zwei bevorzugt drei Doppelbindungen enthalten, isoliert werden. Neben diesen Estern sind die mehrfach ungesättigten Fettsäuren auch als freie Fettsäuren oder gebunden in anderen Verbindungen in den Organismen vorteilhaft den Pflanzen enthalten. In der Regel liegen die verschiedenen vorgenannten Verbindungen (Fettsäureester und frei Fettsäuren) in den Organismen in einer ungefähren Verteilung von 80 bis 90 Gew.-% Triglyceride, 2 bis 5 Gew.-% Diglyceride, 5 bis 10 Gew.-% Monoglyceride, 1 bis 5 Gew.-% freie Fettsäuren, 2 bis 8 Gew.-% Phospholipide vor, wobei sich die Summe der verschiedenen Verbindungen zu 100 Gew.-% ergänzt.

Im erfindungsgemäßen Verfahren werden die hergestellten LCPUFAs mit einem Gehalt von mindestens 3 Gew.-%, vorteilhaft von mindestens 5 Gew.-%, bevorzugt von mindestens 8 Gew.-%, besonders bevorzugt von mindestens 10 Gew.-%, ganz besonders bevorzugt von mindestens 15 Gew.-% bezogen auf die gesamten Fettsäuren in der transgenen Organismen vorteilhaft in einer transgenen Pflanze hergestellt. Da im erfindungsgemäßen Verfahren von den Ausgangsverbindungen Hexadecadiensäure (C16:2), Linolsäure (C18:2) bzw. Linolensäure (C18:3) mehrere Reaktionsschritte durchlaufen werden, fallen die Endprodukte des Verfahrens wie beispielsweise Arachidonsäure (ARA) oder Eicosapentaensäure (EPA) nicht als absolute Reinprodukte an, es sind immer auch geringe Spuren der Vorstufen im Endprodukt enthalten. Sind in dem Ausgangsorganismus bzw. in der Ausgangspflanze beispielsweise sowohl Linolsäure als auch Linolensäure vorhanden, so liegen die Endprodukte wie ARA und EPA als Mischungen vor. Die Vorstufen sollten vorteilhaft nicht mehr als 20 Gew.-%, bevorzugt nicht mehr als 15 Gew.-%, besonders bevorzugt nicht als 10 Gew.-%, ganz besonders bevorzugt nicht mehr als 5 Gew.-% bezogen auf die Menge des jeweilige Endprodukts betragen. Vorteilhaft werden in einer transgenen Pflanze als Endprodukte nur ARA oder nur EPA im erfindungsgemäßen Verfahren gebunden oder als freie Säuren hergestellt Werden beide Verbindungen (ARA + EPA) gleichzeitig hergestellt, werden sie vorteilhaft in einem Verhältnis von mindesten 1:2 (EPA:ARA), vorteilhaft von mindestens 1:3, bevorzugt von 1:4, besonders bevorzugt von 1:5 hergestellt.

Durch die erfindungsgemäßen Nukleinsäuresequenzen kann eine Steigerung der Ausbeute an mehrfach ungesättigten Fettsäuren von mindestens 50 %, vorteilhaft von mindestens 80 %, besonders vorteilhaft von mindestens 100 %, ganz besonders vorteilhaft von mindestens 150 % gegenüber den nicht transgenen Ausgangsorganismus beim Vergleich in der GC-Analyse siehe Beispiele erreicht werden.

Auch chemisch reine mehrfach ungesättigte Fettsäuren oder Fettsäurezusammensetzungen sind nach den vorbeschriebenen Verfahren darstellbar. Dazu werden die Fettsäuren oder die Fettsäurezusammensetzungen aus dem Organismus wie den Mikroorganismen oder den Pflanzen oder dem Kulturmedium, in dem oder auf dem die Organismen angezogen wurden, oder aus dem Organismus und dem Kulturmedium in bekannter Weise beispielsweise über Extraktion, Destillation, Kristallisation, Chromatographie oder Kombinationen dieser Methoden isoliert. Diese chemisch reinen Fettsäuren oder Fettsäurezusammensetzungen sind für Anwendungen im Bereich der Lebensmittelindustrie, der Kosmetikindustrie und besonders der Pharmaindustrie vorteilhaft.

Als Organismus für die Herstellung im erfindungsgemäßen Verfahren kommen prinzipiell alle Organismen wie Pilze wie Mortierella oder Traustochytrium, Hefen wie Saccharomyces oder Schizosaccharomyces, Moose wie Physcomitrella oder Ceratodon, nicht-humane Tiere wie Caenorhabditis, Algen wie Crypthecodinium oder Phaeodactylum oder Pflanzen wie zweikeimblättrige oder einkeimbiättrige Pflanzen in Frage. Vorteilhaft werden Organismen im erfindungsgemäßen Verfahren verwendet, die zu den Öl-produzierenden Organismen gehören, das heißt die für die Herstellung von Ölen verwendet werden, wie Pilze wie Mortierella oder Traustochytrium, Algen wie Crypthecodinium, Phaeodactylum oder Pflanzen, insbesondere Pflanzen bevorzugt Ölfruchtpflanzen, die große Mengen an Lipidverbindungen enthalten, wie Erdnuss, Raps, Canola, Sonnenblume, Safflor (Färberdistel), Mohn, Senf, Hanf, Rizinus, Olive, Sesam, Calendula, Punica, Nachtkerze, Königskerze, Distel, Wildrosen, Haselnuss, Mandel, Macadamia, Avocado, Lorbeer, Kürbis, Lein, Soja, Pistazien, Borretsch, Bäume (Ölpalme; Kokosnuss oder Walnuss) oder Feldfrüchte, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa oder Buschpflanzen (Kaffee, Kakao, Tee), Salix-Arten sowie ausdauernde Gräser und Futterfeldfrüchte. Bevorzugte erfindungsgemäße Pflanzen sind Ölfruchtpflanzen, wie Erdnuss, Raps, Canola, Sonnenblume, Safflor (Färberdistel), Mohn, Senf, Hanf, Rhizinus, Olive, Calendula, Punica, Nachtkerze, Kürbis, Lein, Soja, Borretsch, Bäume (Ölpalme, Kokosnuss). Besonders bevorzugt sind C18:2- und/oder C18:3-Fettsäure reiche Pflanzen wie Sonnenblume, Färberdistel, Tabak, Königskerze, Sesam, Baumwolle, Kürbis, Mohn, Nachtkerze, Walnuss, Lein, Hanf, Distel oder Färberdistel. Ganz besonders bevorzugt sind Pflanzen wie Färberdistel, Sonnenblume, Mohn, Nachtkerze, Walnuss, Lein oder Hanf.

Für das efindungsgemäße beschriebene Verfahren ist es vorteilhaft in den Organismus zusätzlich zu den unter Verfahrensschritt (a) bis (c) eingebrachten Nukleinsäuren zusätzlich weitere Nukleinsäuren einzubringen, die für Enzyme des Fettsäure- oder Lipidstoffwechsels codieren.

Im Prinzip können alle Gene des Fettsäure- oder Lipidstoffwechsels vorteilhaft in Kombination mit der erfinderischen Acyl-CoA:Lysophospholipid-Acyltransferase im Verfahren zur Herstellung mehrfach ungesättigter Fettsäuren verwendet werden vorteilhaft werden Gene des Fettsäure- oder Lipidstoffwechsels ausgewählt aus der Gruppe Acyl-CoA-Dehydrogenase(n), Acyl-ACP[=acyl carrier protein]-Desaturase(n), Acyl-ACP-Thioesterase(n), Fettsäure-Acyl-Transferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) in Kombination mit der Acyl-CoA:Lysophospholipid-Acyltransferase verwendet. Besonders bevorzugt werden Gene ausgewählt aus der Gruppe der Δ-4-Desaturasen, Δ-5-Desaturasen, Δ-6-Desafturasen, Δ-8-Desatuasen, Δ-9-Desaturasen, Δ-12-Desaturasen, Δ-5-Elongasen, Δ-6-Elongasen oder Δ-9-Elongasen in Kombination mit der Acyl-CoA:Lysophospholipid-Acyltransferase im erfindungsgemäßen Verfahren verwendet.

Durch die enzymatische Aktivität der im erfindungsgemäßen Verfahren verwendeten Nukleinsäuren, die für Polypeptide mit Acyl-CoA:Lysophospholipid-Acyltransferase-aktivität codieren, vorteilhaft in Kombination mit Nukleinsäuresequenzen, die für Polypeptide des Fettsäure- oder Lipidstoffwechsels wie Δ-4-, Δ-5-, Δ-6-, Δ-8-Desaturase-oder Δ-5-, Δ-6-oder Δ-9-Elongaseaktivität codieren, können unterschiedlichste mehrfach ungesättigte Fettsäuren im erfindungsgemäßen Verfahren hergestellt werden. Je nach Auswahl der für das erfindungsgemäße Verfahren verwendeten Organismen wie den vorteilhaften Pflanze lassen sich Mischungen der verschiedenen mehrfach ungesättigten Fettsäure oder einzelne mehrfach ungesättigte Fettsäuren wie EPA oder ARA in freier oder gebundener Form herstellen. Je nachdem welche Fettsäurezusammensetzung in der Ausgangspflanze vorherrscht (C18:2- oder C18:3-Fettsäuren) entstehen so Fettsäuren, die sich von C18:2-Fettsäuren ableiten, wie GLA, DGLA oder ARA oder, die sich von C18:3-Fettsäuren ableiten, wie SDA, ETA oder EPA. Liegt in der für das Verfahren verwendeten Pflanze als ungesättigte Fettsäure nur Linolsäure (= LA, C18:2^{Δ9,12}) vor, so können als Produkte des Verfahrens nur GLA, DGLA und ARA entstehen, die als freie Fettsäuren oder gebunden vorliegen können. Ist in der im Verfahren verwendeten Pflanze als ungesättigte Fettsäure nur α-Linolensäure (= ALA, C18:3^{Δ9,12,15}) beispielsweise wie in Lein, so können als Produkte des Verfahrens nur SDA, ETA und EPA entstehen, die wie oben beschrieben als freie Fettsäuren oder gebunden vorliegen können. Durch Modifikation der Aktivität der an der Synthese beteiligten Enzyme Acyl-CoA:Lysophospholipid-Acyltransferase vorteilhaft in Kombination mit der Δ-5-, Δ-6-Desaturase und Δ-6-Elongase, oder der Δ-5-, Δ-8-Desaturase und Δ-9-Elongase oder in Kombination mit nur den ersten beiden Gene Δ-6-Desaturase und Δ-6-Elongase oder Δ-8-Desaturase und Δ-9-Elongase der Synthesekette lassen sich gezielt in den vorgenannten Organismen vorteilhaft in den vorgenannten Pflanzen nur einzelne Produkte herstellten. Durch die Aktivität der Δ-6-Desaturase und Δ-6-Elongase entstehen beispielsweise GLA und DGLA bzw. SDA und ETA, je nach Ausgangspflanze und ungesättigter Fettsäure. Bevorzugt entstehen DGLA bzw. ETA oder deren Mischungen. Wird die Δ-5-Desaturase zusätzlich in die Organismen vorteilhaft in die Pflanze eingebracht, so entstehen zusätzlich ARA oder EPA. Dies gilt auch für Organismen in die vorher die Δ-8-Desaturase und Δ-9-Elongase eingebracht wurde. Vorteilhaft werden nur ARA oder EPA oder deren Mischungen synthetisiert, abhängig von der in im Organismus bzw. in der Pflanze vorliegenden Fettsäure, die als Ausgangssubstanz für die Synthese dient. Da es sich um Biosyntheseketten handelt, liegen die jeweiligen Endprodukte nicht als Reinsubstanzen in den Organismen vor. Es sind immer auch geringe Mengen der Vorläuferverbindungen im Endprodukt enthalten. Diese geringen Mengen betragen weniger als 20 Gew.-%, vorteilhaft weniger als 15 Gew.-%, besonders vorteilhaft weniger als 10 Gew.-%, ganz besonders vorteilhaft weniger als 5, 4, 3, 2 oder 1 Gew.-% bezogen auf das Endprodukt DGLA, ETA oder deren Mischungen bzw. ARA, EPA oder deren Mischungen.

Zur Steigerung der Ausbeute im beschriebenen Verfahren zur Herstellung von Ölen und/oder Triglyceriden mit einem vorteilhaft erhöhten Gehalt an mehrfach ungesättigten Fettsäuren ist es vorteilhaft die Menge an Ausgangsprodukt für die Fettsäuresynthese zu steigern, dies kann beispielsweise durch das Einbringen einer Nukleinsäure in den Organismus, die für ein Polypeptid mit Δ-12-Desaturase codiert, erreicht werden. Dies ist besonders vorteilhaft in Öl-produzierenden Organismen wie Raps, die einen hohen Ölsäuregehalt aufweisen. Da diese Organismen nur einen geringen Gehalt an Linolsäure aufweisen (Mikoklajczak et al., Journal of the American Oil Chemical Society, 38,1961, 678 - 681) ist die Verwendung der genannten Δ-12-Desaturasen zur Herstellung des Ausgangsprodukts Linolsäure vorteilhaft.

Im erfindungsgemäßen Verfahren verwendeten Nukleinsäuren stammen vorteilhaft aus Pflanzen wie Algen wie Isochrysis oder Crypthecodinium, Algen/Diatomeen wie Phaeodactylum, Moose wie Physcomitrella oder Ceratodon oder höheren Pflanzen wie den Primulaceae wie Aleuritia, Calendula stellata, Osteospermum spinescens oder Osteospermum hyoseroides, Mikroorganismen wie Pilzen wie Aspergillus, Thraustochytrium, Phytophtora, Entomophthora, Mucor oder Mortierella, Hefen oder Tieren wie Nematoden wie Caenorhabditis, Insekten oder dem Mensch. Vorteilhaft stammen die Nukleinsäuren aus Pilzen, Tieren oder aus Pflanzen wie Algen oder Moosen, bevorzugt aus Nematoden wie Caenorhabditis.

Vorteilhaft werden im erfindungsgemäßen Verfahren die vorgenannten Nukleinsäuresequenzen oder deren Derivat oder Homologe, die für Polypeptide codieren, die noch die enzymatische Aktivität der durch Nukleinsäuresequenzen codierten Proteine besitzen. Diese Sequenzen werden einzeln oder in Kombination mit der für die Acyl-CoA:Lysophospholipid-Acyltransferase codierenden Nukleinsäuresquenz in Expressionskonstrukte cloniert und zum Einbringen und zur Expression in Organismen verwendet. Diese Expressionskonstrukte ermöglichen durch ihre Konstruktion eine vorteilhafte optimale Synthese der im erfindungsgemäßen Verfahren produzierten mehrfach ungesättigten Fettsäuren.

Bei einer bevorzugten Ausführungsform umfasst das Verfahren ferner den Schritt des Gewinnens einer Zelle oder eines ganzen Organismus, der die im Verfahren verwendeten Nukleinsäuresequenzen enthält, wobei die Zelle und/oder der Organismus mit der erfindungsgemäßen Nukleinsäuresequenz, die für die Acyl-CoA:Lysophospholipid-Acyltransferase codiert einem Genkonstrukt oder einem Vektor wie nachfolgend beschrieben, allein oder in Kombination mit weiteren Nukleinsäuresequenzen, die für Proteine des Fettsäure- oder Lipidsstoffwechsels codieren, transformiert wird. Bei einer weiteren bevorzugten Ausführungsform umfasst dieses Verfahren ferner den Schritt des Gewinnens der Feinchemikalie aus der Kultur. Bei der Kultur kann es sich beispielsweise um eine Fermentationskultur beispielsweise im Falle der Kultivierung von Mikroorganismen wie z.B. Mortierella, Saccharomyces oder Traustochytrium oder um eine Treibhaus oder Feldkultur einer Pflanze handeln. Die so hergestellte Zelle oder der so hergestellte Organismus ist vorteilhaft eine Zelle eines Öl-produzierenden Organismus wie einer Ölfruchtpflanze wie beispielsweise Erdnuss, Raps, Canola, Lein, Hanf, Erdnuss, Soja, Safflower, Hanf, Sonnenblumen oder Borretsch.

Unter Anzucht ist beispielsweise die Kultivierung im Falle von Pflanzenzellen, -gewebe oder -organe auf oder in einem Nährmedium oder der ganzen Pflanze auf bzw. in einem Substrat beispielsweise in Hydrokultur, Blumentopferde oder auf einem Ackerboden zu verstehen.

"Transgen" bzw. "Rekombinant" im Sinne der Erfindung bedeutet bezüglich zum Beispiel einer Nukleinsäuresequenz, einer Expressionskassette (= Genkonstrukt) oder einem Vektor enthaltend die Nukleinsäuresequenz oder einem Organismus transformiert mit den Nukleinsäuresequenzen, Expressionskassette oder Vektor alle solche durch gentechnische Methoden zustandegekommenen Konstruktionen, in denen sich entweder
a) die Nukleinsauresequenz, oder
b) eine mit der Nukleinsäuresequenz funktionell verknüpfte genetische Kontrollsequenz, zum Beispiel ein Promotor, oder
c) (a) und (b)
sich nicht in ihrer natürlichen, genetischen Umgebung befinden oder durch gentechnische Methoden modifiziert wurden, wobei die Modifikation beispielhaft eine Substitution, Addition, Deletion, Inversion oder Insertion eines oder mehrerer Nukleotidreste sein kann. Natürliche genetische Umgebung meint den natürlichen genomischen bzw. chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise de natürlich vorkommende Kombination des natürlichen Promotors der erfindungsgemäßen Nukleinsäuresequenz mit dem entsprechenden Acyl-CoA:Lysophospholipid-Acyltransferase -Gen - wird zu einer transgenen Expressionskassette, wenn diese durch nicht-natürliche, synthetische ("künstliche") Verfahren wie beispielsweise einer Mutagenisierung geändert wird. Entsprechende Verfahren sind beispielsweise beschrieben in US 5,565,350 oder WO 00/15815.

Unter transgenen Organismus bzw. transgener Pflanze im Sinne der Erfindung ist wie vorgenannt zu verstehen, dass die im Verfahren verwendeten Nukleinsäuren nicht an ihrer natürlichen Stelle im Genom eines Organismus sind, dabei können die Nukleinsäuren homolog oder heterolog exprimiert werden. Transgen bedeutet aber auch wie genannt, dass die erfindungsgemäßen Nukleinsäuren an ihrem natürlichen Platz im Genom eines Organismus sind, dass jedoch die Sequenz gegenüber der natürlichen Sequenz verändert wurde und/oder das die Regulationssequenzen, der natürlichen Sequenzen verändert wurden. Bevorzugt ist unter transgen die Expression der erfindungsgemäßen Nukleinsäuren an nicht natürlicher Stelle im-Genom zu verstehen, das heißt eine homologe oder bevorzugt heterologe Expression der Nukleinsäuren liegt vor. Bevorzugte transgene Organismen sind Pilze wie Mortierella oder Pflanzen sind die Öffruchtpflanzen.

Als Organismen bzw. Wirtsorganismen für die im erfindungsgemäßen Verfahren verwendeten Nukleinsäuren, die Expressionskassette oder den Vektor eignen sich prinzipiell vorteilhaft alle Organismen, die in der Lage sind Fettsäuren speziell ungesättigte Fettsäuren zu synthetisieren bzw. für die Expression rekombinanter Gene geeignet sind. Beispielhaft seien Pflanzen wie Arabidopsis, Asteraceae wie Calendula oder Kulturpflanzen wie Soja, Erdnuss, Rizinus, Sonnenblume, Mais, Baumwolle, Flachs, Raps, Kokosnuss, Ölpalme, Färbersafflor (Carthamus tinctorius) oder Kakaobohne, Mikroorganismen wie Pilze beispielsweise die Gattung Mortierella, Saprolegnia oder Pythium, Bakterien wie die Gattung Escherichia, Hefen wie die Gattung Saccharomyces, Cyanobakterien, Ciliaten, Algen oder Protozoen wie Dinoflagellaten wie Crypthecodinium genannt. Bevorzugt werden Organismen, die natürlicherweise Öle in größeren Mengen synthetisieren können wie Pilze wie Mortierella alpina, Pythium insidiosum oder Pflanzen wie Soja, Raps, Kokosnuss, Ölpalme, Färbersafflor, Flachs, Hanf, Rizinus, Calendula, Erdnuss, Kakaobohne oder Sonnenblume oder Hefen wie Saccharomyces cerevisiae, besonders bevorzugt werden Soja, Flachs, Raps, Färbersafflor, Sonnenblume, Calendula, Mortierella oder Saccharomyces cerevisiae. Prinzipiell sind als Wirtsorganismen neben den vorgenannten transgenen Organismen auch transgene Tiere vorteilhaft nicht-humane Tiere geeignet beispielsweise C. elegans.

Nutzbare Wirtszellen sind weiterhin genannt in: Goeddel, Gene Expression Technology: Methods in Enzymology 185, Academic Press, San Diego, CA (1990).

Verwendbare Expressionsstämme z.B. solche, die eine geringere Proteaseaktivität aufweisen sind beschrieben in: Gottesman, S., Gene Expression Technology: Methods in Enzymology 185, Academic Press, San Diego, California (1990) 119-128.

Hierzu gehören Pflanzenzellen und bestimmte Gewebe, Organe und Teile von Pflanzen in all ihren Erscheinungsformen, wie Antheren, Fasern, Wurzelhaare, Stängel, Embryos, Kalli, Kotelydonen, Petiolen, Erntematerial, pflanzliches Gewebe, reproduktives Gewebe und Zellkulturen, das von der eigentlichen transgenen Pflanze abgeleitet ist und/oder dazu verwendet werden kann, die transgene Pflanze hervorzubringen.

Transgene Pflanzen, die die im efindungsgemäßen Verfahren synthetisierten mehrfach ungesättigten Fettsäuren enthalten, können vorteilhaft direkt vermarktet werden ohne dass, die synthetisierten Öle, Upide oder Fettsäuren isoliert werden müssen. Unter Pflanzen im erfindungsgemäßen Verfahren sind ganze Pflanzen sowie alle Pflanzenteile, Pflanzenorgane oder Pflanzenteile wie Blatt, Stiel, Samen, Wurzel, Knollen, Antheren, Fasern, Wurzelhaare, Stängel, Embryos, Kalli, Kotelydonen, Petiolen, Emtematerial, pflanzliches Gewebe, reproduktives Gewebe, Zellkulturen, die sich von der transgenen Pflanze abgeleiten und/oder dazu verwendet werden können, die transgene Pflanze hervorzubringen. Der Samen umfasst dabei alle Samenteile wie die Samenhüllen, Epidermis- und Samenzellen, Endosperm oder Embyrogewebe. Die im erfindungsgemäßen Verfahren hergestellten Verbindungen können aber auch aus den Organismen vorteilhaft Pflanzen in Form ihrer Öle, Fett, Lipide und/oder freien Fettsäuren isoliert werden. Durch dieses Verfahren hergestellte mehrfach ungesättigten Fettsäuren lassen sich durch Ernten der Organismen entweder aus der Kultur, in der sie wachsen, oder vom Feld ernten. Dies kann über Pressen oder Extraktion der Pflanzenteile bevorzugt der Pflanzensamen erfolgen. Dabei können die Öle, Fette, Lipide und/oder freien Fettsäuren durch sogenanntes kalt schlagen oder kalt pressen ohne Zuführung von Wärme durch Pressen gewonnen werden. Damit sich die Pflanzenteile speziell die Samen leichter aufschließen lassen, werden sie vorher zerkleinert, gedämpft oder geröstet. Die so vorbehandelten Samen können anschließend gepresst werden oder mit Lösungsmittel wie warmen Hexan extrahiert werden. Anschließend wird das Lösungsmittel wieder entfernt, Im Falle von Mikroorganismen werden diese nach Ernte beispielsweise direkt ohne weitere Arbeitsschritte extrahiert oder aber nach Aufschluss über verschiedene dem Fachmann bekannte Methoden extrahiert. Auf diese Weise können mehr als 96 % der im Verfahren hergestellten Verbindungen isoliert werden-Anschließend werden die so erhaltenen Produkte weiter bearbeitet, das heißt raffiniert. Dabei werden zunächst beispielsweise die Pflanzenschleime und Trübstoffe entfernt. Die sogenannte Entschleimung kann enzymatisch oder beispielsweise chemisch/physikalisch durch Zugabe von Säure wie Phosphorsäure erfolgen. Anschlieβend werden die freien Fettsäuren durch Behandlung mit einer Base beispielsweise Natronlauge entfernt. Das erhaltene Produkt wird zur Entfernung der im Produkt verbliebenen Lauge mit Wasser gründlich gewaschen und getrocknet Um die noch im Produkt enthaltenen Farbstoffe zu entfernen werden die Produkte einer Bleichung mit beispielsweise Bleicherde oder Aktivkohle unterzogen. Zum Schluss wird das Produkt noch beispielsweise mit Wasserdampf noch desodoriert.

Vorzugsweise sind die durch dieses Verfahren produzierten PUFAs bzw. LCPUFAs C₁₈-, C₂₀- oder C₂₂-Fettsäuremoleküle mit mindestens zwei Doppelbindungen im Fettsäuremolekül, vorzugsweise drei, vier, fünf oder sechs Doppelbindungen. Diese C₁₈-, C₂₀- oder C₂₂-Fettsäuremoleküle lassen sich aus dem Organismus in Form eines Öls, Lipids oder einer freien Fettsäure isolieren. Geeignete Organismen sind beispielsweise die vorstehend erwähnten. Bevorzugte Organismen sind transgene Pflanzen.

Offenbart werden auch Öle, Lipide oder Fettsäuren oder Fraktionen davon, die durch das oben beschriebene Verfahren hergestellt worden sind, besonders bevorzugt Öl, Lipid oder eine Fettsäurezusammensetzung, die PUFAs umfassen und von transgenen Pflanzen herrühren.

Zudem wird die Verwendung des Öls, Lipids, der Fettsäuren und/oder der Fettsäurezusammensetzung in Futtermitteln, Nahrungsmitteln, Kosmetika oder Pharmazeutika.- offenbart

Unter dem Begriff "Öl", "Lipid" oder "Fett" wird ein Fettsäuregemisch verstanden, das ungesättigte, gesättigte, vorzugsweise veresterte Fettsäure(n) enthält Bevorzugt ist, dass das Öl, Lipid oder Fett einen hohen Anteil an mehrfach ungesättigten freien oder vorteilhaft veresterten Fettsäure(n), insbesondere Linolsäure, Y-Linolensäure, Dihomoy-linolensäure, Arachidonsäure, α-Linofensäure, Stearidonsäure, Eicosatetraensäure, Eicosapentaensäure, Docosapentaensäure oder Docosahexaensäure hat Vorzugsweise ist der Anteil an ungesättigten veresterten Fettsäuren ungefähr 30 %, mehr bevorzugt ist ein Anteil von 50 %, noch mehr bevorzugt ist ein Anteil von 60 %, 70 %, 80 % oder mehr. Zur Bestimmung kann z.B. der Anteil an Fettsäure nach Überführung der Fettsäuren in die Methylestern durch Umesterung gaschromatographisch bestimmt werden. Das Öl, Lipid oder Fett kann verschiedene andere gesättigte oder ungesättigte Fettsäuren, z.B. Calendulasäure, Palmitin-, Palmitolein-, Stearin-, Ölsäure etc., enthalten. Insbesondere kann je nach Ausgangsorganismus der Anteil der verschiedenen Fettsäuren in dem Öl oder Fett schwanken.

Bei den im Verfahren hergestellten mehrfach ungesättigte Fettsäuren mit vorteilhaft mindestens zwei Doppelbindungen enthalten, handelt es sich beispielsweise um Sphingolipide, Phosphoglyceride, Lipide, Glycolipide, Phospholipide, Monoacylglycerin, Diacylglycerin, Triacylglycerin oder sonstige Fettsäureester.

Aus den so im erfindungsgemäßen Verfahren hergestellten mehrfach ungesättigte Fettsäuren mit vorteilhaft mindestens zwei Doppelbindungen lassen sich die enthaltenden mehrfach ungesättigten Fettsäuren beispielsweise über eine Alkalibehandlung beispielsweise wäßrige KOH oder NaOH oder saure Hydrolyse vorteilhaft in Gegenwart eines Alkohols wie Methanol oder Methanol oder über eine enzymatische Abspaltung freisetzen und isolieren über beispielsweise Phasentrennung und anschließender Ansäuerung über z.B. H₂SO₄. Die Freisetzung der Fettsäuren kann auch direkt ohne die vorhergehend beschriebene Aufarbeitung erfolgen.

Die im Verfahren verwendeten Nukleinsäuren können nach Einbringung in einem Organismus vorteilhaft einer Pflanzenzelle bzw. Pflanzen entweder auf einem separaten Plasmid liegen oder in das Genom der Wirtszelle integriert sein. Bei Integration in das Genom kann die Integration zufallsgemäß sein oder durch derartige Rekombination erfolgen, dass das native Gen durch die eingebrachte Kopie ersetzt wird, wodurch die Produktion der gewünschten Verbindung durch die Zelle moduliert wird, oder durch Verwendung eines Gens in trans, so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist. Vorteilhaft werden die Nukleinsäuren über Multiexpressionskassetten oder Konstrukte zur multiparallelen Expression in die Organismen vorteilhaft zur multiparallelen samenspezifischen Expression von Genen in die Pflanzen gebracht

Moose und Algen sind die einzigen bekannten Pflanzensysteme, die erhebliche Mengen an mehrfach ungesättigten Fettsäuren, wie Arachidonsäure (ARA) und/oder Eicosapentaensäure (EPA) und/oder Docosahexaensäure (DHA) herstellen. Moose enthalten PUFAs in Membranlipiden während Algen, algenverwandte Organismen und einige Pilze auch nennenswerte Mengen an PUFAs in der Triacylglyceroffraktion akkumulieren. Daher eignen sich Nukleinsäuremoleküle; die aus solchen Stämmen isoliert werden, die PUFAs auch in der Triacylglycerolfraktion akkumulieren, besonders vorteilhaft für das erfindungsgemäße Verfahren und damit zur Modifikation des Lipid- und PUFA-Produktionssystems in einem Wirt, insbesondere Pflanzen, wie Ölfruchtpflanzen, beispielsweise Raps, Canola, Lein, Hanf, Soja, Sonnenblumen, Borretsch. Sie sind deshalb Vorteilhaft im erfindungsgemäßen Verfahren verwendbar.

Als Substrate der Acyl-CoA:Lysophospholipid-Acyltransferasen werden vorteilhaft C₁₆-, C₁₈-, C₂₀- oder C₂₂-Fettsäuren verwendet

Zur Herstellung der langkettiger PUFAs müssen die mehrfach ungesättigten C₁₆- oder C₁₈-Fettsäuren zunächst durch die enzymatische Aktivität einer Desaturase zunächst desaturiert und anschließend über eine Elongase um mindestens zwei Kohlenstoffatome verlängert werden. Nach einer Elongationsrunde führt diese Enzymaktivität zu C₁₈- oder C₂₀-Fettsäuren, und nach zwei oder drei Elongationsrunden zu C₂₂- oder C₂₄-Fettsäuren. Die Aktivität der erfindungsgemäßen Verfahren verwendeten Desaturasen und Elongasen führt vorzugsweise zu C₁₈, C₂₀ und/oder C₂₂-Fettsäuren vorteilhaft mit mindestens zwei Doppelbindungen im Fettsäuremolekül, vorzugsweise mit drei, vier oder fünf Doppelbindungen, besonders bevorzugt zu C₂₀-und/oder C₂₂-Fettsäuren mit mindestens zwei Doppelbindungen im Fettsäuremolekül, vorzugsweise mit drei, vier oder fünf Doppelbindungen im Molekül. Nachdem eine erste Desaturierung und die Verlängerung stattgefunden hat, können weitere Desaturierungsschritte wie z.B. eine solche in A-5-Position erfolgen. Besonders bevorzugt als Produkte des erfindungsgemäßen Verfahrens sind Dihomo-γ-linolensäure, Arachidonsäure, Eicosapentaensäure, Docosapetaensäure und/oder Docosahesaensäure. Die C₁₈-Fettsäuren mit mindestens zwei Doppelbindungen in der Fettsäure können durch die erfindungsgemäße enzymatische Aktivität in Form der freien Fettsäure oder in Form der Ester, wie Phospholipide, Glycolipide, Sphingolipide, Phosphoglyceride, Monoacylglycerin, Diacylglycerin oder Triacylglycerin, verlängert werden.

Der bevorzugte Biosyntheseort von Fettsäuren, Ölen, Lipiden oder Fette in den vorteilhaft verwendeten Pflanzen ist beispielsweise im allgemeinen der Samen oder Zellschichten des Samens, so dass eine sämenspezifische Expression der im Verfahren verwendeten Nukleinsäuren sinnvoll ist Es ist jedoch naheliegend, dass die Biosynthese von Fettsäuren, Ölen oder Lipiden nicht auf das Samengewebe beschränkt sein muss, sondern auch in allen übrigen Teilen der Pflanze - beispielsweise in Epidermiszellen oder in den Knollen - gewebespezifisch erfolgen kann.

Werden im erfindungsgemäßen Verfahren als Organismen Mikroorganismus wie Hefen wie Saccharomyces oder Schizosaccharomyces, Pilze wie Mortierella, Aspergillus, Phytophtora, Entomophthora, Mucor oder Traustochytrium Algen wie lsochrysis, Phaeodactylum oder Crypthecodinium verwendet, so werden diese Organismen vorteilhaft fermentativ angezogen.

Durch die Verwendung der erfindungsgemäßen Nukleinsäuren, die für Acyl-CoA:Lysophospholipid-Acyltransferasen codieren, können im Verfahren die hergestellten mehrfach ungesättigten Fettsäuren mindestens um 10 % , bevorzugt mindestens um 15 %, besonders bevorzugt mindestens um 20 % ganz besonders bevorzugt um mindestens 50 % gegenüber dem Wildtyp der Organismen, die die Nukleinsäuren nicht rekombinant enthalten, erhöht werden.

Durch das erfindungsgemäße Verfahren können die hergestellten mehrfach ungesättigten Fettsäuren in den im Verfahren verwendeten Organismen prinzipiell auf zwei Arten erhöht werden. Es kann vorteilhaft der Pool an freien mehrfach ungesättigten Fettsäuren und/oder der Anteil der über das Verfahren hergestellten veresterten mehrfach ungesättigten Fettsäuren erhöht werden. Vorteilhaft wird durch das erfindungsgemäße Verfahren der Pool an veresterten mehrfach ungesättigten Fettsäuren in den transgenen Organismen erhöht.

Werden im erfindungsgemäßen Verfahren als Organismen Mikroorganismen verwendet, so werden sie je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann batch weise, semi batch weise oder kontinuierlich erfolgen. Nährstoffe können zu Beginn der Fermentation vorgelegt oder semikontinuierlich oder kontinuierlich nachgefüttert werden. Die hergestellten mehrfach ungesättigten Fettsäuren können nach dem Fachmann bekannten Verfahren wie oben beschrieben aus den Organismen isoliert werden. Beispielsweise über Extraktion, Destillation, Kristallisation, ggf. Salzfällung und/oder Chromatographie. Die Organismen können dazu vorher noch vorteilhaft aufgeschlossen werden.

Das erfindungsgemäße Verfahren wird, wenn es sich bei den Wirtsorganismen um Mikroorganismen handelt, vorteilhaft bei einer Temperatur zwischen 0 °C bis 95 °C, bevorzugt zwischen 10 °C bis 85 °C, besonders bevorzugt zwischen 15 °C bis 75 °C, ganz besonders bevorzugt zwischen 15 °C bis 45 °C durchgeführt Der pH-Wert wird dabei vorteilhaft zwischen pH 4 und 12, bevorzugt zwischen pH 6 und 9, besonders bevorzugt zwischen pH 7 und 8 gehalten.

Das erfindungsgemäße Verfahren kann batchweise, semi-batchweise oder kontinuierlich betrieben werden. Eine Zusammenfassung über bekannte Kultivienrngsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbare Medien umfassen wie oben beschrieben gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl, Sonnenblumenöl, Erdnussöl und/oder Kokosfett, Fettsäuren wie z. B. Palmitinsäure, Stearinsäure und/oder Unolsäure, Alkohole und/oder Polyalkohole wie z. B. Glycerin, Methanol und/oder Ethanol und/oder organische Säuren wie z. B. Essigsäure und/oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak in flüssiger- oder gasform oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalzium, Mangan, Zink, Kupfer und Eisen.

Als Schwefelquelle für die Herstellung von schwefelhaltigen Feinchemikalien, insbesondere von Methionin, können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß zur Kultivierung von Mikroorganismen eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vor stufen-zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 019 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experiments konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basischen Verbindungen wie Natriumhydroxid, Kaliumhydroxid, Ammoniak bzw. Ammoniakwasser oder sauren Verbindungen wie Phosphorsäure oder Schwefelsäure kontrollieren. Zur Kontrolle der Schaumentwiklung können Antischaummittel wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff haltige Gasmischungen, wie z. B. Umgebungsluft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht

Die so erhaltenen, insbesondere mehrfach ungesättigte Fettsäuren enthaltenden, Fermentationsbrühen haben üblicherweise eine Trockenmasse von 7,5 bis 25 Gew.-%.

Die Fermentationsbrühe kann anschließend weiterverarbeitet werden. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden. Vorteilhaft wird die Biomasse nach Abtrennung aufgearbeitet.

Die Fermentationsbrühe kann aber auch ohne Zellabtrennung mit bekannten Methoden, wie z. B. mit Hilfe eines Rotationsverdampfers, Dünnschichtverdampfers, Fallfilmverdampfers, durch Umkehrosmose, oder durch Nanofiltration, eingedickt beziehungsweise aufkonzentriert werden. Diese aufkonzentrierte Fermentationsbrühe kann schließlich zur Gewinnung der darin enthaltenen Fettsäuren aufgearbeitet werden.

Die im Verfahren gewonnenen Fettsäuren eignen sich auch als Ausgangsmaterial für die chemische Synthese von weiteren Wertprodukten. Sie können beispielsweise in Kombination miteinander oder allein zur Herstellung von Pharmaka, Nahrungsmittel, Tierfutter oder Kosmetika verwendet werden. Offenbart werden ferner isolierte Nukleinsäuresequenzen, die für Polypeptide mit Acyl-CoA:Lysophospholipid-Acyltransferaseaktivität codieren, wobei die durch die Nukleinsäuresequenzen codierten Acyl-CoA:Lysophospholipid-Acyltransferasen spezifisch C₁₆-, C₁₈-, C₂₀- oder C₂₂-Fettsäuren mit mindestens einer Doppelbindungen im Fettsäuremolekül umsetzen.

Vorteilhafte isolierte Nukleinsäuresequenzen sind Sequenzen ausgewählt aus der Gruppe :
a) einer Nukleinsäuresequenz mit der in SEQ ID NO: 1, SEQ ID NO: 3, SEQ ID NO: 5 oder SEQ ID NO: 7 dargestellten Sequenz,
b) Nukleinsäuresequenzen, die sich als Ergebnis des degenerierten genetischen Codes von der in SEQ ID NO: 1, SEQ ID NO: 3, SEQ ID NO: 5 oder SEQ ID NO: 7 enthaltenden codierenden Sequenz ableiten lassen
c) Derivate der in SEQ ID NO:1, SEQ ID NO: 3, SEQ ID NO: 5 oder SEQ ID NO: 7 dargestellten Nuldeinsäuresequenz, die für Polypeptide mit der in SEQ ID NO: 2, SEQ ID NO: 4, SEQ ID NO: 6 oder SEQ ID NO: 8 dargestellten Aminosäuresequenz codieren und mindestens 90% Homologie auf Aminosäureebene mit SEQ ID NO: 2, SEQ ID NO: 4, SEQ ID NO: 6 oder SEQ ID NO: 8 aufweisen und eine Acyl-CoA:Lysophosphofipid-Acyltransferaseaktivität aufweisen.

Vorteilhaft stammen die oben genannten Nukfeinsäuresequenzen- aus einem eukaryontischen Organismus.

Die im Verfahren verwendeten Nukleinsäuresequenzen, die für Proteine mit Acyl-CoA:Lysophospholipid-Acyltransferase-Aktivität codieren oder für Proteine des Fettsäure- oder Lipidstoffwechsels, werden vorteilhaft in einer Expressionskassette (= Nukleinsäurekonstrukt), die die Expression der Nukleinsäuren in einem Organismus vorteilhaft einer Pflanze oder einem Mikroorganismus ermöglicht, eingebracht.

Zum Einbringen werden die im Verfahren verwendeten Nukleinsäuren vorteilhaft einer Amplifikation und Ugation in bekannter Weise unterworfen. Vorzugsweise geht man in Anlehnung an das Protokoll der Pfu-DNA-Polymerase oder eines Pfu/Taq-DNA-Polymerasegemisches vor. Die Primer werden in Anlehnung an die zu amplifizierende Sequenz gewählt Zweckmäßigerweise sollten die Primer so gewählt werden, dass das Amplifikat die gesamte kodogene Sequenz vom Start- bis zum Stop-Kodon umfasst. Im Anschluss an die Amplifikation wird das Amplifikat zweckmäßigerweise analysiert. Beispielsweise kann die Analyse nach gelelektrophoretischer Auftrennung hinsichtlich Qualität und Quantität erfolgen. Im Anschluss kann das Amplifikat nach einem Standardprotokoll gereinigt werden (z.B. Qiagen). Ein Aliquot des gereinigten Amplifikats steht dann für die nachfolgende Klonierung zur Verfügung. Geeignete Klonierungsvektoren sind dem Fachmann allgemein bekannt. Hierzu gehören insbesondere Vektoren, die in mikrobiellen Systemen replizierbar sind, also vor allem Vektoren, die eine effiziente Klonierung in Hefen oder Pilze gewährleisten, und die stabile Transformation von Pflanzen ermöglichen. Zu nennen sind insbesondere verschiedene für die T-DNAvermittelte Transformation geeignete, binäre und co-integrierte Vektorsysteme. Derartige Vektorsysteme sind in der Regel dadurch gekennzeichnet, dass sie zumindest die für die Agrobakterium-vermittelte Transformation benötigten vir-Gene sowie die T-DNA begrenzenden Sequenzen (T-DNA-Border) beinhalten. Vorzugsweise umfassen diese Vektorsysteme auch weitere cis-regulatorische Regionen wie Promotoren und Terminatoren und/oder Selektionsmarker, mit denen entsprechend transformierte Organismen identifiziert werden können. Während bei co-integrierten Vektorsystemen vir-Gene und T-DMA-Sequenzen auf demselben Vektor angeordnet sind, basieren binäre Systeme auf wenigstens zwei Vektoren, von denen einer vir-Gene, aber keine T-DNA und ein zweiter T-DNA, jedoch kein vir-Gen trägt Dadurch sind letztere Vektoren relativ klein, leicht zu manipulieren und sowohl in E-coli als auch in Agrobacterium zu replizieren. Zu diesen binären Vektoren gehören Vektoren der Serien pBIB-HYG, pPZP, pBecks, pGreen. Erfindungsgemäß bevorzugt verwendet werden Bin19, pB1101, pBinAR, pGPTV und pCAMBIA. Eine Übersicht über binäre Vektoren und ihre Verwendung gibt Hellens et al, Trends in Plant Science (2000) 5, 446-451. Für die Vektorpräparation können die Vektoren zunächst mit Restriktionsendonuklease(n) linearisiert und dann in geeigneter Weise enzymatisch modifiziert werden. Im Anschluss wird der Vektor gereinigt und ein Aliquot für die Klonierung eingesetzt. Bei der Klonierung wird das enzymatisch geschnittenen und erforderlichenfalls gereinigten Amplifikat mit ähnlich präparierten Vektorfragmenten mit Einsatz von Ligase kloniert. Dabei kann ein bestimmtes Nukleinsäurekonstrukt bzw. Vektor oder Plasmidkonstrukt einen oder auch mehrere kodogene Genabschnitte aufweisen. Vorzugsweise sind die kodogenen Genabschnitte In diesen Konstrukten mit regulatorischen Sequenzen funktional verknüpft Zu den regulatorischen Sequenzen gehören insbesondere pflanzliche Sequenzen wie die oben beschriebenen Promotoren und Terminatoren. Die Konstrukte lassen sich vorteilhafterweise in Mikroorganismen, insbesondere Escherichia coli und Agrobacterium tumefaciens, unter selektiven Bedingungen stabil propagieren und ermöglichen einen Transfer von heterologer DNA in Pflanzen oder Mikroorganismen.

Unter der vorteilhaften Verwendung von Klonierungsvektoren können die im Verfahren verwendeten Nukleinsäuren, die Nukleinsäuren und Nukleinsäurekonstrukte in Organismen wie Mikroorganismen oder vorteilhaft Pflanzen eingebracht werden und damit bei der Pflanzentransformation verwendet werden, wie denjenigen, die veröffentlicht sind in und dort zitiert sind: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus, Annu. Rev. Plant Physiol. Plant Molec. Biol. 42 (1991), 205-225)). Die im Verfahren verwendeten Nukleinsäuren, die Nukleinsäuren und Nukleinsäurekonstrukte und/oder Vektoren lassen sich damit zur gentechnologischen Veränderung eines breiten Spektrums an Organismen vorteilhaft an Pflanzen verwenden, so dass diese bessere und/oder effizientere Produzenten von PUFAs werden.

Es gibt eine Reihe von Mechanismen, durch die die Veränderung eines Acyl-CoA:Lysophospholipid-Acyltransferase-Proteins die Ausbeute, Produktion und/oder Effizienz der Produktion einer Feinchemikalie aus einer Ölfruchtpflanze oder einem Mikroorganismus aufgrund eines veränderten Proteins direkt beeinflussen kann. Die Anzahl oder Aktivität des Acyl-CoA:Lysophospholipid-Acyltransferase-Proteins oder -Gens sowie von Genkombinationen von Acyl-CoA:Lysophospholipid-Acyltransferasen, Desaturasen und/oder Elongasen kann erhöht sein, so dass größere Mengen der produzierten Verbindungen de novo hergestellt werden, weil den Organismen diese Aktivität und Fähigkeit zur Biosynthese vor dem Einbringen des/der entsprechenden Gens/Gene fehlte. Entsprechendes gilt für die Kombination mit weiteren Desaturasen oder Elongasen oder weiteren Enzymen aus dem Fettsäure- und Lipidstoffwechsel. Auch die Verwendung verschiedener divergenter, d.h. auf DNA= Sequenzebene unterschiedlicher Sequenzen kann dabei vorteilhaft sein bzw. die Verwendung von Promotoren zur Genexpression, die eine andere zeitliche Genexpression zB. abhängig vom Reifegrad eines Samens oder Öl-speichernden Gewebes ermöglicht.

Durch das Einbringen eines Acyl-CoA:Lysophospholipid-Acyltransferase-, Desaturase-und/oder Elongase-Gens oder mehrerer Acyl-CoA:Lysophospholipid-Acyltransferasen-, Desaturase- und/oder Bongase-Gene in einen Organismus allein oder in Kombination mit anderen Genen in eine Zelle kann nicht nur den Biosynthesefluss zum Endprodukt erhöht, sondern auch die entsprechende Triacylglycerin-Zusammensetzung erhöht oder de novo geschaffen werden. Ebenso kann die Anzahl oder Aktivität anderer Gene, die am import von Nährstoffen, die zur Biosynthese einer oder mehrerer Feinchemikalien (z.B. Fettsäuren, polaren und neutralen Lipiden) nötig sind, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs, wie im folgenden beschrieben, weiter gesteigert wird. Fettsäuren und Lipide sind selbst als Feinchemikalien wünschenswert; durch Optimierung der Aktivität oder Erhöhung der Anzahl einer oder mehrerer Acyl-CoA:Lysophospholipid-Acyltransferase-, Desaturasen und/oder Elongasen, die an der Biosynthese dieser Verbindungen beteiligt sind, oder durch Zerstören der Aktivität einer oder mehrerer Desaturasen, die am Abbau dieser Verbindungen beteiligt sind, kann es möglich sein, die Ausbeute, Produktion und/oder Effizienz der Produktion von Fettsäure- und Lipidmolekülen aus Organismen und vorteilhaft aus Pflanzen zu steigern.

Die im erfindungsgemäßen Verfahren verwendeten isolierten Nukleinsäuremoleküle codieren für Proteine oder Teile von diesen, wobei die Proteine oder das einzelne Protein oder Teile davon eine Aminosäuresequenz enthält, die wie zu vor beschrieben ausreichend homolog zu einer Aminosäuresequenz der Sequenz SEQ ID NO: 2, SEQ ID NO: 4, SEQ ID NO: 6 oder SEQ ID NO: 8 ist, so dass das Protein oder der Teil davon eine Acyl-CoA:Lysophospholipid-Acyltransferase-Aktivität beibehält Vorzugsweise hat das Protein oder der Teil davon, das/der von dem Nukleinsäuremolekül kodiert wird, noch seine wesentliche enzymatische Aktivität und die Fähigkeit, am Stoffwechsel von zum Aufbau von Zellmembranen oder Lipidkörperchen in Organismen vorteilhaft in Pflanzen notwendigen Verbindungen oder am Transport von Molekülen über diese Membranen teilzunehmen noch hat. Das von den Nukleinsäuremolekülen kodierte Protein ist mindestens 90 % identisch zu einer Aminosäuresequenz der Sequenz SEQ ID NO: 2, SEQ ID NO: 4, SEQ ID NO: 6 oder SEQ ID NO: 8. Vorteilhafte Ausführungsformen der erfindungsgemäßen Aminosäuresequenz der sequenz SEQ ID NO: 2 sind Aminosäuresequenzen, die an Position 30 der SEQ ID NO: 2 anstelle des vorhandenen Methionin einen Valinrest haben oder in Position 100 anstelle des vorhandenen Serin einen Glycinrest haben oder in Position 170 anstelle des vorhandenen Phenylalanin einen Serinrest haben. Diese werden in SEQ ID NO: 4, SEQ ID NO: 6 bzw. SEQ ID NO: 8 wiedergegeben. Im Sinne der Erfindung ist unter Homologie oder homolog, Identität oder identisch zu verstehen.

Unter wesentliche enzymatischer Aktivität der verwendeten Acyl-CoA:Lysophospholipid-Acyltransferasen ist zu verstehen, dass sie gegenüber den durch die Sequenz mit SEQ ID NO: 1, SEQ ID NO: 3, SEQ ID NO: 5 oder SEQ ID NO: 7 und deren Derivate codierten Proteinen/Enzymen im Vergleich noch mindestens eine enzymatische Aktivität von mindestens 10 %, bevorzugt 20 %, besonders bevorzugt 30 % und ganz besonders 40 % aufweisen und damit am Stoffwechsel von zum Aufbau von Fettsäuren in einem Organismus vorteilhaft einer Pflanzenzelle notwendigen Verbindungen oder am Transport von Molekülen über Membranen teilnehmen können, wobei desaturierte C₁₆-, C₁₈- oder C20-24-Kohlenstoffketten mit Doppelbindungen an mindesteris zwei, vorteilhaft drei, vier oder fünf Stellen gemeint ist.

Vorteilhaft im Verfahren verwendbare Nukleinsäuren stammen aus Pilzen oder Pflanzen wie Algen oder Moosen wie den Gattungen Physcomitrella, Thraustochytrium, Phytophtora, Ceratodon, Isochrysis, Aleurita, Muscarioides, Mortierella, Borago, Phaeodactylum, Crypthecodinium oder aus Nematoden wie Caenorhabditis, speziell aus den Gattungen und Arten Physcomitrella patens, Phytophtora infestans, Ceratodon purpureus, Isochrysis galbana, Aleurita farinosa, Muscarioides viallii, Mortierella alpina, Borago officinalis, Phaeodactylum tricomutum oder besonders vorteilhaft aus Caenorhabditis elegans.

Alternativ können die verwendeten isolierten Nukleotidsequenzen für Acyl-CoA:Lysophospholipid-Acyltransferasen codieren, die an eine Nukleotidsequenz der SEQ ID NO: 1, SEQ ID NO: 3, SEQ ID NO: 5 oder SEQ ID NO: 7 hybridisieren, z.B. unter stringenten Bedingungen hybridisieren.

Die im Verfahren verwendeten Nukleinsäuresequenzen werden vorteilhaft in einer Expressionskassette, die die Expression der Nukleinsäuren in Organismen wie Mikroorganismen oder Pflanzen ermöglicht, eingebracht

Dabei werden die für die erfinderische Acyl-CoA:Lysophospholipid-Acyltransferasen, die verwendeten Desaturasen und/oder die Elongasen codierenden Nukleinsäuresequenzen mit einem oder mehreren Regulafionssignalen vorteilhafterweise zur Erhöhung der Genexpression funktionell verknüpft. Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen an die Induktoren oder Repressoren binden und so die Expression der Nukleinsäure regulieren. Zusätzlich zu diesen neuen Regulationssequenzen oder anstelle dieser Sequenzen kann die natürliche Regulation dieser Sequenzen vor den eigentlichen Strukturgenen noch vorhanden sein und gegebenenfalls genetisch verändert worden sein, so dass die natürlichen Regulation ausgeschaltet und die Expression der Gene erhöht wurde. Die Expressionskassette (= Expressionskonstrukt = Genkonstrukt) kann aber auch einfacher aufgebaut sein, das heißt es wurden keine zusätzlichen Regulationssignale vor die Nukleinsäuresequenz oder dessen Derivate inseriert und der natürliche Promotor mit seiner Regulation wurde nicht entfernt. Stattdessen wurde die natürliche Regulationssequenz so mutiert, dass keine Regulation mehr erfolgt und/oder die Genexpression gesteigert wird. Diese veränderten Promotoren können in Form von Teilsequenzen (= Promotor mit Teilen der erfindungsgemäßen Nukleinsäuresequenzen) auch allein vor das natürliche Gen zur Steigerung der Aktivität gebracht werden. Das Genkonstrukt kann außerdem vorteilhafterweise auch eine oder mehrere sogenannte "enhancer Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatoren. Die Acyl-CoA:Lysophospholipid-Acyltransferase-Gene sowie die vorteilhaft verwendeten Δ-4-Desaturase-, Δ5-Desaturase-, Δ-6-Desaturase- und/oder Δ-8-Desaturase-Gene und/oder die Δ-5-Elongase-, Δ-6-Elongase- und/oder Δ-9-Elongase-Gene können in einer oder mehreren Kopien in der Expressionskassette (= Genkonstrukt) enthalten sein. Vorteilhaft liegt nur jeweils eine Kopie der Gene in der Expressionskassette vor. Dieses Genkonstrukt oder die Genkonstrukte können zusammen im Wirtsorganismus exprimiert werden. Dabei kann das Genkonstrukt oder die Genkonstrukte in einem - oder mehreren Vektoren inseriert sein und frei in der Zelle vorliegen oder aber im Genom inseriert sein. Es ist vorteilhaft für die Insertion weiterer Gene im Wirtsgenom, wenn die zu exprimierenden Gene zusammen in einem Genkonstrukt vorliegen.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Offenbart werden ein oder mehrere Genkonstrukte, die eine oder mehrere Sequenzen enthalten, die durch SEQ ID NO: 1, SEQ ID NO: 3, SEQ ID NO: 5 oder SEQ ID NO: 7 oder dessen Derivate definiert sind und für Polypeptide gemäß SEQ ID NO: 2, SEQ ID NO: 4, SEQ ID NO: 6 oder SEQ ID NO: 8 kodieren. Die genannten Acyl-CoA:Lysophospholipid-Acyftransferasen führen dabei zu einem Austausch der Fettsäuren zwischen dem Mono-, Di- und/oder Triglyceridpool der Zelle und dem CoA Fettsäureester-Pool, wobei das Substrat vorteilhaft ein, zwei, drei, vier oder fünf. Doppelbindungen aufweist und vorteilhaft 16, 18, 20, 22 oder 24 Kohlenstoffatome im Fettsäuremolekül aufweist. Gleiches gilt für ihre Homologen, Derivate oder Analoga, die funktionsfähig mit einem oder mehreren Regulationssignalen, vorteilhafterweise zur Steigerung der Genexpression, verbunden sind.

Vorteilhafte Regulationssequenzen für das neue Verfahren liegen beispielsweise in Promotoren vor, wie dem cos-, tac-, trp-, tet-, trp-tet , lpp-, lac-, lpp-lac-, laclq-, T7-T5-, T3-, gal-, tro-, ara-, SP6-, λ-PR- oder λ-PL-Promotor und werden vorteilhafterweise in Gram-negativen Bakterien angewendet. Weitere vorteilhafte Regulationssequenzen liegen beispielsweise in den Gram-positiven Promotoren amy und SPO2, in den Hefe- oder Pilzpromotoren ADC1, MFα, AC, P-60, CYC1, GAPDH, TEF, rp28, ADH oder in den Pflanzenpromotoren CaMV/35S [Franck et al., Cell 21 (1980) 285-294], PRP1 [Ward et al., Plant. Mol. Biol. 22 (1993)], SSU, OCS, lib4, usp, STLS1, B33, nos oder im Ubiquitin- oder Phaseolin-Promotor vor. In diesem Zusammenhang vorteilhaft sind ebenfalls induzierbare Promotoren, wie die in EP-A-0 388186 (Benzylsulfonamid-induzierbar), Plant J. 2,1992:397-404 (Gatz et al., Tetracyclin- - induzierbar), EP-A-0 335 528 (Abzisinsäure-induzierbar) oder WO 93/21334 (Ethanol-oder Cyclohexenol-induzierbar) beschriebenen Promotoren. Weitere geeignete Pflanzenpromotoren sind der Promotor von cytosolischer FBPase oder der ST-LSI-Promotor der Kartoffel (Stockhaus et al., EMBO J. 8, 1989, 2445), der Phosphoribosylpyrophosphatamidotransferase-Promotor aus Glycine max (Genbank-Zugangsnr. U87999) oder der in EP-A-0 249 676 beschriebene nodienspezifische Promotor. Besonders vorteilhafte Promotoren sind Promotoren, welche die Expression in Geweben ermöglichen, die an der Fettsäurebipsynthese beteiligt sind. Ganz besonders vorteilhaft sind samenspezirfische Promotoren, wie der ausführungsgemäße USP Promotor aber auch andere Promotoren wie der LeB4-, DC3, Phaseolin- oder Napin-Promotor. Weitere besonders vorteilhafte Promotoren sind samenspezifische Promotoren, die für monokotyle oder dikotyle Pflanzen verwendet werden können und in US 5,608,152 (Napin-Promotor aus Raps), WO 98/45461 (Oleosin-Promotor aus Arobidopsis), US 5,504,200 (Phaseolin-Promotor aus Phaseolus vulgaris), WO 91/13980 (Bce4-Promotor aus Brassica), von Baeumlein et al., Plant J., 2, 2, 1992:233-239 (LeB4-Promotor aus einer Leguminose) beschrieben sind, wobei sich diese Promotoren für Dikotyledonen eignen. Die folgenden Promotoren eignen sich beispielsweise für Monokotyledonen lpt-2- oder Ipt-1-Promotor aus Gerste (WO 95/15389 und

WO 95/23230), Hordeln-Promotor aus Gerste und andere, in WO 99/16890 beschriebene geeignete Promotoren.

Es ist im Prinzip möglich, alle natürlichen Promotoren mit ihren Regulationssequenzen, wie die oben genannten, für das neue Verfahren zu verwenden. Es ist ebenfalls möglich und vorteilhaft, zusätzlich oder alleine synthetische Promotoren zu verwenden, besonders wenn sie eine Samen-spezifische Expression vermitteln, wie z.B. beschrieben in WO 99/16890.

Um einen besonders hohen Gehalt an PUFAs vor allem in transgenen Pflanzen zu erzielen, sollten die PUFA-Biosynthesegene vorteilhaft samenspezifisch in Ölsaaten exprimiert werden. Hierzu können Samen-spezifische Promotoren verwendet werden, bzw. solche Promotoren die im Embryo und/oder im Endosperm aktiv sind. Samenspezifische Promotoren können prinzipiell sowohl aus dikotolydonen als auch aus monokotolydonen Pflanzen isoliert werden. Im folgenden sind vorteilhafte bevorzugte Promotoren aufgeführt: USP (= unknown seed protein) und Vicilin (Vicia faba) [Bäumlein et al., Mol. Gen Genet., 1991, 225(3)], Napin (Raps) [US 5,608,152], Acyl-Carrier Protein (Raps) [US 5,315,001 und WO 92/18634], Oleosin (Arabidopsis thaliana) [WO 98/45461 und WO 93/20216], Phaseolin (Phaseolus vulgaris) [US 5,504,200], Bce4 [WO 91/13980], Leguminosen B4 (LegB4-Promotor) [Bäumlein et al., Plant J., 2,2, 1992], Lpt2 und ipt1 (Gerste) [WO 95/15389 u. WO95/23230], Samen-spezifische Promotoren aus Reis, Mais u. Weizen [WO 99/16890], Amy32b, Amy 6-6 und Aleurain [US 5,677,474], Bce4 (Raps) [US 5,530,149], Glycinin (Soja) [EP 571 741], Phosphoenol-Pyruvatcarboxylase (Soja) [JP 06/62870], ADR12-2 (Soja) [WO 98/08962], Isocitratlyase (Raps) [US 5,689,040] oder α-Amylase (Gerste) [EP 781 849].

Die Pflanzengenexpression lässt sich auch über einen chemisch induzierbare Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Um eine stabile Integration der Biosynthesegene in die transgene Pflanze über mehrere Generation sicherzustellen, sollte jede der im Verfahren verwendeten Nukleinsäuren, die für die Acyl-CoA:Lysophospholipid-Acyltransferase, die vorteilhafte Δ-4-Desaturase, die Δ-5-Desaturase, die Δ-6-Desaturase, die Δ-8-Desaturase und/oder die Δ-5-Elongase, die Δ-6-Elongase und/oder die Δ-9-Elongase codieren, unter der Kontrolle eines eigenen bevorzugt eines unterschiedlichen Promotors exprimiert werden, da sich wiederholende Sequenzmotive zu Instabilität der T-DNA bzw. zu Rekombinationsereignissen führen könnten. Die Expressionskassette ist dabei vorteilhaft so aufgebaut, dass einem Promotor eine geeignete Schnittstelle zur Insertion der zu expremierenden Nukleinsäure folgt vorteilhaft in einem Polylinker anschließend gegebenenfalls ein Terminator hinter dem Polylinker liegt. Diese Abfolge wiederholt sich mehrfach bevorzugt drei-, vier- oder fünfmal, so dass bis zu fünf Gene in einem Konstrukt zusammengeführt werden und so zur Expression in die transgene Pflanze eingebracht werden können. Vorteilhaft wiederholt sich die Abfolge bis zu dreimal. Die Nukleinsäuresequenzen werden zur Expression über die geeignete Schnittstelle beispielsweise im Polylinker hinter den Promotor inseriert. Vorteilhaft hat jede Nukleinsäuresequenz ihren eigenen Promotor und gegebenenfalls ihren eigenen Terminator. Es ist aber auch möglich mehrere Nukleinsäuresequenzen hinter einem Promotor und ggf. vor einem Terminator zu inserieren. Dabei ist die Insertionsstelle bzw. die Abfolge der inserierten Nukleinsäure in der Expressionskassette nicht von entscheidender Bedeutung, das heißt eine Nukleinsäuresequenz kann an erster oder letzter Stelle in der Kassette inseriert sein, ohne dass dadurch die Expression wesentlich beeinflusst wird. Es können in der Expressionskassette vorteilhaft unterschiedliche Promotoren wie beispielsweise der USP-, LegB4 oder DC3-Promotor und unterschiedliche Terminatoren verwendet werden. Es ist aber auch möglich nur einen Promotortyp in der Kassette zu verwenden. Dies kann jedoch zu unerwünschten Rekombinationsereignissen führen.

Wie oben beschrieben sollte die Transkription der eingebrachten Gene vorteilhaft durch geeignete Terminatoren am 3'-Ende der eingebrachten Biosynthesegene (hinter dem Stoppcodon) abgebrochen werden. Verwendet werden kann hier z.B. der OCS1 Terminator. Wie auch für die Promotoren, so sollten hier für jedes Gen unterschiedliche Terminatorsequenzen verwendet werden.

Das Genkonstrukt kann, wie oben beschrieben, auch weitere Gene umfassen, die in die Organismen eingebracht werden sollen. Es ist möglich und vorteilhaft, in die Wirtsorganismen Regulationsgene, wie Gene für Induktoren, Repressoren oder Enzyme, welche durch ihre Enzymaktivität in die Regulation eines oder mehrerer Gene eines Biosynthesewegs eingreifen, einzubringen und darin zu exprimieren. Diese Gene können heterologen oder homologen Ursprungs sein. Weiterhin können vorteilhaft im Nukleinsäurekonstrukt bzw. Genkonstrukt weitere Biosynthesegene des Fettsäure- oder Lipidstoffwechsels enthalten sein oder aber diese Gene können auf einem weiteren oder mehreren weiteren Nukleinsäurekonstrukten liegen. Vorteilhaft werden als Biosynthesegene des Fettsäure- oder Lipidstoffwechsels ein Gen ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Fettsäure-Symthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) oder deren Kombinationen verwendet. Besonders vorteilhafte Nukleinsäuresequenzen sind Biosynthesegene des Fettsäure- oder Lipidstoffwechsels ausgewählt aus der Gruppe der Δ-4-Desaturase-, Δ-5-Desaturase-, Δ-6-Desaturase-, Δ-8-Desatuase-, Δ-9-Desaturase-, Δ-12-Desaturase-, Δ-5-Elongase-, Δ-6-Elongase-oder Δ-9-Elongase.

Dabei können die vorgenannten Desaturasen in Kombination mit anderen Elongasen und Desaturasen in erfindungsgemäßen Expressionskassetten kloniert werden und zur Transformation von Pflanzen mithilfe von Agrobakterium eingesetzt werden.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird. Die Expressionskassetten können prinzipiell direkt zum Einbringen in die Pflanze verwendet werden oder aber in einen Vektoren eingebracht werden.

Diese Vektoren, vorzugsweise Expressionsvektoren, enthalten die im Verfahren verwendeten Nukleinsäuren, die für Acyl-CoA:Lysophospholipid-Acyltransferasen codieren, oder ein Nukleinsäurekonstrukt, die die verwendeten Nukleinsäure allein oder in Kombination mit weiteren Biosynthesegenen des Fettsäure-oder Lipidstoffwechsels wie Δ-4-Desaturase-, Δ-5-Desaturase-, Δ-6-Desaturase-, Δ-8-Desatuase-, Δ-9-Desaturase-, Δ-12-Desaturase-, Δ-5-Elongase-, Δ-6-dongase-und/oder Δ-9-Elongase. Wie hier verwendet, betrifft der Begriff "Vektor" ein Nukleinsäuremolekül, das eine andere Nukleinsäure transportieren kann, an welche es gebunden ist. Ein Vektortyp ist ein "Piasmid", was für eine zirkuläre doppelsträngige DNA-Schleife steht, in die zusätzlichen DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung). Andere Vektoren werden vorteilhaft beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet Gewöhnlich haben Expressionsvektoren, die für DNA-Rekombinationstechniken geeignet sind, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch diese anderen Expressionsvektorformen, wie virale Vektoren, die ähnliche Funktionen ausüben, umfassen. Ferner soll der Begriff Vektor auch andere Vektoren, die dem Fachmann bekannt sind, wie Phagen, Viren, wie SV40, CMV, TMV, Transposons, IS-Elemente, Phasmide, Phagemide, Cosmide, lineare oder zirkuläre DNA, umfassen.

Die im Verfahren vorteilhaft verwendeten rekombinanten Expressionsvektoren umfassen die die unten beschriebenen Nukleinsäuren oder das oben beschriebene GenKonstrukt in einer Form, die sich zur Expression der verwendeten Nukleinsäuren in einer Wirtszelle eignen, was bedeutet, dass die rekombinanten Expressionsvektoren eine oder mehrere Regulationssequenzen, ausgewählt auf der Basis der zur Expression zu verwendenden Wirtszellen, die mit der zu exprimierenden Nukleinsäuresequenz funktionsfähig verbunden ist, umfasst. In einem rekombinanten Expressionsvektor bedeutet "funktionsfähig verbunden", dass die Nukleotidsequenz von Interesse derart an die Regulationssequenz(en) gebunden ist, dass die Expression der Nukleotidsequenz möglich ist und sie aneinander gebunden sind, so dass beide Sequenzen die vorhergesagte, der Sequenz zugeschrieben Funktion erfüllen (z.B. in einem In-vitro-Transkriptions-/Translationssystem oder in einer Wirtszelle, wenn der Vektor in die Wirtszelle eingebracht wird). Der Begriff "Regulationssequenz" soll Promotoren, Enhancer und andere Expressionskontrollelemente (z.B. Polyadenylierungssignale) umfassen. Diese Regulationssequenzen sind z.B. beschrieben in Goeddel: Gene Expression Technology Methods in Enzymology 185, Academic Press, San Diego, CA (1990), oder siehe: Gruber und Crosby, in: Methods in Plant Molecular Biology and Biotechnolgy, CRC Press, Boca Raton, Florida, Hrsgb.: Glick und Thompson, Kapitel 7, 89-108, einschließlich der Literaturstellen darin. Regulationssequenzen umfassen solche, welche die konstitutive Expression einer Nukleotidsequenz in vielen Wirtszelitypen steuern, und solche, welche die direkte Expression der Nukleotidsequenz nur in bestimmten Wirtszellen unter bestimmten Bedingungen steuern. Der Fachmann weiß, dass die Gestaltung des Expressionsvektors von Faktoren, wie der Auswahl der zu transformierenden Wirtszelle, dem Ausmaß der Expression des gewünschten Proteins usw., abhängen kann.

Die verwendeten rekombinanten Expressionsvektoren können zur Expression von Acyl-CoA:Lysophospholipid-Acyltransferasen, Desaturasen und Elongasen in prokaryotischen oder eukaryotischen Zellen gestaltet sein. Dies ist vorteilhaft, da häufig Zwischenschritte der Vektorkonstruktion der Einfachheithalber in Mikroorganismen durchgeführt werden. Beispielsweise können Acyl-CoA:Lysophospholipid-Acyltransferase-, Desaturase- und/oder Elongase-Gene in bakteriellen Zellen, Insektenzellen (unter Verwendung von Baculovirus-Expressionsvektoren), Hefe- und anderen Pilzzellen (siehe Romanos, M.A., et al. (1992) "Foreign gene expression in yeast a review", Yeast 8:423-488; van den Hondel, C.A.M.J.J., et al. (1991) "Heterologous gene expression in filamentous fungi", in: More Gene Manipulations in Fungi, J.W. Bennet & LL Lasure, Hrsgb., S. 396-428: Academic Press: San Diego; und van den Hondel, C.A.M.J.J., & Punt, P.J. (1991) "Gene transfer systems and vector development for filamentous fungi, in: Applied Molecular Genetics of Fungi, Peberdy, J.F., et al., Hrsgb., S. 1-28, Cambridge University Press: Cambridge), Algen (Falciatore et al.,1999, Marine Biotechnology. 1, 3:239-251), Ciliaten der Typen: Holotrichia, Peritrichia, Spirotrichia, Suctoria, Tetrahymena, Paramecium, Colpidium, Glaucoma, Platyophrya, Potomacus, Desaturaseudocohnilembus, Euplotes, Engelmaniella und Stylonychia, insbesondere der Gattung Stylonychia lemnae, mit Vektoren nach einem Transformationsverfahren, wie beschrieben in WO 98/01572, sowie bevorzugt in Zellen vielzelliger Pflanzen (siehe Schmidt, R. und Willmitzer, L (1988) "High efficiency Agrobacterium tumefacions-mediated transformation of Arabidopsis thaliana leaf and cotyledon explants" Plant Cell Rep.:583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993),128-43; Potrykus, Annu. Rev. Plant Physiol. Plant Molec. Biol. 42 (1991), 205-225 (und darin zitierte Literaturstellen)) exprimiert werden. Geeignete Wirtszellen werden ferner erörtert in Goeddel, Gene Expression Technology: Methods in Enzymology 185, Academic Press, San Diego, CA (1990). Der rekombinante Expressionsvektor kann alternativ, zum Beispiel unter Verwendung von T7-Promotor-Regulationssequenzen und T7-Polymerase, in vitro transkribiert und translatiert werden.

Die Expression von Proteinen in Prokaryoten erfolgt meist mit Vektoren, die konstitutive oder induzierbare Promotoren enthalten, welche die Expression von Fusions- oder nicht-Fusionsproteinen steuern. Typische Fusions-Expressionsvektoren sind u.a. pGEX (Pharmacia Biotech Inc; Smith, D.B., und Johnson, K.S. (1988) Gene 67:31-40), pMAL (New England Biolabs, Beverly, MA) und pRIT5 (Pharmacia, Piscataway, NJ), bei denen Glutathion-S-Transferase (GST), Maltose E-bindendes Protein bzw. Protein A an das rekombinante Zielprotein fusioniert wird.

Beispiele für geeignete induzierbare nicht-Fusions-E. coli-Expressionsvektoren sind u.a. pTrc (Amann et al. (1988) Gene 69:301-315) und pET 11d (Studier et al., Gene Expression Technology: Methods in Enzymology 185, Academic Press, San Diego, Kalifornien (1990) 60-89). Die Zielgenexpression vom pTrc-Vektor beruht auf der Transkription durch Wirts-RNA-Polymerase von einem Hybrid-trp-lac-Fusionspromotor. Die Zielgenexpression aus dem pET 11 d-Vektor beruht auf der Transkription von einem T7-gn10-lac-Fusions-Promotor, die von einer coexprimierten viralen RNA-Polymerase (T7 gn1) vermittelt wird. Diese virale Polymerase wird von den Wirtsstämmen BL21 (DE3) oder HMS174 (DE3) von einem residenten λ-Prophagen bereitgestellt, der ein T7 gn1-Gen unter der Transkriptionskontrolle des lacUV 5-Promotors birgt.

Andere in prokaryotischen Organismen geeignete Vektoren sind dem Fachmann bekannt, diese Vektoren sind beispielsweise in E. coli pLG338, pACYC184, die pBR-Reihe, wie pBR322, die pUC-Reihe, wie pUC18 oder pUC19, die M113mp-Reihe, pKC30, pRep4, pHS1, pHS2, pPLc236, pMBL24, pLG200, pUR290, pIN-III113-B1, λgt11 or pBdCl, in Streptomyces pIJ101, plJ364, plJ702 oder pIJ361, in Bacillus pUB110, pC194 oder pBD214, in Corynebacterium pSA77 oder pAJ667.

Bei einer weiteren Ausführungsform ist der Expressionsvektor ein Hefe-Expressionsvektor. Beispiele für Vektoren zur Expression in der Hefe S. cerevisiae umfassen pYe-Desaturasec1 (Baldari et al. (1987) Embo J. 6:229-234), pMFa (Kurjan und Herskowitz (1982) Cell 30:933-943), pJRY88 (Schuitz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie den filamentösen Pilzen, eignen, umfassen diejenigen, die eingehend beschrieben sind in: van den Hondel, C.A.M.J.J., & Punt, P.J. (1991) "Gene transfer systems and vector development for filamentous fungi, in: Applied Molecular Genetics of fungi, J.F. Peberdy et al., Hrsgb., S. 1-28, Cambridge University Press: Cambridge, oder in: More Gene Manipulations in Fungi [J.W. Bennet & L.L. Lasure, Hrsgb., S. 396-428: Academic Press: San Diego]. Weitere geeignete Hefevektoren sind beispielsweise pAG-1, YEp6, YEp13 oder pEMBLYe23.

Alternativ können die Acyl-CoA:Lysophospholipid-Acyitransferasen, Desaturasen und/oder Elongasen in Insektenzellen unter Verwendung von Baculovirus-Expressionsvektoren exprimiert werden. Baculovirus-Vektoren, die zur Expression von Proteinen in gezüchteten Insektenzellen (z.B. Sf9-Zellen) verfügbar sind, umfassen die pAc-Reibe (Smith et al. (1983) Mol. Cell Biol.. 3:2156-2165) und die pVL-Reihe (Lucklow und Summers (1989) Virology 170:31-39).

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018). Weitere geeignete Expressionssysteme für prokaryotische und eukaryotische Zellen siehe in den Kapiteln 16 und 17 von Sambropk, J., Fritsch, E.F., und Maniatis, T., Molecular Cloning: A Laboratory Manual, 2. Auflage, Cold Spring Harbor Laboratory, Cold Spring Harbor Laboratory Press, Cold Spring Harbor, NY, 1989.

Bei einer weiteren Ausführungsform des Verfahrens können die Acyl-CoA:Lysophospholipid-Acyltransferasen, Desaturasen und/oder Elongasen in einzelligen Pflanzenzellen (wie Algen), siehe Falciatore et al., 1999, Marine Biotechnology 1 (3):239-251 und darin zitierte Literaturangaben, und Pflanzenzellen aus höheren Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell, J., und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38.

Eine Pflanzen-Expressionskassette enthält vorzugsweise Regulationssequenzen, welche die Genexpression in Pflanzenzellen steuern können und funktionsfähig verbunden sind, so dass jede Sequenz ihre Funktion, wie Termination der Transkription, erfüllen kann, beispielsweise Polyadenylierungssignale. Bevorzugte Polyadenylierungssignale sind diejenigen, die aus Agrobacterium tumefaciens-T-DNA stammen, wie das als Octopinsynthase bekannte Gen 3 des Ti-Plasmids pTiACH5 (Gielen et al., EMBO J. 3 (1984) 835ff.) oder funktionelle. Äquivalente davon, aber auch alle anderen in Pflanzen funktionell aktiven Terminatoren sind geeignet.

Da die Pflanzengenexpression sehr oft nicht auf Transkriptionsebenen beschränkt ist, enthält eine Pflanzen-Expressionskassette vorzugsweise andere funktionsfähig verbunden Sequenzen, wie Translationsenhancer, beispielsweise die Overdrive-Sequenz, welche die 5'-untranslatierte Leader-Sequenz aus Tabakmosaikvirus, die das Prote-in/RNA-Verhältnis erhöht, enthält (Gallie et al., 1987, Nucl. Acids Research 15:8693-8711).

Die Pflanzengenexpression-muss wie oben beschrieben funktionsfähig mit einem geeigneten Promotor verbunden sein, der die Genexpression auf rechtzeitige, zell- oder gewebespezifische Weise durchführt Nutzbare Promotoren sind konstitutive Promotoren (Benfey et al., EMBO J. 8 (1989) 2195-2202), wie diejenigen, die von Pflanzenviren stammen, wie 35S CAMV (Franck et al., Cell 21 (1980) 285-294), 19S CaMV (siehe auch US 5352605 und WO 84/02913) oder Pflanzenpromotoren, wie der in US 4,962,028 beschriebene der kleinen Untereinheit der Rubisco.

Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment notwendig sind (siehe eine Übersicht in Kermode, Crit Rev. Plant Sci. 15, 4 (1996) 285-423 und darin zitierte Literaturstellen), beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Die Pflanzengenexpression lässt sich auch wie oben beschrieben über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzerbarer Promotor (WO 95/19443), ein Tetracyclin-induzierbarer Promotor (Gatz et al. (1992) Plant J. 2, 397-404) und ein Ethanol-Induzierbarer Promotor.

Auch Promotoren, die auf biotische oder abiotische Stressbedingungen reagieren, sind geeignete Promotoren, beispielsweise der pathogeninduzierte PRP1-Gen-Promotor (Ward et al., Plant. Mol. Biol. 22 (1993) 361-366), der hitzeinduzierbare hsp80-Promotor aus Tomate (US 5,187,267), der kälteinduzierbare Alphaamylase-Promotor aus Kartoffel (WO 96/12814) oder der durch Wunden induzierbare pinll-Promotor (EP-A-0 375 091).

Es sind insbesondere solche Promotoren bevorzugt, welche die Genexpression in Geweben und Organen herbeiführen, in denen die Fettsäure-, Lipid- und Ölbiosynthese stattfindet, in Samenzellen, wie den Zellen des Endosperms und des sich entwickelnden Embryos. Geeignete Promotoren sind der Napingen-Promotor aus Raps (US 5,608,152), der USP-Promotor aus Vicia faba (Baeumlein et al., Mol Gen Genet, 1991, 225 (3):459-67), der Oleosin-Promotor aus Arabidopsis (WO 98/45461), der Phaseolin-Promotor aus Phaseolus vulgaris (US 5,504,200), der Bce4-Promotor aus Brassica (WO 91/13980) oder der Legumin-B4-Promotor (LeB4; Baeumlein et al., 1992, Plant Journal, 2 (2):233-9) sowie Promotoren, welche die samenspezifische Expression in Monokotyledonen-Pflanzen, wie Mais, Gerste, Weizen, Roggen, Reis usw. herbeiführen. Geeignete beachtenswerte Promotoren sind der lpt2- oder lpt1-Gen-Promotor aus Gerste (WO 95/15389 und WO 95/23230) oder die in WO 99/16890 beschriebenen (Promotoren aus dem Gersten-Hordein-Gen, dem Reis-Glutelin-Gen, dem Reis-Oryzin-Gen, dem Reis-Prolamin-Gen, dem Weizen-Gliadin-Gen, Weizen-Glutelin-Gen, dem Mais-Zein-Gen, dem Hafer-Glutelin-Gen, dem Sorghum-Kasirin-Gen, dem Roggen-Secalin-Gen).

Insbesondere kann die multiparallele Expression der im Verfahren verwendeten Acyl-CoA:Lysophospholipid-Acyltransterasen allein oder in Kombination mit Desaturasen und/oder Elongasen gewünscht sein. Die Einführung solcher Expressionskassetten kann über eine simultane Transformation mehrerer einzelner Expressionskonstrukte erfolgen oder bevorzugt durch Kombination mehrerer Expressionskassetten auf einem Konstrukt. Auch können mehrere Vektoren mit jeweils mehreren Expressionskassetten transformiert und auf die Wirtszelle übertragen werden.

Ebenfalls besonders geeignet sind Promotoren, welche die plastidenspezfische Expression herbeiführen, da Plastiden das Kompartiment sind, in dem die Vorläufer sowie einige Endprodukte der Lipidbiosynthese synthetisiert werden. Geeignete Promotoren, wie der virale RNA-Polymerase-Promotor, sind beschrieben in WO 95/16783 und WO 97/06250, und der clpP-Promotor aus Arabidopsis, beschrieben in WO 99/46394.

Vektor-DNA lässt sich in prokaryotische oder eukaryotische Zellen über herkömmliche Transformations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäure (z.B. DNA) in eine Wirtszelle, einschließlich Calciumphosphat- oder Calciumchlorid-Copräzipitation, DEAE-Dextran-vermittelte Transfektion, Lipofektion, natürliche Kompetenz, chemisch vermittelter Transfer, Elektroporation oder Teilchenbeschuss, umfassen. Geeignete Verfahren zur Transformation oder Transfektion von Wirtszellen, einschließlich Pflanzenzellen, lassen sich finden in Sambrook et al. (Molecular Cloning: A Laboratory Manual., 2. Aufl., Cold Spring Harbor Laboratory, Cold Spring Harbor.Laboratory Press, Cold Spring Harbor, NY, 1989) und anderen Labor-Handbüchern, wie Methods in Molecular Biology, 1995, Bd. 44, Agrobacterium protocols, Hrsgb: Gartland und Davey, Humana Press, Totowa, New Jersey.

Wirtszellen, die im Prinzip zum Aufnehmen der erfindungsgemäßen Nukleinsäure, des erfindungsgemäßen Genproduktes oder des erfindungsgemäßen Vektors geeignet sind, sind alle prokaryotischen oder eukaryotischen Organismen. Die vorteilhafterweise verwendeten Wirtsorganismen sind Mikroorganismen, wie Pilze oder Hefen oder Pflanzenzellen vorzugsweise Pflanzen oder Teile davon. Pilze, Hefen oder Pflanzen werden vorzugsweise verwendet, besonders bevorzugt Pflanzen, ganz besonders bevorzugt Pflanzen, wie Ölfruchtpflanzen, die große Mengen an Lipidverbindungen enthalten, wie Raps, Nachtkerze, Hanf, Diestel, Erdnuss, Canola, Lein, Soja, Safflor, Sonnenblume, Borretsch, oder Pflanzen, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa, Buschpflanzen (Kaffee, Kakao, Tee), Salix-Arten, Bäume (Ölplame, Kokosnuss) sowie ausdauernde Gräser und Futterfeldfrüchte. Besonders bevorzugte erfindungsgemäße Pflanzen sind Ölfruchtpflanzen, wie Soja, Erdnuß, Raps, Canola, Lein, Hanf, Nachtkerze, Sonnenblume, Safflor, Bäume (Ölpalme, Kokosnuß).

Offenbart werden zudem isolierte Nukleinsäuresequenzen, die für Polypeptide mit Acyl-CoA:Lysophospholipid-Acyltransferaseaktivität codieren; wobei die durch die Nukleinsäuresequenzen codierten Acyl-CoA:Lysophospholipid-Acyltransferasen spezifisch C₁₆-, C₁₈-, C₂₀- oder C₂₂-Fettsäuren mit mindestens einer Doppelbindungen im Fettsäuremolekül umsetzen.

Vorteilhafte isolierte Nukleinsäuresequenzen sind Sequenzen ausgewählt aus der Gruppe:
d) einer Nukleinsäuresequenz mit der in SEQ ID NO: 1, SEQ ID NO: 3, SEQ ID NO: 5 oder SEQ ID NO: 7 dargestellten Sequenz,
e) Nukleinsäuresequenzen, die sich als Ergebnis des degenerierten genetischen Codes von der in SEQ ID NO: 1, SEQ ID NO: 3, SEQ ID NO: 5 oder SEQ ID NO: 7 enthaltenden codierenden Sequenz ableiten lassen
f) Derivate der in SEQ ID NO:1, SEQ ID NO: 3, SEQ ID NO: 5 oder SEQ ID NO: 7 dargestellten Nukleinsäuresequenz, die für Polypeptide mit der in SEQ ID NO: 2, SEQ ID NO: 4, SEQ ID NO: 6 oder SEQ ID NO: 8 dargestellten Aminosäuresequenz codieren und mindestens 90 % Homologie auf Aminosäureebene mit SEQ ID NO: 2, SEQ ID NO: 4, SEQ ID NO: 6 oder SEQ ID NO: 8 aufweisen und eine Acyl-CoA:Lysophospholipid-Acyltransferaseaktivität aufweisen.

Die oben genannte Nukleinsäuren stammen von Organismen, wie Tieren, Ciliaten, Pilzen, Pflanzen wie Algen oder Dinoflagellaten, die PUFAs synthetisieren können.

Der Begriff "Nukleinsäure(molekül)", wie hier verwendet, umfasst in einer vorteilhaften Ausführungsform zudem die am 3'- und am 5'-Ende des kodierenden Genbereichs gelegene untranslatierte Sequenz: mindestens 500, bevorzugt 200, besonders bevorzugt 100 Nukleotide der Sequenz stromaufwärts des 5'-Endes des kodierenden Bereichs und mindestens 100, bevorzugt 50, besonders bevorzugt 20 Nukleotide der Sequenz stromabwärts des 3'-Endes des kodierenden Genbereichs. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z.B. Sequenzen, die sich an den 5'- und 3'-Enden der Nukleinsäure befinden). Bei verschiedenen Ausführungsformen kann das isolierte Acyl-CoA:Lysophospholipid-Acyltransferasemolekül zum Beispiel weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt flankieren.

Die im-Verfahren verwendeten Nukleinsäuremoleküle, z.B. ein Nukleinsäuremolekül mit einer Nuldeotidsequenz der SEQ ID NO:1, SEQ ID NO: 3, SEQ ID NO: 5 oder SEQ ID NO: 7 oder eines Teils davon, kann unter Verwendung molekularbiologischer Standardtechniken und der hier bereitgestellten Sequenzinformation isoliert werden. Auch kann mithilfe von Vergleichsalgorithmen beispielsweise eine homologe Sequenz oder homologe, konservierte Sequenzbereiche auf DNA oder Aminosäureebene identifiziert werden. Diese können als Hybridisierungssonde sowie Standard-Hybridisierungstechniken (wie z.B. beschrieben in Sambrook et al., Molecular Cloning: A Laboratory Manual. 2. Aufl., Cold Spring Harbor Laboratory, Cold Spring Harbor Laboratory Press, Cold Spring Harbor, NY, 1989) zur Isolierung weiterer im Verfahren nützlicher Nukleinsäuresequenzen verwendet werden. Überdies lässt sich ein Nukleinsäuremolekül, umfassend eine vollständig Sequenz der SEQ ID NO: 1, SEQ ID NO: 3, SEQ ID NO: 5 oder SEQ ID NO: 7 oder einen Teil davon, durch Polymerasekettenreaktion isolieren, wobei Oligonukleotidprimer, die auf der Basis dieser Sequenz oder von Teilen davon, verwendet werden (z.B. kann ein Nukleinsäuremolekül, umfassend die vollständigen Sequenz oder einen Teil davon, durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18:5294-5299) und cDNA mittels Reverser Transkriptase (z.B. Moloney-MLV-Reverse-Transkriptase, erhältlich von Gibco/BRL, Bethesda, MD, oder AMV-Reverse-Transkriptase, erhältlich von Seikagaku America, Inc., St.Petersburg, FL) herstellen. Synthetische Oligonukleotidprimer zur Amplifizierung mittels Polymerasekettenreaktion lassen sich auf der Basis einer der in SEQ ID NO:1, SEQ ID NO: 3, SEQ ID NO: 5 oder SEQ ID NO: 7 gezeigten Sequenzen oder mithilfe der in SEQ ID NO: 2, SEQ ID NO: 4, SEQ ID NO: 6 oder SEQ ID NO: 8 dargestellten Aminosäuresequenzen erstellen. Eine erfindungsgemäße Nukleinsäure kann unter Verwendung von cDNA oder alternativ von genomischer DNA als Matrize und geeigneten Oligonukleotidprimern gemäß Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer Desaturase-Nukleotidsequenz entsprechen, können durch Standard-Syntheseverfahren, beispielsweise mit einem automatischen DNA-Synthesegerät, hergestellt werden.

Homologe der verwendeten Acyl-CoA:Lysophospholipid-Acyltransferase-Nukleinsäuresequenzen mit der Sequenz SEQ ID NO: 1, SEQ ID NO: 3, SEQ ID NO: 5 oder SEQ ID NO: 7 bedeutet beispielsweise allelische Varianten mit mindestens 90 bis 95 % und noch stärker bevorzugt mindestens 95 %, 96 %, 97 %, 98 %, 99 % oder mehr Identität zu einer in SEQ ID NO: 1, SEQ ID NO: 3, SEQ ID NO: 5 oder SEQ ID NO: 7 gezeigten Nukleotidsequenzen oder ihren Homologen, Derivaten oder Analoga oder Teilen davon. Weiterhin sind isolierte Nukleinsäuremoleküle einer Nukleotidsequenz, die an eine der in SEQ ID NO: 1, SEQ ID NO: 3, SEQ ID NO: 5 oder SEQ ID NO: 7 gezeigten Nukleotidsequenzen oder einen Teil davon hybridisieren, z.B. unter stringenten Bedingungen hybridisiert. Allelische Varianten umfassen insbesondere funktionelle Varianten, die sich durch Deletion, Insertion oder Substitution von Nukleotiden aus/in der in SEQ ID NO:1, SEQ ID NO: 3, SEQ ID NO: 5 oder SEQ ID NO: 7 dargestellten Sequenz erhalten lassen, wobei aber die Absicht ist, dass die Enzymaktivität der davon herrührenden synthetisierten Proteine für die Insertion eines oder mehrerer Gene vorteilhafterweise beibehalten wird. Proteine, die noch die enzymatische Aktivität der Acyl-CoA:Lysophospholipid-Acyltransferase besitzen, das heißt deren Aktivität im wesentlichen nicht reduziert ist, bedeutet Proteine mit mindestens 10 %, vorzugsweise 20 %, besonders bevorzugt 30 %, ganz besonders bevorzugt 40 % der ursprünglichen Enzymaktivität, verglichen mit dem durch SEQ ID NO:1, SEQ ID NO: 3, SEQ ID NO: 5 oder SEQ ID NO: 7 kodierten Protein.

Homologen der SEQ ID NO: 1, SEQ ID NO: 3, SEQ ID NO: 5 oder SEQ ID NO: 7 bedeuten beispielsweise auch bakterielle, Pilz- und Pflanzenhomologen, verkürzte Sequenzen, einzelsträngige DNA oder RNA der kodierenden und nicht-kodierenden DNA-Sequenz.

Homologen der SEQ ID NO: 1, SEQ ID NO: 3, SEQ ID NO: 5 oder SEQ ID NO: 7 bedeutet auch Derivate, wie beispielsweise Promotorvarianten. Die Promotoren stromaufwärts der angegebenen Nukleotidsequenzen können durch einen oder mehrere Nukleotidaustausche, durch Insertion(en) und/oder Deletion(en) modifiziert werden, ohne dass jedoch die Funktionalität oder Aktivität der Promotoren gestört wird. Es ist weiterhin möglich, dass die Aktivität der Promotoren durch Modifikation ihrer Sequenz erhöht ist oder dass sie vollständig durch aktivere Promotoren, sogar aus heterologen Organismen, ersetzt werden.

Die vorgenannten Nukleinsäuren und Proteinmoleküle mit Acyl-CoA:Lysophospholipid-Acyltransferaseaktivität, die am Stoffwechsel von Lipiden und Fettsäuren, PUFA-Cofaktoren und Enzymen oder am Transport lipophiler Verbindungen über Membranen beteiligt sind, werden im erfindungsgemäßen Verfahren zur Modulation der Produktion von PUFAs in transgenen Organismen vorteilhaft in Pflanzen, wie Mais, Weizen, Roggen, Hafer, Triticale, Reis, Gerste, Sojabohne, Erdnuss, Baumwolle, Linum Arten wie Öl- oder Faserlein, Brassica-Arten, wie Raps, Canola und Rübsen, Pfeffer, Sonnenblume, Borretsch, Nachtkerze und Tagetes, Solanacaen-Pflanzen, wie Kartoffel, Tabak, Aubergine und Tomate, Vicia-Arten, Erbse, Maniok, Alfalfa, Buschpflanzen (Kaffee, Kakao, Tee), Salix-Arten, Bäume (Ölpalme, Kokosnuss) und ausdauernden Gräsern und Futterfeldfrüchten, entweder direkt (z.B. wenn die Überexpression oder Optimierung eines Fettsäurebiosynthese-Proteins einen direkten Einfluss auf die Ausbeute, Produktion und/oder Effizienz der Produktion der Fettsäure aus modifizierten Organismen hat) verwendet und/oder können eine indirekt Auswirkung haben, die dennoch zu einer Steigerung der Ausbeute, Produktion und/oder Effizienz der Produktion der PU-FAs oder einer Abnahme unerwünschter Verbindungen führt (z.B. wenn die Modulation des Stoffwechsels von Lipiden und Fettsäuren, Cofaktoren und Enzymen zu Veränderungen der Ausbeute, Produktion und/oder Effizienz der Produktion oder der Zusammensetzung der gewünschten Verbindungen innerhalb der Zellen führt, was wiederum die Produktion einer oder mehrerer Fettsäuren beeinflussen kann).

Die Kombination verschiedener Vorläufermoleküle und Biosyntheseenzyme führt zur Herstellung verschiedener Fettsäuremoleküle, was eine entscheidende Auswirkung auf die Zusammensetzung der Lipide hat Da mehrfach ungesättigte Fettsäuren (= PUFAs) nicht nur einfach in Triacylglycerin sondern auch in Membranlipide eingebaut werden.

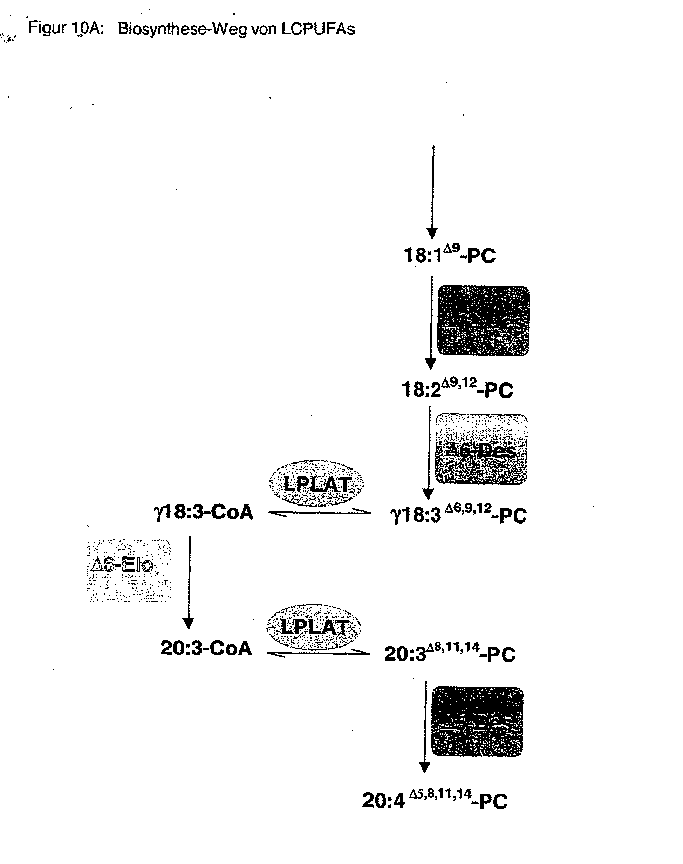

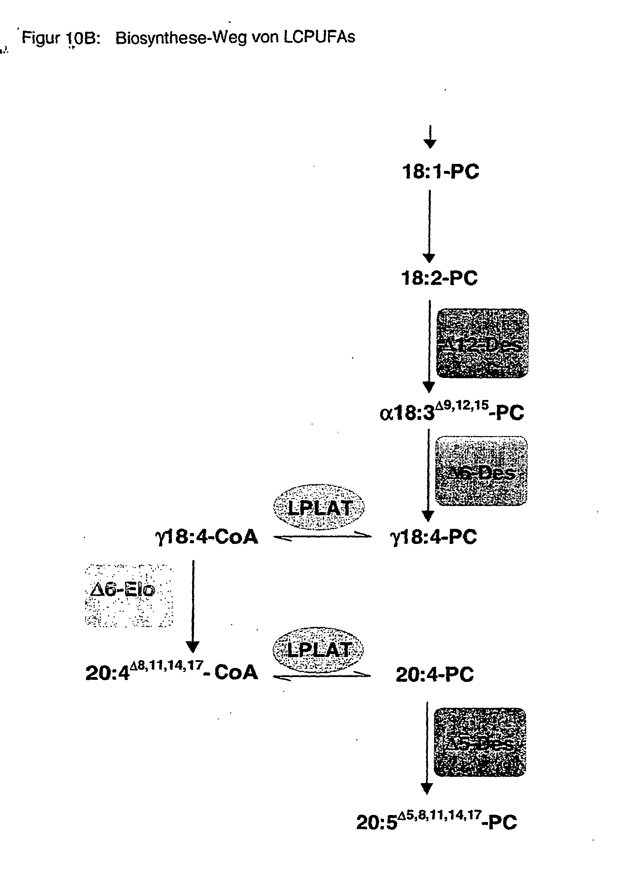

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipid, Glycolipide, Sphingolipide und Phosphoglyceride. Die Fettsäuresynthese beginnt mit der Umwandlung von Acetyl-CoA in Malonyl-CoA durch die Acetyl-CoA-Carboxylase oder in Acetyl-ACP durch die Acetyltransacylase. Nach einer Kondensationsreaktion bilden diese beiden Produktrnoleküte zusammen Acetoacetyl-ACP, das über eine Reihe von Kondensations-, Reduktions- und Dehydratisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen). Die so hergestellten an Phospholipide gebundenen Fettsäuren müssen anschließend wieder für die weitere Elongationen aus den Phospholipiden in den FettsäureCoA-Ester-Pool überführt werden. Dies ermöglichen die erfindungsgemäßen Acyl-CoA:Lysophospholipid-Acyltransferasen. Weiterhin können diese Enzyme die elongierten Fettsäuren wieder von den CoA-Estem auf die Phospholipide übertragen. Diese Reaktionsabfolge kann gegebenenfalls mehrfach durchlaufen werden (siehe Figur 10).

Vorläufer für die PUFA-Biosynthese sind beispielsweise Ölsäure, Linol- und Linolensäure. Diese C₁₈-Kohlenstoff-Fettsäuren müssen auf C₂₀ und C₂₂ verlängert werden, damit Fettsäuren vom Eicosa- und Docosa-Kettentyp erhalten werden. Mithilfe der im Verfahren verwendeten Acyl-CoA:Lysophospholipid-Acyltransferasen, vorteilhaft in Kombination mit Desaturasen wie der Δ-4-, Δ-5-, A-6- und Δ-8-Desaturasen und/oder der Δ-5-, Δ-6-, Δ-9-Elongase können Arachidonsäure, Eicosapentaensäure, Docosapentaensäure oder Docosahexaensäure sowie verschiedene andere langkettige PUFAs erhalten, extrahiert und für verschiedene Zwecke bei Nahrungsmittel-, Futter-, Kosmetik- oder pharmazeutischen Anwendungen verwendet werden. Mit den genannten Enzymen können vorzugsweise C₁₈-, C₂₀-, und/oder C₂₂-Fettsäuren mit mindestens zwei vorteilhaft mindestens drei, vier, fünf oder sechs Doppelbindungen im Fettsäuremolekül, vorzugsweise zu C₂₀-, und/oder C₂₂-Fettsäuren mit vorteilhaft drei, vier oder fünf Doppelbindungen im Fettsäuremolekül hergestellt werden. Die Desaturierung kann vor oder nach Elongation der entsprechenden Fettsäure erfolgen. Daher führen die Produkte der Desaturaseaktivitäten und der möglichen weiteren Desaturierung und Elongation zu bevorzugten PUFAs mit höherem Desaturierungsgrad, einschließlich einer weiteren Elongation von C₂₀ zu C₂₂-Fettsäuren, zu Fettsäuren wie γ-Linolensäure, Dihomo-γ-linolensäure, Arachidonsäure, Stearidonsäure, Eicosatetraensäure oder Eicosapentaensäure. Substrate Substrat der Acyl-CoA:Lysophospholipid-Acyltransferasen im erfindungsgemäßen Verfahren sind C₁₆, C₁₈-, C₂₀ oder C₂₂-Fettsäuren wie zum Beispiel Palmitinsäure, Palmitoleinsäure, Linolsäure, γ-Linolensäure, α-Linolensäure, Dihomo-γ-linolensäure, Eicosatetraensäure oder Stearidonsäure. Bevorzugte Substrate sind Linolsäure, γ-Linofensäure und/oder α-Linolensäure, Dihomo-γ-linolensäure bzw. Arachidonsäure, Eicosatetraensäure oder Eicosaptsntaensäure. Die C₁₈-, C₂₀- oder C₂₂-Fettsäuren mit mindestens zwei Doppelbindungen in der Fettsäure fallen im erfindungsgemäßen Verfahren in Form der freien Fettsäure oder in Form ihrer Ester beispielsweise in Form ihrer Glyceride an.

Unter dem Begriff "Glycerid" wird ein mit ein, zwei oder drei Carbonsäureresten verestertes Glycerin verstanden (Mono-, Di- oder Triglycerid). Unter "Glycerid" wird auch ein Gemisch an verschiedenen Glyceriden verstanden. Das Glycerid oder das Gylceridgemisch kann weitere Zusätze, z.B. freie Fettsäuren, Antioxidantien, Proteine, Kohlenhydrate, Vitamine und/oder andere Substanzen enthalten.

Unter einem "Glycerid" im Sinne des erfindungsgemäßen Verfahrens werden ferner vom Glycerin abgeleitete Derivate verstanden. Dazu zählen neben den oben beschriebenen Fettsäureglyceriden auch Glycerophosphorlipide und Glyceroglycolipide: Bevor zugt seien hier die Glycerophospholipide wie Lecithin (Phosphatidylcholin), Cardiolipin, Phosphatidylglycerin, Phosphatidylserin und Alkylacylglycerophospholipide beispielhaft genannt

Ferner müssen Fettsäuren anschließend an verschiedene Modifikationsorte transportier und in das Triacylglycertn-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyftransferase (siehe Frentzen, 1998, Lipid, 100(4-5):161-166).

Veröffentlichungen über die Pflanzen-Fettsäurebiosynthese, Desaturierung, den Lipidstoffwechsel und Membrantransport von fetthaltigen Verbindungen, die Betaoxidation, Fettsäuremodfikation und Cofaktoren, Triacylglycerin-Speicherung und -Assemblierung einschließlich der Literaturstellen darin siehe in den folgenden Artikeln: Kinney, 1997, Genetic Engeneering, Hrsgb.: JK Setlow, 19:149-166; Ohlrogge und Browse, 1995, Plant Cell 7:957-970; Shanklin und Cahoon, 1998, Annu. Rev. Plant Physiol. Plant Mol. Biol. 49:611-641; Voelker, 1996, Genetic Engeneering, Hrsgb.: JK Setlow, 18:111-13; Gerhardt, 1992, Prog. Lipid R. 31:397-417; Gühnemann-Schäfer & Kindl, 1995, Biochim. Biophys Acta 1256:181-186; Kunau et al., 1995, Prog. Lipid Res. 34:267-342; Stymne et al., 1993, in: Biochemistry and Molecular Biology of Membrane and Storage Lipids of Plants, Hrsgb.: Murata und Somerville, Rockville, American Society of plant Physiologists, 150-158, Murphy & Ross 1998, Plant Journal. 13(1):1-16.

Die im Verfahren hergestellten PUFAs, umfassen eine Gruppe von Molekülen, die höhere Tiere nicht mehr synthetisieren können und somit aufnehmen müssen oder die höhere Tiere nicht mehr ausreichend selbst herstellen können und somit zusätzlich aufnehmen müssen, obwohl sie leicht von anderen Organismen, wie Bakterien, synthetisiert werden, beispielsweise können Katzen Arachidonsäure nicht mehr synthetisieren.

Der Begriff " Acyl-CoA:Lysophospholipid-Acyltransferasen " im Sinne der Erfindung umfasst Proteine, die am Transfer der an Phospholipide gebundenen Fettsäuren in den CoA-Ester-Pool und vice versa teilnehmen, sowie ihre Homologen, Derivaten oder Analoga. ,Unter Phospholipiden im Sinne der Erfindung sind zu verstehen Phosphatidylcholin, Phosphatidylethanoiamin, Phosphatidylserin, Phosphatidylglycerin, und/oder Phosphatidylinositol vorteilhafterweise Phosphatidylcholin. Die Begriffe Acyl-CoA:Lysophospholipid-Acyltransferase-Nukleinsäuresequenz(en) umfassen Nukleinsäuresequenzen, die eine Acyl-CoA:Lysophospholipid-Acyltransferase kodieren und bei denen ein Teil eine kodierende Region und ebenfalls entsprechende 5'- und 3'-untranslatierte Sequenzbereiche sein können. Die Begriffe Produktion oder Produktivität sind im Fachgebiet bekannt und beinhalten die Konzentration des Fermentationsproduktes (Verbindungen der Formel I), das in einer bestimmten Zeitspanne und einem bestimmten Fermentationsvolumen gebildet wird (z.B. kg Produkt pro Stunde pro Liter). Der Begriff Effizienz der Produktion umfasst die Zeit, die zur Erzielung einer bestimmten Produktionsmenge nötig ist (z.B. wie lange die Zelle zur Aufrichtung einer bestimmten Durchsatzrate einer Feinchemikalie benötigt). Der Begriff Ausbeute oder Produkt/Kohtenstoff-Aüsbeute ist im Fachgebiet bekannt und umfasst die Effizienz der Umwandlung der Kohlenstoffquelle in das Produkt (d.h. die Feinchemikalie). Dies wird gewöhnlich beispielsweise ausgedrückt als kg Produkt pro kg Kohlenstoffquelle. Durch Erhöhen der Ausbeute oder Produktion der Verbindung wird die Menge der gewonnenen Moleküle oder der geeigneten gewonnenen Moleküle dieser Verbindung in einer bestimmten Kulturmenge über einen festgelegten Zeitraum erhöht Die Begriffe Biosynthese oder Biosyntheseweg sind im Fachgebiet bekannt und umfassen die Synthese einer Verbindung, vorzugsweise einer organischen Verbindung, durch eine Zelle aus Zwischenverbindungen, beispielsweise in einem Mehrschritt- und stark regulierten Prozess. Die Begriffe Abbau oder Abbauweg sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle) beispielsweise in einem Mehrschritt- und stark regulierten Prozess. Der Begriff Stoffwechsel ist im Fachgebiet bekannt und umfasst die Gesamtheit der biochemischen Reaktionen, die in einem Organismus stattfinden. Der Stoffwechsel einer bestimmten Verbindung (z.B. der Stoffwechsel einer Fettsäure) umfasst dann die Gesamtheit der Biosynthese-, Modifikations- und Abbauwege dieser Verbindung in der Zelle, die diese Verbindung betreffen.

Derivate des efindungsgemäßen Nukleinsäuremoteküls wie hier in offenbart und wieder gegeben in SEQ ID NO: 1, SEQ ID NO: 3, SEQ ID NO: 5 oder SEQ ID NO: 7 Proteine mit mindestens 90 %, 90 bis 95 % und am stärksten bevorzugt mindestens etwa 96 %, 97 %, 98 %, 99 % oder mehr Identität zu einer vollständigen Aminosäuresequenz der SEQ ID NO: 2, SEQ ID NO: 4, SEQ ID NO: 6 oder SEQ ID NO: 8. Die Homologie wurde über den gesamten Aminosäure- bzw. Nukleinsäuresequenzbereich berechnet Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm BestFit über den gesamten Sequenzbereich mit folgenden Einstellungen vermittelt Gap Weight 8, Length Weight 2.

Offenbart werden auch Nukleinsäuremoleküle, die sich von einer der in SEQ ID NO: 1, SEQ ID NO: 3, SEQ ID NO: 5 oder SEQ ID NO: 7 gezeigten Nukleotidsequenzen (und Teilen davon) aufgrund des degenerierten genetischen Codes unterscheiden und somit die gleiche Acyl-CoA:Lysophospholipid-Acyltransferase kodieren wie diejenige, die von den in SEQ ID NO:1, SEQ ID NO: 3, SEQ ID NO: 5 oder SEQ ID NO: 7 gezeigten Nukleotidsequenzen kodiert wird.

Zusätzlich zu den in SEQ ID NO: 1, SEQ ID NO: 3, SEQ ID NO: 5 oder SEQ ID NO: 7 gezeigten Acyl-CoA:Lysophospholipid-Acyltransferase-Nuldeotidsequenzen erkennt der Fachmann, dass DNA-Sequenzpolymorphismen, die zu Änderungen in den Aminosäuresequenzen der Acyl-CoA:Lysophospholipid-Acyltransferasen führen, innerhalb einer Population existieren können. Diese genetischen Polymorphismen im Acyl-CoA:Lysophospholipid-Acyltransferase-Gen können zwischen Individuen innerhalb einer Population aufgrund von natürlicher Variation existieren. Diese natürlichen Varianten bewirken üblicherweise eine Varianz von 1 bis 5 % in der Nukleotidsequenz des Acyl-CoA:Lysophospholipid-Acyltransferase-Gens. Sämtliche und alle dieser Nukleotidvariationen und daraus resultierende Aminosäurepolymorphismen in der Acyl-CoA:Lysophospholipid-Acyltransferase, die das Ergebnis natürlicher Variation sind und die funktionelle Aktivität von Acyl-CoA:Lysophospholipid-Acyltransferasen nicht verändern, sollen im Umfang der Erfindung enthalten sein.

Für das erfindungsgemäße Verfahren vorteilhafte NUkleinsäuremoleküle wie in Anspruch 1 beschreiben können auf der Grundlage ihrer Homologie zu den hier offenbarten Acyl-CoA:Lysophospholipid-Acyttransferase-Nukleinsäuren unter Verwendung der Sequenzen oder eines Teils davon als Hybridisierungssonde gemäß Standard-Hybridisierungstechniken unter stringenten Hybridisierungsbedingungen isoliert werden. Dabei können beispielsweise isolierte Nukleinsäuremoleküle verwendet werden, die mindestens 15 Nukleotide lang sind und unter stringenten Bedingungen mit dem Nukleinsäutemolekülen, die eine Nukteotidsequenz der SEQ ID NO:1, SEQ ID NO: 3, SEQ ID NO: 5 oder SEQ ID NO: 7 umfassen, hybridisieren. Es können auch Nukleinsäuren mindestens 25, 50, 100, 250 oder mehr Nukleotide verwendet werden. Der Begriff hybridisiert unter stringenten Bedingungen", wie hier verwendet, soll Hybridisierungs- und Waschbedingungen beschreiben, unter denen Nukleotidsequenzen, die mindestens 60 % homolog zueinander sind, gewöhnlich aneinander hybridisiert bleiben. Die Bedingungen sind vorzugsweise derart, dass Sequenzen, die mindestens etwa 65 %, stärker bevorzugt mindestens etwa 70 % und noch stärker bevorzugt mindestens etwa 75 % oder stärker zueinander homolog sind, gewöhnlich aneinander hybridisiert bleiben. Diese stringenten Bedingungen sind dem Fachmann bekannt und lassen sich in Current Protocols in Molecular Biology, John Wiley & Sons, N. Y. (1989); 6.3.1-6.3.6., finden. Ein bevorzugtes, nicht einschränkendes Beispiel für stringente Hybridisierungsbedingungen sind Hybridisierungen in 6 x Natriumchlorid/Natriumcitrat (sodium chloride/sodiumcitrate = SSC) bei etwa 45°C, gefolgt von einem oder mehreren Waschschritten in 0,2 x SSC, 0,1 % SDS bei 50 bis 65°C. Dem Fachmann ist bekannt, dass diese Hybridisierungsbedingungen sich je nach dem Typ der Nukleinsäure und, wenn beispielsweise organische Lösungsmittel vorliegen, hinsichtlich der Temperatur und der Konzentration des Puffers unterscheiden. Die Temperatur unterscheidet sich beispielsweise unter "Standard-Hybridisierungsbedingungen" je nach dem Typ der Nukleinsäure zwischen 42°C und 58°C in wässrigem Puffer mit einer Konzentration von 0,1 bis 5 x SSC (pH 7,2). Falls organisches Lösungsmittel im obengenannten Puffer vorliegt, zum Beispiel 50 % Formamid, ist die Temperatur unter Standardbedingungen etwa 42°C. Vorzugsweise sind die Hybridisierungsbedingungen für DNA:DNA-Hybride zum Beispiel 0,1 x SSC und 20°C bis 45°C, vorzugsweise zwischen 30°C und 45°C. Vorzugsweise sind die Hybridisierungsbedingungen für DNA:RNA-Hybride zum Beispiel 0,1 x SSC und 30°C bis 55°C, vorzugsweise zwischen 45°C und 55°C. Die vorstehend genannten Hybridisierungstemperaturen sind beispielsweise für eine Nukleinsäure mit etwa 100 bp (= Basenpaare) Länge und einem G + Gehalt von 50 % in Abwesenheit von Formamid bestimmt Der Fachmann weiß, wie die erforderlichen Hybridisierungsbedingungen anhand von Lehrbüchern, wie dem vorstehend erwähnten oder aus den folgenden Lehrbüchern Sambrook et al., "Molecular Cloning", Cold Spring Harbor Laboratory, 1989; Hames und Higgins (Hrsgb.) 1985, "Nucleic Acids Hybridization: A Practical Approach", IRL Press at Oxford University Press, Oxford; Brown (Hrsgb.) 1991, "Essential Molecular Biology: A Practical Approach", IRL Press at Oxford University Press, Oxford, bestimmt werden können.

Zur Bestimmung der prozentualen Homologie (= Identität) von zwei Aminosäuresequenzen (z.B. einer der Sequenzen der SEQ ID NO: 2, SEQ ID NO: 4, SEQ ID NO: 6 oder SEQ ID NO: 8) oder von zwei Nukleinsäuren (z.B. SEQ ID NO:1, SEQ ID NO: 3, SEQ ID NO: 5 oder SEQ ID NO: 7) werden die Sequenzen zum Zweck des optimalen Vergleichs untereinander geschrieben (z.B. können Lücken in die Sequenz eines Proteins oder einer Nukleinsäure eingefügt werden, um ein optimales Alignment mit dem anderen Protein oder der anderen Nukleinsäure zu erzeugen). Die Aminosäureresten oder Nucleotide an den entsprechenden Aminosäurepositionen oder Nukleotidpositionen werden dann verglichen. Wenn eine Position in einer Sequenz durch den gleichen Aminosäurerest oder das gleiche Nukleotid wie die entsprechende Stelle in der anderen Sequenz belegt wird, dann sind die Moleküle an dieser Position homolog (d.h. Aminosäure- oder Nukteinsäure-"Homologie", wie hier verwendet, entspricht Aminosäure- oder Nukleinsäure-"Identität"). Die prozentuale Homologie zwischen den beiden Sequenzen ist eine Funktion der Anzahl an identischen Positionen, die den Sequenzen gemeinsam sind (d.h. % Homologie = Anzahl der identischen Positionen/Gesamtanzahl der Positionen x 100). Die Begriffe Homologie und Identität sind damit als Synonym anzusehen. Die verwendeten Programme bzw. Algorithmen sind oben beschrieben.

Ein isoliertes Nukleinsäuremolekül, das eine Acyl-CoA:Lysophospholipid-Acyltransferase kodiert, die zu einer Proteinsequenz der SEQ ID NO: 2, SEQ ID NO:4, SEQ ID-NO: 6 oder SEQ ID NO: 8 wie in Anspruch 1 beschrieben homolog ist, kann durch Einbringen einer oder mehrerer Nukieotidsubstitutionen, -additionen oder -deletionen in eine Nukleotidsequenz der SEQ ID NO:1, SEQ ID NO: 3, SEQ ID NO: 5 oder SEQ ID NO: 7 erzeugt werden, so dass eine oder mehrere Aminosäuresubstitutionen, -additionen oder -deletionen in das kodierte Protein eingebracht werden. Mutationen können in eine der Sequenzen der SEQ ID NO: 1, SEQ ID NO: 3, SEQ ID NO: 5 oder SEQ ID NO: 7 durch Standardtechniken, wie stellenspezifische Mutagenese und PCR-vermittette Mutagenese, eingebracht werden. Vorzugsweise werden konservative Aminosäuresubstitutionen an einer oder mehreren der vorhergesagten nicht-essentiellen Aminosäureresten hergestelk. Bei einer "konservativen Aminosäuresubstitution" wird der Aminosäurerest gegen einen Aminosäurerest mit einer ähnlichen Seitenkette ausgetauscht Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (zB. Glycin, Asparagin, Glutamin, Serin, Threonin, Tyrosin, Cystein), unpolaren Seitenketten, (z.B. Alanin, Valin, Leucin, Isoleucin, Prolin, Phenylalanin, Methionin, Tryptophan), beta-verzweigten Seitenketten (z.B. Threonin, Valin, Isoleucin) und aromatischen Seitenketten (zB. Tyrosin, Phenylalanin, Tryptophan, Histidin). Ein vorhergesagter nicht-essentieller Aminosäurerest in einer Acyl-CoA:Lysophospholipid-Acyltransferase.wird somit vorzugsweise durch einen anderen Aminosäurerest aus der gleichen Seitenkettenfamilie ausgetauscht Alternativ können bei einer anderen Ausführungsform die Mutationen zufallsgemäß über die gesamte oder einen Teil der Acyl-CoA:Lysophospholipid-Acyltransferase-kodierenden Sequenz eingebracht werden, z.B. durch Sättigungsmutagenese, und die resultierenden Mutanten können nach der hier beschriebenen Acyl-CoA:Lysophospholipid-Acyttransferase-Aktivität durchmustert werden, um Mutanten zu identifizieren, die die Acyl-COA:Lysophöspholipid-Acyltransferase-Aktivität beibehalten haben. Nach der Mutagenese einer der Sequenzen der SEQ ID NO: 1, SEQ ID NO: 3, SEQ ID NO: 5 oder SEQ ID NO: 7 kann das kodierte Protein rekombinant exprimiert werden, und die Aktivität des Proteins kann z.B. unter Verwendung der hier beschriebenen Tests bestimmt werden. Diese Erfindung wird durch die nachstehenden Beispiele weiter veranschauücht, die nicht als beschränkend aufgefasst werden sollten.

### Beispiel

### Beispiel 1: Allgemeine Verfahren

### a) Allgemeine Klonierungsverfahren:

klonienrungsverfahren, wie beispielsweise Restriktionsspaltungen, Agarosegelelektrophorese, Reinigung von DNA-Fragmenten, Transfer von Nukleinsäuren auf Nitrocellulose- und Nylonmembranen, Verbindung von DNA-Fragmenten, Transformation von Escherichia coli- und Hefe-Zellen, Anzucht von Bakterien und Sequenzanalyse rekombinanter DNA, wurden durchgeführt wie beschrieben in Sambrook et al. (1989) (Cold Spring Harbor Laboratory Press: ISBN 0-87969-309-6) oder Kaiser, Michaelis und Mitchell (1994) "Methods in Yeast Genetics" (Cold Spring Harbor Laboratory Press: ISBN 0-87989-451-3).

### b) Chemikalien

Die verwendeten Chemikalien wurden, wenn im Text nicht anders angegeben, in p. A.-Qualität von den Firmen Fluka (Neu-Ulm), Merck (darmstadt); Roth (Karisruhe), Serva (Heidelberg) und Sigma (Deisenhofen) bezogen. Lösungen wurden unter Verwendung von reinem pyrogenfreiem Wasser, im nachstehenden Text als H₂O bezeichnet, aus einer Milli-Q-Wassersystem-Wasserreinigungsanlage (millipore, Eschbom) hergestellt. Restriktionsendonukleasen, DNA-modifizierende Enzyme und molekutartiologische Kits wurden bezogen von den Firmen AGS (Heidelberg), Amersham (Braunschweig), Biometra (Göttingen), Boehringer (Mannheim), Genomed (Bad Oeynhausen), New England Biolabs (Schwalbach/Taunus), Novagen (Madison, Wisconsin, USA), Perkin-Elmer (Weiterstadt), Pharmacia (Freiburg), Qiagen (Hilden) und Stratagene (Amsterdam, Niederlande). Wenn nicht anders angegeben, wurden sie nach den Anweisungen des Herstellers verwendet

### c) Klonierung und Expression von Desaturasen und Elongasen

Der Escherichia coli-Stamm XL1 Blue MRF' kan (Stratagene) wurde zur Subklonierung der Δ-6-Desaturase aus Physcomitrella patens verwendet. Für die funktionelle Expression dieses Gens wurde der Saccharomyces cerevisiae-Stamm INVSc 1 (Invitrogen Co.) verwendet. E. coli wurde in Luria-Bertani-Brühe (LB, Duchefa, Haarlem, Niederlande) bei 37°C kultiviert. Wenn nötig, wurde Ampicillin (100 mg/Liter) zugegeben, und 1,5,% Agar (Gew./Vol.) wurde für feste LB-Medien hinzugefügt. S. cerevisiae wurde bei 30°C entweder in YPG-Medium oder in komplettem Minimalmedium ohne Uracil (CMdum; siehe in: Ausubel, F.M., Brent, R., Kingston, R.E, Moore, D.D., Seidman, J.G., Smith, J.A., Struhl, K., Albright, L.B., Coen, D.M., und Varki, A. (1995) Current Protocols in Molecular Biology, John Wiley & Sons, New York) mit entweder 2 % (Gew./Vol.) Raffinose oder Glucose kultiviert. Für feste Medien wurden 2 % (Gew./Vol.) Bacto™-Agar (Difco) hinzugefügt Die zur Klonierung und Expression verwendeten Plasmide sind pUC18 (Pharmacia) und pYES2 (Invitrogen Co.).

### d) klonierung und Expression PUFA spezifischer Desaturasen und Elongaen

Für die Expression in Pflanzen wurden cDNA Klone aus SEQ ID NO: 9,11 oder 13 so modifiziert, dass lediglich die Codierregion mittels Polymerase Kettenreaktion unter Zuhilfenahme zweier Oligonukleotide amplifiziert werden. Dabei wurde darauf geachtet, dass eine Konsensusequenz vor dem Startcodon zur effizienten Translation eingehalten wurde. Entweder wurde hierzu die Basenfolge ATA oder AAA gewählt und vor das ATG in die Sequenz eingefügt [Kozak, M. (1986) Point mutations define a sequence flanking the AUG initiator codon that modulates translation by eukaryotic ribosomes, Cell 44, 283-2929]. Vor diesem Konsensustriplett wurde zusätzlich eine Restriktionsschnittstelle eingeführt, die kompatibel sein muss zur Schnittstelle des Zielvektors, in den das Fragment kloniert werden soll und mit dessen Hilfe die Genexpression in Mikroorganismen oder Pflanzen erfolgen soll.

Die PCR-Reaktion wurde mit Plasmid-DNA als Matrize in einem Thermocycler (Biometra) mit der Pfu-DNA-(Stratagene)Polymerase und dem folgenden Temperaturprogramm durchgeführt: 3 min bei 96°C, gefolgt von 30 Zyklen mit 30 s bei 96°C, 30 s bei 55°C und 2 min bei 72°C, 1 Zyklus mit 10 min bei 72°C und Stop bei 4°C. Die Anlagerungstemperatur wurde je nach gewählten Oligonukleotiden variiert Pro Kilobasenpaare DNA ist von einer Synthesezeit von etwa einer Minute auszugehen. Weitere Parameter, die Einfluss auf die PCR haben wie z.B. Mg-Ionen, Salz, DNA Polymerase etc., sind dem Fachmann auf dem Gebiet geläufig und können nach Bedarf variiert werden.

Die korrekte Größe des amplifizierten DNA-Fragments wurde mittels Agarose-TBE-Geletektrophorese bestätigt. Die amplifizierte DNA wurde aus dem Gel mit dem QIAquick-Gelextraktionskit (QIAGEN) extrahiert und in die Smal-Restriktionsstelle des dephosphorylierten Vektors pUC18 unter Verwendung des Sure Clone Ligations Kit (Pharmacia) ligiert, wobei die pUC-Derivate erhalten wurden. Nach der Transformation von E. coli XL1 Blue MRF' kan wurde eine DNA-Minipräparation [Riggs, M.G., & McLachlan, A. (1986) A simplified screening procedure for large numbers of plasmid mini-preparation. BioTechnicjues 4,310-313] an ampicillinresistenten Transformanden durchgeführt, und positive Klone mittels BamHI-Restriktionsanalyse identifiziert. Die Sequenz des klonierten PCR-Produktes wurde mittels Resequenzierung unter Verwendung des ABI PRISM Big Dye Terminator Cycle Sequencing Ready Reaction Kit (Perkin-Elmer, Weiterstadt) bestätigt.

### e) Transformation von Agrobacterium

Die Agrobacterium-vermittelte Pflanzentransformation wurde, wenn nicht anders beschrieben, wie von Deblaere et al. (1984, Nucl. Acids Res. 13, 4777-4788) mit Hilfe eines Agrobacterium tumefaciens-Stamms durchgeführt.

### f) Pflanzentransformation

Die Agrobacterium-vermittette Pflanzentransformation wurde, wenn nicht anders beschrieben, unter Verwendung von Standard-Transformations- und Regenerationstechniken durchgeführt (Gelvin, Stanton B., Schilperoort, Robert A., Plant Molecular Biology Manual, 2. Aufl., Dordrecht Kluwer Academic Publ., 1995, in Sect., Ringbuc Zentrale Signatur: BT11-P ISBN 0-7923-2731-4; Glick, Bemard R., Thompson, John E., Methods in Plant Molecular Biology and Biotechnology, Boca Raton: CRC Press, 1993, 360 S., ISBN 0-8493-5164-2).

Nach diesen kann beispielsweise Raps mittels Kotyledonen- oder Hypokotyltransformation transformiert werden (Moloney et al., Plant Cell 8 (1989) 238-242; De Block et al., Plant Physiol. 91 (1989) 694-701). Die Verwendung von Antibiotika für die Agrobacterium- und Pflanzenselektion hängt von dem für die Transformation verwendeten binären Vektor und Agrobacterium-Stamm ab. Die Rapsselektion wird dabei gewöhnlich unter Verwendung von Kanamycin als selektierbarem Pflanzenmarker durchgeführt.

Die Transformation von Soja kann unter Verwendung von beispielsweise einer in EP-A-0 0424 047 (Pioneer Hi-Bred International) oder in EP-A-0 0397 687, US 5,376,543, US 5,169,770 (University Toledo) beschriebenen Technik durchgeführt werden.

Die Pflanzentransformation unter Verwendung von Teilchenbeschuss, Polyethylenglycol-vermittelter DNA-Aufnahme oder über die Siliziumcarbonatfaser-Technik ist beispielsweise beschrieben von Freeling und Walbot "The maize handbook" (1993) ISBN 3-540-97826-7, Springer Verlag New York).

Der Agrobacterium-vermittelte Gentransfer in Lein (Linum usitatissimum) wurde, wenn nicht anders beschrieben, wie bei Miynarova et al. [(1994) Plant Cell Report 13:282-285] beschriebenen Technik durchführt.

### g) Plasmide für die Pflanzentransformation

Zur Pflanzentransformation wurden binäre Vektoren auf Basis der Vektoren pBinAR (Höfgen und Willmitzer, Plant Science 66 (1990) 221-230) oder pGPTV (Becker et al 1992, Plant Mol. Biol. 20:1195-1197) verwendet. Die Konstruktion der binären Vektoren, die die zu exprimierenden Nukleinsäuren enthalten, erfolgt durch Ligation der cDNA in Sense-Orientierung in die T-DNA erfolgen. 5' der cDNA aktiviert ein Pflanzenpromotor die Transkription der cDNA. Eine Polyadenylierungssequenz befindet sich 3' von der cDNA. Die binären Vektoren können unterschiedliche Markergene tragen wie beispielsweise das Acetolactat Synthasegens (AHAS oder ALS) [Ott et al., J. Mol. Biol. 1996, 263:359-360], das eine Resistenz gegen die imidazolinone vermittelt oder das nptil-Markergen, das für eine Kanamycin-Resistenz vermittelt durch Neomycinphosphotransferase codiert.

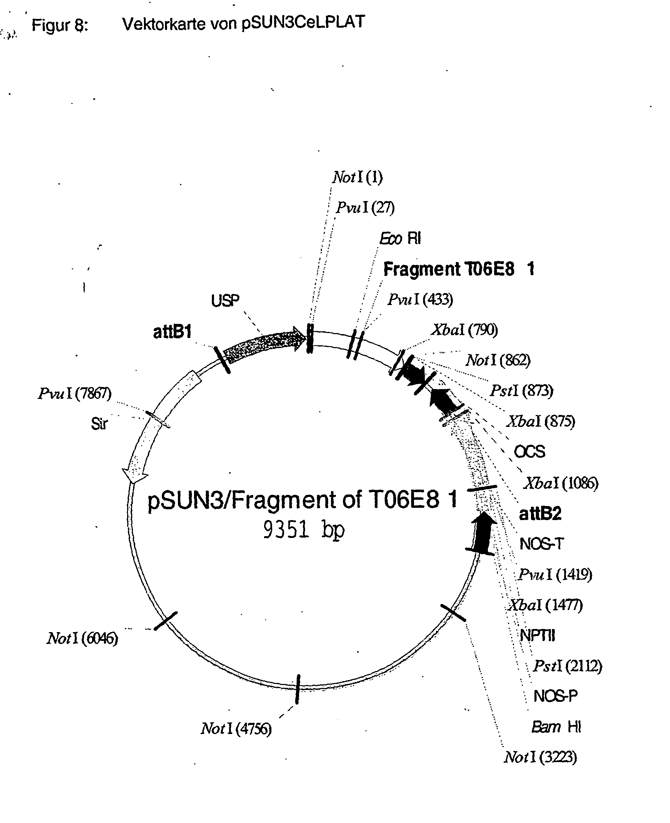

Die gewebespezifische Expression der Nukleinsäuren lässt sich unter Verwendung eines gewebespezifischen Promotors erzielen. Wenn nicht anders beschrieben wurde der LeB4- oder der USP-Promotor oder der Phaseolin-Promotor 5' der cDNA einkloniert wird. Als Terminatoren wurde der NOS-Terminator und der OCS-Terminator verwendet (siehe Figur 8). Figur 8 zeigt eine Vektorkarte des zur Expression verwendeten Vektor pSUN3CeLPLAT.

Auch jedes andere samenspezifische Promotorelement wie z.B. der Napin- oder Arcelin Promotor Goossens et al. 1999, Plant Phys. 120(4):1095-1103 und Gerhardt et al. 2000, Biochimica et Biophysica Acta 1490(1-2):87-98) kann verwendet werden.

Zur konstitutiven Expression in der ganzen Pflanzen lässt sich der CaMV-35S-Promotor oder ein v-ATPase C1 Promotor verwenden.

Die im Verfahren verwendeten Nukleinsäuren/die für die Acyl-CoA:Lysophospholipid-Acyltransferasen; Desaturasen oder Elongasen codieren, wurden durch Konstruktion mehrerer Expressionskassetten hintereinander in einen binären Vektor kloniert, um den Stoftwechselweg in Pflanzen nachzubilden.

Innerhalb einer Expressionskassette kann das zu exprimierende Protein unter Verwendung eines Signalpeptids, beispielsweise für Plastiden, Mitochondrien oder das Endoplasmatische Retikulum, in ein zelluläres Kompartiment dirigiert werden (Kermode, Crit. ReV. Plant Sci. 15,4 (1996) 285-423). Das Signalpeptid wird 5' im Leseraster mit der cDNA einkloniert, um die subzelluläre Lokalisierung des Fusionsprotein zu erreichen.

Beispiele für Multiexpressionskassetten wurden in DE 10219 203 offenbart und sind im folgenden nochmals wiedergegeben.

### i.) Promotor-Terminator-Kassetten

Expressionskassetten bestehen aus wenigstens zwei funktiönellen Einheiten wie einem Promotor und einem Terminator. Zwischen Promotor und Terminator können weitere gewünschte Gensequenzen wie Targetting-Sequenzen, Codierregionen von Genen oder Teilen davon etc. eingefügt werden. Zum Aufbau der Expressionskassetten wurden Promotoren und Terminatoren (USP Promotor: Baeumlein et al., Mol Gen Genet, 1991, 225 (3):459-67); OCS Terminator. Gielen et al. EMBO J. 3 (1984) 835ff.) mithilfe der Potymerasekettenreaktion isoliert und mit flankierenden Sequenzen nach Wahl auf Basis von synthetischen Oligonukleotiden maßgeschneidert.

Folgende Oligonukleotide können beispielsweise verwendet werden:
USP1 vorne:
   CCGGAATTCGGCGCGCCGAGCTCCTCGAGCAAATTTACACATTGCCA -
USP2 vorne:
   - CCGGAATTCGGCGCGCCGAGCTCCTCGAGCAAATTTACACATTGCCA -
USP3 vorne:
   - CCGGAATTCGGCGCGCCGAGCTCCTCGAGCAAATTTACACATTGCCA -
USP1 hinten:
   - AAAACTGCAGGCGGCCGCCCACCGCGGTGGGCTGGCTATGAAGAAATT -
USP2 hinten:
   - CGCGGATCCGCTGGCTATGAAGAAATT -
USP3 hinten:
   - TCCCCCGGGATCGATGCCGGCAGATCTGCTGGCTATGAAGAAATT -
OCS1. vorne:
   - AAAACTGCAGTCTAGAAGGCCTCCTGCTTTAATGAGATAT -
OCS2 vorne:
   - CGCGGATCCGATATCGGGCCCGCTAGCGTTAACCCTGCTTTAATGAGATAT -
OCS3 vorne:
   - TCCCCCGGGCCATGGCCTGCTTTAATGAGATAT -
OCS1 hinten:
   - CCCAAGCTTGGCGCGCCGAGCTCGAATTCGTCGACGGACAATCAGTAAATTGA -
OCS2 hinten:
   - CCCAAGCTTGGCGCGCCGAGCTCGAATTCGTCGACGGACAATCAGTAAATTGA -
OCS3 hinten:
   -CCCAAGCTTGGCGCGCCGAGCTCGTCGACGGACAATCAGTAAATTGA-

Die Methoden sind dem Fachmann auf dem Gebiet bekannt und sind allgemein literaturbekannt

In einem ersten Schritt wurden ein Promotor und ein Terminator über PCR amplifiziert. Dann wurde der Terminator in ein Empfängerplasmid kloniert und in einem zweiten Schritt der Promotor vor den Terminator inseriert. Dadurch wurde eine Expressionskassette in das Basis-Plasmid cloniert. Auf Basis des Plamides pUC19 wurden so die Plasmide pUT1, 2 und 3 erstellt

Die entsprechenden Konstrukt bzw. Plasmide sind in SEQ ID NO: 15, 16 bis 17 definiert. Sie enthalten den USP-Promotor und den OCS Terminator. Auf Basis dieser Plasmide wurde das Konstrukt pUT12 erstellt, indem pUT1 mittels SalI/ScaI geschnitten wurde und pUT2 mittels XhoI/ScaI geschnitten wurde. Die die Expressionskassetten enthaltenden Fragmente wurden ligiert und in E. coli XL1 blue MRF transformiert. Es wurde nach Vereinzelung von ampicillinresistenten Kolonien DNA präpariert und per Restriktionsanalyse solche Klone identifiziert, die zwei Expressionskassetten enthalten. Die XhoI/SalI Ligation kompatibler Enden hat dabei die beiden Schnittstelien XhoI und SalI zwischen den Expressionskassetten eleminiert. Das resultierende Plasmid pUT12 wird in SEQ ID NO: 18 wiedergegeben. Anschließend wurde pUT12 wiederum mittels SaI/ScaI geschnitten und pUT3 mittels XhoI/ScaI geschnitten. Die die Expressionskassetten enthaltenden Fragmente wurden ligiert und in E. coli XLI blue MRF transformiert. Es wurde wieder nach Vereinzelung aus ampicillinresistenten Kolonien DNA präpariert und per Restriktionsanalyse solche Klone identifiziert, die drei Expressionskassetten enthalten. Auf diese Weise wurde ein Set von Multiexpressionskassetten geschaffen, dass für die Insertion gewünschter DNA genutzt werden kann und in Tabelle 1 beschrieben wird und zudem noch weitere Expressionskassetten aufnehmen kann.

Diese enthalten folgende Elemente:

**Tabelle 1**

| PUC19-Derivat | Schnittstellen vor dem USP Promotor | Multiple Klonierwtgs-Schniüstellen | Schnittstellen hinter dem OCS-Terminator |
|---|---|---|---|
| PUT1 | EcoRI/AscI/ SacI/XhoI | BstXI/NotI/PstI/XbaI/StuI | SalI/EcoRI/SacI/AscI/ HindIII |
| PUT2 | EcoRI/AscI/SacI/XhoI | BamHI/EcoRV/ ApaI/NheI/HpaI HinIII | SalI/EcoRI/SacI/AscI/ |
| PUT3 | EcoRI/AscI/SacI/XhoI | Bg1II/NaeI/ClaI/Smal/NcoI | San/SacI/AscI/HindIII |
| PUT12 Doppel-expressions-kasette | EcoRI/AscI/SacI/XhoI | BstXI/NotI/PstI/XbaI/StnI Und BamHI/EcoRV/ApaI/NheI/HpaI | SalI/EcoRI/SacI/AscI/ HindIII |
| PUT123 Tripel-expressions-kassette | EcoRI/AscI/SacI/XhoI | 1.BstXI/NotI/PstI/XbaI/StuI und | SalI/SacI/AscI/HindIII |
| | | 2. BamHI/EcoRV/ ApaI/NheI/HpaI und | |
| | | 3. BgIII/NaeI/ClaI/SmaI/NcoI | |

Weiterhin lassen sich wie beschrieben und wie in Tabelle 2 näher spezifiziert weitere Multiexpressionskassetten mithilfe des
i) USP-Promotors oder-mithilfe des
ii) 700 Basenpaare 3'-Fragmentes des LeB4-Promotors oder mithilfe des
iii) DC3-Promotors erzeugen und für samenspezifische Genexpression einsetzen.

Der DC3-Promotor ist beschrieben bei Thomas, Plant Cell 1996, 263:359-368 und besteht lediglich aus der Region -117 bis +26 weshalb er mithin einer der kleinsten bekannten samenspezifischen Promotoren darstellt Die Expressionskassetten können mehrfach den selbeh Promotor enthalten oder aber über drei verschiedene Promotoren aufgebaut werden.

Vorteilhaft verwendete Polylinker- bzw. Polylinker-Terminator-Polylinker sind den Sequenzen SEQ ID NO: 23 bis 25 zu entnehmen.

**Tabelle 2: Multiple Expressionskassetten**

| Plasmidname des pUC19-Derivates | Schnittstellen vor dem jeweiligen Promotor | . Multiple Klonierungs-Schnittstellen | Schnittstellen hinter dem OCS-Terminator |
|---|---|---|---|
| pUT1 (pUC19 mit USP-OCS1) | EcoRI/AscI/SacI/XhoI | (1) BstXI/NotI/PstI/XbaI/StnI | SalI/EcoRI/SacI/AscI/ HindIII |
| PDCT (pUC19 mit DC3-OCS) | EcoRI/AscI/SacI/XhoI | (2) BamHI/EcoRI/ApaI/NheI HpaI | SalI/EcoRI/SacI/AscI/ HindIII |
| PleBT (pUC19-mit LeB4(700)-OCS) | EcoRI/AscI/SacI/XhoI | (3) BglII/NaeI/ClaI/SmaI/NcoI | SalI/SacI/AscI/HindIII |
| PUD12 (pUC19 mit-mit USP-OCS1 und mit DC3-OCS) | EcoRI/AscI/SacI/XhoI | (1) BstXI/NotI/PstI/XbaI/StaI und | SalI/EcoRI/SacI/AscI/ HindIII |
| | | (2) BamHI/EcoRVI/ApaI/NheI/ HpaI | |
| PUDL123 Triple expression cassette (pUC19 mit USP/DC3 und LeB4-700) | EcoRI/AscI/SacI/XhoI | (1) BstXI/NotI/PstI/XbaI/StuI und | SalI/SacI/AscI/HindIII |
| | | (2) BamHI/ (EcoRV*)/ApaI/NheI/ HpaI und | |
| | | (3) BglII/NaeI/ClaI/SmaI/NcoI | |

| | | | |
|---|---|---|---|
| * EcoRV Schnittstelle schneidet im 700 Basenpaarfragment des LeB4 Promotors (LeB4-700) | | | |

Analog lassen sich weitere Promotoren für Muttigenkonstrukte erzeugen insbesondere unter Verwendung des
a) 2,7 kB Fragmentes des LeB4-Promotors oder mithilfe des
b) Phaseolin-Promotors oder mithilfe des
c) konstitutiven v-ATPase c1-Promotors.

Es kann insbesondere wünschenswert sein, weitere besonders geeignete Promotoren zum Aufbau samenspezfischer Multiexpressionskassetten wie z.B. den Napin-Promotor oder den Arcelin-5 Promotor zu verwenden.

Weitere in Pflanzen nutzbare Vektoren mit einer bzw. zwei oder drei Promotor-Terminator-Expressionkassetten sind den Sequenzen SEQ ID NO: 26 bis SEQ ID NO: 31 zu entnehmen.

### ii.) Erstellung von Expressionskonstrukten, die Promotor, Terminator und gewünschte Gensequenz zur PUFA Genexpression in pflanzlichen Expressionskassetten enthalten.

In pUT123 wird zunächst über BstXI und XbaI die Δ-6-Elongase Pp_PSE1 in die erste Kassette inseriert. Dann wird die Δ-6-Desaturase aus Moos (Pp_des6) über Bam-HI/Nael in die zweite Kassette inseriert und schließlich die Δ-5-Desaturase aus Phaeodactylum (Pt_des5) über BgIII/NcoI in die dritte Kassette inseriert (siehe SEQ ID NO: 19). Das Dreifachkonstrukt erhält den Namen pARA1. Unter Berücksichtigung sequenzspezifischer Restriktionsschnittstellen können weitere Expressionskassetten gemäß Tabelle 3 mit der Bezeichnung pARA2, pARA3 und pARA4 erstellt werden.

**Tabelle 3: Kombinationen von Desaturasen und Elongasen**

| Gen Plasmid | Δ-6-Desaturase | Δ-5-Desaturase | Δ-6-Elongase |
|---|---|---|---|
| pARA1 | Pp_des6 | Pt_des5 | Pp_PSE1 |
| pARA2 | Pt_des6 | Pt_des5 | Pp_PSE1 |
| pARA3 | Pt_des6 | Ce_des5 | Pp_PSE1 |
| PARA4 | Ce_des6 | Ce_des5 | Ce_PSE1 |

des5 = PUFA spezifisch Δ-5-Desaturase
des6 = PUFA spezifische Δ-6-Desaturase
PSE = PUFA spezifische Δ-6-Elongase
Pt_des5 = Δ-5-Desaturase aus Phaeodactylum tricomutum
Pp_des6 oder Pt_des6 = Δ-6-Desaturase aus Physcomitrella patens bzw. Phaeodactylum tricomutum
Pp = Physcomitrella patens, Pt = Phaeodactylum tricomutum
Pp_PSE1 = Δ-6-Elongase aus Physcomitrella patens
Pt_PSE1 = Δ-6-Elongase aus Phaeodactylum tricomutum
Ce_des5 = Δ-5-Desaturase aus Caenorhabditis elegans (Genbank Acc. Nr. AF078796) Ce_des6 = Δ-6-Desaturase aus Caenorhabditis elegans (Genbank Acc. Nr. AF031477, Basen 11-1342)
Ce_PSE1 = Δ-6-Elongase aus Caenorhabditis elegans (Genbank Acc. Nr. AF244356, Basen 1-867)

Auch weitere Desaturasen oder Elongasegensequenzen können in Expressionskassetten beschriebener Art inseriert werden wie z.B. Genbank Acc. Nr. AF231981, NM_013402, AF206662, AF268031, AF226273, AF110510 oder AF110509.

### iii.) Transfer von Expressionskassetten in Vektoren zur Transformation von Agrobakterium tumefaciens und zur Transformation von Pflanzen

Die so erstellten Konstrukte wurden mittels Ascl in den binären Vektor pGPTV inseriert. Die multiple Klonierungssequenz wurde zu diesem Zweck um eine Ascl Schnittstelle erweitert. Zu diesem Zweck wurde der Polylinker als zwei doppelsträngige Oligonukleoside neu synthetisiert, wobei eine zusätzliche Ascl DNA Sequenz eingefügt wird. Das Oligonukleotid wurde mittels EcoRI und HindIII in den Vektor pGPTV inseriert. Die notwendigen Kloniertechniken sind dem Fachmann bekannt und können einfach wie in Beispiel 1 beschrieben nachgelesen werden.

Für die im folgenden beschriebenen Versuche wurden als Nukleinsäuresequenzen für die Δ-5-Desaturase (SEQ ID NO: 13), die Δ-6-Desaturase (SEQ ID NO: 9) und die Δ-6-Elongase (SEQ ID NO: 11), die Sequenzen aus Physcomitrella patens und Phaedactylum tricornutum verwendet. Die entsprechenden Aminosäuresequenzen sind den Sequenzen SEQ ID NO: 10, SEQ ID NO: 12 und SEQ ID NO: 14. Ein Vektor der alle vorgenannten Gene enthält ist in SEQ ID NO: 19 wiedergegeben. Die korrespondierenden Aminosäurensequenzen der Gene sind SEQ ID NO: 20, SEQ ID NO: 21 und SEQ ID NO: 22 zu entnehmen.

### Beispiel 2: Klonierung und Charakterisierung der ceLPLATs

### a) Datenbanken-Suche

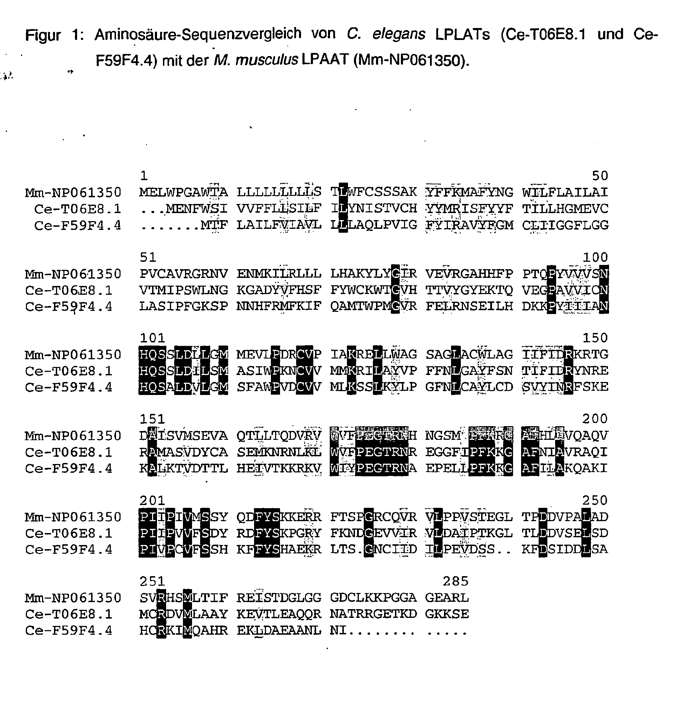

Die Identifizierung der ceLPLATs (= Acyl-CoA:Lysophosphotipid-Acyltransferase aus Caenorhabditis elegans) erfolgte durch Sequenzvergleiche mit bekannten LPA-ATs. Die Suche wurde mit Hilfe des BLAST-Psi-Algorithmus (Altschul et al., J. Mol. Biol. 1990, 215: 403-410) auf das Nematodengenom (*Caenorhabditis elegans*) beschränkt, da dieser Organismus LCPUFAs synthetisiert. Für den Sequenzvergleich diente als Sonde eine LPAAT Proteinsequenz aus *Mus musculus* (MsLPAAT Accession Nr. NP_061350). LPLAT katalysiert durch eine reversible Transferasereaktion die ATPunabhängige Synthese von Acyl-CoAs aus Phospholipiden mit Hilfe von CoA als Cofactor (Yamashita et al., J. Biol. Chem. 2001, 20: 26745-26752). Durch Sequenzvergleiche konnten zwei putative ceLPLAT-Sequenzen identifiziert werden (Accession Nr. *T06E8.1* bzw. *F59F4.4*). Die identifizierten Sequenzen weisen die größte Ähnlichkeit jeweils zueinander und zu MsLPAATs auf (Figur 1). Das Alignment wurde mit dem Programm Clustal erstellt.

### b) Klonierung der CeLPLATs

Auf der Basis der CeLPLAT-Nukleinsäuresequenzen wurden Primerpaare synthetisiert (Tab. 1) und mittels PCR-Verfahren die zugehörigen cDNAs aus einer *C. elegans*cDNA-Bank isoliert. Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der LPLAT-cDNAs wurde jeweils mit 2 µl cDNA-Bank-Lösung als Template, 200 µM dNTPs, 2,5 U "proof-reading" pfu-Polymerase und 50 pmol eines jeden Primers in einem Gesamtvolumen von 50 µl durchgeführt. Die Bedingungen für die PCR waren wie folgt Erste Denaturierung bei 95°C für 5 Minuten, gefolgt von 30 Zyklen bei 94°C für 30 Sekunden, 58°C für eine Minute und 72°C für 2 Minuten sowie ein letzter Verlängerungsschritt bei 72°C für 10 Minuten. Die Sequenz der LPLAT-cDNAs wurde durch DNA-Sequenzierung bestätigt.

**Tabelle 4: Nukleotidsequenzen der PCR-Primer zur Klonierung von CeLPLATs**

| Primer | Nukleotidsequenz |
|---|---|
| 5' T06E8.1f* | 5' ACATAATGGAGAACTTCTGGTCGATCGTC 3' |
| 3' T06E8.1 r* | 5' TTACTCAGATTTCTTCCCGTCTTT 3' |
| 5' F59F4.4f* | 5' ACATAATGACCTTCCTAGCCATATTA 3' . |
| 3' F59F4.4r* | 5' TCAGATATTCAAATTGGCGGCTTC 3' |

| | |
|---|---|
| * f: forward, r: reverse | |

### Beispiel 3: Analyse der Auswirkung der rekombinanten Proteine auf die Produktion des gewünschten Produktes

### a) Aufarbeitungsmöglichkeiten

Die Auswirkung der genetischen Modifikation in Pilzen, Algen, Ciliaten oder wie in den Beispielen weiter oben beschrieben in Hefen auf die Produktion der mehrfach ungesättigten Fettsäuren oder Pflanzen kann bestimmt werden, indem die modifizierten Mikroorganismen oder die modifizierten Pflanze unter geeigneten Bedingungen (wie den vorstehend beschriebenen) gezüchtet werden und das Medium und/oder die zellulären Komponenten auf die erhöhte Produktion der Lipide oder Fettsäuren untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren zum Nachweis von Fettsäuren in Hefen werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992,1X, 307 S. (Oily Press Lipid Library; 1); "Progress in Lipid Research, Oxford: Pergamon Press, 1 (1952) -16 (1977) u.d.T.: Progress in the Chemistry of Fats and Other Lipids CODEN.

So kann die Analyse von Fettsäuren oder Triacylglycerin (= TAG, Abkürzungen in Klammern angegeben) z.B. mittels Fettsäuremethylester (= FAME), Gas-Flüssigkeitschromatographie-Massenspektrometrie (= GC-MS) oder Dünnschichtchromatographie (TLC) erfolgen.

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Pflanzenmaterial kann dazu entweder durch Ultraschallbehandlung, Mahlen in der Glasmühle, flüssigen Stickstoff und Mahlen oder über andere anwendbare Verfahren aufgebrochen werden. Das Material wird dann anschließend nach dem Aufbrechen zentrifugiert. Das Sediment wird danach in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Upide ergeben. Diese Fettsäuremethylester können anschließend in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen werden. Die Identität der erhaltenen Fettsäuremethylester lassen sich unter Verwendung von Standards, die aus kommerziellen Quellen erhältlich sind (d.h. Sigma), definieren.

Bei Fettsäuren, für die keine Standards verfügbar sind, kann die Identität über Derivatisierung und anschließende GC-MS-Analyse gezeigt werden. Beispielsweise wird die Lokalisierung von Fettsäuren mit Dreifachbindung über GC-MS nach Derivatisierung mit 4,4-Dimethoxyoxazolin-Derivaten (Christie, 1998, siehe oben) gezeigt.

### b) Fettsäureanalyse in Pflanzen

Die Gesamt-Fettsäuren wurden aus Pflanzensamen extrahiert und mittels Gaschromatographie analysiert.

Die Samen wurden mit 1 % Natriummethanolat in Methoanol aufgenommen und 20 min bei RT (ca. 22 °C) inkubiert. Anschließend wurde mit NaCl Lösung gewaschen und die FAME in 0,3 ml Heptan aufgenommen.

Die Proben wurden auf einer ZEBRON-ZB-Wax Kapillarsäule (30 m, 0,32 mm, 0,25 mikro m; Phenomenex) in einem Hewlett Packard-6850-Gaschromatograph mit einem Flammenionisationsdetektor aufgetrennt Die Ofentemperatur wurde von 70°C (1 min halten) bis 200°C mit einer Rate von 20°C/min, dann auf 250°C (5 min halten) mit einer Rate von 5°C/min und schließlich auf 260°C mit einer Rate von 5°C/min programmiert. Stickstoff wurde als Trägergas verwendet (4,5 ml/min bei 70°C). Die Fettsäuren wurden durch Vergleich mit Retentionszeiten von FAME-Standards (SIGMA) identifiziert.

### Beispiel 4: Funktionelle Charakterierung der CeLPLATs in Hefe

### a) Heterologe Expression in Saccharomyces cerevisiae

Zur Charakterisierung der Funktion der CeLPLATs aus *C. elegans* wurden die offenen Leserahmen der jeweilgen cDNAs stromabwärts des Galactose-induzierbaren GAL1-Promotors von pYes2.1Topo unter Verwendung des pYes2.1TOPO TA Expression Kit (Invitrogen) kloniert, wobei pYes2-T06E8.1 und pYes2-F59F4.4 erhalten wurden.

Da die Expression der CeLPLATs zu einem effizienten Austausch der Acyl-Substrate führen sollte, wurde weiterhin das Doppelkonstrukt pESCLeu-PpD6-Pse1 hergestellt, das die offenen Leserahmen einer Δ6-Desaturase (PpD6) und einer Δ6-Elongase (PSE1) aus *Physcomitrella patens* (siehe DE 102 19 203) beinhaltet. Die Nukleinsäuresequenz der Δ6-Desaturase (PpD6) und der Δ6-Elongase (Pse1) werden jeweils in SEQ ID NO: 9 und SEQ ID NO: 11 wiedergegeben. Die korrespondierenden Aminosäuresequenzen sind SEQ ID NO:10 und SEQ ID NO: 12 zu entnehmen.

Die *Saccharomyces cerevisiae*-Stämme C13ABYS86 (Protease-defizient) und INVSc1 wurde mittels eines modifizierten PEG/Lithiumacetat-Protokolls gleichzeitig mit den Vektoren pYes2-T06E8.1 und pESCLeu-PpD6-Pse1 bzw. pYes2-F59F4.4 und pESCLeu-PpD6-Pse1 transformiert. Als Kontrolle wurde eine Hefe verwendet, die mit dem Vektor pESCLeu-PpD6-Pse1 und dem leeren Vektor pYes2 transformiert wurde. Die Selektion der transformierten Hefen-erfolgte auf Komplett-Minimalmedium (CMdum)-Agarplatten mit 2% Glucose, aber ohne Uracil und Leucin. Nach der Selektion wurden 4 Transformanten, zwei pYes2-T06E8.1/pESCLeu-PpD6-Pse1 und zwei pYes2-F59F4.4/pESCLeu-PpD6-Pse1 und eine pESCLeu-PpD6-Pse1/ pYes2 zur weiteren funktionellen Expression ausgewählt. Die beschriebenen Experimente wurden auch im Hefestamm INVSc1 durchgeführt.

Für die Expresssion der CeLPAATs wurden zunächst Vorkulturen aus jeweils 2 ml CMdum-Flüssigmedium mit 2% (w/v) Raffinose, aber ohne Uracil und Leucin mit den ausgewählten Transformanten angeimpft und 2 Tage bei 30°C, 200rpm inkubiert. 5 ml CMdum-Flüssigmedium (ohne Uracil und Leucin) mit 2% Raffinose, 1% (v/v) Tergitol NP-40 und 250 µM Linolsäure (18:2^{Δ9,12}) oder Linolensäure (18:3^{Δ9,12,15}) wurden dann mit den Vorkulturen auf eine OD₆₀₀ von 0,08 angeimpft. Die Expression wurde bei einer OD₆₀₀ von 0,2-0,4 durch die Zugabe von 2% (w/v) Galactose induziert. Die Kulturen wurden für weitere 48 h bei 20°C inkubiert.

### Fettsäureanalyse

Die Hefezellen aus den Hauptkulturen wurden durch Zentrifugation (100 x g, 10 min, 20°C) geerntet und mit 100 mM NaHCO₃, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM NaHCO₃, pH 8,0 und 2 ml Aqua dest. gewaschen. Anschließend wurden die PE-Phasen mit Na₂SO₄ getrocknet, unter Argon eingedampft und in 100 µl PE aufgenommen. Die Proben wurden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate 5°C/min und schließlich 10 min bei 250°C (halten) programmiert.

Die identifikation der Signale erfolgte durch Vergleiche der Retentionszeiten mit entsprechenden Fettsäurestandards (Sigma).

### Acyl-CoA Analyse

Die Acyl-CoA-Analyse erfolgte wie bei Larson and Graham (2001; Plant Journal 25: 115-125) beschrieben.

### Expressionsanalyse

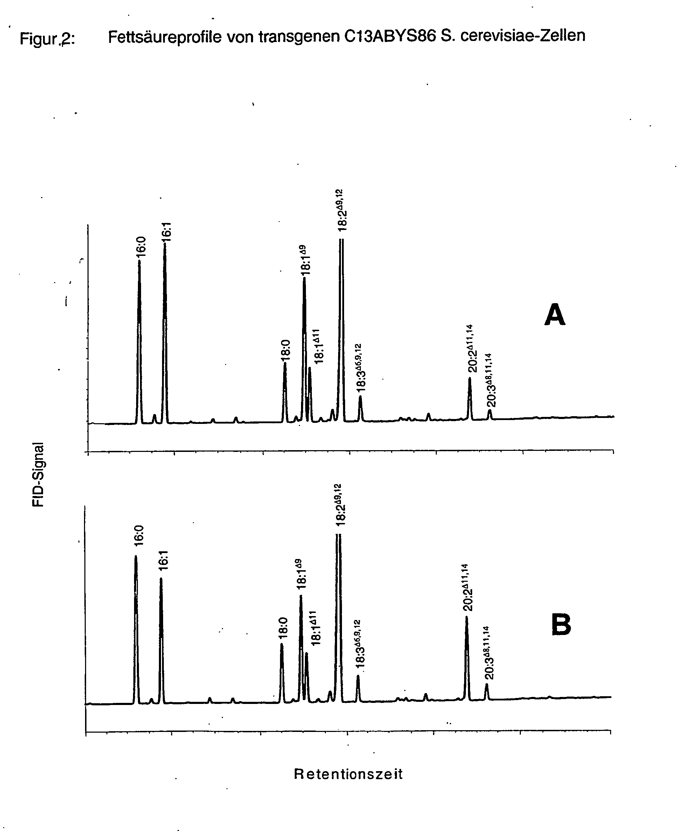

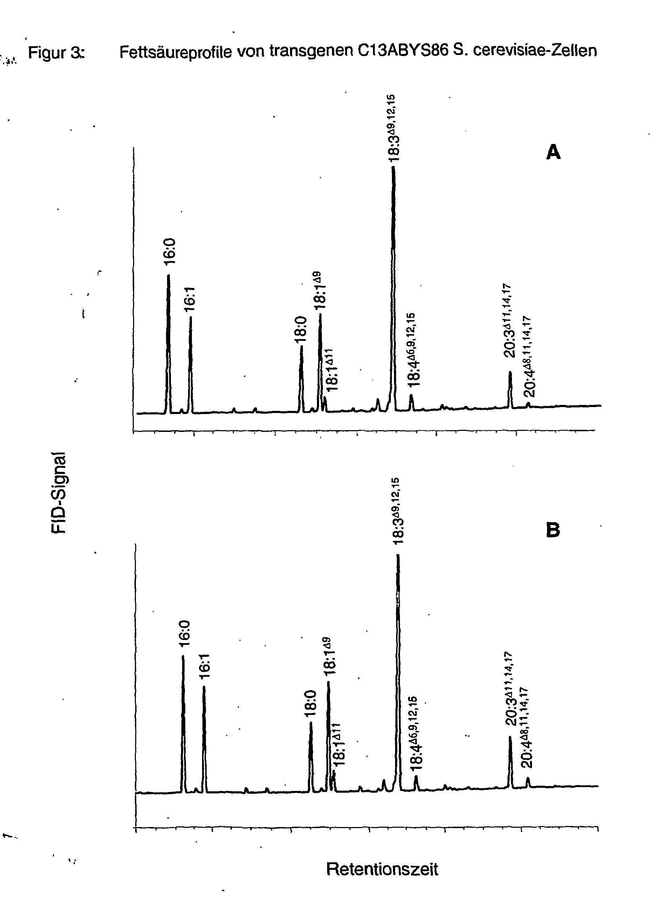

Figuren 2A und B sowie 3A und B zeigen die Feftsäureprofile von transgenen C13ABYS86 Hefen, die mit 18:2^{Δ9,12} bzw. 18:3^{Δ9,12,15} gefüttert wurden. Die gefütterten Substrate sind in großen Mengen in allen transgenen Hefen nachzuweisen. Alle vier transgenen Hefen zeigen eine Synthese von 18:3^{Δ6,9,12} und 20:3^{Δ8,11,14} bzw. 18:4^{Δ,6,9,12,15} und 20:4^{Δ8,11,14,17}, den Produkten der Δ-6- Desaturase und Δ-6-Elongase Reaktionen. Dies bedeutet, dass die Gene Pp96 und Pse1 funktional exprimiert werden konnten.

Figur 2 gibt wie oben beschrieben die Fettsäureprofile von transgenen C13ABYS86 *S. cerevisiae*-Zellen. Die Synthese der Fettsäuremethylester erfolgte durch saure Methanolyse intakter Zellen, die entweder mit den Vektoren pESCLeu-PpD6-Pse1/pYes2 (A) oder pYes2-T06E8.1/pESCLeu-PpD6-Pse1 (B) transformiert worden waren. Die Hefen wurden in Minimalmedium in Gegenwart von 18:2^{Δ9,12} kultiviert. Anschließend wurden die Fettsäuremethylester über GLC analysiert.

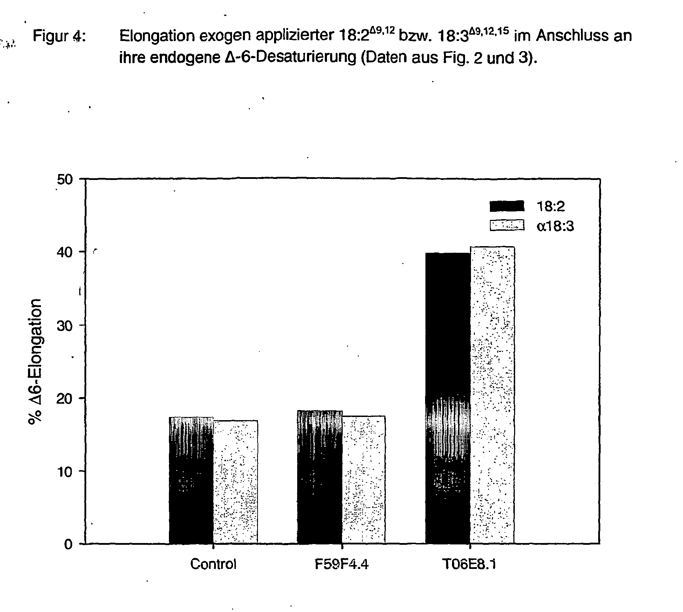

In den Kontroll-Hefen, die mit den Vektoren pESCLeu-PpD6-Pse1/pYes2 transformiert wurden, ist der Anteil von 20:3^{Δ8,11,14}, zu dem 18:3^{Δ6,9,12} durch Pse1 elongiert wird, wesentlich niedriger als in den Hefen, die zusätzlich die LPLAT T06E8.1 exprimieren. Tatsächlich konnte die Elongation von 18:3^{Δ6,9,12} und 18:4^{Δ6,9,12,15} durch die zusätzliche Expression von CeLPLAT (T06E8.1) um 100-150% verbessert werden (Figur 4). Diese signifikante Erhöhung des LCPUFA-Gehalts ist nur wie folgt zu erklären: die exogen gefütterten Fettsäuren (18:2^{Δ9,12} bzw. 18:3^{Δ9,12,15}) werden zunächst in Phospholipide eingebaut und dort von der Δ-6-Desaturase zu 18:3^{Δ6,9,12} und 18:4^{Δ6,9,12,15} desaturiert. Erst nach Reäquilibrierung mit dem Acyl-CoA-Pool können 18:3^{Δ6,9,12} und 18:4^{Δ6,9,12,15} durch die Elongase zu 20:3^{Δ8,11,14}- bzw. 20:4^{Δ8,11,14,17}-CoA elongiert und dann wieder in die Lipide eingebaut werden. Die LPLAT T06E8.1 ist in der Lage, die Δ6-desaturierten Acylgruppen sehr effizient in CoA-Thioester zurückzuverwandeln. Interessanterweise konnte auch die Elongation der gefütterten Fettsäuren 18:2^{Δ9,12} und 18:3^{Δ9,12,15} verbessert werden. (Figur 2A und B bzw. 3A und B).

Figur 3 gibt die Fettsäureprofile von transgenen C13ABYS86 *S. cerevisiae*-Zellen. Die Synthese der Fettsäuremethylester erfolgte durch saure Methanolyse intakter Zellen, die entweder mit den Vektoren pESCLeu-PpD6-Pse1/pYes2 **(A)** oder pYes2-T06E8.1/pESCLeu-PpD6-Pse1 **(B)** transformiert worden waren. Die Hefen wurden in Minimalmedium in Gegenwart von 18:3^{Δ9,12,15} kultiviert. Anschließend wurden die Fettsäuremethylester über GLC analysiert.

Die Expression einer anderen CeLPLAT (F59F4.4) hat dagegen keinen Einfluss auf die Elongation (Figur 4). Offenbar kodiert F59F4.4 nicht für eine LPLAT. Nicht jede der putativen LPLAT Nukleinsäuresequenzen ist also enzymatisch aktiv in der erfindungsgemäß gefundenen Reaktion.

Figur 4 gibt die Elongation exogen applizierter 18:2^{Δ9,12} bzw. 18:3^{Δ9,12,15} im Anschluss an ihre endogene Δ-6-Desaturierung (Daten aus Fig. 2 und 3) wieder. Die exogen gefütterten Fettsäuren werden zunächst in Phospholipide eingebaut und dort zu 18:3^{Δ6,9,12} und 18:4^{Δ6,9,12,15} desaturiert. Erst nach Reäquilibrierung mit dem Acyl-CoA-Pool können 18:3^{Δ6,9,12} und 18:4^{Δ6,9,12,15} durch die Elongase zu 20:3^{Δ8,11,14}- bzw. 20:4^{Δ8,11,14,17}-CoA elongiert und dann wieder in die Lipide eingebaut werden. Die LPLAT T06E8.1 ist in der Lage, die Δ-6-desaturierten Acylgruppen effizient in CoA-Thioester zurückzuverwandeln.

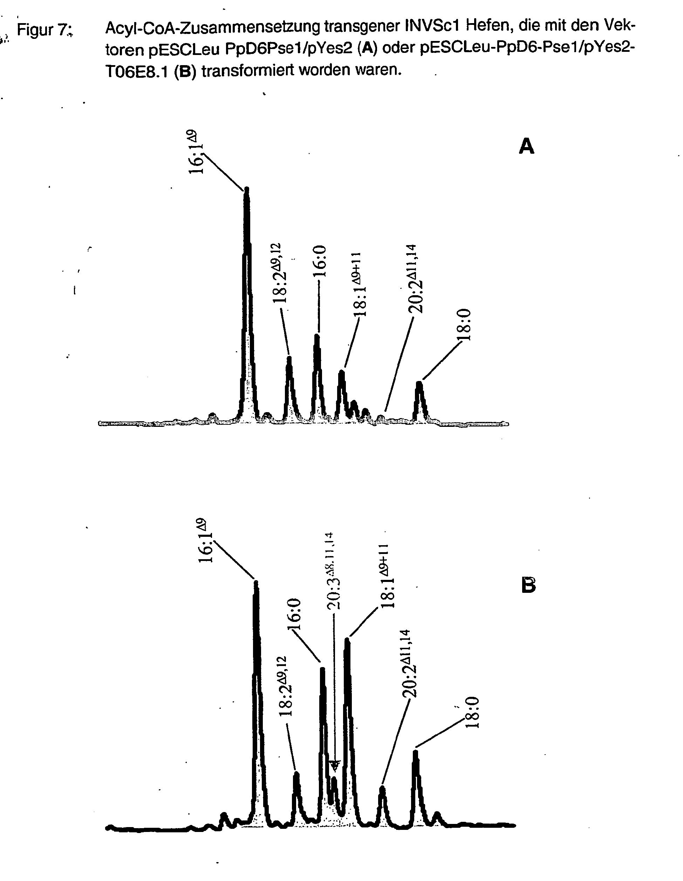

Diese Ergebnisse zeigen, dass die CeLPLAT (T06E8.1) nach Co-expression mit der Δ-6-Desaturase und Δ-6-Elongase zu einer effizienten Produktion von C20-PUFAs führt. Diese Ergebnisse sind dadurch zu erklären, dass die CeLPLAT (T06E8.1) einen effizienten Austausch der neusynthetisierten Fettsäuren zwischen Lipiden und dem Acyl-CoA-Pool ermöglicht (siehe Figur 7).

Figur 7 gibt die Acyl-CoA-Zusammensetzung transgener INVSc1 Hefen, die mit den Vektoren pESCLeu PpD6Pse1/pYes2 **(A)** oder pESCLeu-PpD6-Pse1/pYes2-T06E8.1 **(B)** transformiert worden waren, wieder. Die Hefezellen wurden in Minimalmedium ohne Uracil und Leucin in Gegenwart von 250 µM 18:2^{Δ9,12} kultiviert. Die Acyl-CoA-Derivate wurden über HPLC analysiert.

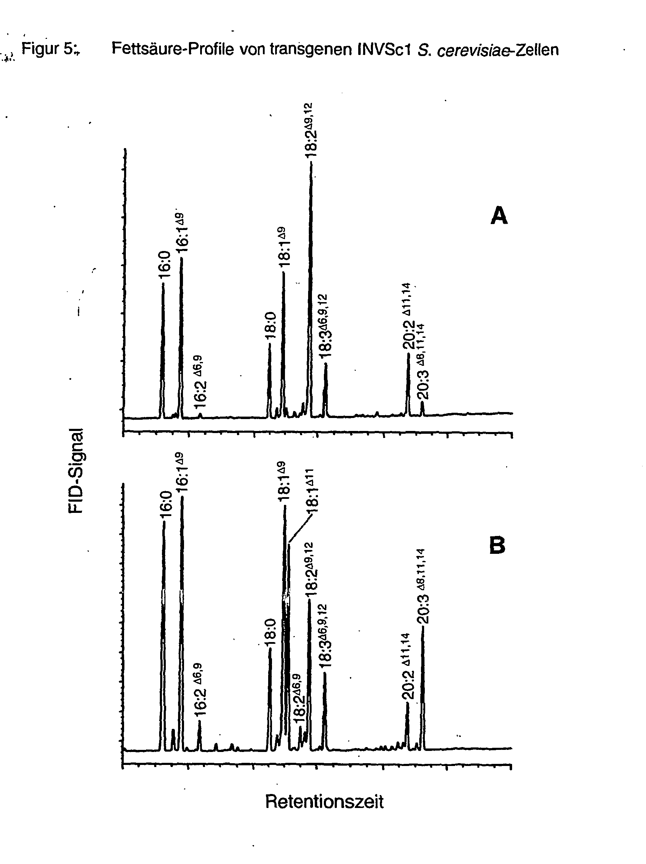

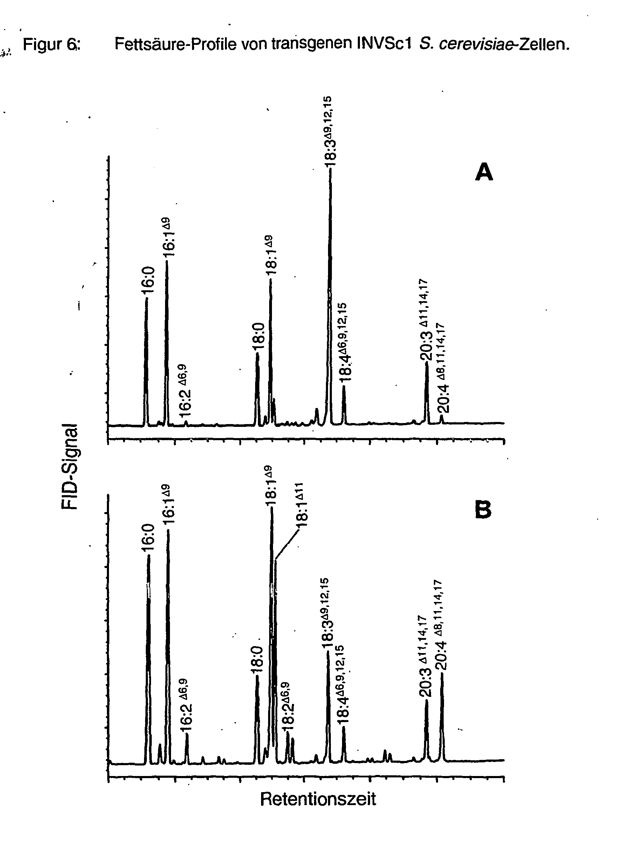

Bei Verwendung des Hefe-Stammes INVSc1 zur Co-Expression von CeLPLAT (T06E8.1) zusammen mit PpD6 und Pse1 ergibt sich folgendes Bild: Kontrollhefen, die PpD6 und Pse1 exprimieren, enthalten wie schon bei Verwendung des Stammes C13ABYS86 gezeigt nur geringe Mengen des Elongationsprodukts (20:3^{Δ8,11,14} bei Fütterung von 18:2 bzw. 20:4^{Δ8,11,14,17} bei Fütterung von 18:3; siehe Figur 5A und 6A). Bei zusätzlicher Expression von CeLPLAT (T06E8.1) erfolgt ein deutlicher Anstieg dieser Elongationsprodukte (siehe Figur 5B und 6B). Tabelle 6 zeigt, dass die zusätzliche Expression von CeLPLAT überraschenderweise eine 8-fache Erhöhung des Gehaltes an 20:3^{Δ8,11,14} (bei Fütterung von 18:2) bzw. 20:4^{Δ8,11,14,17} (bei Fütterung von 18:3) bewirkt. Daneben zeigt sich, dass auch C16:2^{Δ6,9} zu C18:2^{Δ6,9} effizienter elongiert wird.

**Tabelle 5: Fettsäure-Zusammensetzung (in mol %) transgener Hefen, die mit den Vektoren pESCLeu PpD6Pse1/pYes2 (PpD6 Pse1) oder pESCLeu-PpD6-Pse1/pYes2-T06E8.1 (PpD6 Pse1 + T06E8) transformiert worden waren. Die Hefezellen wurden in Minimalmedium ohne Uracil und Leucin in Gegenwart von 250 µM 18:2^{Δ9,12} oder 18:3^{Δ9,12,15} kultiviert. Die Fettsäuremethylester wurden durch saure Methanolyse ganzer Zellen gewonnen und über GLC analysiert. Jeder Wert gibt den Mittelwert (n = 4) ± Standardabweichung wieder.**

| Fettsäuren | Fütterung mit 250 µM 18:2^{Δ9,12} | | Fütterung mit 250 µM 18:3^{Δ9,12,15} | |
|---|---|---|---|---|
| | PpΔ6/Pse1 | PpΔ6/Pse1+ T06E8 | PpΔ6/Pse1 | PpΔ6/Pse1+ T06E8 |
| 16:0 | 15,31 ± 1,36 | 15,60 ± 1,36 | 12,20 ± 0,62 | 16,25 ± 1,85 |
| 16:1^{Δ9} | 23,22 ± 2,16 | 15,80±3,92 | 17,61 ± 1,05 | 14,58 ± 1,93 |
| 18:0 | 5,11 ± 0,63 | 7,98 ± 1,28 | 5,94 ± 0,71 | 7,52 ± 0,89 |
| 18:1^{Δ9} | 15,09±0,59 | 16,01 ± 2,53 | 15,62 ± 0,34 | 15,14 ± 2,61 |
| 18:1^{Δ11} | 4,64±1,09 | 11,80±1,12 | 4,56±0,18 | 13,07±1,66 |
| 18:2^{Δ9,12} | 28,72±3,25 | 14,44 ± 1,61 | - | - |
| 18:3^{Δ6,9,12} | 3,77±0,41 | 4,72 ± 0,72 | - | - |
| 18:3 ^{Δ9,12,15} | - | - | 32,86 ± 1,20 | 14,14 ± 2,52 |
| 18:4^{Δ6,9,12,15} | - | - | 5,16±1,04 | 3,31 ±1,15 |
| 20:2^{Δ11,14} | 2,12±0,86 | 4,95 ± 4,71 | - | - |
| 20:3^{Δ8,11,14} | 1,03 ±0,14 | 8,23±1,59 | - | - |
| 20:3 ^{Δ11,14,17} | - | - | 4,12 ± 1,54 | 6,95 ±2,52 |
| 20:4^{Δ8,11,14,17} | - | - | 1,34±0,28 | 8,70±1,11 |

Figur 5 ist das Fettsäure-Profile von transgenen INVSc1 S. cerevisiae-Zellen zu entnehmen. Die Synthese der Fettsäuremethylester erfolgte durch saure Methanolyse intakter Zellen, die entweder mit den Vektoren pESCLeu-PpD6-Pse1/pYes2 (A) oder pYes2-T06E8.1/pESCLeu-PpD6-Pse1 (B) transformiert worden waren. Die Hefen wurden in Minimalmedium in Gegenwart von 18:2^{Δ9,12} kultiviert. Anschließend wurden die Fettsäuremethylester über GLC analysiert.

Figur 6 gibt die Fettsäure-Profile von transgenen INVSc1 *S. cerevisiae*-Zellen wieder. Die Synthese der Fettsäuremethylester erfolgte durch saure Methanolyse intakter Zeilen, die entweder mit den Vektoren pESCLeu-PpD6-Pse1/pYes2 **(A)** oder pYes2-T06E8.1/pESCLeu-PpD6-Pse1 **(B)** transformiert worden waren. Die Hefen wurden in Minimalmedium in Gegenwart von 18:3^{Δ,12,15} kultiviert. Anschließend wurden die Fettsäuremethylester über GLC analysiert.

Ein Maß für die Effizienz der LCPUFA-Biosynthese in transgener Hefe stellt der Quotient aus Gehalt der erwünschten Δ-6-Elongationsprodukt nach Δ-6-Desaturierung (20:3^{Δ8,11,14} bzw. 20:4^{Δ8,11,14,17}) zu Gehalt an zugefütterter Fettsäure (18:2^{Δ9,12} bzw. 18:3^{Δ9,12,15}) dar. Dieser Quotient beträgt 0,04 in INVSc1 Kontrollhefen, die PpD6 und Pse1 exprimieren, und 0,60 in Hefen die zusätzlich zu PpD6 und Pse1 CeLPLAT exprimieren. In anderen Worten: der Gehalt an erwünschtem Δ-6-Elongationsprodukt nach Δ-6-Desaturierung bei Co-Expression von CeLPLAT beträgt 60% des Gehalts der jeweils zugefütterten Fettsäure. In Kontrollhefen beträgt dieser Gehalt nur ca. 4%. Dies bedeutet eine 15-fache Erhöhung der Effizienz der LCPUFA-Biosynthese in transgener Hefe durch Co-Expression von LPLAT.

Interessanterweise bewirkt die Co-Expression von CeLPLAT nicht nur eine Erhöhung der genannten Elongationsprodukte 20:3^{Δ8,11,14} bzw. 20:4^{Δ8,11,14,17}, sondern auch eine Erhöhung des Verhältnisses 20:3^{Δ8,11,14} : 20:2^{Δ11,14} bzw. 20:4^{Δ8,11,14,17} : 20:3^{Δ11,14,17}. Dies bedeutet, dass in Anwesenheit der LPLAT die Δ-6-Elongase bevorzugt mehrfach ungesättigte Fettsäuren (18:3^{Δ6,9,12} und 18:4^{Δ6,9,12,15}) als Substrat verwendet, während bei Abwesenheit der LPLAT keine ausgeprägte Substratspezifität zu erkennen ist (auch 18:2^{Δ9,12} und 18:3^{Δ9,12,15} werden elongiert). Grund hierfür können Protein-Protein-Wechselwirkungen zwischen Δ-6-Elongase, Δ-6-Desturase und LPLAT oder posttranslationale Modifikationen (z.B. partielle Proteolyse) sein. Dies würde auch erklären, warum der oben beschriebene Anstieg von Δ-6-Elongationsprodukten bei Co-Expression von Δ-6-Desaturase, Δ-6-Elongase und LPLAT bei Verwendung eines proteasedefizienten Hefestamms geringer ausfällt.

Acyl-CoA Analysen von transgenen INVSc1 Hefen, die mit 18:2^{Δ9,12} gefüttert wurden, ergaben folgendes Ergebnis: in Kontrollhefen, die PpD6 und Pse1 exprimieren, ist kein 18:3^{Δ6,9,12}-CoA und 20:3^{Δ8,11,14}-CoA nachweisbar. Dies weist darauf hin, dass weder das Substrat (18:3^{Δ6,9,12}-CoA) noch das Produkt (20:3^{Δ8,11,14}-CoA) der Δ-6-Elongase in Kontrollhefen in nachweisbaren Mengen vorhanden ist. Dies lässt darauf schließen, das der Transfer von 18:3^{Δ6,9,12} aus Membranlipiden in den Acyl-CoA Pool nicht oder nicht richtig stattfindet. Das bedeutet, dass kaum Substrat für die vorhandene Δ-6-Elongase zur Verfügung steht, was wiederum den geringen Gehalt an Elongationsprodukt in Kontrollhefen erklärt. INVSc1 Hefen, die zusätzlich zur PpD6 und Pse1 die CeLPLAT exprimieren und mit 182^{Δ9,12} gefüttert worden waren, weisen keine signifikanten Mengen an 18:3^{Δ6,9,12}-CoA auf, wohl aber 20:3^{Δ8,11,14}-CoA. Dies deutet darauf hin, dass LPLAT sehr effizient 18:3^{Δ6,9,12} aus den Membranlipiden in den Acyl-CoA-Pool überführt. 18:3^{Δ6,9,12}-CoA wird dann von der Δ-6-Elongase elongiert, so dass kein 18:3^{Δ6,9,12}-CoA, wohl aber 20:3^{Δ8,11,14}-CoA nachweisbar ist.

### b) Funktionelle Charakterierung der CeLPLATs in transgenen Pflanzen

### Expression funktionaler CeLPLAT in transgenen Pflanzen

In DE 10219 203 wurden transgene Pflanzen beschrieben, deren Samenöl durch samenspezifische Expression funktioneller Gene kodierend für Δ-6-Desaturase, Δ-6-Elongase und Δ-5-Desaturase geringe Mengen an ARA und EPA enthält. Der zur Transformation dieser Pflanzen benutzte Vektor ist SEQ ID NO: 19 zu entnehmen. Um den Gehalt an diesen LCPUFAs zu erhöhen, wurde in den genannten transgenen Pflanzen zusätzlich das Gen CeLPLAT (T06E8.1) in Samen exprimiert.

Zu diesem Zweck wurde der kodierende Bereich von CeLPLAT über PCR amplifiziert.

In Tabelle 6 sind die Primer wiedergegeben, die zur Klonierung eines weiteren Clones der ceLPLAT in binäre Vektoren verwendet wurden.

**Tabelle 6: Nukleotidsequenzen der PCR-Primer zur Klonierung von CeLPLAT (T06E8.1) in den binären Vektor pSUN3**

| Primer | Nukleotidsequenz |
|---|---|
| ARe503f* | 5' TTAAGCGCGGCCGCATGGAGAACTTCTGGTCG 3' |
| ARe504r* | 5' ACCTCGGCGGCCGCCCTTTTACTCAGATTTC 3' |

| | |
|---|---|
| * f: forward, r: reverse | |

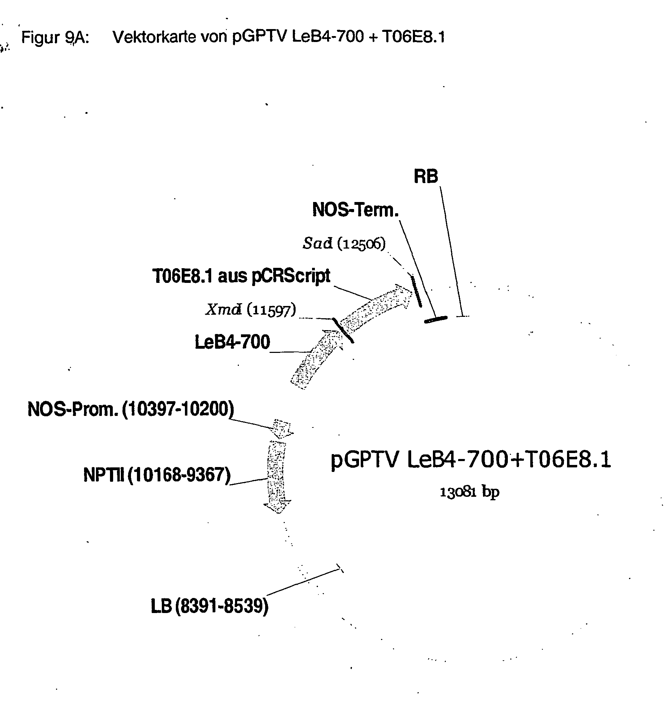

Das PCR-Produkt wurde in einen pENTRY Vektor zwischen USP Promotor und OCS-Terminator kloniert. Anschließend wurde die Expressionskassette in die binären Vektoren pSUN300 kloniert. Der entstandene Vektor wurde mit pSUN3CeLPLAT (Figur 8) bezeichnet. Darüber hinaus wurde der kodierende Bereiche von CeLPLAT amplifiziert und zwischen LegB4 Promotor und OCS-Terminator kloniert: Dieser Vektor wurde mit pGPTVCeLPLAT bezeichnet (Figur 9A).

Darüberhinaus wurde der kodierende Bereich von CeLPLAT über PCR amplifiziert und zwischen LegB4 Promotor und OCS-Terminator kloniert. Die hierfür verwendeten PCR Primer wurden so ausgewählt, dass in das PCR-Produkt eine effiziente Kosaksequenz eingeführt wurde. Außerdem wurde die DNA-Sequenz von CeLPLAT so verändert, dass sie der codon usage von höheren Pflanzen angepasst war.

Folgende Primer wurden für die PCR verwendet

Forward primer: 5'-ACATAATGGAGAACTTCTGGTCTATTGTTGTGTTTTTTCTA-3'

Reverse primer: 5'-CTAGCTAGCTTACTCAGATTTCTTCCCGTCTTTTGTTTCTG-3'

Das PCR Produkt wurde in den Klonierungsvektor pCR Script kloniert und über die Restriktionsenzyme Xmal und Sacl in den Vektor pGPTV LegB4-700 kloniert. Das entstandene Plasmid wurde mit pGPTV LegB4-700 + T06E8.1 bezeichnet (Figur 9A).

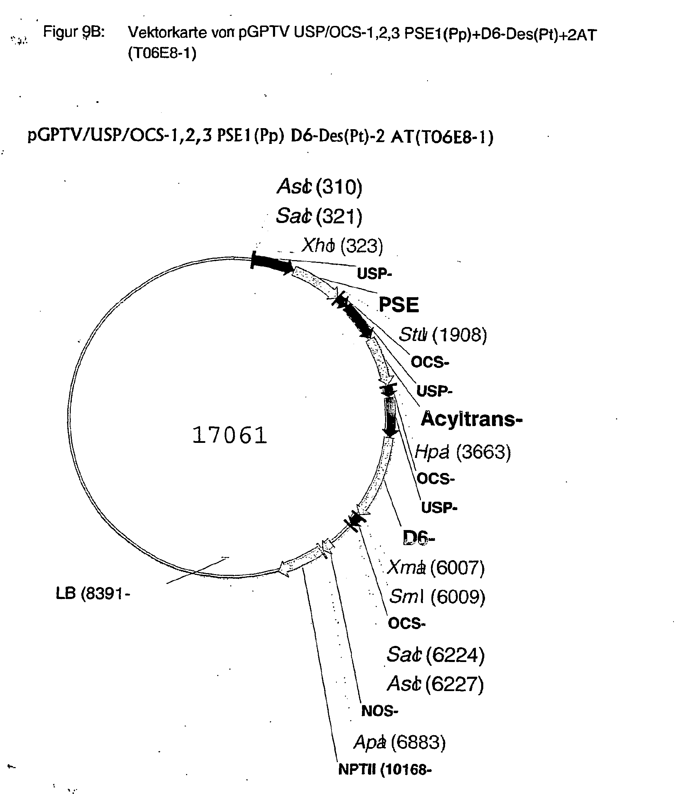

Das gleiche PCR Produkt wurde darüber hinaus in einen Multigen-Expressionsvektor kloniert, der bereits die Gene für eine Delta-6-Desaturase aus Phaeodactylum tricomutum (SEQ ID NO: 32, Aminosäuresequenz SEQ ID NO: 33) und einer Delta-6-Elongase aus P. patens enthielt. Das entstandene Plasmid wurde mit pGPTV USP/OCS-1,2,3 PSE1 (Pp)+D6-Des(Pt)+2AT (T06E8-1) bezeichnet (Figur 9B). Die Sequenzen des Vektors sowie der Gene sind SEQ ID NO:-34, SEQ ID NO: 35, SEQ ID NO: 36 und SEQ ID NO: 37 zu entnehmen. Die Δ-6-Desaturase aus Phaeodactylum ticomutum reicht von Nukleotid 4554 bis 5987 in der SEQ ID NO: 34. Die A-6-Elongase aus Physcomitrella patens reicht von Nukleotid 1026 bis 1898 und die der LPLAT aus Caenorhabditis elegans reicht von Nukleotid 2805 bis 3653 in der SEQ ID NO: 34.

Tabakpflanzen wurden co-transformiert mit dem Vektor pSUN3CeLPLAT und dem in DE 10219 203 und SEQ ID NO: 19 beschriebenen Vektor enthaltend Gene kodierend für Δ-6-Desaturase, Δ-6-Elongase und Δ-5-Desaturase, wobei die Selektion transgener Pflanzen mit Kanamycin erfolgte.

Tabakpflanzen wurden außerdem transformiert mit dem Vektor pGPTV USP/OCS-1,2,3 PSE1(Pp)+D6-Des(Pt)+2AT (T06E8-1) [siehe SEQ ID NO:.34, SEQ ID NO: 35, SEQ ID NO: 36 und SEQ ID NO: 37].

Lein wurde mit dem Vektor pSUN3CeLPLAT transformiert. Die entstandenen transgenen Pflanzen wurden mit solchen transgenen Leinpflanzen gekreuzt, die bereits geringe Mengen an ARA und EPA aufgrund der funktionellen Genexpression von Δ-6-Desaturase, Δ-6-Elongase und Δ-5-Desaturase enthielten.

Weiterhin wurde Lein mit dem Vektor pGPTV LegB4-700 + T06E8.1 transformiert. Die entstandenen transgenen Pflanzen wurden mit solchen transgene Leinpflanzen gekreuzt, die bereits geringe Mengen an ARA und EPA aufgrund der funktionellen Expression von Δ-6-Desaturase, Δ-6-Elongase und Δ-5-Desaturase enthielten.

Die Samen von tränsgenen Tabak- und Leinpflanzen wurden wie weiter vorne beschrieben [Beispiel 3 b)] auf erhöhte Gehalte an LCPUFAs in untersucht.

Aus den hier vorliegenden.Arbeiten lässt sich die Funktion der Acyl-CoA:Lysophopholipid-Acyltranserase (LPLAT) wie in Figur 10 dargestellt ableiten. Der Biosynthese-Weg der LCPUFAS stellt sich damit wie folgt dar.

Desaturasen katalysieren die Einführung von Doppelbindungen in lipidgekoppelte Fettsäuren (sn2-Acyl-Phosphatidylcholin), während die Elongasen exklusiv die Elongation Coenzym A-veresterter Fettsäuren (Acyl-CoAs) katalysieren. Nach diesem Mechanismus erfordert die alternierende Wirkung von Desaturasen und Elongasen einen ständigen Austausch von Acyl-Substraten zwischen Phospholipiden und Acyl-CoA-Pool und somit die Existenz einer zusätzlichen Aktivität, die die Acyl-Substrate in die jeweils notwendige Substratform, d.h. Lipide (für Desaturasen) oder CoA-Thioester (für Elongasen), überführt. Dieser Austausch zwischen Acyl-CoA Pool und Phospholipiden wird durch LCPUFA-spezifische LPLAT ermöglicht. Die Biosynthese von ARA (A) erfolgt analog zu EPA (B), mit dem Unterschied, dass bei EPA der Δ-6-Desaturierung eine Δ-15-Desaturierung vorgeschaltet ist, so dass α18:3-PC als Substrat für die Δ-6-Desaturase fungiert. Die Biosynthese von DHA macht einen weiteren Austausch zwischen Phospholipiden und Acyl-CoA-Pool über LPLAT notwendig: 20:5^{Δ5,8,11,14,17} wird vom Phospholipid- zum CoA-Pool transferiert und nach erfolgter Δ-5-Elongation wird 22:5^{□7,10,13,16,19} vom CoA- zum Phospholipid-Pool transferiert und schließlich durch Δ-4-Desaturase zu DHA umgesetzt. Gleiches gilt für den Austausch im Biosyntheseweg unter Verwendung der Δ-8-Desaturase, der Δ-9-Elongase und der Δ-5-Desaturase.

## Patentansprüche

1. Verfahren zur Herstellung mehrfach ungesättigter Fettsäuren in einem Organismus, **dadurch gekennzeichnet, dass** das Verfahren folgende Schritte umfasst:
a) Einbringen mindestens einer Nukleinsäuresequenz in den Organismus mit der in SEQ ID NO: 1, SEQ ID NO: 3, SEQ ID NO: 5 oder SEQ ID NO: 7 dargestellten Sequenz, die für ein Polypeptid mit einer Acyl-CoA:Lysophospholipid-Acyltransferaseaktivität codiert; oder
b) Einbringen mindestens einer Nukleinsäuresequenz in den Organismus, die sich als Ergebnis des degenerierten genetischen Codes von der in SEQ ID NO: 1, SEQ ID NO: 3, SEQ ID NO: 5 oder SEQ ID NO: 7 enthaltenden codierenden Sequenz ableiten lässt, oder
c) Einbringen mindestens eines Derivates der in SEQ ID NO: 1, SEQ ID NO: 3, SEQ ID NO: 5 oder SEQ ID NO: 7 dargestellten Nukleinsäuresequenz in den Organismus, die für Polypeptide mit der in SEQ ID NO: 2, SEQ ID NO: 4, SEQ ID NO: 6 oder SEQ ID NO: 8 dargestellten Aminosäuresequenz codieren und mindestens 90 % Identität auf Aminosäureebene mit SEQ ID NO: 2, SEQ ID NO: 4, SEQ ID NO: 6 oder SEQ ID NO: 8 aufweisen und Acyl-CoA:Lysophospholipid-Acyltransferaseaktivität aufweisen, und
d) Kultivieren und ernten des Organismus.

2. Verfahren zur Herstellung mehrfach ungesättigter Fettsäuren nach Anspruch 1, **dadurch gekennzeichnet, dass** zusätzlich zu den unter (a) bis (c) genannten Nukleinsäuresequenzen weitere Nukleinsäuresequenzen in den Organismus eingebracht wurden, die für Polypeptide des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-AcylTransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) codieren.

3. Verfahren zur Herstellung mehrfach ungesättigter Fettsäuren nach Anspruch 2 oder 13, **dadurch gekennzeichnet, dass** zusätzlich zu den unter (a) bis (c) genannten Nukleinsäuresequenzen weitere Nukleinsäuresequenzen in den Organismus eingebracht wurden, die für Polypeptide ausgewählt aus der Gruppe Δ-4-Desaturase-, Δ-5-Desaturase-, Δ-6-Desaturase-, Δ-8-Desatuase-, Δ-9-Desaturase-, Δ-12-Desaturase-, Δ-5-Elongase-, Δ-6-Elongase- oder Δ-9-Elongaseaktivität codieren.

4. Verfahren zur Herstellung mehrfach ungesättigter Fettsäuren nach den Ansprüchen 1 bis 3, **dadurch gekennzeichnet, dass** als Substrat der Acyl-CoA:Lysophospholipid-Acyltransferasen C₁₆-, C₁₈-, C₂₀- oder C₂₂-Fettsäuren verwendet werden.

5. Verfahren zur Herstellung mehrfach ungesättigter Fettsäuren nach den Ansprüchen 1 bis 3, **dadurch gekennzeichnet, dass** die mehrfach ungesättigten Fettsäuren aus dem Organismus in Form eines Öls, Lipids oder einer freien Fettsäure isoliert werden.

6. Verfahren zur Herstellung mehrfach ungesättigter Fettsäuren nach den Ansprüchen 1 bis 5, **dadurch gekennzeichnet, dass** die im Verfahren hergestellte mehrfach ungesättigten Fettsäure eine C₁₈-, C₂₀- oder C₂₂-Fettsäuren mit mindestens zwei Doppelbindungen im Molekül ist.

7. Verfahren zur Herstellung mehrfach ungesättigter Fettsäuren nach den Ansprüchen 1 bis 6 **dadurch gekennzeichnet, dass** im Verfahren eine mehrfach ungesättigte Fettsäure ausgewählt aus der Gruppe Dihomo-γ-linolensäure, Arachidonsäure, Eisosapentaensäure, Docosapentaensäure und Docosahexaensäure hergestellten wird.

8. Verfahren zur Herstellung mehrfach ungesättigter Fettsäuren nach den Ansprüchen 1 bis 7, **dadurch gekennzeichnet, dass** der Organismus ein Mikroorganismus, ein nicht-humanes Tier oder eine Pflanze ist.

9. Verfahren zur Herstellung mehrfach ungesättigter Fettsäuren nach den Ansprüchen 1 bis 8, **dadurch gekennzeichnet, dass** der Organismus eine transgene Pflanze ist.

10. Verfahren zur Herstellung mehrfach ungesättigter Fettsäuren nach den Ansprüchen 1 bis 9, **dadurch gekennzeichnet, dass** die transgene Pflanze eine Ölfruchtpflanze ist.

11. Verwendung einer Nukleinsäuresequenz zur Produktion von mehrfach ungesättigten Fettsäuren (PUFAs) in Pflanzen, wobei die Nukleinsäuresequenz ausgewählt ist aus der Gruppe bestehend aus:
a) einer Nukleinsäuresequenz mit der in SEQ ID NO: 1, SEQ ID NO: 3, SEQ ID NO: 5 oder SEQ ID NO: 7 dargestellten Sequenz, die für ein Polypeptid mit einer Acyl-CoA:Lysophospholipid-Acyltransferaseaktivität codiert;
b) einer Nukleinsäuresequenz, die sich als Ergebnis des degenerierten genetischen Codes von der in SEQ ID NO: 1, SEQ ID NO: 3, SEQ ID NO: 5 oder SEQ ID NO: 7 enthaltenden codierenden Sequenz ableiten lässt, und
c) einem Derivat der in SEQ ID NO: 1, SEQ ID NO: 3, SEQ ID NO: 5 oder SEQ ID NO: 7 dargestellten Nukleinsäuresequenz, die für Polypeptide mit der in SEQ ID NO: 2, SEQ ID NO: 4, SEQ ID NO: 6 oder SEQ ID NO: 8 dargestellten Aminosäuresequenz codiert und mindestens 90 % Identität auf Aminosäureebene mit SEQ ID NO: 2, SEQ ID NO: 4, SEQ ID NO: 6 oder SEQ ID NO: 8 aufweist und Acyl-CoA:Lysophospholipid-Acyltransferaseaktivität aufweist.

## Claims

1. A process for producing polyunsaturated fatty acids in an organism, wherein said process comprises the following steps:
a) introducing into the organism at least one nucleic acid sequence having the sequence depicted in SEQ ID NO: 1, SEQ ID NO: 3, SEQ ID NO: 5 or SEQ ID NO: 7, which sequence codes for a polypeptide having an acyl-CoA:lysophospholipid-acyltransferase activity; or
b) introducing into said organism at least one nucleic acid sequence which can be derived, as a result of the degenerate genetic code, from the coding sequence comprised in SEQ ID NO: 1, SEQ ID NO: 3, SEQ ID NO: 5 or SEQ ID NO: 7, or
c) introducing into said organism at least one derivative of the nucleic acid sequence depicted in SEQ ID NO: 1, SEQ ID NO: 3, SEQ ID NO: 5 or SEQ ID NO: 7, which code for polypeptides having the amino acid sequence depicted in SEQ ID NO: 2, SEQ ID NO: 4, SEQ ID NO: 6 or SEQ ID NO: 8 and which are at least 90% identical at the amino acid level to SEQ ID NO: 2, SEQ ID NO: 4, SEQ ID NO: 6 or SEQ ID NO: 8 and have acyl-CoA:lysophospholipidacyltransferase activity, and
d) culturing and harvesting said organism.

2. The process for producing polyunsaturated fatty acids according to claim 1, wherein, in addition to the nucleic acid sequences mentioned under (a) to (c), further nucleic acid sequences have been introduced into said organism, which code for polypeptides of the fatty acid or lipid metabolism, selected from the group consisting of acyl-CoA-dehydrogenase(s), acyl-ACP[= acyl carrier protein] desaturase(s), acyl-ACP thioesterase(s), fatty acid acyltransferase(s), fatty acid synthase(s), fatty acid hydroxylase(s), acetyl-coenzyme A carboxylase(s), acyl-coenzyme A oxidase(s), fatty acid desaturase(s), fatty acid acetylenases, lipoxygenases, triacylglycerol lipases, allenoxide synthases, hydroperoxide lyases or fatty acid elongase(s).

3. The process for producing polyunsaturated fatty acids according to claim 2, wherein, in addition to the nucleic acid sequences mentioned under (a) to (c), further nucleic acid sequences have been introduced into the organism, which code for polypeptides selected from the group consisting of Δ4-desaturase, Δ5-desaturase, Δ6-desaturase, Δ8-desaturase, Δ9-desaturase, Δ12-desaturase, Δ5-elongase, Δ6-elongate or Δ9-elongate activity.

4. The process for producing polyunsaturated fatty acids according to claims 1 to 3, wherein C₁₆-, C₁₈-, C₂₀- or C₂₂- fatty acids are used as substrate of the acyl-CoA:lysophospholipid acyltransferases.

5. The process for producing polyunsaturated fatty acids according to claims 1 to 3, wherein the polyunsaturated fatty acids are isolated from the organism in the form of an oil, lipid or a free fatty acid.

6. The process for producing polyunsaturated fatty acids according to claims 1 to 5, wherein the polyunsaturated fatty acid produced in said process is a C₁₈-, C₂₀- or C₂₂- fatty acid having at least two double bonds in the molecule.

7. The process for producing polyunsaturated fatty acids according to claims 1 to 6, wherein a polyunsaturated fatty acid selected from the group consisting of dihomo-γ-linolenic acid, arachidonic acid, eicosapentaenoic acid, docosapentaenoic acid and docosahexaenoic acid is produced in said process.

8. The process for producing polyunsaturated fatty acids according to claims 1 to 7, wherein the organism is a microorganism, a nonhuman animal or a plant.

9. The process for producing polyunsaturated fatty acids according to claims 1 to 8, wherein the organism is a transgenic plant.

10. The process for producing polyunsaturated fatty acids according to claims 1 to 9, wherein the transgenic plant is an oil crop plant.

11. The use of a nucleic acid sequence for the production of polyunsaturated fatty acids (PUFAs) in plants, the nucleic acid sequence being selected from the group consisting of:
a) a nucleic acid sequence having the sequence depicted in SEQ ID NO: 1, SEQ ID NO: 3, SEQ ID NO: 5 or SEQ ID NO: 7, which sequence codes for a polypeptide having an acyl-CoA:lysophospholipid-acyltransferase activity;
b) a nucleic acid sequence which can be derived from the coding sequence comprised in SEQ ID NO: 1, SEQ ID NO: 3, SEQ ID NO: 5 or SEQ ID NO: 7 as a result of the degenerate genetic code, and
c) a derivative of the nucleic acid sequence depicted in SEQ ID NO: 1, SEQ ID NO: 3, SEQ ID NO: 5 or SEQ ID NO: 7 which codes for polypeptides having the amino acid sequence depicted in SEQ ID NO: 2, SEQ ID NO: 4, SEQ ID NO: 6 or SEQ ID NO: 8 and is at least 90% identical at the amino acid level to SEQ ID NO: 2, SEQ ID NO: 4, SEQ ID NO: 6 or SEQ ID NO: 8 and has acyl-CoA:lysophospholipid-acyltransferase activity.

## Revendications

1. Procédé de préparation d'acides gras polyinsaturés dans un organisme, **caractérisé en ce que** le procédé comprend les étapes suivantes :
a) introduction d'au moins une séquence d'acides nucléiques dans l'organisme, ayant la séquence présentée dans SEQ ID N°1, SEQ ID N°3, SEQ ID N°5 ou SEQ ID N°7, qui code pour un polypeptide ayant une activité d'acyl-CoA:lysophospholipide-acyltransférase ; ou
b) introduction d'au moins une séquence d'acides nucléiques dans l'organisme, qui peut dériver, en conséquence du code génétique dégénéré, de la séquence codante contenue dans SEQ ID N°1, SEQ ID N°3, SEQ ID N°5 ou SEQ ID N°7, ou
c) introduction d'au moins un dérivé de la séquence d'acides nucléiques présentée dans SEQ ID N°1, SEQ ID N°3, SEQ ID N°5 ou SEQ ID N°7 dans l'organisme, qui code pour les polypeptides ayant la séquence d'acides aminés présentée dans SEQ ID N°2, SEQ ID N°4, SEQ ID N°6 ou SEQ ID N°8, et qui présente une identité d'au moins 90 % au niveau des acides aminés avec SEQ ID N°2, SEQ ID N°4, SEQ ID N°6 ou SEQ ID N°8 et présente une activité d'acyl-CoA:lysophospholipide-acyltransférase, et
c) culture et récolte de l'organisme.

2. Procédé de préparation d'acides gras polyinsaturés selon la revendication 1, **caractérisé en ce que**, outre les séquences d'acides nucléiques mentionnées en a) à c), on a introduit dans l'organisme d'autres séquences d'acides nucléiques, qui codent pour des polypeptides du métabolisme des acides gras ou des lipides, choisis dans le groupe de la ou des acyl-CoA-déshydrogénases, acyl-ACP(= protéine porteuse d'acyle)-désaturases, acyl-ACP-thioestérases, acide gras-acyl-transférases, acide gras-synthases, acide gras-hydroxylases, acétylcoenzyme A-carboxylases, acyl-coenzyme-A-oxydases, acide gras-désaturases, acide gras-acétylénases, lipoxygénases, triacylglycérol-lipases, oxyde d'allène-synthases, hydroperoxyde-lyases ou acide gras-élongases.

3. Procédé de préparation d'acides gras polyinsaturés selon la revendication 2, **caractérisé en ce que**, outre les séquences d'acides nucléiques mentionnées en a) à c), on introduit dans l'organisme d'autres séquences d'acides nucléiques, qui codent pour des polypeptides choisis dans le groupe consistant en une activité de Δ-4-désaturase, de Δ-5-désaturase, de Δ-6-désaturase, de Δ-8-désaturase, de Δ-9-désaturase, de Δ-12-désaturase, de Δ-5-élongase, de Δ-6-élongase ou de Δ-9-élongase.

4. Procédé de préparation d'acides gras polyinsaturés selon les revendications 1 à 3, **caractérisé en ce qu'**on utilise en tant que substrats des acyl-CoA:lysophospholipide-acyltransférases des acides gras en C₁₆, C₁₈, C₂₀ ou C₂₂.

5. Procédé de préparation d'acides gras polyinsaturés selon les revendications 1 à 3, **caractérisé en ce que** les acides gras insaturés sont isolés de l'organisme sous forme d'une huile, d'un lipide ou d'un acide gras libre.

6. Procédé de préparation d'acides gras polyinsaturés selon les revendications 1 à 5, **caractérisé en ce que** l'acide gras polyinsaturé préparé dans le procédé est un acide gras en C₁₈, C₂₀ ou C₂₂ ayant dans sa molécule au moins deux doubles liaisons.

7. Procédé de préparation d'acides gras polyinsaturés selon les revendications 1 à 6, **caractérisé en ce qu'**on prépare dans le procédé un acide gras polyinsaturé choisi dans le groupe consistant en l'acide dihomo-γ-linolénique, l'acide arachidonique, l'acide eicosapentaénoïque, l'acide docosapentaénoïque et l'acide docosahexaénoïque.

8. Procédé de préparation d'acides gras polyinsaturés selon les revendications 1 à 7, **caractérisé en ce que** l'organisme est un microorganisme, un animal non humain ou une plante.

9. Procédé de préparation d'acides gras polyinsaturés selon les revendications 1 à 8, **caractérisé en ce que** l'organisme est une plante transgénique.

10. Procédé de préparation d'acides gras polyinsaturés selon les revendications 1 à 9, **caractérisé en ce que** la plante transgénique est une plante oléagineuse.

11. Utilisation d'une séquence d'acides nucléiques pour la production d'acides gras polyinsaturés (PUFA) dans des plantes, la séquence d'acides nucléiques étant choisie dans le groupe consistant en :
a) une séquence d'acides nucléiques ayant la séquence présentée dans SEQ ID N°1, SEQ ID N°3, SEQ ID N°5 ou SEQ ID N°7, qui code pour un polypeptide ayant une activité d'acyl-CoA:lysophospholipide-acyltransférase ;
b) une séquence d'acides nucléiques qui peut, en conséquence du code génétique dégénéré, dériver de la séquence codante contenue dans SEQ ID N°1, SEQ ID N°3, SEQ ID N°5 ou SEQ ID N°7, et
c) un dérivé de la séquence d'acides nucléiques présentée dans SEQ ID N°1, SEQ ID N°3, SEQ ID N°5 ou SEQ ID N°7, qui code pour des polypeptides ayant la séquence d'acides nucléiques présentée dans SEQ ID N°2, SEQ ID N°4, SEQ ID N°6 ou SEQ ID N°8 et présente une identité d'au moins 90 % au niveau des acides aminés avec SEQ ID N°2, SEQ ID N°4, SEQ ID N°6 ou SEQ ID N°8, et présente une activité d'acyl-CoA: lysophospholipide-acyltransférase.


### SEQUENCE LISTING

<110> BASF Plant Science GmbH
<120> Verfahren zur Herstellung mehrfach ungesättigter Fettsäuren
<130> 20030015
<160> 37
<170> PatentIn version 3.1
<210> 1
   <211> 849
   <212> DNA
   <213> Caenorhabditis elegans
<220>
   <221> CDS
   <222> (1)..(849)
   <223> Acyl-CoA:Lysophospholipid-Acyltransferase
<400> 1
   atg gag aac ttc tgg tcg atc gtc gtg ttt ttt cta ctc tca att ctc      48
   Met Glu Asn Phe Trp Ser Ile Val Val Phe Phe Leu Leu Ser Ile Leu
   1               5                   10                  15
   ttc att tta tat aac ata tcg aca gta tgc cac tac tat atg cgg att      96
   Phe Ile Leu Tyr Asn Ile Ser Thr Val Cys His Tyr Tyr Met Arg Ile
               20                  25                  30
   tcg ttt tat tac ttc aca att tta ttg cat gga atg gaa gtt tgt gtt     144
   Ser Phe Tyr Tyr Phe Thr Ile Leu Leu His Gly Met Glu Val Cys Val
           35                  40                  45
   aca atg atc cct tct tgg cta aat ggg aag ggt gct gat tac gtg ttt     192
   Thr Met Ile Pro Ser Trp Leu Asn Gly Lys Gly Ala Asp Tyr Val Phe
       50                  55                  60
   cac tcg ttt ttc tat tgg tgt aaa tgg act ggt gtt cat aca aca gtc     240
   His Ser Phe Phe Tyr Trp Cys Lys Trp Thr Gly Val His Thr Thr Val
   65                  70                  75                  80
   tat gga tat gaa aaa aca caa gtt gaa ggt ccg gct gta gtt att tgt     288
   Tyr Gly Tyr Glu Lys Thr Gln Val Glu Gly Pro Ala Val Val Ile Cys
                   85                  90                  95
   aat cat cag agt tct ctc gac att cta tcg atg gca tca atc tgg ccg     336
   Asn His Gln Ser Ser Leu Asp Ile Leu Ser Met Ala Ser Ile Trp Pro
               100                 105                 110
   aag aat tgt gtt gta atg atg aaa cga att ctt gcc tat gtt cca ttc     384
   Lys Asn Cys Val Val Met Met Lys Arg Ile Leu Ala Tyr Val Pro Phe
           115                 120                 125
   ttc aat ctc gga gcc tac ttt tcc aac aca atc ttc atc gat cga tat     432
   Phe Asn Leu Gly Ala Tyr Phe Ser Asn Thr Ile Phe Ile Asp Arg Tyr
       130                 135                 140
   aac cgt gaa cgt gcg atg gct tca gtt gat tat tgt gca tct gaa atg     480
   Asn Arg Glu Arg Ala Met Ala Ser Val Asp Tyr Cys Ala Ser Glu Met
   145 150 155 160
   aag aac aga aat ctt aaa ctt tgg gta ttt ccg gaa gga aca aga aat 528
   Lys Asn Arg Asn Leu Lys Leu Trp Val Phe Pro Glu Gly Thr Arg Asn
   165 170 175
   cgt gaa gga ggg ttc att cca ttc aag aaa gga gca ttc aat att gca 576
   Arg Glu Gly Gly Phe Ile Pro Phe Lys Lys Gly Ala Phe Asn Ile Ala
   180 185 190
   gtt cgt gcg cag att ccc att att cca gtt gta ttc tca gac tat cgg 624
   Val Arg Ala Gln Ile Pro Ile Ile Pro Val Val Phe Ser Asp Tyr Arg
   195 200 205
   gat ttc tac tca aag cca ggc cga tat ttc aag aat gat gga gaa gtt 672
   Asp Phe Tyr Ser Lys Pro Gly Arg Tyr Phe Lys Asn Asp Gly Glu Val
   210 215 220
   gtt att cga gtt ctg gat gcg att cca aca aaa ggg ctc act ctt gat 720
   Val Ile Arg Val Leu Asp Ala Ile Pro Thr Lys Gly Leu Thr Leu Asp
   225 230 235 240
   gac gtc agc gag ttg tct gat atg tgt cgg gac gtt atg ttg gca gcc 768
   Asp Val Ser Glu Leu Ser Asp Met Cys Arg Asp Val Met Leu Ala Ala
   245 250 255
   tat aag gaa gtt act cta gaa gct cag caa cga aat gcg aca cgg cgt 816
   Tyr Lys Glu Val Thr Leu Glu Ala Gln Gln Arg Asn Ala Thr Arg Arg
   260 265 270
   gga gaa aca aaa gac ggg aag aaa tct gag taa 849
   Gly Glu Thr Lys Asp Gly Lys Lys Ser Glu
   275 280
<210> 2
   <211> 282
   <212> PRT
   <213> Caenorhabditis elegans
<400> 2
   Met Glu Asn Phe Trp Ser Ile Val Val Phe Phe Leu Leu Ser Ile Leu
   1               5                   10                  15
   Phe Ile Leu Tyr Asn Ile Ser Thr Val Cys His Tyr Tyr Met Arg Ile
               20                  25                  30
   Ser Phe Tyr Tyr Phe Thr Ile Leu Leu His Gly Met Glu Val Cys Val
           35                  40                  45
   Thr Met Ile Pro Ser Trp Leu Asn Gly Lys Gly Ala Asp Tyr Val Phe
       50                  55                  60
   His Ser Phe Phe Tyr Trp Cys Lys Trp Thr Gly Val His Thr Thr Val
   65                  70                  75                  80
   Tyr Gly Tyr Glu Lys Thr Gln Val Glu Gly Pro Ala Val Val Ile Cys
                   85                  90                  95
   Asn His Gln Ser Ser Leu Asp Ile Leu Ser Met Ala Ser Ile Trp Pro
               100                 105                 110
   Lys Asn Cys Val Val Met Met Lys Arg Ile Leu Ala Tyr Val Pro Phe
           115                 120                 125
   Phe Asn Leu Gly Ala Tyr Phe Ser Asn Thr Ile Phe Ile Asp Arg Tyr
       130                 135                 140
   Asn Arg Glu Arg Ala Met Ala Ser Val Asp Tyr Cys Ala Ser Glu Met
   145                 150                 155                 160
   Lys Asn Arg Asn Leu Lys Leu Trp Val Phe Pro Glu Gly Thr Arg Asn
                   165                 170                 175
   Arg Glu Gly Gly Phe Ile Pro Phe Lys Lys Gly Ala Phe Asn Ile Ala
               180                 185                 190
   Val Arg Ala Gln Ile Pro Ile Ile Pro Val Val Phe Ser Asp Tyr Arg
           195                 200                 205
   Asp Phe Tyr Ser Lys Pro Gly Arg Tyr Phe Lys Asn Asp Gly Glu Val
       210                 215                 220
   Val Ile Arg Val Leu Asp Ala Ile Pro Thr Lys Gly Leu Thr Leu Asp
   225                 230                 235                 240
   Asp Val Ser Glu Leu Ser Asp Met Cys Arg Asp Val Met Leu Ala Ala
                   245                 250                 255
   Tyr Lys Glu Val Thr Leu Glu Ala Gln Gln Arg Asn Ala Thr Arg Arg
               260                 265                 270
   Gly Glu Thr Lys Asp Gly Lys Lys Ser Glu
           275                 280
<210> 3
   <211> 849
   <212> DNA
   <213> Caenorhabditis elegans
<220>
   <221> CDS
   <222> (1)..(849)
   <223> Acyl-CoA:Lysophospholipid-Acyltransferase
   <400> 3
   atg gag aac ttc tgg tcg atc gtc gtg ttt ttt cta ctc tca att ctc      48
   Met Glu Asn Phe Trp Ser Ile Val Val Phe Phe Leu Leu Ser Ile Leu
   1               5                   10                  15
   ttc att tta tat aac ata tcg aca gta tgc cac tac tat atg cgg att      96
   Phe Ile Leu Tyr Asn Ile Ser Thr Val Cys His Tyr Tyr Met Arg Ile
               20                  25                  30
   tcg ttt tat tac ttc aca att tta ttg cat gga atg gaa gtt tgt gtt     144
   Ser Phe Tyr Tyr Phe Thr Ile Leu Leu His Gly Met Glu Val Cys Val
           35                  40                  45
   aca atg atc cct tct tgg cta aat ggg aag ggt gct gat tac gtg ttt     192
   Thr Met Ile Pro Ser Trp Leu Asn Gly Lys Gly Ala Asp Tyr Val Phe
       50                  55                  60
   cac tcg ttt ttc tat tgg tgt aaa tgg act ggt gtt cat aca aca gtc     240
   His Ser Phe Phe Tyr Trp Cys Lys Trp Thr Gly Val His Thr Thr Val
   65                  70                  75                  80
   tat gga tat gaa aaa aca caa gtt gaa ggt ccg gct gta gtt att tgt     288
   Tyr Gly Tyr Glu Lys Thr Gln Val Glu Gly Pro Ala Val Val Ile Cys
                   85                  90                  95
   aat cat cag agt tct ctc gac att cta tcg atg gca tca atc tgg ccg     336
   Asn His Gln Ser Ser Leu Asp Ile Leu Ser Met Ala Ser Ile Trp Pro
               100                 105                 110
   aag aat tgt gtt gta atg atg aaa cga att ctt gcc tat gtt cca ttc     384
   Lys Asn Cys Val Val Met Met Lys Arg Ile Leu Ala Tyr Val Pro Phe
           115                 120                 125
   ttc aat ctc gga gcc tac ttt tcc aac aca atc ttc atc gat cga tat     432
   Phe Asn Leu Gly Ala Tyr Phe Ser Asn Thr Ile Phe Ile Asp Arg Tyr
       130                 135                 140
   aac cgt gaa cgt gcg atg gct tca gtt gat tat tgt gca tct gaa atg     480
   Asn Arg Glu Arg Ala Met Ala Ser Val Asp Tyr Cys Ala Ser Glu Met
   145                 150                 155                 160
   aag aac aga aat ctt aaa ctt tgg gta tct ccg gaa gga aca aga aat     528
   Lys Asn Arg Asn Leu Lys Leu Trp Val Ser Pro Glu Gly Thr Arg Asn
                   165                 170                 175
   cgt gaa gga ggg ttc att cca ttc aag aaa gga gca ttc aat att gca     576
   Arg Glu Gly Gly Phe Ile Pro Phe Lys Lys Gly Ala Phe Asn Ile Ala
               180                 185                 190
   gtt cgt gcg cag att ccc att att cca gtt gta ttc tca gac tat cgg     624
   Val Arg Ala Gln Ile Pro Ile Ile Pro Val Val Phe Ser Asp Tyr Arg
           195                 200                 205
   gat ttc tac tca aag cca ggc cga tat ttc aag aat gat gga gaa gtt     672
   Asp Phe Tyr Ser Lys Pro Gly Arg Tyr Phe Lys Asn Asp Gly Glu Val
       210                 215                 220
   gtt att cga gtt ctg gat gcg att cca aca aaa ggg ctc act ctt gat     720
   Val Ile Arg Val Leu Asp Ala Ile Pro Thr Lys Gly Leu Thr Leu Asp
   225                 230                 235                 240
   gac gtc agc gag ttg tct gat atg tgt cgg gac gtt atg ttg gca gcc        768
   Asp Val Ser Glu Leu Ser Asp Met Cys Arg Asp Val Met Leu Ala Ala
                   245                 250                 255
   tat aag gaa gtt act cta gaa gct cag caa cga aat gcg aca cgg cgt        816
   Tyr Lys Glu Val Thr Leu Glu Ala Gln Gln Arg Asn Ala Thr Arg Arg
               260                 265                 270
   gga gaa aca aaa gac ggg aag aaa tct gag taa                            849
   Gly Glu Thr Lys Asp Gly Lys Lys Ser Glu
           275                 280
<210> 4
   <211> 282
   <212> PRT
   <213> Caenorhabditis elegans
<400> 4
   Met Glu Asn Phe Trp Ser Ile Val Val Phe Phe Leu Leu Ser Ile Leu
   1 5 10 15
   Phe Ile Leu Tyr Asn Ile Ser Thr Val Cys His Tyr Tyr Met Arg Ile
   20 25 30
   Ser Phe Tyr Tyr Phe Thr Ile Leu Leu His Gly Met Glu Val Cys Val
   35 40 45
   Thr Met Ile Pro Ser Trp Leu Asn Gly Lys Gly Ala Asp Tyr Val Phe
   50 55 60
   His Ser Phe Phe Tyr Trp Cys Lys Trp Thr Gly Val His Thr Thr Val
   65 70 75 80
   Tyr Gly Tyr Glu Lys Thr Gln Val Glu Gly Pro Ala Val Val Ile Cys
   85 90 95
   Asn His Gln Ser Ser Leu Asp Ile Leu Ser Met Ala Ser Ile Trp Pro
   100 105 110
   Lys Asn Cys Val Val Met Met Lys Arg Ile Leu Ala Tyr Val Pro Phe
   115 120 125
   Phe Asn Leu Gly Ala Tyr Phe Ser Asn Thr Ile Phe Ile Asp Arg Tyr
   130 135 140
   Asn Arg Glu Arg Ala Met Ala Ser Val Asp Tyr Cys Ala Ser Glu Met
   145 150 155 160
   Lys Asn Arg Asn Leu Lys Leu Trp Val Ser Pro Glu Gly Thr Arg Asn
   165 170 175
   Arg Glu Gly Gly Phe Ile Pro Phe Lys Lys Gly Ala Phe Asn Ile Ala
   180 185 190
   Val Arg Ala Gln Ile Pro Ile Ile Pro Val Val Phe Ser Asp Tyr Arg
   195 200 205
   Asp Phe Tyr Ser Lys Pro Gly Arg Tyr Phe Lys Asn Asp Gly Glu Val
   210 215 220
   Val Ile Arg Val Leu Asp Ala Ile Pro Thr Lys Gly Leu Thr Leu Asp
   225 230 235 240
   Asp Val Ser Glu Leu Ser Asp Met Cys Arg Asp Val Met Leu Ala Ala
   245 250 255
   Tyr Lys Glu Val Thr Leu Glu Ala Gln Gln Arg Asn Ala Thr Arg Arg
   260 265 270
   Gly Glu Thr Lys Asp Gly Lys Lys Ser Glu
   275 280
<210> 5
   <211> 849
   <212> DNA
   <213> Caenorhabditis elegans
<220>
   <221> CDS
   <222> (1)..(849)
   <223> Acyl-CoA:Lysophospholipid-Acyltransferase
<400> 5
   atg gag aac ttc tgg tcg atc gtc gtg ttt ttt cta ctc tca att ctc       48
   Met Glu Asn Phe Trp Ser Ile Val Val Phe Phe Leu Leu Ser Ile Leu
   1               5                   10                  15
   ttc att tta tat aac ata tcg aca gta tgc cac tac tat gtg cgg att       96
   Phe Ile Leu Tyr Asn Ile Ser Thr Val Cys His Tyr Tyr Val Arg Ile
               20                  25                  30
   tcg ttt tat tac ttc aca att tta ttg cat gga atg gaa gtt tgt gtt      144
   Ser Phe Tyr Tyr Phe Thr Ile Leu Leu His Gly Met Glu Val Cys Val
           35                  40                  45
   aca atg atc cct tct tgg cta aat ggg aag ggt gct gat tac gtg ttt      192
   Thr Met Ile Pro Ser Trp Leu Asn Gly Lys Gly Ala Asp Tyr Val Phe
       50                  55                  60
   cac tcg ttt ttc tat tgg tgt aaa tgg act ggt gtt cat aca aca gtc      240
   His Ser Phe Phe Tyr Trp Cys Lys Trp Thr Gly Val His Thr Thr Val
   65                  70                  75                  80
   tat gga tat gaa aaa aca caa gtt gaa ggt ccg gct gta gtt att tgt      288
   Tyr Gly Tyr Glu Lys Thr Gln Val Glu Gly Pro Ala Val Val Ile Cys
                   85                  90                  95
   aat cat cag agt tct ctc gac att cta tcg atg gca tca atc tgg ccg      336
   Asn His Gln Ser Ser Leu Asp Ile Leu Ser Met Ala Ser Ile Trp Pro
               100                 105                 110
   aag aat tgt gtt gta atg atg aaa cga att ctt gcc tat gtt cca ttc      384
   Lys Asn Cys Val Val Met Met Lys Arg Ile Leu Ala Tyr Val Pro Phe
           115                 120                 125
   ttc aat ctc gga gcc tac ttt tcc aac aca atc ttc atc gat cga tat      432
   Phe Asn Leu Gly Ala Tyr Phe Ser Asn Thr Ile Phe Ile Asp Arg Tyr
       130                 135                 140
   aac cgt gaa cgt gcg atg gct tca gtt gat tat tgt gca tct gaa atg      480
   Asn Arg Glu Arg Ala Met Ala Ser Val Asp Tyr Cys Ala Ser Glu Met
   145                 150                 155                 160
   aag aac aga aat ctt aaa ctt tgg gta ttt ccg gaa gga aca aga aat      528
   Lys Asn Arg Asn Leu Lys Leu Trp Val Phe Pro Glu Gly Thr Arg Asn
                   165                 170                 175
   cgt gaa gga ggg ttc att cca ttc aag aaa gga gca ttc aat att gca      576
   Arg Glu Gly Gly Phe Ile Pro Phe Lys Lys Gly Ala Phe Asn Ile Ala
               180                 185                 190
   gtt cgt gcg cag att ccc att att cca gtt gta ttc tca gac tat cgg      624
   Val Arg Ala Gln Ile Pro Ile Ile Pro Val Val Phe Ser Asp Tyr Arg
           195                 200                 205
   gat ttc tac tca aag cca ggc cga tat ttc aag aat gat gga gaa gtt      672
   Asp Phe Tyr Ser Lys Pro Gly Arg Tyr Phe Lys Asn Asp Gly Glu Val
       210                 215                 220
   gtt att cga gtt ctg gat gcg att cca aca aaa ggg ctc act ctt gat      720
   Val Ile Arg Val Leu Asp Ala Ile Pro Thr Lys Gly Leu Thr Leu Asp
   225                 230                 235                 240
   gac gtc agc gag ttg tct gat atg tgt cgg gac gtt atg ttg gca gcc      768
   Asp Val Ser Glu Leu Ser Asp Met Cys Arg Asp Val Met Leu Ala Ala
                   245                 250                 255
   tat aag gaa gtt act cta gaa gct cag caa cga aat gcg aca cgg cgt      816
   Tyr Lys Glu Val Thr Leu Glu Ala Gln Gln Arg Asn Ala Thr Arg Arg
               260                 265                 270
   gga gaa aca aaa gac ggg aag aaa tct gag taa                          849
   Gly Glu Thr Lys Asp Gly Lys Lys Ser Glu
           275                 280
<210> 6
   <211> 282
   <212> PRT
   <213> Caenorhabditis elegans
<400> 6
   Met Glu Asn Phe Trp Ser Ile Val Val Phe Phe Leu Leu Ser Ile Leu
   1 5 10 15
   Phe Ile Leu Tyr Asn Ile Ser Thr Val Cys His Tyr Tyr Val Arg Ile
   20 25 30
   Ser Phe Tyr Tyr Phe Thr Ile Leu Leu His Gly Met Glu Val Cys Val
   35 40 45
   Thr Met Ile Pro Ser Trp Leu Asn Gly Lys Gly Ala Asp Tyr Val Phe
   50 55 60
   His Ser Phe Phe Tyr Trp Cys Lys Trp Thr Gly Val His Thr Thr Val
   65 70 75 80
   Tyr Gly Tyr Glu Lys Thr Gln Val Glu Gly Pro Ala Val Val Ile Cys
   85 90 95
   Asn His Gln Ser Ser Leu Asp Ile Leu Ser Met Ala Ser Ile Trp Pro
   100 105 110
   Lys Asn Cys Val Val Met Met Lys Arg Ile Leu Ala Tyr Val Pro Phe
   115 120 125
   Phe Asn Leu Gly Ala Tyr Phe Ser Asn Thr Ile Phe Ile Asp Arg Tyr
   130 135 140
   Asn Arg Glu Arg Ala Met Ala Ser Val Asp Tyr Cys Ala Ser Glu Met
   145 150 155 160
   Lys Asn Arg Asn Leu Lys Leu Trp Val Phe Pro Glu Gly Thr Arg Asn
   165 170 175
   Arg Glu Gly Gly Phe Ile Pro Phe Lys Lys Gly Ala Phe Asn Ile Ala
   180 185 190
   Val Arg Ala Gln Ile Pro Ile Ile Pro Val Val Phe Ser Asp Tyr Arg
   195 200 205
   Asp Phe Tyr Ser Lys Pro Gly Arg Tyr Phe Lys Asn Asp Gly Glu Val
   210 215 220
   Val Ile Arg Val Leu Asp Ala Ile Pro Thr Lys Gly Leu Thr Leu Asp
   225 230 235 240
   Asp Val Ser Glu Leu Ser Asp Met Cys Arg Asp Val Met Leu Ala Ala
   245 250 255
   Tyr Lys Glu Val Thr Leu Glu Ala Gln Gln Arg Asn Ala Thr Arg Arg
   260 265 270
   Gly Glu Thr Lys Asp Gly Lys Lys Ser Glu
   275 280
<210> 7
   <211> 849
   <212> DNA
   <213> Caenorhabditis elegans
<220>
   <221> CDS
   <222> (1)..(849)
   <223> Acyl-CoA:Lysophospholipid-Acyltransferase
<400> 7
   atg gag aac ttc tgg tcg atc gtc gtg ttt ttt cta ctc tca att ctc      48
   Met Glu Asn Phe Trp Ser Ile Val Val Phe Phe Leu Leu Ser Ile Leu
   1               5                   10                  15
   ttc att tta tat aac ata tcg aca gta tgc cac tac tat atg cgg att      96
   Phe Ile Leu Tyr Asn Ile Ser Thr Val Cys His Tyr Tyr Met Arg Ile
               20                  25                  30
   tcg ttt tat tac ttc aca att tta ttg cat gga atg gaa gtt tgt gtt      144
   Ser Phe Tyr Tyr Phe Thr Ile Leu Leu His Gly Met Glu Val Cys Val
           35                  40                  45
   aca atg atc cct tct tgg cta aat ggg aag ggt gct gat tac gtg ttt      192
   Thr Met Ile Pro Ser Trp Leu Asn Gly Lys Gly Ala Asp Tyr Val Phe
       50                  55                  60
   cac tcg ttt ttc tat tgg tgt aaa tgg act ggt gtt cat aca aca gtc      240
   His Ser Phe Phe Tyr Trp Cys Lys Trp Thr Gly Val His Thr Thr Val
   65                  70                  75                  80
   tat gga tat gaa aaa aca caa gtt gaa ggt ccg gcc gta gtt att tgt      288
   Tyr Gly Tyr Glu Lys Thr Gln Val Glu Gly Pro Ala Val Val Ile Cys
                   85                  90                  95
   aat cat cag ggt tct ctc gac att cta tcg atg gca tca atc tgg ccg      336
   Asn His Gln Gly Ser Leu Asp Ile Leu Ser Met Ala Ser Ile Trp Pro
               100                 105                 110
   aag aat tgt gtt gta atg atg aaa cga att ctt gcc tat gtt cca ttc      384
   Lys Asn Cys Val Val Met Met Lys Arg Ile Leu Ala Tyr Val Pro Phe
           115                 120                 125
   ttc aat ctc gga gcc tac ttt tcc aac aca atc ttc atc gat cga tat      432
   Phe Asn Leu Gly Ala Tyr Phe Ser Asn Thr Ile Phe Ile Asp Arg Tyr
       130                 135                 140
   aac cgt gaa cgt gcg atg gct tca gtt gat tat tgt gca tct gaa atg      480
   Asn Arg Glu Arg Ala Met Ala Ser Val Asp Tyr Cys Ala Ser Glu Met
   145                 150                 155                 160
   aag aac aga aat ctt aaa ctt tgg gta ttt ccg gaa gga aca aga aat      528
   Lys Asn Arg Asn Leu Lys Leu Trp Val Phe Pro Glu Gly Thr Arg Asn
                   165                 170                 175
   cgt gaa gga ggg ttc att cca ttc aag aaa gga gca ttc aat att gca      576
   Arg Glu Gly Gly Phe Ile Pro Phe Lys Lys Gly Ala Phe Asn Ile Ala
               180                 185                 190
   gtt cgt gcg cag att ccc att att cca gtt gta ttc tca gac tat cgg      624
   Val Arg Ala Gln Ile Pro Ile Ile Pro Val Val Phe Ser Asp Tyr Arg
           195                 200                 205
   gat ttc tac tca aag cca ggc cga tat ttc aag aat gat gga gaa gtt      672
   Asp Phe Tyr Ser Lys Pro Gly Arg Tyr Phe Lys Asn Asp Gly Glu Val
       210                 215                 220
   gtt att cga gtt ctg gat gcg att cca aca aaa ggg ctc act ctt gat      720
   Val Ile Arg Val Leu Asp Ala Ile Pro Thr Lys Gly Leu Thr Leu Asp
   225                 230                 235                 240
   gac gtc agc gag ttg tct gat atg tgt cgg gac gtt atg ttg gca gcc      768
   Asp Val Ser Glu Leu Ser Asp Met Cys Arg Asp Val Met Leu Ala Ala
                   245                 250                 255
   tat aag gaa gtt act cta gaa gct cag caa cga aat gcg aca cgg cgt      816
   Tyr Lys Glu Val Thr Leu Glu Ala Gln Gln Arg Asn Ala Thr Arg Arg
               260                 265                 270
   gga gaa aca aaa gac ggg aag aaa tct gag taa                          849
   Gly Glu Thr Lys Asp Gly Lys Lys Ser Glu
           275                 280
<210> 8
   <211> 282
   <212> PRT
   <213> Caenorhabditis elegans
<400> 8
   Met Glu Asn Phe Trp Ser Ile Val Val Phe Phe Leu Leu Ser Ile Leu
   1 5 10 15
   Phe Ile Leu Tyr Asn Ile Ser Thr Val Cys His Tyr Tyr Met Arg Ile
   20 25 30
   Ser Phe Tyr Tyr Phe Thr Ile Leu Leu His Gly Met Glu Val Cys Val
   35 40 45
   Thr Met Ile Pro Ser Trp Leu Asn Gly Lys Gly Ala Asp Tyr Val Phe
   50 55 60
   His Ser Phe Phe Tyr Trp Cys Lys Trp Thr Gly Val His Thr Thr Val
   65 70 75 80
   Tyr Gly Tyr Glu Lys Thr Gln Val Glu Gly Pro Ala Val Val Ile Cys
   85 90 95
   Asn His Gln Gly Ser Leu Asp Ile Leu Ser Met Ala Ser Ile Trp Pro
               100                 105                 110
   Lys Asn Cys Val Val Met Met Lys Arg Ile Leu Ala Tyr Val Pro Phe
           115                 120                 125
   Phe Asn Leu Gly Ala Tyr Phe Ser Asn Thr Ile Phe Ile Asp Arg Tyr
       130                 135                 140
   Asn Arg Glu Arg Ala Met Ala Ser Val Asp Tyr Cys Ala Ser Glu Met
   145                 150                 155                 160
   Lys Asn Arg Asn Leu Lys Leu Trp Val Phe Pro Glu Gly Thr Arg Asn
                   165                 170                 175
   Arg Glu Gly Gly Phe Ile Pro Phe Lys Lys Gly Ala Phe Asn Ile Ala
               180                 185                 190
   Val Arg Ala Gln Ile Pro Ile Ile Pro Val Val Phe Ser Asp Tyr Arg
           195                 200                 205
   Asp Phe Tyr Ser Lys Pro Gly Arg Tyr Phe Lys Asn Asp Gly Glu Val
       210                 215                 220
   Val Ile Arg Val Leu Asp Ala Ile Pro Thr Lys Gly Leu Thr Leu Asp
   225                 230                 235                 240
   Asp Val Ser Glu Leu Ser Asp Met Cys Arg Asp Val Met Leu Ala Ala
                   245                 250                 255
   Tyr Lys Glu Val Thr Leu Glu Ala Gln Gln Arg Asn Ala Thr Arg Arg
               260                 265                 270
   Gly Glu Thr Lys Asp Gly Lys Lys Ser Glu
           275                 280
<210> 9
   <211> 1578
   <212> DNA
   <213> Physcomitrella patens
<220>
   <221> CDS
   <222> (1)..(1578)
   <223> Delta-6-Desaturase
<400> 9
   atg gta ttc gcg ggc ggt gga ctt cag cag ggc tct ctc gaa gaa aac 48
   Met Val Phe Ala Gly Gly Gly Leu Gln Gln Gly Ser Leu Glu Glu Asn
   1               5                   10                  15
   atc gac gtc gag cac att gcc agt atg tct ctc ttc agc gac ttc ttc      96
   Ile Asp Val Glu His Ile Ala Ser Met Ser Leu Phe Ser Asp Phe Phe
               20                  25                  30
   agt tat gtg tct tca act gtt ggt tcg tgg agc gta cac agt ata caa     144
   Ser Tyr Val Ser Ser Thr Val Gly Ser Trp Ser Val His Ser Ile Gln
           35                  40                  45
   cct ttg aag cgc ctg acg agt aag aag cgt gtt tcg gaa agc gct gcc     192
   Pro Leu Lys Arg Leu Thr Ser Lys Lys Arg Val Ser Glu Ser Ala Ala
       50                  55                  60
   gtg caa tgt ata tca gct gaa gtt cag aga aat tcg agt acc cag gga     240
   Val Gln Cys Ile Ser Ala Glu Val Gln Arg Asn Ser Ser Thr Gln Gly
   65                  70                  75                  80
   act gcg gag gca ctc gca gaa tca gtc gtg aag ccc acg aga cga agg     288
   Thr Ala Glu Ala Leu Ala Glu Ser Val Val Lys Pro Thr Arg Arg Arg
                   85                  90                  95
   tca tct cag tgg aag aag tcg aca cac ccc cta tca gaa gta gca gta     336
   Ser Ser Gln Trp Lys Lys Ser Thr His Pro Leu Ser Glu Val Ala Val
               100                 105                 110
   cac aac aag cca agc gat tgc tgg att gtt gta aaa aac aag gtg tat     384
   His Asn Lys Pro Ser Asp Cys Trp Ile Val Val Lys Asn Lys Val Tyr
           115                 120                 125
   gat gtt tcc aat ttt gcg gac gag cat ccc gga gga tca gtt att agt     432
   Asp Val Ser Asn Phe Ala Asp Glu His Pro Gly Gly Ser Val Ile Ser
       130                 135                 140
   act tat ttt gga cga gac ggc aca gat gtt ttc tct agt ttt cat gca     480
   Thr Tyr Phe Gly Arg Asp Gly Thr Asp Val Phe Ser Ser Phe His Ala
   145                 150                 155                 160
   gct tct aca tgg aaa att ctt caa gac ttt tac att ggt gac gtg gag     528
   Ala Ser Thr Trp Lys Ile Leu Gln Asp Phe Tyr Ile Gly Asp Val Glu
                   165                 170                 175
   agg gtg gag ccg act cca gag ctg ctg aaa gat ttc cga gaa atg aga     576
   Arg Val Glu Pro Thr Pro Glu Leu Leu Lys Asp Phe Arg Glu Met Arg
               180                 185                 190
   gct ctt ttc ctg agg gag caa ctt ttc aaa agt tcg aaa ttg tac tat     624
   Ala Leu Phe Leu Arg Glu Gln Leu Phe Lys Ser Ser Lys Leu Tyr Tyr
           195                 200                 205
   gtt atg aag ctg ctc acg aat gtt gct att ttt gct gcg agc att gca     672
   Val Met Lys Leu Leu Thr Asn Val Ala Ile Phe Ala Ala Ser Ile Ala
       210                 215                 220
   ata ata tgt tgg agc aag act att tca gcg gtt ttg gct tca gct tgt     720
   Ile Ile Cys Trp Ser Lys Thr Ile Ser Ala Val Leu Ala Ser Ala Cys
   225                 230                 235                 240
   atg atg gct ctg tgt ttc caa cag tgc gga tgg cta tcc cat gat ttt     768
   Met Met Ala Leu Cys Phe Gln Gln Cys Gly Trp Leu Ser His Asp Phe
                   245                 250                 255
   ctc cac aat cag gtg ttt gag aca cgc tgg ctt aat gaa gtt gtc ggg     816
   Leu His Asn Gln Val Phe Glu Thr Arg Trp Leu Asn Glu Val Val Gly
               260                 265                 270
   tat gtg atc ggc aac gcc gtt ctg ggg ttt agt aca ggg tgg tgg aag     864
   Tyr Val Ile Gly Asn Ala Val Leu Gly Phe Ser Thr Gly Trp Trp Lys
           275                 280                 285
   gag aag cat aac ctt cat cat gct gct cca aat gaa tgc gat cag act     912
   Glu Lys His Asn Leu His His Ala Ala Pro Asn Glu Cys Asp Gln Thr
       290                 295                 300
   tac caa cca att gat gaa gat att gat act ctc ccc ctc att gcc tgg     960
   Tyr Gln Pro Ile Asp Glu Asp Ile Asp Thr Leu Pro Leu Ile Ala Trp
   305                 310                 315                 320
   agc aag gac ata ctg gcc aca gtt gag aat aag aca ttc ttg cga atc    1008
   Ser Lys Asp Ile Leu Ala Thr Val Glu Asn Lys Thr Phe Leu Arg Ile
                   325                 330                 335
   ctc caa tac cag cat ctg ttc ttc atg ggt ctg tta ttt ttc gcc cgt    1056
   Leu Gln Tyr Gln His Leu Phe Phe Met Gly Leu Leu Phe Phe Ala Arg
               340                 345                 350
   ggt agt tgg ctc ttt tgg agc tgg aga tat acc tct aca gca gtg ctc    1104
   Gly Ser Trp Leu Phe Trp Ser Trp Arg Tyr Thr Ser Thr Ala Val Leu
           355                 360                 365
   tca cct gtc gac agg ttg ttg gag aag gga act gtt ctg ttt cac tac    1152
   Ser Pro Val Asp Arg Leu Leu Glu Lys Gly Thr Val Leu Phe His Tyr
       370                 375                 380
   ttt tgg ttc gtc ggg aca gcg tgc tat ctt ctc cct ggt tgg aag cca    1200
   Phe Trp Phe Val Gly Thr Ala Cys Tyr Leu Leu Pro Gly Trp Lys Pro
   385                 390                 395                 400
   tta gta tgg atg gcg gtg act gag ctc atg tcc ggc atg ctg ctg ggc    1248
   Leu Val Trp Met Ala Val Thr Glu Leu Met Ser Gly Met Leu Leu Gly
                   405                 410                 415
   ttt gta ttt gta ctt agc cac aat ggg atg gag gtt tat aat tcg tct    1296
   Phe Val Phe Val Leu Ser His Asn Gly Met Glu Val Tyr Asn Ser Ser
               420                 425                 430
   aaa gaa ttc gtg agt gca cag atc gta tcc aca cgg gat atc aaa gga    1344
   Lys Glu Phe Val Ser Ala Gln Ile Val Ser Thr Arg Asp Ile Lys Gly
           435                 440                 445
   aac ata ttc aac gac tgg ttc act ggt ggc ctt aac agg caa ata gag    1392
   Asn Ile Phe Asn Asp Trp Phe Thr Gly Gly Leu Asn Arg Gln Ile Glu
       450                 455                 460
   cat cat ctt ttc cca aca atg ccc agg cat aat tta aac aaa ata gca    1440
   His His Leu Phe Pro Thr Met Pro Arg His Asn Leu Asn Lys Ile Ala
   465                 470                 475                 480
   cct aga gtg gag gtg ttc tgt aag aaa cac ggt ctg gtg tac gaa gac    1488
   Pro Arg Val Glu Val Phe Cys Lys Lys His Gly Leu Val Tyr Glu Asp
                   485                 490                 495
   gta tct att gct acc ggc act tgc aag gtt ttg aaa gca ttg aag gaa    1536
   Val Ser Ile Ala Thr Gly Thr Cys Lys Val Leu Lys Ala Leu Lys Glu
   500 505 510
   gtc gcg gag gct gcg gca gag cag cat gct acc acc agt taa 1578
   Val Ala Glu Ala Ala Ala Glu Gln His Ala Thr Thr Ser
   515 520 525
<210> 10
   <211> 525
   <212> PRT
   <213> Physcomitrella patens'
<400> 10
   Met Val Phe Ala Gly Gly Gly Leu Gln Gln Gly Ser Leu Glu Glu Asn
   1 5 10 15
   Ile Asp Val Glu His Ile Ala Ser Met Ser Leu Phe Ser Asp Phe Phe
   20 25 30
   Ser Tyr Val Ser Ser Thr Val Gly Ser Trp Ser Val His Ser Ile Gln
   35 40 45
   Pro Leu Lys Arg Leu Thr Ser Lys Lys Arg Val Ser Glu Ser Ala Ala
   50 55 60
   Val Gln Cys Ile Ser Ala Glu Val Gln Arg Asn Ser Ser Thr Gln Gly
   65 70 75 80
   Thr Ala Glu Ala Leu Ala Glu Ser Val Val Lys Pro Thr Arg Arg Arg
   85 90 95
   Ser Ser Gln Trp Lys Lys Ser Thr His Pro Leu Ser Glu Val Ala Val
   100 105 110
   His Asn Lys Pro Ser Asp Cys Trp Ile Val Val Lys Asn Lys Val Tyr
   115 120 125
   Asp Val Ser Asn Phe Ala Asp Glu His Pro Gly Gly Ser Val Ile Ser
   130 135 140
   Thr Tyr Phe Gly Arg Asp Gly Thr Asp Val Phe Ser Ser Phe His Ala
   145 150 155 160
   Ala Ser Thr Trp Lys Ile Leu Gln Asp Phe Tyr Ile Gly Asp Val Glu
   165 170 175
   Arg Val Glu Pro Thr Pro Glu Leu Leu Lys Asp Phe Arg Glu Met Arg
   180 185 190
   Ala Leu Phe Leu Arg Glu Gln Leu Phe Lys Ser Ser Lys Leu Tyr Tyr
           195                 200                 205
   Val Met Lys Leu Leu Thr Asn Val Ala Ile Phe Ala Ala Ser Ile Ala
       210                 215                 220
   Ile Ile Cys Trp Ser Lys Thr Ile Ser Ala Val Leu Ala Ser Ala Cys
   225                 230                 235                 240
   Met Met Ala Leu Cys Phe Gln Gln Cys Gly Trp Leu Ser His Asp Phe
                   245                 250                 255
   Leu His Asn Gln Val Phe Glu Thr Arg Trp Leu Asn Glu Val Val Gly
               260                 265                 270
   Tyr Val Ile Gly Asn Ala Val Leu Gly Phe Ser Thr Gly Trp Trp Lys
           275                 280                 285
   Glu Lys His Asn Leu His His Ala Ala Pro Asn Glu Cys Asp Gln Thr
       290                 295                 300
   Tyr Gln Pro Ile Asp Glu Asp Ile Asp Thr Leu Pro Leu Ile Ala Trp
   305                 310                 315                 320
   Ser Lys Asp Ile Leu Ala Thr Val Glu Asn Lys Thr Phe Leu Arg Ile
                   325                 330                 335
   Leu Gln Tyr Gln His Leu Phe Phe Met Gly Leu Leu Phe Phe Ala Arg
               340                 345                 350
   Gly Ser Trp Leu Phe Trp Ser Trp Arg Tyr Thr Ser Thr Ala Val Leu
           355                 360                 365
   Ser Pro Val Asp Arg Leu Leu Glu Lys Gly Thr Val Leu Phe His Tyr
       370                 375                 380
   Phe Trp Phe Val Gly Thr Ala Cys Tyr Leu Leu Pro Gly Trp Lys Pro
   385                 390                 395                 400
   Leu Val Trp Met Ala Val Thr Glu Leu Met Ser Gly Met Leu Leu Gly
                   405                 410                 415
   Phe Val Phe Val Leu Ser His Asn Gly Met Glu Val Tyr Asn Ser Ser
               420                 425                 430
   Lys Glu Phe Val Ser Ala Gln Ile Val Ser Thr Arg Asp Ile Lys Gly
           435                 440                 445
   Asn Ile Phe Asn Asp Trp Phe Thr Gly Gly Leu Asn Arg Gln Ile Glu
   450 455 460
   His His Leu Phe Pro Thr Met Pro Arg His Asn Leu Asn Lys Ile Ala
   465 470 475 480
   Pro Arg Val Glu Val Phe Cys Lys Lys His Gly Leu Val Tyr Glu Asp
   485 490 495
   Val Ser Ile Ala Thr Gly Thr Cys Lys Val Leu Lys Ala Leu Lys Glu
   500 505 510
   Val Ala Glu Ala Ala Ala Glu Gln His Ala Thr Thr Ser
   515 520 525
<210> 11
   <211> 1192
   <212> DNA
   <213> Physcomitrella patens
<220>
   <221> CDS
   <222> (58)..(930)
   <223> Delta-6-Elongase
<400> 11
   ctgcttcgtc tcatcttggg ggtgtgattc gggagtgggt tgagttggtg gagcgca        57
   atg gag gtc gtg gag aga ttc tac ggt gag ttg gat ggg aag gtc tcg       105
   Met Glu Val Val Glu Arg Phe Tyr Gly Glu Leu Asp Gly Lys Val Ser
   1               5                   10                  15
   cag ggc gtg aat gca ttg ctg ggt agt ttt ggg gtg gag ttg acg gat       153
   Gln Gly Val Asn Ala Leu Leu Gly Ser Phe Gly Val Glu Leu Thr Asp
               20                  25                  30
   acg ccc act acc aaa ggc ttg ccc ctc gtt gac agt ccc aca ccc atc       201
   Thr Pro Thr Thr Lys Gly Leu Pro Leu Val Asp Ser Pro Thr Pro Ile
           35                  40                  45
   gtc ctc ggt gtt tct gta tac ttg act att gtc att gga ggg ctt ttg       249
   Val Leu Gly Val Ser Val Tyr Leu Thr Ile Val Ile Gly Gly Leu Leu
       50                  55                  60
   tgg ata aag gcc agg gat ctg aaa ccg cgc gcc tcg gag cca ttt ttg       297
   Trp Ile Lys Ala Arg Asp Leu Lys Pro Arg Ala Ser Glu Pro Phe Leu
   65                  70                  75                  80
   ctc caa gct ttg gtg ctt gtg cac aac ctg ttc tgt ttt gcg ctc agt       345
   Leu Gln Ala Leu Val Leu Val His Asn Leu Phe Cys Phe Ala Leu Ser
                   85                  90                  95
   ctg tat atg tgc gtg ggc atc gct tat cag gct att acc tgg cgg tac       393
   Leu Tyr Met Cys Val Gly Ile Ala Tyr Gln Ala Ile Thr Trp Arg Tyr
               100                 105                 110
   tct ctc tgg ggc aat gca tac aat cct aaa cat aaa gag atg gcg att      441
   Ser Leu Trp Gly Asn Ala Tyr Asn Pro Lys His Lys Glu Met Ala Ile
           115                 120                 125
   ctg gta tac ttg ttc tac atg tct aag tac gtg gaa ttc atg gat acc      489
   Leu Val Tyr Leu Phe Tyr Met Ser Lys Tyr Val Glu Phe Met Asp Thr
       130                 135                 140
   gtt atc atg ata ctg aag cgc agc acc agg caa ata agc ttc ctc cac      537
   Val Ile Met Ile Leu Lys Arg Ser Thr Arg Gln Ile Ser Phe Leu His
   145                 150                 155                 160
   gtt tat cat cat tct tca att tcc ctc att tgg tgg gct att gct cat      585
   Val Tyr His His Ser Ser Ile Ser Leu Ile Trp Trp Ala Ile Ala His
                   165                 170                 175
   cac gct cct ggc ggt gaa gca tat tgg tct gcg gct ctg aac tca gga      633
   His Ala Pro Gly Gly Glu Ala Tyr Trp Ser Ala Ala Leu Asn Ser Gly
               180                 185                 190
   gtg cat gtt ctc atg tat gcg tat tac ttc ttg gct gcc tgc ctt cga      681
   Val His Val Leu Met Tyr Ala Tyr Tyr Phe Leu Ala Ala Cys Leu Arg
           195                 200                 205
   agt agc cca aag tta aaa aat aag tac ctt ttt tgg ggc agg tac ttg      729
   Ser Ser Pro Lys Leu Lys Asn Lys Tyr Leu Phe Trp Gly Arg Tyr Leu
       210                 215                 220
   aca caa ttc caa atg ttc cag ttt atg ctg aac tta gtg cag gct tac      777
   Thr Gln Phe Gln Met Phe Gln Phe Met Leu Asn Leu Val Gln Ala Tyr
   225                 230                 235                 240
   tac gac atg aaa acg aat gcg cca tat cca caa tgg ctg atc aag att      825
   Tyr Asp Met Lys Thr Asn Ala Pro Tyr Pro Gln Trp Leu Ile Lys Ile
                   245                 250                 255
   ttg ttc tac tac atg atc tcg ttg ctg ttt ctt ttc ggc aat ttt tac      873
   Leu Phe Tyr Tyr Met Ile Ser Leu Leu Phe Leu Phe Gly Asn Phe Tyr
               260                 265                 270
   gta caa aaa tac atc aaa ccc tct gac gga aag caa aag gga gct aaa      921
   Val Gln Lys Tyr Ile Lys Pro Ser Asp Gly Lys Gln Lys Gly Ala Lys
           275                 280                 285
   act gag tga gctgtatcaa gccatagaaa ctctattatg ttagaacctg              970
   Thr Glu
       290
   aagttggtgc tttcttatct ccacttatct tttaagcagc atcagttttg aaatgatgtg   1030
   tgggcgtggt ctgcaagtag tcatcaatat aatcggcctg agcacttcag atggattgtt   1090
   agaacatgag taaaagcggt tattacggtg tttattttgt accaaatcac cgcacgggtg   1150
   aattgaaata tttcagattt gatcaatttc atctgaaaaa aa                      1192
<210> 12
   <211> 290
   <212> PRT
   <213> Physcomitrella patens
<400> 12
   Met Glu Val Val Glu Arg Phe Tyr Gly Glu Leu Asp Gly Lys Val Ser
   1               5                   10                  15
   Gln Gly Val Asn Ala Leu Leu Gly Ser Phe Gly Val Glu Leu Thr Asp
               20                  25                  30
   Thr Pro Thr Thr Lys Gly Leu Pro Leu Val Asp Ser Pro Thr Pro Ile
           35                  40                  45
   Val Leu Gly Val Ser Val Tyr Leu Thr Ile Val Ile Gly Gly Leu Leu
       50                  55                  60
   Trp Ile Lys Ala Arg Asp Leu Lys Pro Arg Ala Ser Glu Pro Phe Leu
   65                  70                  75                  80
   Leu Gln Ala Leu Val Leu Val His Asn Leu Phe Cys Phe Ala Leu Ser
                   85                  90                  95
   Leu Tyr Met Cys Val Gly Ile Ala Tyr Gln Ala Ile Thr Trp Arg Tyr
               100                 105                 110
   Ser Leu Trp Gly Asn Ala Tyr Asn Pro Lys His Lys Glu Met Ala Ile
           115                 120                 125
   Leu Val Tyr Leu Phe Tyr Met Ser Lys Tyr Val Glu Phe Met Asp Thr
       130                 135                 140
   Val Ile Met Ile Leu Lys Arg Ser Thr Arg Gln Ile Ser Phe Leu His
   145                 150                 155                 160
   Val Tyr His His Ser Ser Ile Ser Leu Ile Trp Trp Ala Ile Ala His
                   165                 170                 175
   His Ala Pro Gly Gly Glu Ala Tyr Trp Ser Ala Ala Leu Asn Ser Gly
               180                 185                 190
   Val His Val Leu Met Tyr Ala Tyr Tyr Phe Leu Ala Ala Cys Leu Arg
           195                 200                 205
   Ser Ser Pro Lys Leu Lys Asn Lys Tyr Leu Phe Trp Gly Arg Tyr Leu
       210                 215                 220
   Thr Gln Phe Gln Met Phe Gln Phe Met Leu Asn Leu Val Gln Ala Tyr
   225                 230                 235                 240
   Tyr Asp Met Lys Thr Asn Ala Pro Tyr Pro Gln Trp Leu Ile Lys Ile
                   245                 250                 255
   Leu Phe Tyr Tyr Met Ile Ser Leu Leu Phe Leu Phe Gly Asn Phe Tyr
               260                 265                 270
   Val Gln Lys Tyr Ile Lys Pro Ser Asp Gly Lys Gln Lys Gly Ala Lys
           275                 280                 285
   Thr Glu
       290
<210> 13
   <211> 1410
   <212> DNA
   <213> Phaeodactylum tricornutum
<220>
   <221> CDS
   <222> (1)..(1410)
   <223> Delta-5-Desaturase
<400> 13
   atg gct ccg gat gcg gat aag ctt cga caa cgc cag acg act gcg gta      48
   Met Ala Pro Asp Ala Asp Lys Leu Arg Gln Arg Gln Thr Thr Ala Val
   1               5                   10                  15
   gcg aag cac aat gct gct acc ata tcg acg cag gaa cgc ctt tgc agt      96
   Ala Lys His Asn Ala Ala Thr Ile Ser Thr Gln Glu Arg Leu Cys Ser
               20                  25                  30
   ctg tct tcg ctc aaa ggc gaa gaa gtc tgc atc gac gga atc atc tat     144
   Leu Ser Ser Leu Lys Gly Glu Glu Val Cys Ile Asp Gly Ile Ile Tyr
           35                  40                  45
   gac ctc caa tca ttc gat cat ccc ggg ggt gaa acg atc aaa atg ttt     192
   Asp Leu Gln Ser Phe Asp His Pro Gly Gly Glu Thr Ile Lys Met Phe
       50                  55                  60
   ggt ggc aac gat gtc act gta cag tac aag atg att cac ccg tac cat     240
   Gly Gly Asn Asp Val Thr Val Gln Tyr Lys Met Ile His Pro Tyr His
   65                  70                  75                  80
   acc gag aag cat ttg gaa aag atg aag cgt gtc ggc aag gtg acg gat     288
   Thr Glu Lys His Leu Glu Lys Met Lys Arg Val Gly Lys Val Thr Asp
                   85                  90                  95
   ttc gtc tgc gag tac aag ttc gat acc gaa ttt gaa cgc gaa atc aaa     336
   Phe Val Cys Glu Tyr Lys Phe Asp Thr Glu Phe Glu Arg Glu Ile Lys
               100                 105                 110
   cga gaa gtc ttc aag att gtg cga cga ggc aag gat ttc ggt act ttg     384
   Arg Glu Val Phe Lys Ile Val Arg Arg Gly Lys Asp Phe Gly Thr Leu
           115                 120                 125
   gga tgg ttc ttc cgt gcg ttt tgc tac att gcc att ttc ttc tac ctg      432
   Gly Trp Phe Phe Arg Ala Phe Cys Tyr Ile Ala Ile Phe Phe Tyr Leu
       130                 135                 140
   cag tac cat tgg gtc acc acg gga acc tct tgg ctg ctg gcc gtg gcc      480
   Gln Tyr His Trp Val Thr Thr Gly Thr Ser Trp Leu Leu Ala Val Ala
   145                 150                 155                 160
   tac gga atc tcc caa gcg atg att ggc atg aat gtc cag cac gat gcc      528
   Tyr Gly Ile Ser Gln Ala Met Ile Gly Met Asn Val Gln His Asp Ala
                   165                 170                 175
   aac cac ggg gcc acc tcc aag cgt ccc tgg gtc aac gac atg cta ggc      576
   Asn His Gly Ala Thr Ser Lys Arg Pro Trp Val Asn Asp Met Leu Gly
               180                 185                 190
   ctc ggt gcg gat ttt att ggt ggt tcc aag tgg ctc tgg cag gaa caa      624
   Leu Gly Ala Asp Phe Ile Gly Gly Ser Lys Trp Leu Trp Gln Glu Gln
           195                 200                 205
   cac tgg acc cac cac gct tac acc aat cac gcc gag atg gat ccc gat      672
   His Trp Thr His His Ala Tyr Thr Asn His Ala Glu Met Asp Pro Asp
       210                 215                 220
   agc ttt ggt gcc gaa cca atg ctc cta ttc aac gac tat ccc ttg gat      720
   Ser Phe Gly Ala Glu Pro Met Leu Leu Phe Asn Asp Tyr Pro Leu Asp
   225                 230                 235                 240
   cat ccc gct cgt acc tgg cta cat cgc ttt caa gca ttc ttt tac atg      768
   His Pro Ala Arg Thr Trp Leu His Arg Phe Gln Ala Phe Phe Tyr Met
                   245                 250                 255
   ccc gtc ttg gct gga tac tgg ttg tcc gct gtc ttc aat cca caa att      816
   Pro Val Leu Ala Gly Tyr Trp Leu Ser Ala Val Phe Asn Pro Gln Ile
               260                 265                 270
   ctt gac ctc cag caa cgc ggc gca ctt tcc gtc ggt atc cgt ctc gac      864
   Leu Asp Leu Gln Gln Arg Gly Ala Leu Ser Val Gly Ile Arg Leu Asp
           275                 280                 285
   aac gct ttc att cac tcg cga cgc aag tat gcg gtt ttc tgg cgg gct      912
   Asn Ala Phe Ile His Ser Arg Arg Lys Tyr Ala Val Phe Trp Arg Ala
       290                 295                 300
   gtg tac att gcg gtg aac gtg att gct ccg ttt tac aca aac tcc ggc      960
   Val Tyr Ile Ala Val Asn Val Ile Ala Pro Phe Tyr Thr Asn Ser Gly
   305                 310                 315                 320
   ctc gaa tgg tcc tgg cgt gtc ttt gga aac atc atg ctc atg ggt gtg     1008
   Leu Glu Trp Ser Trp Arg Val Phe Gly Asn Ile Met Leu Met Gly Val
                   325                 330                 335
   gcg gaa tcg ctc gcg ctg gcg gtc ctg ttt tcg ttg tcg cac aat ttc     1056
   Ala Glu Ser Leu Ala Leu Ala Val Leu Phe Ser Leu Ser His Asn Phe
               340                 345                 350
   gaa tcc gcg gat cgc gat ccg acc gcc cca ctg aaa aag acg gga gaa     1104
   Glu Ser Ala Asp Arg Asp Pro Thr Ala Pro Leu Lys Lys Thr Gly Glu
           355                 360                 365
   cca gtc gac tgg ttc aag aca cag gtc gaa act tcc tgc act tac ggt     1152
   Pro Val Asp Trp Phe Lys Thr Gln Val Glu Thr Ser Cys Thr Tyr Gly
   370 375 380
   gga ttc ctt tcc ggt tgc ttc acg gga ggt ctc aac ttt cag gtt gaa 1200
   Gly Phe Leu Ser Gly Cys Phe Thr Gly Gly Leu Asn Phe Gln Val Glu
   385 390 395 400
   cac cac ttg ttc cca cgc atg agc agc gct tgg tat ccc tac att gcc 1248
   His His Leu Phe Pro Arg Met Ser Ser Ala Trp Tyr Pro Tyr Ile Ala
   405 410 415
   ccc aag gtc cgc gaa att tgc gcc aaa cac ggc gtc cac tac gcc tac 1296
   Pro Lys Val Arg Glu Ile Cys Ala Lys His Gly Val His Tyr Ala Tyr
   420 425 430
   tac ccg tgg atc cac caa aac ttt ctc tcc acc gtc cgc tac atg cac 1344
   Tyr Pro Trp Ile His Gln Asn Phe Leu Ser Thr Val Arg Tyr Met His
   435 440 445
   gcg gcc ggg acc ggt gcc aac tgg cgc cag atg gcc aga gaa aat ccc 1392
   Ala Ala Gly Thr Gly Ala Asn Trp Arg Gln Met Ala Arg Glu Asn Pro
   450 455 460
   ttg acc gga cgg gcg taa 1410
   Leu Thr Gly Arg Ala
   465
<210> 14
   <211> 469
   <212> PRT
   <213> Phaeodactylum tricornutum
<400> 14
   Met Ala Pro Asp Ala Asp Lys Leu Arg Gln Arg Gln Thr Thr Ala Val
   1               5                   10                  15
   Ala Lys His Asn Ala Ala Thr Ile Ser Thr Gln Glu Arg Leu Cys Ser
               20                  25                  30
   Leu Ser Ser Leu Lys Gly Glu Glu Val Cys Ile Asp Gly Ile Ile Tyr
           35                  40                  45
   Asp Leu Gln Ser Phe Asp His Pro Gly Gly Glu Thr Ile Lys Met Phe
       50                  55                  60
   Gly Gly Asn Asp Val Thr Val Gln Tyr Lys Met Ile His Pro Tyr His
   65                  70                  75                  80
   Thr Glu Lys His Leu Glu Lys Met Lys Arg Val Gly Lys Val Thr Asp
                   85                  90                  95
   Phe Val Cys Glu Tyr Lys Phe Asp Thr Glu Phe Glu Arg Glu Ile Lys
               100                 105                 110
   Arg Glu Val Phe Lys Ile Val Arg Arg Gly Lys Asp Phe Gly Thr Leu
   115 120 125
   Gly Trp Phe Phe Arg Ala Phe Cys Tyr Ile Ala Ile Phe Phe Tyr Leu
   130 135 140
   Gln Tyr His Trp Val Thr Thr Gly Thr Ser Trp Leu Leu Ala Val Ala
   145 150 155 160
   Tyr Gly Ile Ser Gln Ala Met Ile Gly Met Asn Val Gln His Asp Ala
   165 170 175
   Asn His Gly Ala Thr Ser Lys Arg Pro Trp Val Asn Asp Met Leu Gly
   180 185 190
   Leu Gly Ala Asp Phe Ile Gly Gly Ser Lys Trp Leu Trp Gln Glu Gln
   195 200 205
   His Trp Thr His His Ala Tyr Thr Asn His Ala Glu Met Asp Pro Asp
   210 215 220
   Ser Phe Gly Ala Glu Pro Met Leu Leu Phe Asn Asp Tyr Pro Leu Asp
   225 230 235 240
   His Pro Ala Arg Thr Trp Leu His Arg Phe Gln Ala Phe Phe Tyr Met
   245 250 255
   Pro Val Leu Ala Gly Tyr Trp Leu Ser Ala Val Phe Asn Pro Gln Ile
   260 265 270
   Leu Asp Leu Gln Gln Arg Gly Ala Leu Ser Val Gly Ile Arg Leu Asp
   275 280 285
   Asn Ala Phe Ile His Ser Arg Arg Lys Tyr Ala Val Phe Trp Arg Ala
   290 295 300
   Val Tyr Ile Ala Val Asn Val Ile Ala Pro Phe Tyr Thr Asn Ser Gly
   305 310 315 320
   Leu Glu Trp Ser Trp Arg Val Phe Gly Asn Ile Met Leu Met Gly Val
   325 330 335
   Ala Glu Ser Leu Ala Leu Ala Val Leu Phe Ser Leu Ser His Asn Phe
   340 345 350
   Glu Ser Ala Asp Arg Asp Pro Thr Ala Pro Leu Lys Lys Thr Gly Glu
   355 360 365
   Pro Val Asp Trp Phe Lys Thr Gln Val Glu Thr Ser Cys Thr Tyr Gly
   370 375 380
   Gly Phe Leu Ser Gly Cys Phe Thr Gly Gly Leu Asn Phe Gln Val Glu
   385 390 395 400
   His His Leu Phe Pro Arg Met Ser Ser Ala Trp Tyr Pro Tyr Ile Ala
   405 410 415
   Pro Lys Val Arg Glu Ile Cys Ala Lys His Gly Val His Tyr Ala Tyr
   420 425 430
   Tyr Pro Trp Ile His Gln Asn Phe Leu Ser Thr Val Arg Tyr Met His
   435 440 445
   Ala Ala Gly Thr Gly Ala Asn Trp Arg Gln Met Ala Arg Glu Asn Pro
   450 455 460
   Leu Thr Gly Arg Ala
   465
<210> 15
   <211> 3598
   <212> DNA
   <213> artificial sequence
<220>
   <221> misc_feature
   <223> Sequenz stellt eine pflanzliche Promotor-Terminator-Expressionska ssette in Vektor pUC19 dar
<400> 15
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgcc     240
   attcgccatt caggctgcgc aactgttggg aagggcgatc ggtgcgggcc tcttcgctat     300
   tacgccagct ggcgaaaggg ggatgtgctg caaggcgatt aagttgggta acgccagggt     360
   tttcccagtc acgacgttgt aaaacgacgg ccagtgaatt cggcgcgccg agctcctcga     420
   gcaaatttac acattgccac taaacgtcta aacccttgta atttgttttt gttttactat     480
   gtgtgttatg tatttgattt gcgataaatt tttatatttg gtactaaatt tataacacct     540
   tttatgctaa cgtttgccaa cacttagcaa tttgcaagtt gattaattga ttctaaatta     600
   tttttgtctt ctaaatacat atactaatca actggaaatg taaatatttg ctaatatttc    660
   tactatagga gaattaaagt gagtgaatat ggtaccacaa ggtttggaga tttaattgtt    720
   gcaatgctgc atggatggca tatacaccaa acattcaata attcttgagg ataataatgg    780
   taccacacaa gatttgaggt gcatgaacgt cacgtggaca aaaggtttag taatttttca    840
   agacaacaat gttaccacac acaagttttg aggtgcatgc atggatgccc tgtggaaagt    900
   ttaaaaatat tttggaaatg atttgcatgg aagccatgtg taaaaccatg acatccactt    960
   ggaggatgca ataatgaaga aaactacaaa tttacatgca actagttatg catgtagtct   1020
   atataatgag gattttgcaa tactttcatt catacacact cactaagttt tacacgatta   1080
   taatttcttc atagccagcc caccgcggtg ggcggccgcc tgcagtctag aaggcctcct   1140
   gctttaatga gatatgcgag acgcctatga tcgcatgata tttgctttca attctgttgt   1200
   gcacgttgta aaaaacctga gcatgtgtag ctcagatcct taccgccggt ttcggttcat   1260
   tctaatgaat atatcacccg ttactatcgt atttttatga ataatattct ccgttcaatt   1320
   tactgattgt ccgtcgacga attcgagctc ggcgcgccaa gcttggcgta atcatggtca   1380
   tagctgtttc ctgtgtgaaa ttgttatccg ctcacaattc cacacaacat acgagccgga   1440
   agcataaagt gtaaagcctg gggtgcctaa tgagtgagct aactcacatt aattgcgttg   1500
   cgctcactgc ccgctttcca gtcgggaaac ctgtcgtgcc agctgcatta atgaatcggc   1560
   caacgcgcgg ggagaggcgg tttgcgtatt gggcgctctt ccgcttcctc gctcactgac   1620
   tcgctgcgct cggtcgttcg gctgcggcga gcggtatcag ctcactcaaa ggcggtaata   1680
   cggttatcca cagaatcagg ggataacgca ggaaagaaca tgtgagcaaa aggccagcaa   1740
   aaggccagga accgtaaaaa ggccgcgttg ctggcgtttt tccataggct ccgcccccct   1800
   gacgagcatc acaaaaatcg acgctcaagt cagaggtggc gaaacccgac aggactataa   1860
   agataccagg cgtttccccc tggaagctcc ctcgtgcgct ctcctgttcc gaccctgccg   1920
   cttaccggat acctgtccgc ctttctccct tcgggaagcg tggcgctttc tcatagctca   1980
   cgctgtaggt atctcagttc ggtgtaggtc gttcgctcca agctgggctg tgtgcacgaa   2040
   ccccccgttc agcccgaccg ctgcgcctta tccggtaact atcgtcttga gtccaacccg   2100
   gtaagacacg acttatcgcc actggcagca gccactggta acaggattag cagagcgagg   2160
   tatgtaggcg gtgctacaga gttcttgaag tggtggccta actacggcta cactagaagg   2220
   acagtatttg gtatctgcgc tctgctgaag ccagttacct tcggaaaaag agttggtagc   2280
   tcttgatccg gcaaacaaac caccgctggt agcggtggtt tttttgtttg caagcagcag   2340
   attacgcgca gaaaaaaagg atctcaagaa gatcctttga tcttttctac ggggtctgac   2400
   gctcagtgga acgaaaactc acgttaaggg attttggtca tgagattatc aaaaaggatc   2460
   ttcacctaga tccttttaaa ttaaaaatga agttttaaat caatctaaag tatatatgag 2520
   taaacttggt ctgacagtta ccaatgctta atcagtgagg cacctatctc agcgatctgt 2580
   ctatttcgtt catccatagt tgcctgactc cccgtcgtgt agataactac gatacgggag 2640
   ggcttaccat ctggccccag tgctgcaatg ataccgcgag acccacgctc accggctcca 2700
   gatttatcag caataaacca gccagccgga agggccgagc gcagaagtgg tcctgcaact 2760
   ttatccgcct ccatccagtc tattaattgt tgccgggaag ctagagtaag tagttcgcca 2820
   gttaatagtt tgcgcaacgt tgttgccatt gctacaggca tcgtggtgtc acgctcgtcg 2880
   tttggtatgg cttcattcag ctccggttcc caacgatcaa ggcgagttac atgatccccc 2940
   atgttgtgca aaaaagcggt tagctccttc ggtcctccga tcgttgtcag aagtaagttg 3000
   gccgcagtgt tatcactcat ggttatggca gcactgcata attctcttac tgtcatgcca 3060
   tccgtaagat gcttttctgt gactggtgag tactcaacca agtcattctg agaatagtgt 3120
   atgcggcgac cgagttgctc ttgcccggcg tcaatacggg ataataccgc gccacatagc 3180
   agaactttaa aagtgctcat cattggaaaa cgttcttcgg ggcgaaaact ctcaaggatc 3240
   ttaccgctgt tgagatccag ttcgatgtaa cccactcgtg cacccaactg atcttcagca 3300
   tcttttactt tcaccagcgt ttctgggtga gcaaaaacag gaaggcaaaa tgccgcaaaa 3360
   aagggaataa gggcgacacg gaaatgttga atactcatac tcttcctttt tcaatattat 3420
   tgaagcattt atcagggtta ttgtctcatg agcggataca tatttgaatg tatttagaaa 3480
   aataaacaaa taggggttcc gcgcacattt ccccgaaaag tgccacctga cgtctaagaa 3540
   accattatta tcatgacatt aacctataaa aataggcgta tcacgaggcc ctttcgtc 3598
<210> 16
   <211> 3590
   <212> DNA
   <213> artificial sequence
<220>
   <221> misc_feature
   <223> Sequenz stellt eine pflanzliche Promotor-Terminator-Expressionska ssette in Vektor pUC19 dar
<400> 16
   tcgcgcgttt cggtgatgac ggtgaaaacc tctgacacat gcagctcccg gagacggtca     60
   cagcttgtct gtaagcggat gccgggagca gacaagcccg tcagggcgcg tcagcgggtg    120
   ttggcgggtg tcggggctgg cttaactatg cggcatcaga gcagattgta ctgagagtgc    180
   accatatgcg gtgtgaaata ccgcacagat gcgtaaggag aaaataccgc atcaggcgcc    240
   attcgccatt caggctgcgc aactgttggg aagggcgatc ggtgcgggcc tcttcgctat    300
   tacgccagct ggcgaaaggg ggatgtgctg caaggcgatt aagttgggta acgccagggt    360
   tttcccagtc acgacgttgt aaaacgacgg ccagtgaatt cggcgcgccg agctcctcga 420
   gcaaatttac acattgccac taaacgtcta aacccttgta atttgttttt gttttactat 480
   gtgtgttatg tatttgattt gcgataaatt tttatatttg gtactaaatt tataacacct 540
   tttatgctaa cgtttgccaa cacttagcaa tttgcaagtt gattaattga ttctaaatta 600
   tttttgtctt ctaaatacat atactaatca actggaaatg taaatatttg ctaatatttc 660
   tactatagga gaattaaagt gagtgaatat ggtaccacaa ggtttggaga tttaattgtt 720
   gcaatgctgc atggatggca tatacaccaa acattcaata attcttgagg ataataatgg 780
   taccacacaa gatttgaggt gcatgaacgt cacgtggaca aaaggtttag taatttttca 840
   agacaacaat gttaccacac acaagttttg aggtgcatgc atggatgccc tgtggaaagt 900
   ttaaaaatat tttggaaatg atttgcatgg aagccatgtg taaaaccatg acatccactt 960
   ggaggatgca ataatgaaga aaactacaaa tttacatgca actagttatg catgtagtct 1020
   atataatgag gattttgcaa tactttcatt catacacact cactaagttt tacacgatta 1080
   taatttcttc atagccagcg gatccgatat cgggcccgct agcgttaacc ctgctttaat 1140
   gagatatgcg agacgcctat gatcgcatga tatttgcttt caattctgtt gtgcacgttg 1200
   taaaaaacct gagcatgtgt agctcagatc cttaccgccg gtttcggttc attctaatga 1260
   atatatcacc cgttactatc gtatttttat gaataatatt ctccgttcaa tttactgatt 1320
   gtccgtcgac gaattcgagc tcggcgcgcc aagcttggcg taatcatggt catagctgtt 1380
   tcctgtgtga aattgttatc cgctcacaat tccacacaac atacgagccg gaagcataaa 1440
   gtgtaaagcc tggggtgcct aatgagtgag ctaactcaca ttaattgcgt tgcgctcact 1500
   gcccgctttc cagtcgggaa acctgtcgtg ccagctgcat taatgaatcg gccaacgcgc 1560
   ggggagaggc ggtttgcgta ttgggcgctc ttccgcttcc tcgctcactg actcgctgcg 1620
   ctcggtcgtt cggctgcggc gagcggtatc agctcactca aaggcggtaa tacggttatc 1680
   cacagaatca ggggataacg caggaaagaa catgtgagca aaaggccagc aaaaggccag 1740
   gaaccgtaaa aaggccgcgt tgctggcgtt tttccatagg ctccgccccc ctgacgagca 1800
   tcacaaaaat cgacgctcaa gtcagaggtg gcgaaacccg acaggactat aaagatacca 1860
   ggcgtttccc cctggaagct ccctcgtgcg ctctcctgtt ccgaccctgc cgcttaccgg 1920
   atacctgtcc gcctttctcc cttcgggaag cgtggcgctt tctcatagct cacgctgtag 1980
   gtatctcagt tcggtgtagg tcgttcgctc caagctgggc tgtgtgcacg aaccccccgt 2040
   tcagcccgac cgctgcgcct tatccggtaa ctatcgtctt gagtccaacc cggtaagaca 2100
   cgacttatcg ccactggcag cagccactgg taacaggatt agcagagcga ggtatgtagg 2160
   cggtgctaca gagttcttga agtggtggcc taactacggc tacactagaa ggacagtatt 2220
   tggtatctgc gctctgctga agccagttac cttcggaaaa agagttggta gctcttgatc 2280
   cggcaaacaa accaccgctg gtagcggtgg tttttttgtt tgcaagcagc agattacgcg 2340
   cagaaaaaaa ggatctcaag aagatccttt gatcttttct acggggtctg acgctcagtg 2400
   gaacgaaaac tcacgttaag ggattttggt catgagatta tcaaaaagga tcttcaccta 2460
   gatcctttta aattaaaaat gaagttttaa atcaatctaa agtatatatg agtaaacttg 2520
   gtctgacagt taccaatgct taatcagtga ggcacctatc tcagcgatct gtctatttcg 2580
   ttcatccata gttgcctgac tccccgtcgt gtagataact acgatacggg agggcttacc 2640
   atctggcccc agtgctgcaa tgataccgcg agacccacgc tcaccggctc cagatttatc 2700
   agcaataaac cagccagccg gaagggccga gcgcagaagt ggtcctgcaa ctttatccgc 2760
   ctccatccag tctattaatt gttgccggga agctagagta agtagttcgc cagttaatag 2820
   tttgcgcaac gttgttgcca ttgctacagg catcgtggtg tcacgctcgt cgtttggtat 2880
   ggcttcattc agctccggtt cccaacgatc aaggcgagtt acatgatccc ccatgttgtg 2940
   caaaaaagcg gttagctcct tcggtcctcc gatcgttgtc agaagtaagt tggccgcagt 3000
   gttatcactc atggttatgg cagcactgca taattctctt actgtcatgc catccgtaag 3060
   atgcttttct gtgactggtg agtactcaac caagtcattc tgagaatagt gtatgcggcg 3120
   accgagttgc tcttgcccgg cgtcaatacg ggataatacc gcgccacata gcagaacttt 3180
   aaaagtgctc atcattggaa aacgttcttc ggggcgaaaa ctctcaagga tcttaccgct 3240
   gttgagatcc agttcgatgt aacccactcg tgcacccaac tgatcttcag catcttttac 3300
   tttcaccagc gtttctgggt gagcaaaaac aggaaggcaa aatgccgcaa aaaagggaat 3360
   aagggcgaca cggaaatgtt gaatactcat actcttcctt tttcaatatt attgaagcat 3420
   ttatcagggt tattgtctca tgagcggata catatttgaa tgtatttaga aaaataaaca 3480
   aataggggtt ccgcgcacat ttccccgaaa agtgccacct gacgtctaag aaaccattat 3540
   tatcatgaca ttaacctata aaaataggcg tatcacgagg ccctttcgtc 3590
<210> 17
   <211> 3584
   <212> DNA
   <213> artificial sequence
<220>
   <221> misc_feature
   <223> Sequenz stellt eine pflanzliche Promotor-Terminator-Expressionska ssette in Vektor pUC19 dar
<400> 17
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg    120
   ttggcgggtg tcggggctgg cttaactatg cggcatcaga gcagattgta ctgagagtgc    180
   accatatgcg gtgtgaaata ccgcacagat gcgtaaggag aaaataccgc atcaggcgcc    240
   attcgccatt caggctgcgc aactgttggg aagggcgatc ggtgcgggcc tcttcgctat    300
   tacgccagct ggcgaaaggg ggatgtgctg caaggcgatt aagttgggta acgccagggt    360
   tttcccagtc acgacgttgt aaaacgacgg ccagtgaatt cggcgcgccg agctcctcga    420
   gcaaatttac acattgccac taaacgtcta aacccttgta atttgttttt gttttactat    480
   gtgtgttatg tatttgattt gcgataaatt tttatatttg gtactaaatt tataacacct    540
   tttatgctaa cgtttgccaa cacttagcaa tttgcaagtt gattaattga ttctaaatta    600
   tttttgtctt ctaaatacat atactaatca actggaaatg taaatatttg ctaatatttc    660
   tactatagga gaattaaagt gagtgaatat ggtaccacaa ggtttggaga tttaattgtt    720
   gcaatgctgc atggatggca tatacaccaa acattcaata attcttgagg ataataatgg    780
   taccacacaa gatttgaggt gcatgaacgt cacgtggaca aaaggtttag taatttttca    840
   agacaacaat gttaccacac acaagttttg aggtgcatgc atggatgccc tgtggaaagt    900
   ttaaaaatat tttggaaatg atttgcatgg aagccatgtg taaaaccatg acatccactt    960
   ggaggatgca ataatgaaga aaactacaaa tttacatgca actagttatg catgtagtct   1020
   atataatgag gattttgcaa tactttcatt catacacact cactaagttt tacacgatta   1080
   taatttcttc atagccagca gatctgccgg catcgatccc gggccatggc ctgctttaat   1140
   gagatatgcg agacgcctat gatcgcatga tatttgcttt caattctgtt gtgcacgttg   1200
   taaaaaacct gagcatgtgt agctcagatc cttaccgccg gtttcggttc attctaatga   1260
   atatatcacc cgttactatc gtatttttat gaataatatt ctccgttcaa tttactgatt   1320
   gtccgtcgac gagctcggcg cgccaagctt ggcgtaatca tggtcatagc tgtttcctgt   1380
   gtgaaattgt tatccgctca caattccaca caacatacga gccggaagca taaagtgtaa   1440
   agcctggggt gcctaatgag tgagctaact cacattaatt gcgttgcgct cactgcccgc   1500
   tttccagtcg ggaaacctgt cgtgccagct gcattaatga atcggccaac gcgcggggag   1560
   aggcggtttg cgtattgggc gctcttccgc ttcctcgctc actgactcgc tgcgctcggt   1620
   cgttcggctg cggcgagcgg tatcagctca ctcaaaggcg gtaatacggt tatccacaga   1680
   atcaggggat aacgcaggaa agaacatgtg agcaaaaggc cagcaaaagg ccaggaaccg   1740
   taaaaaggcc gcgttgctgg cgtttttcca taggctccgc ccccctgacg agcatcacaa   1800
   aaatcgacgc tcaagtcaga ggtggcgaaa cccgacagga ctataaagat accaggcgtt   1860
   tccccctgga agctccctcg tgcgctctcc tgttccgacc ctgccgctta ccggatacct   1920
   gtccgccttt ctcccttcgg gaagcgtggc gctttctcat agctcacgct gtaggtatct 1980
   cagttcggtg taggtcgttc gctccaagct gggctgtgtg cacgaacccc ccgttcagcc 2040
   cgaccgctgc gccttatccg gtaactatcg tcttgagtcc aacccggtaa gacacgactt 2100
   atcgccactg gcagcagcca ctggtaacag gattagcaga gcgaggtatg taggcggtgc 2160
   tacagagttc ttgaagtggt ggcctaacta cggctacact agaaggacag tatttggtat 2220
   ctgcgctctg ctgaagccag ttaccttcgg aaaaagagtt ggtagctctt gatccggcaa 2280
   acaaaccacc gctggtagcg gtggtttttt tgtttgcaag cagcagatta cgcgcagaaa 2340
   aaaaggatct caagaagatc ctttgatctt ttctacgggg tctgacgctc agtggaacga 2400
   aaactcacgt taagggattt tggtcatgag attatcaaaa aggatcttca cctagatcct 2460
   tttaaattaa aaatgaagtt ttaaatcaat ctaaagtata tatgagtaaa cttggtctga 2520
   cagttaccaa tgcttaatca gtgaggcacc tatctcagcg atctgtctat ttcgttcatc 2580
   catagttgcc tgactccccg tcgtgtagat aactacgata cgggagggct taccatctgg 2640
   ccccagtgct gcaatgatac cgcgagaccc acgctcaccg gctccagatt tatcagcaat 2700
   aaaccagcca gccggaaggg ccgagcgcag aagtggtcct gcaactttat ccgcctccat 2760
   ccagtctatt aattgttgcc gggaagctag agtaagtagt tcgccagtta atagtttgcg 2820
   caacgttgtt gccattgcta caggcatcgt ggtgtcacgc tcgtcgtttg gtatggcttc 2880
   attcagctcc ggttcccaac gatcaaggcg agttacatga tcccccatgt tgtgcaaaaa 2940
   agcggttagc tccttcggtc ctccgatcgt tgtcagaagt aagttggccg cagtgttatc 3000
   actcatggtt atggcagcac tgcataattc tcttactgtc atgccatccg taagatgctt 3060
   ttctgtgact ggtgagtact caaccaagtc attctgagaa tagtgtatgc ggcgaccgag 3120
   ttgctcttgc ccggcgtcaa tacgggataa taccgcgcca catagcagaa ctttaaaagt 3180
   gctcatcatt ggaaaacgtt cttcggggcg aaaactctca aggatcttac cgctgttgag 3240
   atccagttcg atgtaaccca ctcgtgcacc caactgatct tcagcatctt ttactttcac 3300
   cagcgtttct gggtgagcaa aaacaggaag gcaaaatgcc gcaaaaaagg gaataagggc 3360
   gacacggaaa tgttgaatac tcatactctt cctttttcaa tattattgaa gcatttatca 3420
   gggttattgt ctcatgagcg gatacatatt tgaatgtatt tagaaaaata aacaaatagg 3480
   ggttccgcgc acatttcccc gaaaagtgcc acctgacgtc taagaaacca ttattatcat 3540
   gacattaacc tataaaaata ggcgtatcac gaggcccttt cgtc 3584
<210> 18
   <211> 4507
   <212> DNA
   <213> artificial sequence
   <220>
   <221> misc_feature
   <223> Sequenz stellt eine pflanzliche Promotor-Terminator-Expressionska ssette in Vektor pUC19 dar
<400> 18
   tcgcgcgttt cggtgatgac ggtgaaaacc tctgacacat gcagctcccg gagacggtca     60
   cagcttgtct gtaagcggat gccgggagca gacaagcccg tcagggcgcg tcagcgggtg    120
   ttggcgggtg tcggggctgg cttaactatg cggcatcaga gcagattgta ctgagagtgc    180
   accatatgcg gtgtgaaata ccgcacagat gcgtaaggag aaaataccgc atcaggcgcc    240
   attcgccatt caggctgcgc aactgttggg aagggcgatc ggtgcgggcc tcttcgctat    300
   tacgccagct ggcgaaaggg ggatgtgctg caaggcgatt aagttgggta acgccagggt    360
   tttcccagtc acgacgttgt aaaacgacgg ccagtgaatt cggcgcgccg agctcctcga    420
   gcaaatttac acattgccac taaacgtcta aacccttgta atttgttttt gttttactat    480
   gtgtgttatg tatttgattt gcgataaatt tttatatttg gtactaaatt tataacacct    540
   tttatgctaa cgtttgccaa cacttagcaa tttgcaagtt gattaattga ttctaaatta    600
   tttttgtctt ctaaatacat atactaatca actggaaatg taaatatttg ctaatatttc    660
   tactatagga gaattaaagt gagtgaatat ggtaccacaa ggtttggaga tttaattgtt    720
   gcaatgctgc atggatggca tatacaccaa acattcaata attcttgagg ataataatgg    780
   taccacacaa gatttgaggt gcatgaacgt cacgtggaca aaaggtttag taatttttca    840
   agacaacaat gttaccacac acaagttttg aggtgcatgc atggatgccc tgtggaaagt    900
   ttaaaaatat tttggaaatg atttgcatgg aagccatgtg taaaaccatg acatccactt    960
   ggaggatgca ataatgaaga aaactacaaa tttacatgca actagttatg catgtagtct   1020
   atataatgag gattttgcaa tactttcatt catacacact cactaagttt tacacgatta   1080
   taatttcttc atagccagcc caccgcggtg ggcggccgcc tgcagtctag aaggcctcct   1140
   gctttaatga gatatgcgag acgcctatga tcgcatgata tttgctttca attctgttgt   1200
   gcacgttgta aaaaacctga gcatgtgtag ctcagatcct taccgccggt ttcggttcat   1260
   tctaatgaat atatcacccg ttactatcgt atttttatga ataatattct ccgttcaatt   1320
   tactgattgt ccgtcgagca aatttacaca ttgccactaa acgtctaaac ccttgtaatt   1380
   tgtttttgtt ttactatgtg tgttatgtat ttgatttgcg ataaattttt atatttggta   1440
   ctaaatttat aacacctttt atgctaacgt ttgccaacac ttagcaattt gcaagttgat   1500
   taattgattc taaattattt ttgtcttcta aatacatata ctaatcaact ggaaatgtaa   1560
   atatttgcta atatttctac tataggagaa ttaaagtgag tgaatatggt accacaaggt   1620
   ttggagattt aattgttgca atgctgcatg gatggcatat acaccaaaca ttcaataatt   1680
   cttgaggata ataatggtac cacacaagat ttgaggtgca tgaacgtcac gtggacaaaa 1740
   ggtttagtaa tttttcaaga caacaatgtt accacacaca agttttgagg tgcatgcatg 1800
   gatgccctgt ggaaagttta aaaatatttt ggaaatgatt tgcatggaag ccatgtgtaa 1860
   aaccatgaca tccacttgga ggatgcaata atgaagaaaa ctacaaattt acatgcaact 1920
   agttatgcat gtagtctata taatgaggat tttgcaatac tttcattcat acacactcac 1980
   taagttttac acgattataa tttcttcata gccagcggat ccgatatcgg gcccgctagc 2040
   gttaaccctg ctttaatgag atatgcgaga cgcctatgat cgcatgatat ttgctttcaa 2100
   ttctgttgtg cacgttgtaa aaaacctgag catgtgtagc tcagatcctt accgccggtt 2160
   tcggttcatt ctaatgaata tatcacccgt tactatcgta tttttatgaa taatattctc 2220
   cgttcaattt actgattgtc cgtcgacgaa ttcgagctcg gcgcgccaag cttggcgtaa 2280
   tcatggtcat agctgtttcc tgtgtgaaat tgttatccgc tcacaattcc acacaacata 2340
   cgagccggaa gcataaagtg taaagcctgg ggtgcctaat gagtgagcta actcacatta 2400
   attgcgttgc gctcactgcc cgctttccag tcgggaaacc tgtcgtgcca gctgcattaa 2460
   tgaatcggcc aacgcgcggg gagaggcggt ttgcgtattg ggcgctcttc cgcttcctcg 2520
   ctcactgact cgctgcgctc ggtcgttcgg ctgcggcgag cggtatcagc tcactcaaag 2580
   gcggtaatac ggttatccac agaatcaggg gataacgcag gaaagaacat gtgagcaaaa 2640
   ggccagcaaa aggccaggaa ccgtaaaaag gccgcgttgc tggcgttttt ccataggctc 2700
   cgcccccctg acgagcatca caaaaatcga cgctcaagtc agaggtggcg aaacccgaca 2760
   ggactataaa gataccaggc gtttccccct ggaagctccc tcgtgcgctc tcctgttccg 2820
   accctgccgc ttaccggata cctgtccgcc tttctccctt cgggaagcgt ggcgctttct 2880
   catagctcac gctgtaggta tctcagttcg gtgtaggtcg ttcgctccaa gctgggctgt 2940
   gtgcacgaac cccccgttca gcccgaccgc tgcgccttat ccggtaacta tcgtcttgag 3000
   tccaacccgg taagacacga cttatcgcca ctggcagcag ccactggtaa caggattagc 3060
   agagcgaggt atgtaggcgg tgctacagag ttcttgaagt ggtggcctaa ctacggctac 3120
   actagaagga cagtatttgg tatctgcgct ctgctgaagc cagttacctt cggaaaaaga 3180
   gttggtagct cttgatccgg caaacaaacc accgctggta gcggtggttt ttttgtttgc 3240
   aagcagcaga ttacgcgcag aaaaaaagga tctcaagaag atcctttgat cttttctacg 3300
   gggtctgacg ctcagtggaa cgaaaactca cgttaaggga ttttggtcat gagattatca 3360
   aaaaggatct tcacctagat ccttttaaat taaaaatgaa gttttaaatc aatctaaagt 3420
   atatatgagt aaacttggtc tgacagttac caatgcttaa tcagtgaggc acctatctca 3480
   gcgatctgtc tatttcgttc atccatagtt gcctgactcc ccgtcgtgta gataactacg 3540
   atacgggagg gcttaccatc tggccccagt gctgcaatga taccgcgaga cccacgctca   3600
   ccggctccag atttatcagc aataaaccag ccagccggaa gggccgagcg cagaagtggt   3660
   cctgcaactt tatccgcctc catccagtct attaattgtt gccgggaagc tagagtaagt   3720
   agttcgccag ttaatagttt gcgcaacgtt gttgccattg ctacaggcat cgtggtgtca   3780
   cgctcgtcgt ttggtatggc ttcattcagc tccggttccc aacgatcaag gcgagttaca   3840
   tgatccccca tgttgtgcaa aaaagcggtt agctccttcg gtcctccgat cgttgtcaga   3900
   agtaagttgg ccgcagtgtt atcactcatg gttatggcag cactgcataa ttctcttact   3960
   gtcatgccat ccgtaagatg cttttctgtg actggtgagt actcaaccaa gtcattctga   4020
   gaatagtgta tgcggcgacc gagttgctct tgcccggcgt caatacggga taataccgcg   4080
   ccacatagca gaactttaaa agtgctcatc attggaaaac gttcttcggg gcgaaaactc   4140
   tcaaggatct taccgctgtt gagatccagt tcgatgtaac ccactcgtgc acccaactga   4200
   tcttcagcat cttttacttt caccagcgtt tctgggtgag caaaaacagg aaggcaaaat   4260
   gccgcaaaaa agggaataag ggcgacacgg aaatgttgaa tactcatact cttccttttt   4320
   caatattatt gaagcattta tcagggttat tgtctcatga gcggatacat atttgaatgt   4380
   atttagaaaa ataaacaaat aggggttccg cgcacatttc cccgaaaagt gccacctgac   4440
   gtctaagaaa ccattattat catgacatta acctataaaa ataggcgtat cacgaggccc   4500
   tttcgtc                                                              4507
<210> 19
   <211> 17752
   <212> DNA
   <213> Phaeodactylum tricornutum, Physcomitrella patens
<220>
   <221> CDS
   <222> (11543)..(12415)
   <223> Delta-6-Elongase
<220>
   <221> CDS
   <222> (13313)..(14890)
   <223> Delta-6-Desaturase
<220>
   <221> CDS
   <222> (15791)..(17200)
   <223> Delta-5-Desaturase
<400> 19
   gatctggcgc cggccagcga gacgagcaag attggccgcc gcccgaaacg atccgacagc 60
   gcgcccagca caggtgcgca ggcaaattgc accaacgcat acagcgccag cagaatgcca 120
   tagtgggcgg tgacgtcgtt cgagtgaacc agatcgcgca ggaggcccgg cagcaccggc 180
   ataatcaggc cgatgccgac agcgtcgagc gcgacagtgc tcagaattac gatcaggggt 240
   atgttgggtt tcacgtctgg cctccggacc agcctccgct ggtccgattg aacgcgcgga 300
   ttctttatca ctgataagtt ggtggacata ttatgtttat cagtgataaa gtgtcaagca 360
   tgacaaagtt gcagccgaat acagtgatcc gtgccgccct ggacctgttg aacgaggtcg 420
   gcgtagacgg tctgacgaca cgcaaactgg cggaacggtt gggggttcag cagccggcgc 480
   tttactggca cttcaggaac aagcgggcgc tgctcgacgc actggccgaa gccatgctgg 540
   cggagaatca tacgcattcg gtgccgagag ccgacgacga ctggcgctca tttctgatcg 600
   ggaatgcccg cagcttcagg caggcgctgc tcgcctaccg cgatggcgcg cgcatccatg 660
   ccggcacgcg accgggcgca ccgcagatgg aaacggccga cgcgcagctt cgcttcctct 720
   gcgaggcggg tttttcggcc ggggacgccg tcaatgcgct gatgacaatc agctacttca 780
   ctgttggggc cgtgcttgag gagcaggccg gcgacagcga tgccggcgag cgcggcggca 840
   ccgttgaaca ggctccgctc tcgccgctgt tgcgggccgc gatagacgcc ttcgacgaag 900
   ccggtccgga cgcagcgttc gagcagggac tcgcggtgat tgtcgatgga ttggcgaaaa 960
   ggaggctcgt tgtcaggaac gttgaaggac cgagaaaggg tgacgattga tcaggaccgc 1020
   tgccggagcg caacccactc actacagcag agccatgtag acaacatccc ctcccccttt 1080
   ccaccgcgtc agacgcccgt agcagcccgc tacgggcttt ttcatgccct gccctagcgt 1140
   ccaagcctca cggccgcgct cggcctctct ggcggccttc tggcgctctt ccgcttcctc 1200
   gctcactgac tcgctgcgct cggtcgttcg gctgcggcga gcggtatcag ctcactcaaa 1260
   ggcggtaata cggttatcca cagaatcagg ggataacgca ggaaagaaca tgtgagcaaa 1320
   aggccagcaa aaggccagga accgtaaaaa ggccgcgttg ctggcgtttt tccataggct 1380
   ccgcccccct gacgagcatc acaaaaatcg acgctcaagt cagaggtggc gaaacccgac 1440
   aggactataa agataccagg cgtttccccc tggaagctcc ctcgtgcgct ctcctgttcc 1500
   gaccctgccg cttaccggat acctgtccgc ctttctccct tcgggaagcg tggcgctttt 1560
   ccgctgcata accctgcttc ggggtcatta tagcgatttt ttcggtatat ccatcctttt 1620
   tcgcacgata tacaggattt tgccaaaggg ttcgtgtaga ctttccttgg tgtatccaac 1680
   ggcgtcagcc gggcaggata ggtgaagtag gcccacccgc gagcgggtgt tccttcttca 1740
   ctgtccctta ttcgcacctg gcggtgctca acgggaatcc tgctctgcga ggctggccgg 1800
   ctaccgccgg cgtaacagat gagggcaagc ggatggctga tgaaaccaag ccaaccagga 1860
   agggcagccc acctatcaag gtgtactgcc ttccagacga acgaagagcg attgaggaaa 1920
   aggcggcggc ggccggcatg agcctgtcgg cctacctgct ggccgtcggc cagggctaca 1980
   aaatcacggg cgtcgtggac tatgagcacg tccgcgagct ggcccgcatc aatggcgacc 2040
   tgggccgcct gggcggcctg ctgaaactct ggctcaccga cgacccgcgc acggcgcggt 2100
   tcggtgatgc cacgatcctc gccctgctgg cgaagatcga agagaagcag gacgagcttg 2160
   gcaaggtcat gatgggcgtg gtccgcccga gggcagagcc atgacttttt tagccgctaa 2220
   aacggccggg gggtgcgcgt gattgccaag cacgtcccca tgcgctccat caagaagagc 2280
   gacttcgcgg agctggtgaa gtacatcacc gacgagcaag gcaagaccga gcgcctttgc 2340
   gacgctcacc gggctggttg ccctcgccgc tgggctggcg gccgtctatg gccctgcaaa 2400
   cgcgccagaa acgccgtcga agccgtgtgc gagacaccgc ggccgccggc gttgtggata 2460
   cctcgcggaa aacttggccc tcactgacag atgaggggcg gacgttgaca cttgaggggc 2520
   cgactcaccc ggcgcggcgt tgacagatga ggggcaggct cgatttcggc cggcgacgtg 2580
   gagctggcca gcctcgcaaa tcggcgaaaa cgcctgattt tacgcgagtt tcccacagat 2640
   gatgtggaca agcctgggga taagtgccct gcggtattga cacttgaggg gcgcgactac 2700
   tgacagatga ggggcgcgat ccttgacact tgaggggcag agtgctgaca gatgaggggc 2760
   gcacctattg acatttgagg ggctgtccac aggcagaaaa tccagcattt gcaagggttt 2820
   ccgcccgttt ttcggccacc gctaacctgt cttttaacct gcttttaaac caatatttat 2880
   aaaccttgtt tttaaccagg gctgcgccct gtgcgcgtga ccgcgcacgc cgaagggggg 2940
   tgccccccct tctcgaaccc tcccggcccg ctaacgcggg cctcccatcc ccccaggggc 3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa 3480
   tactgataag ataatatatc ttttatatag aagatatcgc cgtatgtaag gatttcaggg 3540
   ggcaaggcat aggcagcgcg cttatcaata tatctataga atgggcaaag cataaaaact 3600
   tgcatggact aatgcttgaa acccaggaca ataaccttat agcttgtaaa ttctatcata 3660
   attgggtaat gactccaact tattgatagt gttttatgtt cagataatgc ccgatgactt 3720
   tgtcatgcag ctccaccgat tttgagaacg acagcgactt ccgtcccagc cgtgccaggt 3780
   gctgcctcag attcaggtta tgccgctcaa ttcgctgcgt atatcgcttg ctgattacgt 3840
   gcagctttcc cttcaggcgg gattcataca gcggccagcc atccgtcatc catatcacca 3900
   cgtcaaaggg tgacagcagg ctcataagac gccccagcgt cgccatagtg cgttcaccga 3960
   atacgtgcgc aacaaccgtc ttccggagac tgtcatacgc gtaaaacagc cagcgctggc 4020
   gcgatttagc cccgacatag ccccactgtt cgtccatttc cgcgcagacg atgacgtcac 4080
   tgcccggctg tatgcgcgag gttaccgact gcggcctgag ttttttaagt gacgtaaaat 4140
   cgtgttgagg ccaacgccca taatgcgggc tgttgcccgg catccaacgc cattcatggc 4200
   catatcaatg attttctggt gcgtaccggg ttgagaagcg gtgtaagtga actgcagttg 4260
   ccatgtttta cggcagtgag agcagagata gcgctgatgt ccggcggtgc ttttgccgtt 4320
   acgcaccacc ccgtcagtag ctgaacagga gggacagctg atagacacag aagccactgg 4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg attttttaaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa 5640
   ggcaccaggc gggtcaaatc aggaataagg gcacattgcc ccggcgtgag tcggggcaat 5700
   cccgcaagga gggtgaatga atcggacgtt tgaccggaag gcatacaggc aagaactgat 5760
   cgacgcgggg ttttccgccg aggatgccga aaccatcgca agccgcaccg tcatgcgtgc 5820
   gccccgcgaa accttccagt ccgtcggctc gatggtccag caagctacgg ccaagatcga 5880
   gcgcgacagc gtgcaactgg ctccccctgc cctgcccgcg ccatcggccg ccgtggagcg 5940
   ttcgcgtcgt ctcgaacagg aggcggcagg tttggcgaag tcgatgacca tcgacacgcg 6000
   aggaactatg acgaccaaga agcgaaaaac cgccggcgag gacctggcaa aacaggtcag 6060
   cgaggccaag caggccgcgt tgctgaaaca cacgaagcag cagatcaagg aaatgcagct 6120
   ttccttgttc gatattgcgc cgtggccgga cacgatgcga gcgatgccaa acgacacggc 6180
   ccgctctgcc ctgttcacca cgcgcaacaa gaaaatcccg cgcgaggcgc tgcaaaacaa 6240
   ggtcattttc cacgtcaaca aggacgtgaa gatcacctac accggcgtcg agctgcgggc 6300
   cgacgatgac gaactggtgt ggcagcaggt gttggagtac gcgaagcgca cccctatcgg 6360
   cgagccgatc accttcacgt tctacgagct ttgccaggac ctgggctggt cgatcaatgg 6420
   ccggtattac acgaaggccg aggaatgcct gtcgcgccta caggcgacgg cgatgggctt 6480
   cacgtccgac cgcgttgggc acctggaatc ggtgtcgctg ctgcaccgct tccgcgtcct 6540
   ggaccgtggc aagaaaacgt cccgttgcca ggtcctgatc gacgaggaaa tcgtcgtgct 6600
   gtttgctggc gaccactaca cgaaattcat atgggagaag taccgcaagc tgtcgccgac 6660
   ggcccgacgg atgttcgact atttcagctc gcaccgggag ccgtacccgc tcaagctgga 6720
   aaccttccgc ctcatgtgcg gatcggattc cacccgcgtg aagaagtggc gcgagcaggt 6780
   cggcgaagcc tgcgaagagt tgcgaggcag cggcctggtg gaacacgcct gggtcaatga 6840
   tgacctggtg cattgcaaac gctagggcct tgtggggtca gttccggctg ggggttcagc 6900
   agccagcgct ttactggcat ttcaggaaca agcgggcact gctcgacgca cttgcttcgc 6960
   tcagtatcgc tcgggacgca cggcgcgctc tacgaactgc cgataaacag aggattaaaa 7020
   ttgacaattg tgattaaggc tcagattcga cggcttggag cggccgacgt gcaggatttc 7080
   cgcgagatcc gattgtcggc cctgaagaaa gctccagaga tgttcgggtc cgtttacgag 7140
   cacgaggaga aaaagcccat ggaggcgttc gctgaacggt tgcgagatgc cgtggcattc 7200
   ggcgcctaca tcgacggcga gatcattggg ctgtcggtct tcaaacagga ggacggcccc 7260
   aaggacgctc acaaggcgca tctgtccggc gttttcgtgg agcccgaaca gcgaggccga 7320
   ggggtcgccg gtatgctgct gcgggcgttg ccggcgggtt tattgctcgt gatgatcgtc 7380
   cgacagattc caacgggaat ctggtggatg cgcatcttca tcctcggcgc acttaatatt 7440
   tcgctattct ggagcttgtt gtttatttcg gtctaccgcc tgccgggcgg ggtcgcggcg 7500
   acggtaggcg ctgtgcagcc gctgatggtc gtgttcatct ctgccgctct gctaggtagc 7560
   ccgatacgat tgatggcggt cctgggggct atttgcggaa ctgcgggcgt ggcgctgttg 7620
   gtgttgacac caaacgcagc gctagatcct gtcggcgtcg cagcgggcct ggcgggggcg 7680
   gtttccatgg cgttcggaac cgtgctgacc cgcaagtggc aacctcccgt gcctctgctc 7740
   acctttaccg cctggcaact ggcggccgga ggacttctgc tcgttccagt agctttagtg 7800
   tttgatccgc caatcccgat gcctacagga accaatgttc tcggcctggc gtggctcggc 7860
   ctgatcggag cgggtttaac ctacttcctt tggttccggg ggatctcgcg actcgaacct 7920
   acagttgttt ccttactggg ctttctcagc cccagatctg gggtcgatca gccggggatg 7980
   catcaggccg acagtcggaa cttcgggtcc ccgacctgta ccattcggtg agcaatggat 8040
   aggggagttg atatcgtcaa cgttcacttc taaagaaata gcgccactca gcttcctcag 8100
   cggctttatc cagcgatttc ctattatgtc ggcatagttc tcaagatcga cagcctgtca 8160
   cggttaagcg agaaatgaat aagaaggctg ataattcgga tctctgcgag ggagatgata 8220
   tttgatcaca ggcagcaacg ctctgtcatc gttacaatca acatgctacc ctccgcgaga 8280
   tcatccgtgt ttcaaacccg gcagcttagt tgccgttctt ccgaatagca tcggtaacat 8340
   gagcaaagtc tgccgcctta caacggctct cccgctgacg ccgtcccgga ctgatgggct 8400
   gcctgtatcg agtggtgatt ttgtgccgag ctgccggtcg gggagctgtt ggctggctgg 8460
   tggcaggata tattgtggtg taaacaaatt gacgcttaga caacttaata acacattgcg 8520
   gacgttttta atgtactggg gtggtttttc ttttcaccag tgagacgggc aacagctgat 8580
   tgcccttcac cgcctggccc tgagagagtt gcagcaagcg gtccacgctg gtttgcccca 8640
   gcaggcgaaa atcctgtttg atggtggttc cgaaatcggc aaaatccctt ataaatcaaa 8700
   agaatagccc gagatagggt tgagtgttgt tccagtttgg aacaagagtc cactattaaa 8760
   gaacgtggac tccaacgtca aagggcgaaa aaccgtctat cagggcgatg gcccactacg 8820
   tgaaccatca cccaaatcaa gtttttgggg gtcgaggtgc cgtaaagcac taaatcggaa 8880
   ccctaaaggg agcccccgat ttagagcttg acggggaaag ccggcgaacg tggcgagaaa 8940
   ggaagggaag aaagcgaaag gagcgggcgc cattcaggct gcgcaactgt tgggaagggc 9000
   gatcggtgcg ggcctcttcg ctattacgcc agctggcgaa agggggatgt gctgcaaggc 9060
   gattaagttg ggtaacgcca gggttttccc agtcacgacg ttgtaaaacg acggccagtg 9120
   aattaattcc catcttgaaa gaaatatagt ttaaatattt attgataaaa taacaagtca 9180
   ggtattatag tccaagcaaa aacataaatt tattgatgca agtttaaatt cagaaatatt 9240
   tcaataactg attatatcag ctggtacatt gccgtagatg aaagactgag tgcgatatta 9300
   tgtgtaatac ataaattgat gatatagcta gcttagctca tcgggggatc cgtcgaagct 9360
   agcttgggtc ccgctcagaa gaactcgtca agaaggcgat agaaggcgat gcgctgcgaa 9420
   tcgggagcgg cgataccgta aagcacgagg aagcggtcag cccattcgcc gccaagctct 9480
   tcagcaatat cacgggtagc caacgctatg tcctgatagc ggtccgccac acccagccgg 9540
   ccacagtcga tgaatccaga aaagcggcca ttttccacca tgatattcgg caagcaggca 9600
   tcgccatggg tcacgacgag atcctcgccg tcgggcatgc gcgccttgag cctggcgaac 9660
   agttcggctg gcgcgagccc ctgatgctct tcgtccagat catcctgatc gacaagaccg 9720
   gcttccatcc gagtacgtgc tcgctcgatg cgatgtttcg cttggtggtc gaatgggcag 9780
   gtagccggat caagcgtatg cagccgccgc attgcatcag ccatgatgga tactttctcg 9840
   gcaggagcaa ggtgagatga caggagatcc tgccccggca cttcgcccaa tagcagccag 9900
   tcccttcccg cttcagtgac aacgtcgagc acagctgcgc aaggaacgcc cgtcgtggcc 9960
   agccacgata gccgcgctgc ctcgtcctgc agttcattca gggcaccgga caggtcggtc 10020
   ttgacaaaaa gaaccgggcg cccctgcgct gacagccgga acacggcggc atcagagcag 10080
   ccgattgtct gttgtgccca gtcatagccg aatagcctct ccacccaagc ggccggagaa 10140
   cctgcgtgca atccatcttg ttcaatccaa gctcccatgg gccctcgact agagtcgaga 10200
   tctggattga gagtgaatat gagactctaa ttggataccg aggggaattt atggaacgtc 10260
   agtggagcat ttttgacaag aaatatttgc tagctgatag tgaccttagg cgacttttga 10320
   acgcgcaata atggtttctg acgtatgtgc ttagctcatt aaactccaga aacccgcggc 10380
   tgagtggctc cttcaacgtt gcggttctgt cagttccaaa cgtaaaacgg cttgtcccgc 10440
   gtcatcggcg ggggtcataa cgtgactccc ttaattctcc gctcatgatc ttgatcccct 10500
   gcgccatcag atccttggcg gcaagaaagc catccagttt actttgcagg gcttcccaac 10560
   cttaccagag ggcgccccag ctggcaattc cggttcgctt gctgtccata aaaccgccca 10620
   gtctagctat cgccatgtaa gcccactgca agctacctgc tttctctttg cgcttgcgtt 10680
   ttcccttgtc cagatagccc agtagctgac attcatccgg ggtcagcacc gtttctgcgg 10740
   actggctttc tacgtgttcc gcttccttta gcagcccttg cgccctgagt gcttgcggca 10800
   gcgtgaagct tgcatgcctg caggtcgacg gcgcgccgag ctcctcgagc aaatttacac 10860
   attgccacta aacgtctaaa cccttgtaat ttgtttttgt tttactatgt gtgttatgta 10920
   tttgatttgc gataaatttt tatatttggt actaaattta taacaccttt tatgctaacg 10980
   tttgccaaca cttagcaatt tgcaagttga ttaattgatt ctaaattatt tttgtcttct 11040
   aaatacatat actaatcaac tggaaatgta aatatttgct aatatttcta ctataggaga 11100
   attaaagtga gtgaatatgg taccacaagg tttggagatt taattgttgc aatgctgcat 11160
   ggatggcata tacaccaaac attcaataat tcttgaggat aataatggta ccacacaaga 11220
   tttgaggtgc atgaacgtca cgtggacaaa aggtttagta atttttcaag acaacaatgt  11280
   taccacacac aagttttgag gtgcatgcat ggatgccctg tggaaagttt aaaaatattt  11340
   tggaaatgat ttgcatggaa gccatgtgta aaaccatgac atccacttgg aggatgcaat  11400
   aatgaagaaa actacaaatt tacatgcaac tagttatgca tgtagtctat ataatgagga  11460
   ttttgcaata ctttcattca tacacactca ctaagtttta cacgattata atttcttcat  11520
   agccagccca ccgcggtgga aa atg gag gtc gtg gag aga ttc tac ggt gag   11572
                            Met Glu Val Val Glu Arg Phe Tyr Gly Glu
                            1               5                   10
   ttg gat ggg aag gtc tcg cag ggc gtg aat gca ttg ctg ggt agt ttt   11620
   Leu Asp Gly Lys Val Ser Gln Gly Val Asn Ala Leu Leu Gly Ser Phe
                   15                  20                  25
   ggg gtg gag ttg acg gat acg ccc act acc aaa ggc ttg ccc ctc gtt   11668
   Gly Val Glu Leu Thr Asp Thr Pro Thr Thr Lys Gly Leu Pro Leu Val
               30                  35                  40
   gac agt ccc aca ccc atc gtc ctc ggt gtt tct gta tac ttg act att   11716
   Asp Ser Pro Thr Pro Ile Val Leu Gly Val Ser Val Tyr Leu Thr Ile
           45                  50                  55
   gtc att gga ggg ctt ttg tgg ata aag gcc agg gat ctg aaa ccg cgc   11764
   Val Ile Gly Gly Leu Leu Trp Ile Lys Ala Arg Asp Leu Lys Pro Arg
       60                  65                  70
   gcc tcg gag cca ttt ttg ctc caa gct ttg gtg ctt gtg cac aac ctg   11812
   Ala Ser Glu Pro Phe Leu Leu Gln Ala Leu Val Leu Val His Asn Leu
   75                  80                  85                  90
   ttc tgt ttt gcg ctc agt ctg tat atg tgc gtg ggc atc gct tat cag   11860
   Phe Cys Phe Ala Leu Ser Leu Tyr Met Cys Val Gly Ile Ala Tyr Gln
                   95                  100                 105
   gct att acc tgg cgg tac tct ctc tgg ggc aat gca tac aat cct aaa   11908
   Ala Ile Thr Trp Arg Tyr Ser Leu Trp Gly Asn Ala Tyr Asn Pro Lys
               110                 115                 120
   cat aaa gag atg gcg att ctg gta tac ttg ttc tac atg tct aag tac   11956
   His Lys Glu Met Ala Ile Leu Val Tyr Leu Phe Tyr Met Ser Lys Tyr
           125                 130                 135
   gtg gaa ttc atg gat acc gtt atc atg ata ctg aag cgc agc acc agg   12004
   Val Glu Phe Met Asp Thr Val Ile Met Ile Leu Lys Arg Ser Thr Arg
       140                 145                 150
   caa ata agc ttc ctc cac gtt tat cat cat tct tca att tcc ctc att   12052
   Gln Ile Ser Phe Leu His Val Tyr His His Ser Ser Ile Ser Leu Ile
   155                 160                 165                 170
   tgg tgg gct att gct cat cac gct cct ggc ggt gaa gca tat tgg tct   12100
   Trp Trp Ala Ile Ala His His Ala Pro Gly Gly Glu Ala Tyr Trp Ser
                   175                 180                 185
   gcg gct ctg aac tca gga gtg cat gtt ctc atg tat gcg tat tac ttc   12148
   Ala Ala Leu Asn Ser Gly Val His Val Leu Met Tyr Ala Tyr Tyr Phe
               190                 195                 200
   ttg gct gcc tgc ctt cga agt agc cca aag tta aaa aat aag tac ctt     12196
   Leu Ala Ala Cys Leu Arg Ser Ser Pro Lys Leu Lys Asn Lys Tyr Leu
           205                 210                     215
   ttt tgg ggc agg tac ttg aca caa ttc caa atg ttc cag ttt atg ctg     12244
   Phe Trp Gly Arg Tyr Leu Thr Gln Phe Gln Met Phe Gln Phe Met Leu
       220                 225                     230
   aac tta gtg cag gct tac tac gac atg aaa acg aat gcg cca tat cca     12292
   Asn Leu Val Gln Ala Tyr Tyr Asp Met Lys Thr Asn Ala Pro Tyr Pro
   235                 240                     245                 250
   caa tgg ctg atc aag att ttg ttc tac tac atg atc tcg ttg ctg ttt     12340
   Gln Trp Leu Ile Lys Ile Leu Phe Tyr Tyr Met Ile Ser Leu Leu Phe
                   255                     260                 265
   ctt ttc ggc aat ttt tac gta caa aaa tac atc aaa ccc tct gac gga     12388
   Leu Phe Gly Asn Phe Tyr Val Gln Lys Tyr Ile Lys Pro Ser Asp Gly
               270                     275                     280
   aag caa aag gga gct aaa act gag tga tctagaaggc ctcctgcttt           12435
   Lys Gln Lys Gly Ala Lys Thr Glu
           285                 290
   aatgagatat gcgagacgcc tatgatcgca tgatatttgc tttcaattct gttgtgcacg  12495
   ttgtaaaaaa cctgagcatg tgtagctcag atccttaccg ccggtttcgg ttcattctaa  12555
   tgaatatatc acccgttact atcgtatttt tatgaataat attctccgtt caatttactg  12615
   attgtccgtc gagcaaattt acacattgcc actaaacgtc taaacccttg taatttgttt  12675
   ttgttttact atgtgtgtta tgtatttgat ttgcgataaa tttttatatt tggtactaaa  12735
   tttataacac cttttatgct aacgtttgcc aacacttagc aatttgcaag ttgattaatt  12795
   gattctaaat tatttttgtc ttctaaatac atatactaat caactggaaa tgtaaatatt  12855
   tgctaatatt tctactatag gagaattaaa gtgagtgaat atggtaccac aaggtttgga  12915
   gatttaattg ttgcaatgct gcatggatgg catatacacc aaacattcaa taattcttga  12975
   ggataataat ggtaccacac aagatttgag gtgcatgaac gtcacgtgga caaaaggttt  13035
   agtaattttt caagacaaca atgttaccac acacaagttt tgaggtgcat gcatggatgc  13095
   cctgtggaaa gtttaaaaat attttggaaa tgatttgcat ggaagccatg tgtaaaacca  13155
   tgacatccac ttggaggatg caataatgaa gaaaactaca aatttacatg caactagtta  13215
   tgcatgtagt ctatataatg aggattttgc aatactttca ttcatacaca ctcactaagt  13275
   tttacacgat tataatttct tcatagccag cggatcc atg gta ttc gcg ggc ggt   13330
                                            Met Val Phe Ala Gly Gly
                                                            295
   gga ctt cag cag ggc tct ctc gaa gaa aac atc gac gtc gag cac att     13378
   Gly Leu Gln Gln Gly Ser Leu Glu Glu Asn Ile Asp Val Glu His Ile
               300                     305                     310
   gcc agt atg tct ctc ttc agc gac ttc ttc agt tat gtg tct tca act     13426
   Ala Ser Met Ser Leu Phe Ser Asp Phe Phe Ser Tyr Val Ser Ser Thr
               315                  320                  325
   gtt ggt tcg tgg agc gta cac agt ata caa cct ttg aag cgc ctg acg      13474
   Val Gly Ser Trp Ser Val His Ser Ile Gln Pro Leu Lys Arg Leu Thr
       330                 335                 340
   agt aag aag cgt gtt tcg gaa agc gct gcc gtg caa tgt ata tca gct      13522
   Ser Lys Lys Arg Val Ser Glu Ser Ala Ala Val Gln Cys Ile Ser Ala
   345                 350                 355                 360
   gaa gtt cag aga aat tcg agt acc cag gga act gcg gag gca ctc gca      13570
   Glu Val Gln Arg Asn Ser Ser Thr Gln Gly Thr Ala Glu Ala Leu Ala
                   365                 370                 375
   gaa tca gtc gtg aag ccc acg aga cga agg tca tct cag tgg aag aag      13618
   Glu Ser Val Val Lys Pro Thr Arg Arg Arg Ser Ser Gln Trp Lys Lys
               380                 385                 390
   tcg aca cac ccc cta tca gaa gta gca gta cac aac aag cca agc gat      13666
   Ser Thr His Pro Leu Ser Glu Val Ala Val His Asn Lys Pro Ser Asp
           395                 400                 405
   tgc tgg att gtt gta aaa aac aag gtg tat gat gtt tcc aat ttt gcg      13714
   Cys Trp Ile Val Val Lys Asn Lys Val Tyr Asp Val Ser Asn Phe Ala
       410                 415                 420
   gac gag cat ccc gga gga tca gtt att agt act tat ttt gga cga gac      13762
   Asp Glu His Pro Gly Gly Ser Val Ile Ser Thr Tyr Phe Gly Arg Asp
   425                 430                 435                 440
   ggc aca gat gtt ttc tct agt ttt cat gca gct tct aca tgg aaa att      13810
   Gly Thr Asp Val Phe Ser Ser Phe His Ala Ala Ser Thr Trp Lys Ile
                   445                 450                 455
   ctt caa gac ttt tac att ggt gac gtg gag agg gtg gag ccg act cca      13858
   Leu Gln Asp Phe Tyr Ile Gly Asp Val Glu Arg Val Glu Pro Thr Pro
               460                 465                 470
   gag ctg ctg aaa gat ttc cga gaa atg aga gct ctt ttc ctg agg gag      13906
   Glu Leu Leu Lys Asp Phe Arg Glu Met Arg Ala Leu Phe Leu Arg Glu
           475                 480                 485
   caa ctt ttc aaa agt tcg aaa ttg tac tat gtt atg aag ctg ctc acg      13954
   Gln Leu Phe Lys Ser Ser Lys Leu Tyr Tyr Val Met Lys Leu Leu Thr
       490                 495                 500
   aat gtt gct att ttt gct gcg agc att gca ata ata tgt tgg agc aag      14002
   Asn Val Ala Ile Phe Ala Ala Ser Ile Ala Ile Ile Cys Trp Ser Lys
   505                 510                 515                 520
   act att tca gcg gtt ttg gct tca gct tgt atg atg gct ctg tgt ttc      14050
   Thr Ile Ser Ala Val Leu Ala Ser Ala Cys Met Met Ala Leu Cys Phe
                   525                 530                 535
   caa cag tgc gga tgg cta tcc cat gat ttt ctc cac aat cag gtg ttt      14098
   Gln Gln Cys Gly Trp Leu Ser His Asp Phe Leu His Asn Gln Val Phe
               540                 545                 550
   gag aca cgc tgg ctt aat gaa gtt gtc ggg tat gtg atc ggc aac gcc      14146
   Glu Thr Arg Trp Leu Asn Glu Val Val Gly Tyr Val Ile Gly Asn Ala
           555                 560                 565
   gtt ctg ggg ttt agt aca ggg tgg tgg aag gag aag cat aac ctt cat      14194
   Val Leu Gly Phe Ser Thr Gly Trp Trp Lys Glu Lys His Asn Leu His
       570                 575                 580
   cat gct gct cca aat gaa tgc gat cag act tac caa cca att gat gaa      14242
   His Ala Ala Pro Asn Glu Cys Asp Gln Thr Tyr Gln Pro Ile Asp Glu
   585                 590                 595                 600
   gat att gat act ctc ccc ctc att gcc tgg agc aag gac ata ctg gcc      14290
   Asp Ile Asp Thr Leu Pro Leu Ile Ala Trp Ser Lys Asp Ile Leu Ala
                   605                 610                 615
   aca gtt gag aat aag aca ttc ttg cga atc ctc caa tac cag cat ctg      14338
   Thr Val Glu Asn Lys Thr Phe Leu Arg Ile Leu Gln Tyr Gln His Leu
               620                 625                 630
   ttc ttc atg ggt ctg tta ttt ttc gcc cgt ggt agt tgg ctc ttt tgg      14386
   Phe Phe Met Gly Leu Leu Phe Phe Ala Arg Gly Ser Trp Leu Phe Trp
           635                 640                 645
   agc tgg aga tat acc tct aca gca gtg ctc tca cct gtc gac agg ttg      14434
   Ser Trp Arg Tyr Thr Ser Thr Ala Val Leu Ser Pro Val Asp Arg Leu
       650                 655                 660
   ttg gag aag gga act gtt ctg ttt cac tac ttt tgg ttc gtc ggg aca      14482
   Leu Glu Lys Gly Thr Val Leu Phe His Tyr Phe Trp Phe Val Gly Thr
   665                 670                 675                 680
   gcg tgc tat ctt ctc cct ggt tgg aag cca tta gta tgg atg gcg gtg      14530
   Ala Cys Tyr Leu Leu Pro Gly Trp Lys Pro Leu Val Trp Met Ala Val
                   685                 690                 695
   act gag ctc atg tcc ggc atg ctg ctg ggc ttt gta ttt gta ctt agc      14578
   Thr Glu Leu Met Ser Gly Met Leu Leu Gly Phe Val Phe Val Leu Ser
               700                 705                 710
   cac aat ggg atg gag gtt tat aat tcg tct aaa gaa ttc gtg agt gca      14626
   His Asn Gly Met Glu Val Tyr Asn Ser Ser Lys Glu Phe Val Ser Ala
           715                 720                 725
   cag atc gta tcc aca cgg gat atc aaa gga aac ata ttc aac gac tgg      14674
   Gln Ile Val Ser Thr Arg Asp Ile Lys Gly Asn Ile Phe Asn Asp Trp
       730                 735                 740
   ttc act ggt ggc ctt aac agg caa ata gag cat cat ctt ttc cca aca      14722
   Phe Thr Gly Gly Leu Asn Arg Gln Ile Glu His His Leu Phe Pro Thr
   745                 750                 755                 760
   atg ccc agg cat aat tta aac aaa ata gca cct aga gtg gag gtg ttc      14770
   Met Pro Arg His Asn Leu Asn Lys Ile Ala Pro Arg Val Glu Val Phe
                   765                 770                 775
   tgt aag aaa cac ggt ctg gtg tac gaa gac gta tct att gct acc ggc      14818
   Cys Lys Lys His Gly Leu Val Tyr Glu Asp Val Ser Ile Ala Thr Gly
               780                 785                 790
   act tgc aag gtt ttg aaa gca ttg aag gaa gtc gcg gag gct gcg gca      14866
   Thr Cys Lys Val Leu Lys Ala Leu Lys Glu Val Ala Glu Ala Ala Ala
           795                 800                 805
   gag cag cat gct acc acc agt taa gctagcgtta accctgcttt aatgagatat    14920
   Glu Gln His Ala Thr Thr Ser
       810                 815
   gcgagacgcc tatgatcgca tgatatttgc tttcaattct gttgtgcacg ttgtaaaaaa  14980
   cctgagcatg tgtagctcag atccttaccg ccggtttcgg ttcattctaa tgaatatatc  15040
   acccgttact atcgtatttt tatgaataat attctccgtt caatttactg attgtccgtc  15100
   gagcaaattt acacattgcc actaaacgtc taaacccttg taatttgttt ttgttttact  15160
   atgtgtgtta tgtatttgat ttgcgataaa tttttatatt tggtactaaa tttataacac  15220
   cttttatgct aacgtttgcc aacacttagc aatttgcaag ttgattaatt gattctaaat  15280
   tatttttgtc ttctaaatac atatactaat caactggaaa tgtaaatatt tgctaatatt  15340
   tctactatag gagaattaaa gtgagtgaat atggtaccac aaggtttgga gatttaattg  15400
   ttgcaatgct gcatggatgg catatacacc aaacattcaa taattcttga ggataataat  15460
   ggtaccacac aagatttgag gtgcatgaac gtcacgtgga caaaaggttt agtaattttt  15520
   caagacaaca atgttaccac acacaagttt tgaggtgcat gcatggatgc cctgtggaaa  15580
   gtttaaaaat attttggaaa tgatttgcat ggaagccatg tgtaaaacca tgacatccac  15640
   ttggaggatg caataatgaa gaaaactaca aatttacatg caactagtta tgcatgtagt  15700
   ctatataatg aggattttgc aatactttca ttcatacaca ctcactaagt tttacacgat  15760
   tataatttct tcatagccag cagatctaaa atg gct ccg gat gcg gat aag ctt   15814
                                    Met Ala Pro Asp Ala Asp Lys Leu
                                                    820
   cga caa cgc cag acg act gcg gta gcg aag cac aat gct gct acc ata    15862
   Arg Gln Arg Gln Thr Thr Ala Val Ala Lys His Asn Ala Ala Thr Ile
       825                 830                 835
   tcg acg cag gaa cgc ctt tgc agt ctg tct tcg ctc aaa ggc gaa gaa    15910
   Ser Thr Gln Glu Arg Leu Cys Ser Leu Ser Ser Leu Lys Gly Glu Glu
   840                 845                 850                 855
   gtc tgc atc gac gga atc atc tat gac ctc caa tca ttc gat cat ccc    15958
   Val Cys Ile Asp Gly Ile Ile Tyr Asp Leu Gln Ser Phe Asp His Pro
                   860                 865                 870
   ggg ggt gaa acg atc aaa atg ttt ggt ggc aac gat gtc act gta cag    16006
   Gly Gly Glu Thr Ile Lys Met Phe Gly Gly Asn Asp Val Thr Val Gln
               875                 880                 885
   tac aag atg att cac ccg tac cat acc gag aag cat ttg gaa aag atg    16054
   Tyr Lys Met Ile His Pro Tyr His Thr Glu Lys His Leu Glu Lys Met
           890                 895                 900
   aag cgt gtc ggc aag gtg acg gat ttc gtc tgc gag tac aag ttc gat    16102
   Lys Arg Val Gly Lys Val Thr Asp Phe Val Cys Glu Tyr Lys Phe Asp
       905                 910                 915
   acc gaa ttt gaa cgc gaa atc aaa cga gaa gtc ttc aag att gtg cga    16150
   Thr Glu Phe Glu Arg Glu Ile Lys Arg Glu Val Phe Lys Ile Val Arg
   920                 925                 930                 935
   cga ggc aag gat ttc ggt act ttg gga tgg ttc ttc cgt gcg ttt tgc   16198
   Arg Gly Lys Asp Phe Gly Thr Leu Gly Trp Phe Phe Arg Ala Phe Cys
                   940                 945                 950
   tac att gcc att ttc ttc tac ctg cag tac cat tgg gtc acc acg gga   16246
   Tyr Ile Ala Ile Phe Phe Tyr Leu Gln Tyr His Trp Val Thr Thr Gly
               955                 960                 965
   acc tct tgg ctg ctg gcc gtg gcc tac gga atc tcc caa gcg atg att   16294
   Thr Ser Trp Leu Leu Ala Val Ala Tyr Gly Ile Ser Gln Ala Met Ile
           970                 975                 980
   ggc atg aat gtc cag cac gat gcc aac cac ggg gcc acc tcc aag cgt   16342
   Gly Met Asn Val Gln His Asp Ala Asn His Gly Ala Thr Ser Lys Arg
       985                 990                 995
   ccc  tgg gtc aac gac atg  cta ggc ctc ggt gcg  gat ttt att ggt   16387
   Pro  Trp Val Asn Asp Met  Leu Gly Leu Gly Ala  Asp Phe Ile Gly
   1000                 1005                 1010
   ggt  tcc aag tgg ctc tgg  cag gaa caa cac tgg  acc cac cac gct   16432
   Gly  Ser Lys Trp Leu Trp  Gln Glu Gln His Trp  Thr His His Ala
   1015                 1020                 1025
   tac  acc aat cac gcc gag  atg gat ccc gat agc  ttt ggt gcc gaa   16477
   Tyr  Thr Asn His Ala Glu  Met Asp Pro Asp Ser  Phe Gly Ala Glu
   1030                 1035                 1040
   cca  atg ctc cta ttc aac  gac tat ccc ttg gat  cat ccc gct cgt   16522
   Pro  Met Leu Leu Phe Asn  Asp Tyr Pro Leu Asp  His Pro Ala Arg
   1045                 1050                 1055
   acc  tgg cta cat cgc ttt  caa gca ttc ttt tac  atg ccc gtc ttg   16567
   Thr  Trp Leu His Arg Phe  Gln Ala Phe Phe Tyr  Met Pro Val Leu
   1060                 1065                 1070
   gct  gga tac tgg ttg tcc  gct gtc ttc aat cca  caa att ctt gac   16612
   Ala  Gly Tyr Trp Leu Ser  Ala Val Phe Asn Pro  Gln Ile Leu Asp
   1075                 1080                 1085
   ctc  cag caa cgc ggc gca  ctt tcc gtc ggt atc  cgt ctc gac aac   16657
   Leu  Gln Gln Arg Gly Ala  Leu Ser Val Gly Ile  Arg Leu Asp Asn
   1090                 1095                 1100
   gct  ttc att cac tcg cga  cgc aag tat gcg gtt  ttc tgg cgg gct   16702
   Ala  Phe Ile His Ser Arg  Arg Lys Tyr Ala Val  Phe Trp Arg Ala
   1105                 1110                 1115
   gtg  tac att gcg gtg aac  gtg att gct ccg ttt  tac aca aac tcc   16747
   Val  Tyr Ile Ala Val Asn  Val Ile Ala Pro Phe  Tyr Thr Asn Ser
   1120                 1125                 1130
   ggc  ctc gaa tgg tcc tgg  cgt gtc ttt gga aac  atc atg ctc atg   16792
   Gly  Leu Glu Trp Ser Trp  Arg Val Phe Gly Asn  Ile Met Leu Met
   1135                 1140                 1145
   ggt  gtg gcg gaa tcg ctc  gcg ctg gcg gtc ctg  ttt tcg ttg tcg   16837
   Gly  Val Ala Glu Ser Leu  Ala Leu Ala Val Leu  Phe Ser Leu Ser
   1150                 1155                 1160
   cac  aat ttc gaa tcc gcg  gat cgc gat ccg acc  gcc cca ctg aaa   16882
   His  Asn Phe Glu Ser Ala  Asp Arg Asp Pro Thr  Ala Pro Leu Lys
   1165                    1170                    1175
   aag  acg gga gaa cca gtc  gac tgg ttc aag aca  cag gtc gaa act      16927
   Lys  Thr Gly Glu Pro Val  Asp Trp Phe Lys Thr  Gln Val Glu Thr
   1180                 1185                 1190
   tcc  tgc act tac ggt gga  ttc ctt tcc ggt tgc  ttc acg gga ggt      16972
   Ser  Cys Thr Tyr Gly Gly  Phe Leu Ser Gly Cys  Phe Thr Gly Gly
   1195                 1200                 1205
   ctc  aac ttt cag gtt gaa  cac cac ttg ttc cca  cgc atg agc agc      17017
   Leu  Asn Phe Gln Val Glu  His His Leu Phe Pro  Arg Met Ser Ser
   1210                 1215                 1220
   gct  tgg tat ccc tac att  gcc ccc aag gtc cgc  gaa att tgc gcc      17062
   Ala  Trp Tyr Pro Tyr Ile  Ala Pro Lys Val Arg  Glu Ile Cys Ala
   1225                 1230                 1235
   aaa  cac ggc gtc cac tac  gcc tac tac ccg tgg  atc cac caa aac      17107
   Lys  His Gly Val His Tyr  Ala Tyr Tyr Pro Trp  Ile His Gln Asn
   1240                 1245                 1250
   ttt  ctc tcc acc gtc cgc  tac atg cac gcg gcc  ggg acc ggt gcc      17152
   Phe  Leu Ser Thr Val Arg  Tyr Met His Ala Ala  Gly Thr Gly Ala
   1255                 1260                 1265
   aac  tgg cgc cag atg gcc  aga gaa aat ccc ttg  acc gga cgg gcg      17197
   Asn  Trp Arg Gln Met Ala  Arg Glu Asn Pro Leu  Thr Gly Arg Ala
   1270                 1275                 1280
   taa agatctgccg gcatcgatcc cgggccatgg cctgctttaa tgagatatgc          17250
   gagacgccta tgatcgcatg atatttgctt tcaattctgt tgtgcacgtt gtaaaaaacc  17310
   tgagcatgtg tagctcagat ccttaccgcc ggtttcggtt cattctaatg aatatatcac  17370
   ccgttactat cgtattttta tgaataatat tctccgttca atttactgat tgtccgtcga  17430
   cgagctcggc gcgcctctag aggatcgatg aattcagatc ggctgagtgg ctccttcaac  17490
   gttgcggttc tgtcagttcc aaacgtaaaa cggcttgtcc cgcgtcatcg gcgggggtca  17550
   taacgtgact cccttaattc tccgctcatg atcagattgt cgtttcccgc cttcagttta  17610
   aactatcagt gtttgacagg atatattggc gggtaaacct aagagaaaag agcgtttatt  17670
   agaataatcg gatatttaaa agggcgtgaa aaggtttatc cttcgtccat ttgtatgtgc  17730
   atgccaacca cagggttccc ca                                            17752
<210> 20
   <211> 290
   <212> PRT
   <213> Phaeodactylum tricornutum, Physcomitrella patens
<400> 20
   Met Glu Val Val Glu Arg Phe Tyr Gly Glu Leu Asp Gly Lys Val Ser
   1               5                   10                  15
   Gln Gly Val Asn Ala Leu Leu Gly Ser Phe Gly Val Glu Leu Thr Asp
               20                  25                  30
   Thr Pro Thr Thr Lys Gly Leu Pro Leu Val Asp Ser Pro Thr Pro Ile
           35                  40                  45
   Val Leu Gly Val Ser Val Tyr Leu Thr Ile Val Ile Gly Gly Leu Leu
       50                  55                  60
   Trp Ile Lys Ala Arg Asp Leu Lys Pro Arg Ala Ser Glu Pro Phe Leu
   65                  70                  75                  80
   Leu Gln Ala Leu Val Leu Val His Asn Leu Phe Cys Phe Ala Leu Ser
                   85                  90                  95
   Leu Tyr Met Cys Val Gly Ile Ala Tyr Gln Ala Ile Thr Trp Arg Tyr
               100                 105                 110
   Ser Leu Trp Gly Asn Ala Tyr Asn Pro Lys His Lys Glu Met Ala Ile
           115                 120                 125
   Leu Val Tyr Leu Phe Tyr Met Ser Lys Tyr Val Glu Phe Met Asp Thr
       130                 135                 140
   Val Ile Met Ile Leu Lys Arg Ser Thr Arg Gln Ile Ser Phe Leu His
   145                 150                 155                 160
   Val Tyr His His Ser Ser Ile Ser Leu Ile Trp Trp Ala Ile Ala His
                   165                 170                 175
   His Ala Pro Gly Gly Glu Ala Tyr Trp Ser Ala Ala Leu Asn Ser Gly
               180                 185                 190
   Val His Val Leu Met Tyr Ala Tyr Tyr Phe Leu Ala Ala Cys Leu Arg
           195                 200                 205
   Ser Ser Pro Lys Leu Lys Asn Lys Tyr Leu Phe Trp Gly Arg Tyr Leu
       210                 215                 220
   Thr Gln Phe Gln Met Phe Gln Phe Met Leu Asn Leu Val Gln Ala Tyr
   225                 230                 235                 240
   Tyr Asp Met Lys Thr Asn Ala Pro Tyr Pro Gln Trp Leu Ile Lys Ile
                   245                 250                 255
   Leu Phe Tyr Tyr Met Ile Ser Leu Leu Phe Leu Phe Gly Asn Phe Tyr
               260                 265                 270
   Val Gln Lys Tyr Ile Lys Pro Ser Asp Gly Lys Gln Lys Gly Ala Lys
   275 280 285
   Thr Glu
   290
<210> 21
   <211> 525
   <212> PRT
   <213> Phaeodactylum tricornutum, Physcomitrella patens
<400> 21
   Met Val Phe Ala Gly Gly Gly Leu Gln Gln Gly Ser Leu Glu Glu Asn
   1               5                   10                  15
   Ile Asp Val Glu His Ile Ala Ser Met Ser Leu Phe Ser Asp Phe Phe
               20                  25                  30
   Ser Tyr Val Ser Ser Thr Val Gly Ser Trp Ser Val His Ser Ile Gln
           35                  40                  45
   Pro Leu Lys Arg Leu Thr Ser Lys Lys Arg Val Ser Glu Ser Ala Ala
       50                  55                  60
   Val Gln Cys Ile Ser Ala Glu Val Gln Arg Asn Ser Ser Thr Gln Gly
   65                  70                  75                  80
   Thr Ala Glu Ala Leu Ala Glu Ser Val Val Lys Pro Thr Arg Arg Arg
                   85                  90                  95
   Ser Ser Gln Trp Lys Lys Ser Thr His Pro Leu Ser Glu Val Ala Val
               100                 105                 110
   His Asn Lys Pro Ser Asp Cys Trp Ile Val Val Lys Asn Lys Val Tyr
           115                 120                 125
   Asp Val Ser Asn Phe Ala Asp Glu His Pro Gly Gly Ser Val Ile Ser
       130                 135                 140
   Thr Tyr Phe Gly Arg Asp Gly Thr Asp Val Phe Ser Ser Phe His Ala
   145                 150                 155                 160
   Ala Ser Thr Trp Lys Ile Leu Gln Asp Phe Tyr Ile Gly Asp Val Glu
                   165                 170                 175
   Arg Val Glu Pro Thr Pro Glu Leu Leu Lys Asp Phe Arg Glu Met Arg
   180 185 190
   Ala Leu Phe Leu Arg Glu Gln Leu Phe Lys Ser Ser Lys Leu Tyr Tyr
   195 200 205
   Val Met Lys Leu Leu Thr Asn Val Ala Ile Phe Ala Ala Ser Ile Ala
   210 215 220
   Ile Ile Cys Trp Ser Lys Thr Ile Ser Ala Val Leu Ala Ser Ala Cys
   225 230 235 240
   Met Met Ala Leu Cys Phe Gln Gln Cys Gly Trp Leu Ser His Asp Phe
   245 250 255
   Leu His Asn Gln Val Phe Glu Thr Arg Trp Leu Asn Glu Val Val Gly
   260 265 270
   Tyr Val Ile Gly Asn Ala Val Leu Gly Phe Ser Thr Gly Trp Trp Lys
   275 280 285
   Glu Lys His Asn Leu His His Ala Ala Pro Asn Glu Cys Asp Gln Thr
   290 295 300
   Tyr Gln Pro Ile Asp Glu Asp Ile Asp Thr Leu Pro Leu Ile Ala Trp
   305 310 315 320
   Ser Lys Asp Ile Leu Ala Thr Val Glu Asn Lys Thr Phe Leu Arg Ile
   325 330 335
   Leu Gln Tyr Gln His Leu Phe Phe Met Gly Leu Leu Phe Phe Ala Arg
   340 345 350
   Gly Ser Trp Leu Phe Trp Ser Trp Arg Tyr Thr Ser Thr Ala Val Leu
   355 360 365
   Ser Pro Val Asp Arg Leu Leu Glu Lys Gly Thr Val Leu Phe His Tyr
   370 375 380
   Phe Trp Phe Val Gly Thr Ala Cys Tyr Leu Leu Pro Gly Trp Lys Pro
   385 390 395 400
   Leu Val Trp Met Ala Val Thr Glu Leu Met Ser Gly Met Leu Leu Gly
   405 410 415
   Phe Val Phe Val Leu Ser His Asn Gly Met Glu Val Tyr Asn Ser Ser
   420 425 430
   Lys Glu Phe Val Ser Ala Gln Ile Val Ser Thr Arg Asp Ile Lys Gly
   435 440 445
   Asn Ile Phe Asn Asp Trp Phe Thr Gly Gly Leu Asn Arg Gln Ile Glu
   450 455 460
   His His Leu Phe Pro Thr Met Pro Arg His Asn Leu Asn Lys Ile Ala
   465 470 475 480
   Pro Arg Val Glu Val Phe Cys Lys Lys His Gly Leu Val Tyr Glu Asp
   485 490 495
   Val Ser Ile Ala Thr Gly Thr Cys Lys Val Leu Lys Ala Leu Lys Glu
   500 505 510
   Val Ala Glu Ala Ala Ala Glu Gln His Ala Thr Thr Ser
   515 520 525
<210> 23
   <211> 26
   <212> DNA
   <213> artificial sequence
<400> 23
   gaattcggcg cgccgagctc ctcgag 26
<210> 24
   <211> 265
   <212> DNA
   <213> artificial sequence
<400> 24
   ccaccgcggt gggcggccgc ctgcagtcta gaaggcctcc tgctttaatg agatatgcga 60
   gacgcctatg atcgcatgat atttgctttc aattctgttg tgcacgttgt aaaaaacctg 120
   agcatgtgta gctcagatcc ttaccgccgg tttcggttca ttctaatgaa tatatcaccc 180
   gttactatcg tatttttatg aataatattc tccgttcaat ttactgattg tccgtcgacg 240
   aattcgagct cggcgcgcca agctt 265
<210> 25
   <211> 257
   <212> DNA
   <213> artificial sequence
<400> 25
   ggatccgata tcgggcccgc tagcgttaac cctgctttaa tgagatatgc gagacgccta 60
   tgatcgcatg atatttgctt tcaattctgt tgtgcacgtt gtaaaaaacc tgagcatgtg 120
   tagctcagat ccttaccgcc ggtttcggtt cattctaatg aatatatcac ccgttactat 180
   cgtattttta tgaataatat tctccgttca atttactgat tgtccgtcga cgaattcgag 240
   ctcggcgcgc caagctt 257
<210> 26
   <211> 5410
   <212> DNA
   <213> artificial sequence
<400> 26
   ttttggaaat gatttgcatg gaagccatgt gtaaaaccat gacatccact tggaggatgc 60
   aataatgaag aaaactacaa atttacatgc aactagttat gcatgtagtc tatataatga 120
   ggattttgca atactttcat tcatacacac tcactaagtt ttacacgatt ataatttctt 180
   catagccagc ggatccgata tcgggcccgc tagcgttaac cctgctttaa tgagatatgc 240
   gagacgccta tgatcgcatg atatttgctt tcaattctgt tgtgcacgtt gtaaaaaacc 300
   tgagcatgtg tagctcagat ccttaccgcc ggtttcggtt cattctaatg aatatatcac 360
   ccgttactat cgtattttta tgaataatat tctccgttca atttactgat tgtccgtcga 420
   gcaaatttac acattgccac taaacgtcta aacccttgta atttgttttt gttttactat 480
   gtgtgttatg tatttgattt gcgataaatt tttatatttg gtactaaatt tataacacct 540
   tttatgctaa cgtttgccaa cacttagcaa tttgcaagtt gattaattga ttctaaatta 600
   tttttgtctt ctaaatacat atactaatca actggaaatg taaatatttg ctaatatttc 660
   tactatagga gaattaaagt gagtgaatat ggtaccacaa ggtttggaga tttaattgtt 720
   gcaatgctgc atggatggca tatacaccaa acattcaata attcttgagg ataataatgg 780
   taccacacaa gatttgaggt gcatgaacgt cacgtggaca aaaggtttag taatttttca 840
   agacaacaat gttaccacac acaagttttg aggtgcatgc atggatgccc tgtggaaagt 900
   ttaaaaatat tttggaaatg atttgcatgg aagccatgtg taaaaccatg acatccactt 960
   ggaggatgca ataatgaaga aaactacaaa tttacatgca actagttatg catgtagtct 1020
   atataatgag gattttgcaa tactttcatt catacacact cactaagttt tacacgatta 1080
   taatttcttc atagccagca gatctgccgg catcgatccc gggccatggc ctgctttaat 1140
   gagatatgcg agacgcctat gatcgcatga tatttgcttt caattctgtt gtgcacgttg 1200
   taaaaaacct gagcatgtgt agctcagatc cttaccgccg gtttcggttc attctaatga 1260
   atatatcacc cgttactatc gtatttttat gaataatatt ctccgttcaa tttactgatt 1320
   gtccgtcgac gagctcggcg cgccaagctt ggcgtaatca tggtcatagc tgtttcctgt 1380
   gtgaaattgt tatccgctca caattccaca caacatacga gccggaagca taaagtgtaa 1440
   agcctggggt gcctaatgag tgagctaact cacattaatt gcgttgcgct cactgcccgc 1500
   tttccagtcg ggaaacctgt cgtgccagct gcattaatga atcggccaac gcgcggggag 1560
   aggcggtttg cgtattgggc gctcttccgc ttcctcgctc actgactcgc tgcgctcggt 1620
   cgttcggctg cggcgagcgg tatcagctca ctcaaaggcg gtaatacggt tatccacaga 1680
   atcaggggat aacgcaggaa agaacatgtg agcaaaaggc cagcaaaagg ccaggaaccg 1740
   taaaaaggcc gcgttgctgg cgtttttcca taggctccgc ccccctgacg agcatcacaa 1800
   aaatcgacgc tcaagtcaga ggtggcgaaa cccgacagga ctataaagat accaggcgtt 1860
   tccccctgga agctccctcg tgcgctctcc tgttccgacc ctgccgctta ccggatacct 1920
   gtccgccttt ctcccttcgg gaagcgtggc gctttctcat agctcacgct gtaggtatct 1980
   cagttcggtg taggtcgttc gctccaagct gggctgtgtg cacgaacccc ccgttcagcc 2040
   cgaccgctgc gccttatccg gtaactatcg tcttgagtcc aacccggtaa gacacgactt 2100
   atcgccactg gcagcagcca ctggtaacag gattagcaga gcgaggtatg taggcggtgc 2160
   tacagagttc ttgaagtggt ggcctaacta cggctacact agaaggacag tatttggtat 2220
   ctgcgctctg ctgaagccag ttaccttcgg aaaaagagtt ggtagctctt gatccggcaa 2280
   acaaaccacc gctggtagcg gtggtttttt tgtttgcaag cagcagatta cgcgcagaaa 2340
   aaaaggatct caagaagatc ctttgatctt ttctacgggg tctgacgctc agtggaacga 2400
   aaactcacgt taagggattt tggtcatgag attatcaaaa aggatcttca cctagatcct 2460
   tttaaattaa aaatgaagtt ttaaatcaat ctaaagtata tatgagtaaa cttggtctga 2520
   cagttaccaa tgcttaatca gtgaggcacc tatctcagcg atctgtctat ttcgttcatc 2580
   catagttgcc tgactccccg tcgtgtagat aactacgata cgggagggct taccatctgg 2640
   ccccagtgct gcaatgatac cgcgagaccc acgctcaccg gctccagatt tatcagcaat 2700
   aaaccagcca gccggaaggg ccgagcgcag aagtggtcct gcaactttat ccgcctccat 2760
   ccagtctatt aattgttgcc gggaagctag agtaagtagt tcgccagtta atagtttgcg 2820
   caacgttgtt gccattgcta caggcatcgt ggtgtcacgc tcgtcgtttg gtatggcttc 2880
   attcagctcc ggttcccaac gatcaaggcg agttacatga tcccccatgt tgtgcaaaaa 2940
   agcggttagc tccttcggtc ctccgatcgt tgtcagaagt aagttggccg cagtgttatc 3000
   actcatggtt atggcagcac tgcataattc tcttactgtc atgccatccg taagatgctt 3060
   ttctgtgact ggtgagtact caaccaagtc attctgagaa tagtgtatgc ggcgaccgag 3120
   ttgctcttgc ccggcgtcaa tacgggataa taccgcgcca catagcagaa ctttaaaagt 3180
   gctcatcatt ggaaaacgtt cttcggggcg aaaactctca aggatcttac cgctgttgag 3240
   atccagttcg atgtaaccca ctcgtgcacc caactgatct tcagcatctt ttactttcac 3300
   cagcgtttct gggtgagcaa aaacaggaag gcaaaatgcc gcaaaaaagg gaataagggc    3360
   gacacggaaa tgttgaatac tcatactctt cctttttcaa tattattgaa gcatttatca    3420
   gggttattgt ctcatgagcg gatacatatt tgaatgtatt tagaaaaata aacaaatagg    3480
   ggttccgcgc acatttcccc gaaaagtgcc acctgacgtc taagaaacca ttattatcat    3540
   gacattaacc tataaaaata ggcgtatcac gaggcccttt cgtctcgcgc gtttcggtga    3600
   tgacggtgaa aacctctgac acatgcagct cccggagacg gtcacagctt gtctgtaagc    3660
   ggatgccggg agcagacaag cccgtcaggg cgcgtcagcg ggtgttggcg ggtgtcgggg    3720
   ctggcttaac tatgcggcat cagagcagat tgtactgaga gtgcaccata tgcggtgtga    3780
   aataccgcac agatgcgtaa ggagaaaata ccgcatcagg cgccattcgc cattcaggct    3840
   gcgcaactgt tgggaagggc gatcggtgcg ggcctcttcg ctattacgcc agctggcgaa    3900
   agggggatgt gctgcaaggc gattaagttg ggtaacgcca gggttttccc agtcacgacg    3960
   ttgtaaaacg acggccagtg aattcggcgc gccgagctcc tcgagcaaat ttacacattg    4020
   ccactaaacg tctaaaccct tgtaatttgt ttttgtttta ctatgtgtgt tatgtatttg    4080
   atttgcgata aatttttata tttggtacta aatttataac accttttatg ctaacgtttg    4140
   ccaacactta gcaatttgca agttgattaa ttgattctaa attatttttg tcttctaaat    4200
   acatatacta atcaactgga aatgtaaata tttgctaata tttctactat aggagaatta    4260
   aagtgagtga atatggtacc acaaggtttg gagatttaat tgttgcaatg ctgcatggat    4320
   ggcatataca ccaaacattc aataattctt gaggataata atggtaccac acaagatttg    4380
   aggtgcatga acgtcacgtg gacaaaaggt ttagtaattt ttcaagacaa caatgttacc    4440
   acacacaagt tttgaggtgc atgcatggat gccctgtgga aagtttaaaa atattttgga    4500
   aatgatttgc atggaagcca tgtgtaaaac catgacatcc acttggagga tgcaataatg    4560
   aagaaaacta caaatttaca tgcaactagt tatgcatgta gtctatataa tgaggatttt    4620
   gcaatacttt cattcataca cactcactaa gttttacacg attataattt cttcatagcc    4680
   agcccaccgc ggtgggcggc cgcctgcagt ctagaaggcc tcctgcttta atgagatatg    4740
   cgagacgcct atgatcgcat gatatttgct ttcaattctg ttgtgcacgt tgtaaaaaac    4800
   ctgagcatgt gtagctcaga tccttaccgc cggtttcggt tcattctaat gaatatatca    4860
   cccgttacta tcgtattttt atgaataata ttctccgttc aatttactga ttgtccgtcg    4920
   agcaaattta cacattgcca ctaaacgtct aaacccttgt aatttgtttt tgttttacta    4980
   tgtgtgttat gtatttgatt tgcgataaat ttttatattt ggtactaaat ttataacacc    5040
   ttttatgcta acgtttgcca acacttagca atttgcaagt tgattaattg attctaaatt    5100
   atttttgtct tctaaataca tatactaatc aactggaaat gtaaatattt gctaatattt    5160
   ctactatagg agaattaaag tgagtgaata tggtaccaca aggtttggag atttaattgt 5220
   tgcaatgctg catggatggc atatacacca aacattcaat aattcttgag gataataatg 5280
   gtaccacaca agatttgagg tgcatgaacg tcacgtggac aaaaggttta gtaatttttc 5340
   aagacaacaa tgttaccaca cacaagtttt gaggtgcatg catggatgcc ctgtggaaag 5400
   tttaaaaata 5410
<210> 27
   <211> 12093
   <212> DNA
   <213> artificial sequence
<400> 27
<210> 28
   <211> 12085
   <212> DNA
   <213> artificial sequence
<220>
   <221> misc_feature
   <223> pflanzlicher Expressionsvektor mit einer Promotor-Terminator-Expr essionskassette
<400> 28
   gatctggcgc cggccagcga gacgagcaag attggccgcc gcccgaaacg atccgacagc 60
   gcgcccagca caggtgcgca ggcaaattgc accaacgcat acagcgccag cagaatgcca 120
   tagtgggcgg tgacgtcgtt cgagtgaacc agatcgcgca ggaggcccgg cagcaccggc 180
   ataatcaggc cgatgccgac agcgtcgagc gcgacagtgc tcagaattac gatcaggggt 240
   atgttgggtt tcacgtctgg cctccggacc agcctccgct ggtccgattg aacgcgcgga 300
   ttctttatca ctgataagtt ggtggacata ttatgtttat cagtgataaa gtgtcaagca 360
   tgacaaagtt gcagccgaat acagtgatcc gtgccgccct ggacctgttg aacgaggtcg 420
   gcgtagacgg tctgacgaca cgcaaactgg cggaacggtt gggggttcag cagccggcgc 480
   tttactggca cttcaggaac aagcgggcgc tgctcgacgc actggccgaa gccatgctgg 540
   cggagaatca tacgcattcg gtgccgagag ccgacgacga ctggcgctca tttctgatcg 600
   ggaatgcccg cagcttcagg caggcgctgc tcgcctaccg cgatggcgcg cgcatccatg 660
   ccggcacgcg accgggcgca ccgcagatgg aaacggccga cgcgcagctt cgcttcctct 720
   gcgaggcggg tttttcggcc ggggacgccg tcaatgcgct gatgacaatc agctacttca 780
   ctgttggggc cgtgcttgag gagcaggccg gcgacagcga tgccggcgag cgcggcggca 840
   ccgttgaaca ggctccgctc tcgccgctgt tgcgggccgc gatagacgcc ttcgacgaag 900
   ccggtccgga cgcagcgttc gagcagggac tcgcggtgat tgtcgatgga ttggcgaaaa 960
   ggaggctcgt tgtcaggaac gttgaaggac cgagaaaggg tgacgattga tcaggaccgc 1020
   tgccggagcg caacccactc actacagcag agccatgtag acaacatccc ctcccccttt 1080
   ccaccgcgtc agacgcccgt agcagcccgc tacgggcttt ttcatgccct gccctagcgt 1140
   ccaagcctca cggccgcgct cggcctctct ggcggccttc tggcgctctt ccgcttcctc 1200
   gctcactgac tcgctgcgct cggtcgttcg gctgcggcga gcggtatcag ctcactcaaa 1260
   ggcggtaata cggttatcca cagaatcagg ggataacgca ggaaagaaca tgtgagcaaa 1320
   aggccagcaa aaggccagga accgtaaaaa ggccgcgttg ctggcgtttt tccataggct 1380
   ccgcccccct gacgagcatc acaaaaatcg acgctcaagt cagaggtggc gaaacccgac 1440
   aggactataa agataccagg cgtttccccc tggaagctcc ctcgtgcgct ctcctgttcc 1500
   gaccctgccg cttaccggat acctgtccgc ctttctccct tcgggaagcg tggcgctttt 1560
   ccgctgcata accctgcttc ggggtcatta tagcgatttt ttcggtatat ccatcctttt 1620
   tcgcacgata tacaggattt tgccaaaggg ttcgtgtaga ctttccttgg tgtatccaac 1680
   ggcgtcagcc gggcaggata ggtgaagtag gcccacccgc gagcgggtgt tccttcttca 1740
   ctgtccctta ttcgcacctg gcggtgctca acgggaatcc tgctctgcga ggctggccgg 1800
   ctaccgccgg cgtaacagat gagggcaagc ggatggctga tgaaaccaag ccaaccagga 1860
   agggcagccc acctatcaag gtgtactgcc ttccagacga acgaagagcg attgaggaaa 1920
   aggcggcggc ggccggcatg agcctgtcgg cctacctgct ggccgtcggc cagggctaca 1980
   aaatcacggg cgtcgtggac tatgagcacg tccgcgagct ggcccgcatc aatggcgacc 2040
   tgggccgcct gggcggcctg ctgaaactct ggctcaccga cgacccgcgc acggcgcggt 2100
   tcggtgatgc cacgatcctc gccctgctgg cgaagatcga agagaagcag gacgagcttg 2160
   gcaaggtcat gatgggcgtg gtccgcccga gggcagagcc atgacttttt tagccgctaa 2220
   aacggccggg gggtgcgcgt gattgccaag cacgtcccca tgcgctccat caagaagagc 2280
   gacttcgcgg agctggtgaa gtacatcacc gacgagcaag gcaagaccga gcgcctttgc 2340
   gacgctcacc gggctggttg ccctcgccgc tgggctggcg gccgtctatg gccctgcaaa 2400
   cgcgccagaa acgccgtcga agccgtgtgc gagacaccgc ggccgccggc gttgtggata 2460
   cctcgcggaa aacttggccc tcactgacag atgaggggcg gacgttgaca cttgaggggc 2520
   cgactcaccc ggcgcggcgt tgacagatga ggggcaggct cgatttcggc cggcgacgtg 2580
   gagctggcca gcctcgcaaa tcggcgaaaa cgcctgattt tacgcgagtt tcccacagat 2640
   gatgtggaca agcctgggga taagtgccct gcggtattga cacttgaggg gcgcgactac 2700
   tgacagatga ggggcgcgat ccttgacact tgaggggcag agtgctgaca gatgaggggc 2760
   gcacctattg acatttgagg ggctgtccac aggcagaaaa tccagcattt gcaagggttt 2820
   ccgcccgttt ttcggccacc gctaacctgt cttttaacct gcttttaaac caatatttat 2880
   aaaccttgtt tttaaccagg gctgcgccct gtgcgcgtga ccgcgcacgc cgaagggggg 2940
   tgccccccct tctcgaaccc tcccggcccg ctaacgcggg cctcccatcc ccccaggggc 3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa 3480
   tactgataag ataatatatc ttttatatag aagatatcgc cgtatgtaag gatttcaggg 3540
   ggcaaggcat aggcagcgcg cttatcaata tatctataga atgggcaaag cataaaaact 3600
   tgcatggact aatgcttgaa acccaggaca ataaccttat agcttgtaaa ttctatcata 3660
   attgggtaat gactccaact tattgatagt gttttatgtt cagataatgc ccgatgactt 3720
   tgtcatgcag ctccaccgat tttgagaacg acagcgactt ccgtcccagc cgtgccaggt 3780
   gctgcctcag attcaggtta tgccgctcaa ttcgctgcgt atatcgcttg ctgattacgt 3840
   gcagctttcc cttcaggcgg gattcataca gcggccagcc atccgtcatc catatcacca 3900
   cgtcaaaggg tgacagcagg ctcataagac gccccagcgt cgccatagtg cgttcaccga 3960
   atacgtgcgc aacaaccgtc ttccggagac tgtcatacgc gtaaaacagc cagcgctggc 4020
   gcgatttagc cccgacatag ccccactgtt cgtccatttc cgcgcagacg atgacgtcac 4080
   tgcccggctg tatgcgcgag gttaccgact gcggcctgag ttttttaagt gacgtaaaat 4140
   cgtgttgagg ccaacgccca taatgcgggc tgttgcccgg catccaacgc cattcatggc 4200
   catatcaatg attttctggt gcgtaccggg ttgagaagcg gtgtaagtga actgcagttg 4260
   ccatgtttta cggcagtgag agcagagata gcgctgatgt ccggcggtgc ttttgccgtt 4320
   acgcaccacc ccgtcagtag ctgaacagga gggacagctg atagacacag aagccactgg 4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg attttttaaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa    5640
   ggcaccaggc gggtcaaatc aggaataagg gcacattgcc ccggcgtgag tcggggcaat    5700
   cccgcaagga gggtgaatga atcggacgtt tgaccggaag gcatacaggc aagaactgat    5760
   cgacgcgggg ttttccgccg aggatgccga aaccatcgca agccgcaccg tcatgcgtgc    5820
   gccccgcgaa accttccagt ccgtcggctc gatggtccag caagctacgg ccaagatcga    5880
   gcgcgacagc gtgcaactgg ctccccctgc cctgcccgcg ccatcggccg ccgtggagcg    5940
   ttcgcgtcgt ctcgaacagg aggcggcagg tttggcgaag tcgatgacca tcgacacgcg    6000
   aggaactatg acgaccaaga agcgaaaaac cgccggcgag gacctggcaa aacaggtcag    6060
   cgaggccaag caggccgcgt tgctgaaaca cacgaagcag cagatcaagg aaatgcagct    6120
   ttccttgttc gatattgcgc cgtggccgga cacgatgcga gcgatgccaa acgacacggc    6180
   ccgctctgcc ctgttcacca cgcgcaacaa gaaaatcccg cgcgaggcgc tgcaaaacaa    6240
   ggtcattttc cacgtcaaca aggacgtgaa gatcacctac accggcgtcg agctgcgggc    6300
   cgacgatgac gaactggtgt ggcagcaggt gttggagtac gcgaagcgca cccctatcgg    6360
   cgagccgatc accttcacgt tctacgagct ttgccaggac ctgggctggt cgatcaatgg    6420
   ccggtattac acgaaggccg aggaatgcct gtcgcgccta caggcgacgg cgatgggctt    6480
   cacgtccgac cgcgttgggc acctggaatc ggtgtcgctg ctgcaccgct tccgcgtcct    6540
   ggaccgtggc aagaaaacgt cccgttgcca ggtcctgatc gacgaggaaa tcgtcgtgct    6600
   gtttgctggc gaccactaca cgaaattcat atgggagaag taccgcaagc tgtcgccgac    6660
   ggcccgacgg atgttcgact atttcagctc gcaccgggag ccgtacccgc tcaagctgga    6720
   aaccttccgc ctcatgtgcg gatcggattc cacccgcgtg aagaagtggc gcgagcaggt    6780
   cggcgaagcc tgcgaagagt tgcgaggcag cggcctggtg gaacacgcct gggtcaatga    6840
   tgacctggtg cattgcaaac gctagggcct tgtggggtca gttccggctg ggggttcagc    6900
   agccagcgct ttactggcat ttcaggaaca agcgggcact gctcgacgca cttgcttcgc    6960
   tcagtatcgc tcgggacgca cggcgcgctc tacgaactgc cgataaacag aggattaaaa    7020
   ttgacaattg tgattaaggc tcagattcga cggcttggag cggccgacgt gcaggatttc    7080
   cgcgagatcc gattgtcggc cctgaagaaa gctccagaga tgttcgggtc cgtttacgag    7140
   cacgaggaga aaaagcccat ggaggcgttc gctgaacggt tgcgagatgc cgtggcattc    7200
   ggcgcctaca tcgacggcga gatcattggg ctgtcggtct tcaaacagga ggacggcccc    7260
   aaggacgctc acaaggcgca tctgtccggc gttttcgtgg agcccgaaca gcgaggccga    7320
   ggggtcgccg gtatgctgct gcgggcgttg ccggcgggtt tattgctcgt gatgatcgtc    7380
   cgacagattc caacgggaat ctggtggatg cgcatcttca tcctcggcgc acttaatatt    7440
   tcgctattct ggagcttgtt gtttatttcg gtctaccgcc tgccgggcgg ggtcgcggcg 7500
   acggtaggcg ctgtgcagcc gctgatggtc gtgttcatct ctgccgctct gctaggtagc 7560
   ccgatacgat tgatggcggt cctgggggct atttgcggaa ctgcgggcgt ggcgctgttg 7620
   gtgttgacac caaacgcagc gctagatcct gtcggcgtcg cagcgggcct ggcgggggcg 7680
   gtttccatgg cgttcggaac cgtgctgacc cgcaagtggc aacctcccgt gcctctgctc 7740
   acctttaccg cctggcaact ggcggccgga ggacttctgc tcgttccagt agctttagtg 7800
   tttgatccgc caatcccgat gcctacagga accaatgttc tcggcctggc gtggctcggc 7860
   ctgatcggag cgggtttaac ctacttcctt tggttccggg ggatctcgcg actcgaacct 7920
   acagttgttt ccttactggg ctttctcagc cccagatctg gggtcgatca gccggggatg 7980
   catcaggccg acagtcggaa cttcgggtcc ccgacctgta ccattcggtg agcaatggat 8040
   aggggagttg atatcgtcaa cgttcacttc taaagaaata gcgccactca gcttcctcag 8100
   cggctttatc cagcgatttc ctattatgtc ggcatagttc tcaagatcga cagcctgtca 8160
   cggttaagcg agaaatgaat aagaaggctg ataattcgga tctctgcgag ggagatgata 8220
   tttgatcaca ggcagcaacg ctctgtcatc gttacaatca acatgctacc ctccgcgaga 8280
   tcatccgtgt ttcaaacccg gcagcttagt tgccgttctt ccgaatagca tcggtaacat 8340
   gagcaaagtc tgccgcctta caacggctct cccgctgacg ccgtcccgga ctgatgggct 8400
   gcctgtatcg agtggtgatt ttgtgccgag ctgccggtcg gggagctgtt ggctggctgg 8460
   tggcaggata tattgtggtg taaacaaatt gacgcttaga caacttaata acacattgcg 8520
   gacgttttta atgtactggg gtggtttttc ttttcaccag tgagacgggc aacagctgat 8580
   tgcccttcac cgcctggccc tgagagagtt gcagcaagcg gtccacgctg gtttgcccca 8640
   gcaggcgaaa atcctgtttg atggtggttc cgaaatcggc aaaatccctt ataaatcaaa 8700
   agaatagccc gagatagggt tgagtgttgt tccagtttgg aacaagagtc cactattaaa 8760
   gaacgtggac tccaacgtca aagggcgaaa aaccgtctat cagggcgatg gcccactacg 8820
   tgaaccatca cccaaatcaa gtttttgggg gtcgaggtgc cgtaaagcac taaatcggaa 8880
   ccctaaaggg agcccccgat ttagagcttg acggggaaag ccggcgaacg tggcgagaaa 8940
   ggaagggaag aaagcgaaag gagcgggcgc cattcaggct gcgcaactgt tgggaagggc 9000
   gatcggtgcg ggcctcttcg ctattacgcc agctggcgaa agggggatgt gctgcaaggc 9060
   gattaagttg ggtaacgcca gggttttccc agtcacgacg ttgtaaaacg acggccagtg 9120
   aattaattcc catcttgaaa gaaatatagt ttaaatattt attgataaaa taacaagtca 9180
   ggtattatag tccaagcaaa aacataaatt tattgatgca agtttaaatt cagaaatatt 9240
   tcaataactg attatatcag ctggtacatt gccgtagatg aaagactgag tgcgatatta 9300
   tgtgtaatac ataaattgat gatatagcta gcttagctca tcgggggatc cgtcgaagct 9360
   agcttgggtc ccgctcagaa gaactcgtca agaaggcgat agaaggcgat gcgctgcgaa 9420
   tcgggagcgg cgataccgta aagcacgagg aagcggtcag cccattcgcc gccaagctct 9480
   tcagcaatat cacgggtagc caacgctatg tcctgatagc ggtccgccac acccagccgg 9540
   ccacagtcga tgaatccaga aaagcggcca ttttccacca tgatattcgg caagcaggca 9600
   tcgccatggg tcacgacgag atcctcgccg tcgggcatgc gcgccttgag cctggcgaac 9660
   agttcggctg gcgcgagccc ctgatgctct tcgtccagat catcctgatc gacaagaccg 9720
   gcttccatcc gagtacgtgc tcgctcgatg cgatgtttcg cttggtggtc gaatgggcag 9780
   gtagccggat caagcgtatg cagccgccgc attgcatcag ccatgatgga tactttctcg 9840
   gcaggagcaa ggtgagatga caggagatcc tgccccggca cttcgcccaa tagcagccag 9900
   tcccttcccg cttcagtgac aacgtcgagc acagctgcgc aaggaacgcc cgtcgtggcc 9960
   agccacgata gccgcgctgc ctcgtcctgc agttcattca gggcaccgga caggtcggtc 10020
   ttgacaaaaa gaaccgggcg cccctgcgct gacagccgga acacggcggc atcagagcag 10080
   ccgattgtct gttgtgccca gtcatagccg aatagcctct ccacccaagc ggccggagaa 10140
   cctgcgtgca atccatcttg ttcaatccaa gctcccatgg gccctcgact agagtcgaga 10200
   tctggattga gagtgaatat gagactctaa ttggataccg aggggaattt atggaacgtc 10260
   agtggagcat ttttgacaag aaatatttgc tagctgatag tgaccttagg cgacttttga 10320
   acgcgcaata atggtttctg acgtatgtgc ttagctcatt aaactccaga aacccgcggc 10380
   tgagtggctc cttcaacgtt gcggttctgt cagttccaaa cgtaaaacgg cttgtcccgc 10440
   gtcatcggcg ggggtcataa cgtgactccc ttaattctcc gctcatgatc ttgatcccct 10500
   gcgccatcag atccttggcg gcaagaaagc catccagttt actttgcagg gcttcccaac 10560
   cttaccagag ggcgccccag ctggcaattc cggttcgctt gctgtccata aaaccgccca 10620
   gtctagctat cgccatgtaa gcccactgca agctacctgc tttctctttg cgcttgcgtt 10680
   ttcccttgtc cagatagccc agtagctgac attcatccgg ggtcagcacc gtttctgcgg 10740
   actggctttc tacgtgttcc gcttccttta gcagcccttg cgccctgagt gcttgcggca 10800
   gcgtgaagct tgcatgcctg caggtcgacg gcgcgccgag ctcctcgagc aaatttacac 10860
   attgccacta aacgtctaaa cccttgtaat ttgtttttgt tttactatgt gtgttatgta 10920
   tttgatttgc gataaatttt tatatttggt actaaattta taacaccttt tatgctaacg 10980
   tttgccaaca cttagcaatt tgcaagttga ttaattgatt ctaaattatt tttgtcttct 11040
   aaatacatat actaatcaac tggaaatgta aatatttgct aatatttcta ctataggaga 11100
   attaaagtga gtgaatatgg taccacaagg tttggagatt taattgttgc aatgctgcat 11160
   ggatggcata tacaccaaac attcaataat tcttgaggat aataatggta ccacacaaga 11220
   tttgaggtgc atgaacgtca cgtggacaaa aggtttagta atttttcaag acaacaatgt 11280
   taccacacac aagttttgag gtgcatgcat ggatgccctg tggaaagttt aaaaatattt 11340
   tggaaatgat ttgcatggaa gccatgtgta aaaccatgac atccacttgg aggatgcaat 11400
   aatgaagaaa actacaaatt tacatgcaac tagttatgca tgtagtctat ataatgagga 11460
   ttttgcaata ctttcattca tacacactca ctaagtttta cacgattata atttcttcat 11520
   agccagcgga tccgatatcg ggcccgctag cgttaaccct gctttaatga gatatgcgag 11580
   acgcctatga tcgcatgata tttgctttca attctgttgt gcacgttgta aaaaacctga 11640
   gcatgtgtag ctcagatcct taccgccggt ttcggttcat tctaatgaat atatcacccg 11700
   ttactatcgt atttttatga ataatattct ccgttcaatt tactgattgt ccgtcgacga 11760
   attcgagctc ggcgcgcctc tagaggatcg atgaattcag atcggctgag tggctccttc 11820
   aacgttgcgg ttctgtcagt tccaaacgta aaacggcttg tcccgcgtca tcggcggggg 11880
   tcataacgtg actcccttaa ttctccgctc atgatcagat tgtcgtttcc cgccttcagt 11940
   ttaaactatc agtgtttgac aggatatatt ggcgggtaaa cctaagagaa aagagcgttt 12000
   attagaataa tcggatattt aaaagggcgt gaaaaggttt atccttcgtc catttgtatg 12060
   tgcatgccaa ccacagggtt cccca 12085
<210> 29
   <211> 12079
   <212> DNA
   <213> artificial sequence
<400> 29
   gatctggcgc cggccagcga gacgagcaag attggccgcc gcccgaaacg atccgacagc     60
   gcgcccagca caggtgcgca ggcaaattgc accaacgcat acagcgccag cagaatgcca    120
   tagtgggcgg tgacgtcgtt cgagtgaacc agatcgcgca ggaggcccgg cagcaccggc    180
   ataatcaggc cgatgccgac agcgtcgagc gcgacagtgc tcagaattac gatcaggggt    240
   atgttgggtt tcacgtctgg cctccggacc agcctccgct ggtccgattg aacgcgcgga    300
   ttctttatca ctgataagtt ggtggacata ttatgtttat cagtgataaa gtgtcaagca    360
   tgacaaagtt gcagccgaat acagtgatcc gtgccgccct ggacctgttg aacgaggtcg    420
   gcgtagacgg tctgacgaca cgcaaactgg cggaacggtt gggggttcag cagccggcgc    480
   tttactggca cttcaggaac aagcgggcgc tgctcgacgc actggccgaa gccatgctgg    540
   cggagaatca tacgcattcg gtgccgagag ccgacgacga ctggcgctca tttctgatcg    600
   ggaatgcccg cagcttcagg caggcgctgc tcgcctaccg cgatggcgcg cgcatccatg    660
   ccggcacgcg accgggcgca ccgcagatgg aaacggccga cgcgcagctt cgcttcctct 720
   gcgaggcggg tttttcggcc ggggacgccg tcaatgcgct gatgacaatc agctacttca 780
   ctgttggggc cgtgcttgag gagcaggccg gcgacagcga tgccggcgag cgcggcggca 840
   ccgttgaaca ggctccgctc tcgccgctgt tgcgggccgc gatagacgcc ttcgacgaag 900
   ccggtccgga cgcagcgttc gagcagggac tcgcggtgat tgtcgatgga ttggcgaaaa 960
   ggaggctcgt tgtcaggaac gttgaaggac cgagaaaggg tgacgattga tcaggaccgc 1020
   tgccggagcg caacccactc actacagcag agccatgtag acaacatccc ctcccccttt 1080
   ccaccgcgtc agacgcccgt agcagcccgc tacgggcttt ttcatgccct gccctagcgt 1140
   ccaagcctca cggccgcgct cggcctctct ggcggccttc tggcgctctt ccgcttcctc 1200
   gctcactgac tcgctgcgct cggtcgttcg gctgcggcga gcggtatcag ctcactcaaa 1260
   ggcggtaata cggttatcca cagaatcagg ggataacgca ggaaagaaca tgtgagcaaa 1320
   aggccagcaa aaggccagga accgtaaaaa ggccgcgttg ctggcgtttt tccataggct 1380
   ccgcccccct gacgagcatc acaaaaatcg acgctcaagt cagaggtggc gaaacccgac 1440
   aggactataa agataccagg cgtttccccc tggaagctcc ctcgtgcgct ctcctgttcc 1500
   gaccctgccg cttaccggat acctgtccgc ctttctccct tcgggaagcg tggcgctttt 1560
   ccgctgcata accctgcttc ggggtcatta tagcgatttt ttcggtatat ccatcctttt 1620
   tcgcacgata tacaggattt tgccaaaggg ttcgtgtaga ctttccttgg tgtatccaac 1680
   ggcgtcagcc gggcaggata ggtgaagtag gcccacccgc gagcgggtgt tccttcttca 1740
   ctgtccctta ttcgcacctg gcggtgctca acgggaatcc tgctctgcga ggctggccgg 1800
   ctaccgccgg cgtaacagat gagggcaagc ggatggctga tgaaaccaag ccaaccagga 1860
   agggcagccc acctatcaag gtgtactgcc ttccagacga acgaagagcg attgaggaaa 1920
   aggcggcggc ggccggcatg agcctgtcgg cctacctgct ggccgtcggc cagggctaca 1980
   aaatcacggg cgtcgtggac tatgagcacg tccgcgagct ggcccgcatc aatggcgacc 2040
   tgggccgcct gggcggcctg ctgaaactct ggctcaccga cgacccgcgc acggcgcggt 2100
   tcggtgatgc cacgatcctc gccctgctgg cgaagatcga agagaagcag gacgagcttg 2160
   gcaaggtcat gatgggcgtg gtccgcccga gggcagagcc atgacttttt tagccgctaa 2220
   aacggccggg gggtgcgcgt gattgccaag cacgtcccca tgcgctccat caagaagagc 2280
   gacttcgcgg agctggtgaa gtacatcacc gacgagcaag gcaagaccga gcgcctttgc 2340
   gacgctcacc gggctggttg ccctcgccgc tgggctggcg gccgtctatg gccctgcaaa 2400
   cgcgccagaa acgccgtcga agccgtgtgc gagacaccgc ggccgccggc gttgtggata 2460
   cctcgcggaa aacttggccc tcactgacag atgaggggcg gacgttgaca cttgaggggc 2520
   cgactcaccc ggcgcggcgt tgacagatga ggggcaggct cgatttcggc cggcgacgtg   2580
   gagctggcca gcctcgcaaa tcggcgaaaa cgcctgattt tacgcgagtt tcccacagat   2640
   gatgtggaca agcctgggga taagtgccct gcggtattga cacttgaggg gcgcgactac   2700
   tgacagatga ggggcgcgat ccttgacact tgaggggcag agtgctgaca gatgaggggc   2760
   gcacctattg acatttgagg ggctgtccac aggcagaaaa tccagcattt gcaagggttt   2820
   ccgcccgttt ttcggccacc gctaacctgt cttttaacct gcttttaaac caatatttat   2880
   aaaccttgtt tttaaccagg gctgcgccct gtgcgcgtga ccgcgcacgc cgaagggggg   2940
   tgccccccct tctcgaaccc tcccggcccg ctaacgcggg cctcccatcc ccccaggggc   3000
   tgcgcccctc ggccgcgaac ggcctcaccc caaaaatggc agcgctggca gtccttgcca   3060
   ttgccgggat cggggcagta acgggatggg cgatcagccc gagcgcgacg cccggaagca   3120
   ttgacgtgcc gcaggtgctg gcatcgacat tcagcgacca ggtgccgggc agtgagggcg   3180
   gcggcctggg tggcggcctg cccttcactt cggccgtcgg ggcattcacg gacttcatgg   3240
   cggggccggc aatttttacc ttgggcattc ttggcatagt ggtcgcgggt gccgtgctcg   3300
   tgttcggggg tgcgataaac ccagcgaacc atttgaggtg ataggtaaga ttataccgag   3360
   gtatgaaaac gagaattgga cctttacaga attactctat gaagcgccat atttaaaaag   3420
   ctaccaagac gaagaggatg aagaggatga ggaggcagat tgccttgaat atattgacaa   3480
   tactgataag ataatatatc ttttatatag aagatatcgc cgtatgtaag gatttcaggg   3540
   ggcaaggcat aggcagcgcg cttatcaata tatctataga atgggcaaag cataaaaact   3600
   tgcatggact aatgcttgaa acccaggaca ataaccttat agcttgtaaa ttctatcata   3660
   attgggtaat gactccaact tattgatagt gttttatgtt cagataatgc ccgatgactt   3720
   tgtcatgcag ctccaccgat tttgagaacg acagcgactt ccgtcccagc cgtgccaggt   3780
   gctgcctcag attcaggtta tgccgctcaa ttcgctgcgt atatcgcttg ctgattacgt   3840
   gcagctttcc cttcaggcgg gattcataca gcggccagcc atccgtcatc catatcacca   3900
   cgtcaaaggg tgacagcagg ctcataagac gccccagcgt cgccatagtg cgttcaccga   3960
   atacgtgcgc aacaaccgtc ttccggagac tgtcatacgc gtaaaacagc cagcgctggc   4020
   gcgatttagc cccgacatag ccccactgtt cgtccatttc cgcgcagacg atgacgtcac   4080
   tgcccggctg tatgcgcgag gttaccgact gcggcctgag ttttttaagt gacgtaaaat   4140
   cgtgttgagg ccaacgccca taatgcgggc tgttgcccgg catccaacgc cattcatggc   4200
   catatcaatg attttctggt gcgtaccggg ttgagaagcg gtgtaagtga actgcagttg   4260
   ccatgtttta cggcagtgag agcagagata gcgctgatgt ccggcggtgc ttttgccgtt   4320
   acgcaccacc ccgtcagtag ctgaacagga gggacagctg atagacacag aagccactgg   4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg attttttaaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa 5640
   ggcaccaggc gggtcaaatc aggaataagg gcacattgcc ccggcgtgag tcggggcaat 5700
   cccgcaagga gggtgaatga atcggacgtt tgaccggaag gcatacaggc aagaactgat 5760
   cgacgcgggg ttttccgccg aggatgccga aaccatcgca agccgcaccg tcatgcgtgc 5820
   gccccgcgaa accttccagt ccgtcggctc gatggtccag caagctacgg ccaagatcga 5880
   gcgcgacagc gtgcaactgg ctccccctgc cctgcccgcg ccatcggccg ccgtggagcg 5940
   ttcgcgtcgt ctcgaacagg aggcggcagg tttggcgaag tcgatgacca tcgacacgcg 6000
   aggaactatg acgaccaaga agcgaaaaac cgccggcgag gacctggcaa aacaggtcag 6060
   cgaggccaag caggccgcgt tgctgaaaca cacgaagcag cagatcaagg aaatgcagct 6120
   ttccttgttc gatattgcgc cgtggccgga cacgatgcga gcgatgccaa acgacacggc 6180
   ccgctctgcc ctgttcacca cgcgcaacaa gaaaatcccg cgcgaggcgc tgcaaaacaa 6240
   ggtcattttc cacgtcaaca aggacgtgaa gatcacctac accggcgtcg agctgcgggc 6300
   cgacgatgac gaactggtgt ggcagcaggt gttggagtac gcgaagcgca cccctatcgg 6360
   cgagccgatc accttcacgt tctacgagct ttgccaggac ctgggctggt cgatcaatgg 6420
   ccggtattac acgaaggccg aggaatgcct gtcgcgccta caggcgacgg cgatgggctt 6480
   cacgtccgac cgcgttgggc acctggaatc ggtgtcgctg ctgcaccgct tccgcgtcct 6540
   ggaccgtggc aagaaaacgt cccgttgcca ggtcctgatc gacgaggaaa tcgtcgtgct 6600
   gtttgctggc gaccactaca cgaaattcat atgggagaag taccgcaagc tgtcgccgac 6660
   ggcccgacgg atgttcgact atttcagctc gcaccgggag ccgtacccgc tcaagctgga 6720
   aaccttccgc ctcatgtgcg gatcggattc cacccgcgtg aagaagtggc gcgagcaggt 6780
   cggcgaagcc tgcgaagagt tgcgaggcag cggcctggtg gaacacgcct gggtcaatga 6840
   tgacctggtg cattgcaaac gctagggcct tgtggggtca gttccggctg ggggttcagc 6900
   agccagcgct ttactggcat ttcaggaaca agcgggcact gctcgacgca cttgcttcgc 6960
   tcagtatcgc tcgggacgca cggcgcgctc tacgaactgc cgataaacag aggattaaaa 7020
   ttgacaattg tgattaaggc tcagattcga cggcttggag cggccgacgt gcaggatttc 7080
   cgcgagatcc gattgtcggc cctgaagaaa gctccagaga tgttcgggtc cgtttacgag 7140
   cacgaggaga aaaagcccat ggaggcgttc gctgaacggt tgcgagatgc cgtggcattc 7200
   ggcgcctaca tcgacggcga gatcattggg ctgtcggtct tcaaacagga ggacggcccc 7260
   aaggacgctc acaaggcgca tctgtccggc gttttcgtgg agcccgaaca gcgaggccga 7320
   ggggtcgccg gtatgctgct gcgggcgttg ccggcgggtt tattgctcgt gatgatcgtc 7380
   cgacagattc caacgggaat ctggtggatg cgcatcttca tcctcggcgc acttaatatt 7440
   tcgctattct ggagcttgtt gtttatttcg gtctaccgcc tgccgggcgg ggtcgcggcg 7500
   acggtaggcg ctgtgcagcc gctgatggtc gtgttcatct ctgccgctct gctaggtagc 7560
   ccgatacgat tgatggcggt cctgggggct atttgcggaa ctgcgggcgt ggcgctgttg 7620
   gtgttgacac caaacgcagc gctagatcct gtcggcgtcg cagcgggcct ggcgggggcg 7680
   gtttccatgg cgttcggaac cgtgctgacc cgcaagtggc aacctcccgt gcctctgctc 7740
   acctttaccg cctggcaact ggcggccgga ggacttctgc tcgttccagt agctttagtg 7800
   tttgatccgc caatcccgat gcctacagga accaatgttc tcggcctggc gtggctcggc 7860
   ctgatcggag cgggtttaac ctacttcctt tggttccggg ggatctcgcg actcgaacct 7920
   acagttgttt ccttactggg ctttctcagc cccagatctg gggtcgatca gccggggatg 7980
   catcaggccg acagtcggaa cttcgggtcc ccgacctgta ccattcggtg agcaatggat 8040
   aggggagttg atatcgtcaa cgttcacttc taaagaaata gcgccactca gcttcctcag 8100
   cggctttatc cagcgatttc ctattatgtc ggcatagttc tcaagatcga cagcctgtca 8160
   cggttaagcg agaaatgaat aagaaggctg ataattcgga tctctgcgag ggagatgata 8220
   tttgatcaca ggcagcaacg ctctgtcatc gttacaatca acatgctacc ctccgcgaga 8280
   tcatccgtgt ttcaaacccg gcagcttagt tgccgttctt ccgaatagca tcggtaacat 8340
   gagcaaagtc tgccgcctta caacggctct cccgctgacg ccgtcccgga ctgatgggct 8400
   gcctgtatcg agtggtgatt ttgtgccgag ctgccggtcg gggagctgtt ggctggctgg 8460
   tggcaggata tattgtggtg taaacaaatt gacgcttaga caacttaata acacattgcg 8520
   gacgttttta atgtactggg gtggtttttc ttttcaccag tgagacgggc aacagctgat 8580
   tgcccttcac cgcctggccc tgagagagtt gcagcaagcg gtccacgctg gtttgcccca 8640
   gcaggcgaaa atcctgtttg atggtggttc cgaaatcggc aaaatccctt ataaatcaaa 8700
   agaatagccc gagatagggt tgagtgttgt tccagtttgg aacaagagtc cactattaaa 8760
   gaacgtggac tccaacgtca aagggcgaaa aaccgtctat cagggcgatg gcccactacg 8820
   tgaaccatca cccaaatcaa gttttttggg gtcgaggtgc cgtaaagcac taaatcggaa 8880
   ccctaaaggg agcccccgat ttagagcttg acggggaaag ccggcgaacg tggcgagaaa 8940
   ggaagggaag aaagcgaaag gagcgggcgc cattcaggct gcgcaactgt tgggaagggc 9000
   gatcggtgcg ggcctcttcg ctattacgcc agctggcgaa agggggatgt gctgcaaggc 9060
   gattaagttg ggtaacgcca gggttttccc agtcacgacg ttgtaaaacg acggccagtg 9120
   aattaattcc catcttgaaa gaaatatagt ttaaatattt attgataaaa taacaagtca 9180
   ggtattatag tccaagcaaa aacataaatt tattgatgca agtttaaatt cagaaatatt 9240
   tcaataactg attatatcag ctggtacatt gccgtagatg aaagactgag tgcgatatta 9300
   tgtgtaatac ataaattgat gatatagcta gcttagctca tcgggggatc cgtcgaagct 9360
   agcttgggtc ccgctcagaa gaactcgtca agaaggcgat agaaggcgat gcgctgcgaa 9420
   tcgggagcgg cgataccgta aagcacgagg aagcggtcag cccattcgcc gccaagctct 9480
   tcagcaatat cacgggtagc caacgctatg tcctgatagc ggtccgccac acccagccgg 9540
   ccacagtcga tgaatccaga aaagcggcca ttttccacca tgatattcgg caagcaggca 9600
   tcgccatggg tcacgacgag atcctcgccg tcgggcatgc gcgccttgag cctggcgaac 9660
   agttcggctg gcgcgagccc ctgatgctct tcgtccagat catcctgatc gacaagaccg 9720
   gcttccatcc gagtacgtgc tcgctcgatg cgatgtttcg cttggtggtc gaatgggcag 9780
   gtagccggat caagcgtatg cagccgccgc attgcatcag ccatgatgga tactttctcg 9840
   gcaggagcaa ggtgagatga caggagatcc tgccccggca cttcgcccaa tagcagccag 9900
   tcccttcccg cttcagtgac aacgtcgagc acagctgcgc aaggaacgcc cgtcgtggcc 9960
   agccacgata gccgcgctgc ctcgtcctgc agttcattca gggcaccgga caggtcggtc 10020
   ttgacaaaaa gaaccgggcg cccctgcgct gacagccgga acacggcggc atcagagcag 10080
   ccgattgtct gttgtgccca gtcatagccg aatagcctct ccacccaagc ggccggagaa 10140
   cctgcgtgca atccatcttg ttcaatccaa gctcccatgg gccctcgact agagtcgaga 10200
   tctggattga gagtgaatat gagactctaa ttggataccg aggggaattt atggaacgtc 10260
   agtggagcat ttttgacaag aaatatttgc tagctgatag tgaccttagg cgacttttga 10320
   acgcgcaata atggtttctg acgtatgtgc ttagctcatt aaactccaga aacccgcggc 10380
   tgagtggctc cttcaacgtt gcggttctgt cagttccaaa cgtaaaacgg cttgtcccgc 10440
   gtcatcggcg ggggtcataa cgtgactccc ttaattctcc gctcatgatc ttgatcccct 10500
   gcgccatcag atccttggcg gcaagaaagc catccagttt actttgcagg gcttcccaac 10560
   cttaccagag ggcgccccag ctggcaattc cggttcgctt gctgtccata aaaccgccca 10620
   gtctagctat cgccatgtaa gcccactgca agctacctgc tttctctttg cgcttgcgtt 10680
   ttcccttgtc cagatagccc agtagctgac attcatccgg ggtcagcacc gtttctgcgg 10740
   actggctttc tacgtgttcc gcttccttta gcagcccttg cgccctgagt gcttgcggca 10800
   gcgtgaagct tgcatgcctg caggtcgacg gcgcgccgag ctcctcgagc aaatttacac 10860
   attgccacta aacgtctaaa cccttgtaat ttgtttttgt tttactatgt gtgttatgta 10920
   tttgatttgc gataaatttt tatatttggt actaaattta taacaccttt tatgctaacg 10980
   tttgccaaca cttagcaatt tgcaagttga ttaattgatt ctaaattatt tttgtcttct 11040
   aaatacatat actaatcaac tggaaatgta aatatttgct aatatttcta ctataggaga 11100
   attaaagtga gtgaatatgg taccacaagg tttggagatt taattgttgc aatgctgcat 11160
   ggatggcata tacaccaaac attcaataat tcttgaggat aataatggta ccacacaaga 11220
   tttgaggtgc atgaacgtca cgtggacaaa aggtttagta atttttcaag acaacaatgt 11280
   taccacacac aagttttgag gtgcatgcat ggatgccctg tggaaagttt aaaaatattt 11340
   tggaaatgat ttgcatggaa gccatgtgta aaaccatgac atccacttgg aggatgcaat 11400
   aatgaagaaa actacaaatt tacatgcaac tagttatgca tgtagtctat ataatgagga 11460
   ttttgcaata ctttcattca tacacactca ctaagtttta cacgattata atttcttcat 11520
   agccagcaga tctgccggca tcgatcccgg gccatggcct gctttaatga gatatgcgag 11580
   acgcctatga tcgcatgata tttgctttca attctgttgt gcacgttgta aaaaacctga 11640
   gcatgtgtag ctcagatcct taccgccggt ttcggttcat tctaatgaat atatcacccg 11700
   ttactatcgt atttttatga ataatattct ccgttcaatt tactgattgt ccgtcgacga 11760
   gctcggcgcg cctctagagg atcgatgaat tcagatcggc tgagtggctc cttcaacgtt 11820
   gcggttctgt cagttccaaa cgtaaaacgg cttgtcccgc gtcatcggcg ggggtcataa 11880
   cgtgactccc ttaattctcc gctcatgatc agattgtcgt ttcccgcctt cagtttaaac 11940
   tatcagtgtt tgacaggata tattggcggg taaacctaag agaaaagagc gtttattaga 12000
   ataatcggat atttaaaagg gcgtgaaaag gtttatcctt cgtccatttg tatgtgcatg 12060
   ccaaccacag ggttcccca 12079
<210> 30
   <211> 13002
   <212> DNA
   <213> artificial sequence
<220>
   <223> pflanzlicher Expressionsvektor mit zwei Promotor-Terminator-Expre ssionskassetten
<400> 30
   gatctggcgc cggccagcga gacgagcaag attggccgcc gcccgaaacg atccgacagc      60
   gcgcccagca caggtgcgca ggcaaattgc accaacgcat acagcgccag cagaatgcca     120
   tagtgggcgg tgacgtcgtt cgagtgaacc agatcgcgca ggaggcccgg cagcaccggc     180
   ataatcaggc cgatgccgac agcgtcgagc gcgacagtgc tcagaattac gatcaggggt     240
   atgttgggtt tcacgtctgg cctccggacc agcctccgct ggtccgattg aacgcgcgga     300
   ttctttatca ctgataagtt ggtggacata ttatgtttat cagtgataaa gtgtcaagca     360
   tgacaaagtt gcagccgaat acagtgatcc gtgccgccct ggacctgttg aacgaggtcg     420
   gcgtagacgg tctgacgaca cgcaaactgg cggaacggtt gggggttcag cagccggcgc     480
   tttactggca cttcaggaac aagcgggcgc tgctcgacgc actggccgaa gccatgctgg     540
   cggagaatca tacgcattcg gtgccgagag ccgacgacga ctggcgctca tttctgatcg     600
   ggaatgcccg cagcttcagg caggcgctgc tcgcctaccg cgatggcgcg cgcatccatg     660
   ccggcacgcg accgggcgca ccgcagatgg aaacggccga cgcgcagctt cgcttcctct     720
   gcgaggcggg tttttcggcc ggggacgccg tcaatgcgct gatgacaatc agctacttca     780
   ctgttggggc cgtgcttgag gagcaggccg gcgacagcga tgccggcgag cgcggcggca     840
   ccgttgaaca ggctccgctc tcgccgctgt tgcgggccgc gatagacgcc ttcgacgaag     900
   ccggtccgga cgcagcgttc gagcagggac tcgcggtgat tgtcgatgga ttggcgaaaa     960
   ggaggctcgt tgtcaggaac gttgaaggac cgagaaaggg tgacgattga tcaggaccgc    1020
   tgccggagcg caacccactc actacagcag agccatgtag acaacatccc ctcccccttt    1080
   ccaccgcgtc agacgcccgt agcagcccgc tacgggcttt ttcatgccct gccctagcgt    1140
   ccaagcctca cggccgcgct cggcctctct ggcggccttc tggcgctctt ccgcttcctc    1200
   gctcactgac tcgctgcgct cggtcgttcg gctgcggcga gcggtatcag ctcactcaaa    1260
   ggcggtaata cggttatcca cagaatcagg ggataacgca ggaaagaaca tgtgagcaaa 1320
   aggccagcaa aaggccagga accgtaaaaa ggccgcgttg ctggcgtttt tccataggct 1380
   ccgcccccct gacgagcatc acaaaaatcg acgctcaagt cagaggtggc gaaacccgac 1440
   aggactataa agataccagg cgtttccccc tggaagctcc ctcgtgcgct ctcctgttcc 1500
   gaccctgccg cttaccggat acctgtccgc ctttctccct tcgggaagcg tggcgctttt 1560
   ccgctgcata accctgcttc ggggtcatta tagcgatttt ttcggtatat ccatcctttt 1620
   tcgcacgata tacaggattt tgccaaaggg ttcgtgtaga ctttccttgg tgtatccaac 1680
   ggcgtcagcc gggcaggata ggtgaagtag gcccacccgc gagcgggtgt tccttcttca 1740
   ctgtccctta ttcgcacctg gcggtgctca acgggaatcc tgctctgcga ggctggccgg 1800
   ctaccgccgg cgtaacagat gagggcaagc ggatggctga tgaaaccaag ccaaccagga 1860
   agggcagccc acctatcaag gtgtactgcc ttccagacga acgaagagcg attgaggaaa 1920
   aggcggcggc ggccggcatg agcctgtcgg cctacctgct ggccgtcggc cagggctaca 1980
   aaatcacggg cgtcgtggac tatgagcacg tccgcgagct ggcccgcatc aatggcgacc 2040
   tgggccgcct gggcggcctg ctgaaactct ggctcaccga cgacccgcgc acggcgcggt 2100
   tcggtgatgc cacgatcctc gccctgctgg cgaagatcga agagaagcag gacgagcttg 2160
   gcaaggtcat gatgggcgtg gtccgcccga gggcagagcc atgacttttt tagccgctaa 2220
   aacggccggg gggtgcgcgt gattgccaag cacgtcccca tgcgctccat caagaagagc 2280
   gacttcgcgg agctggtgaa gtacatcacc gacgagcaag gcaagaccga gcgcctttgc 2340
   gacgctcacc gggctggttg ccctcgccgc tgggctggcg gccgtctatg gccctgcaaa 2400
   cgcgccagaa acgccgtcga agccgtgtgc gagacaccgc ggccgccggc gttgtggata 2460
   cctcgcggaa aacttggccc tcactgacag atgaggggcg gacgttgaca cttgaggggc 2520
   cgactcaccc ggcgcggcgt tgacagatga ggggcaggct cgatttcggc cggcgacgtg 2580
   gagctggcca gcctcgcaaa tcggcgaaaa cgcctgattt tacgcgagtt tcccacagat 2640
   gatgtggaca agcctgggga taagtgccct gcggtattga cacttgaggg gcgcgactac 2700
   tgacagatga ggggcgcgat ccttgacact tgaggggcag agtgctgaca gatgaggggc 2760
   gcacctattg acatttgagg ggctgtccac aggcagaaaa tccagcattt gcaagggttt 2820
   ccgcccgttt ttcggccacc gctaacctgt cttttaacct gcttttaaac caatatttat 2880
   aaaccttgtt tttaaccagg gctgcgccct gtgcgcgtga ccgcgcacgc cgaagggggg 2940
   tgccccccct tctcgaaccc tcccggcccg ctaacgcggg cctcccatcc ccccaggggc 3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa 3480
   tactgataag ataatatatc ttttatatag aagatatcgc cgtatgtaag gatttcaggg 3540
   ggcaaggcat aggcagcgcg cttatcaata tatctataga atgggcaaag cataaaaact 3600
   tgcatggact aatgcttgaa acccaggaca ataaccttat agcttgtaaa ttctatcata 3660
   attgggtaat gactccaact tattgatagt gttttatgtt cagataatgc ccgatgactt 3720
   tgtcatgcag ctccaccgat tttgagaacg acagcgactt ccgtcccagc cgtgccaggt 3780
   gctgcctcag attcaggtta tgccgctcaa ttcgctgcgt atatcgcttg ctgattacgt 3840
   gcagctttcc cttcaggcgg gattcataca gcggccagcc atccgtcatc catatcacca 3900
   cgtcaaaggg tgacagcagg ctcataagac gccccagcgt cgccatagtg cgttcaccga 3960
   atacgtgcgc aacaaccgtc ttccggagac tgtcatacgc gtaaaacagc cagcgctggc 4020
   gcgatttagc cccgacatag ccccactgtt cgtccatttc cgcgcagacg atgacgtcac 4080
   tgcccggctg tatgcgcgag gttaccgact gcggcctgag ttttttaagt gacgtaaaat 4140
   cgtgttgagg ccaacgccca taatgcgggc tgttgcccgg catccaacgc cattcatggc 4200
   catatcaatg attttctggt gcgtaccggg ttgagaagcg gtgtaagtga actgcagttg 4260
   ccatgtttta cggcagtgag agcagagata gcgctgatgt ccggcggtgc ttttgccgtt 4320
   acgcaccacc ccgtcagtag ctgaacagga gggacagctg atagacacag aagccactgg 4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg attttttaaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa 5640
   ggcaccaggc gggtcaaatc aggaataagg gcacattgcc ccggcgtgag tcggggcaat 5700
   cccgcaagga gggtgaatga atcggacgtt tgaccggaag gcatacaggc aagaactgat 5760
   cgacgcgggg ttttccgccg aggatgccga aaccatcgca agccgcaccg tcatgcgtgc 5820
   gccccgcgaa accttccagt ccgtcggctc gatggtccag caagctacgg ccaagatcga 5880
   gcgcgacagc gtgcaactgg ctccccctgc cctgcccgcg ccatcggccg ccgtggagcg 5940
   ttcgcgtcgt ctcgaacagg aggcggcagg tttggcgaag tcgatgacca tcgacacgcg 6000
   aggaactatg acgaccaaga agcgaaaaac cgccggcgag gacctggcaa aacaggtcag 6060
   cgaggccaag caggccgcgt tgctgaaaca cacgaagcag cagatcaagg aaatgcagct 6120
   ttccttgttc gatattgcgc cgtggccgga cacgatgcga gcgatgccaa acgacacggc 6180
   ccgctctgcc ctgttcacca cgcgcaacaa gaaaatcccg cgcgaggcgc tgcaaaacaa 6240
   ggtcattttc cacgtcaaca aggacgtgaa gatcacctac accggcgtcg agctgcgggc 6300
   cgacgatgac gaactggtgt ggcagcaggt gttggagtac gcgaagcgca cccctatcgg 6360
   cgagccgatc accttcacgt tctacgagct ttgccaggac ctgggctggt cgatcaatgg 6420
   ccggtattac acgaaggccg aggaatgcct gtcgcgccta caggcgacgg cgatgggctt 6480
   cacgtccgac cgcgttgggc acctggaatc ggtgtcgctg ctgcaccgct tccgcgtcct 6540
   ggaccgtggc aagaaaacgt cccgttgcca ggtcctgatc gacgaggaaa tcgtcgtgct 6600
   gtttgctggc gaccactaca cgaaattcat atgggagaag taccgcaagc tgtcgccgac 6660
   ggcccgacgg atgttcgact atttcagctc gcaccgggag ccgtacccgc tcaagctgga 6720
   aaccttccgc ctcatgtgcg gatcggattc cacccgcgtg aagaagtggc gcgagcaggt 6780
   cggcgaagcc tgcgaagagt tgcgaggcag cggcctggtg gaacacgcct gggtcaatga 6840
   tgacctggtg cattgcaaac gctagggcct tgtggggtca gttccggctg ggggttcagc 6900
   agccagcgct ttactggcat ttcaggaaca agcgggcact gctcgacgca cttgcttcgc 6960
   tcagtatcgc tcgggacgca cggcgcgctc tacgaactgc cgataaacag aggattaaaa 7020
   ttgacaattg tgattaaggc tcagattcga cggcttggag cggccgacgt gcaggatttc 7080
   cgcgagatcc gattgtcggc cctgaagaaa gctccagaga tgttcgggtc cgtttacgag 7140
   cacgaggaga aaaagcccat ggaggcgttc gctgaacggt tgcgagatgc cgtggcattc 7200
   ggcgcctaca tcgacggcga gatcattggg ctgtcggtct tcaaacagga ggacggcccc 7260
   aaggacgctc acaaggcgca tctgtccggc gttttcgtgg agcccgaaca gcgaggccga 7320
   ggggtcgccg gtatgctgct gcgggcgttg ccggcgggtt tattgctcgt gatgatcgtc 7380
   cgacagattc caacgggaat ctggtggatg cgcatcttca tcctcggcgc acttaatatt 7440
   tcgctattct ggagcttgtt gtttatttcg gtctaccgcc tgccgggcgg ggtcgcggcg 7500
   acggtaggcg ctgtgcagcc gctgatggtc gtgttcatct ctgccgctct gctaggtagc 7560
   ccgatacgat tgatggcggt cctgggggct atttgcggaa ctgcgggcgt ggcgctgttg 7620
   gtgttgacac caaacgcagc gctagatcct gtcggcgtcg cagcgggcct ggcgggggcg 7680
   gtttccatgg cgttcggaac cgtgctgacc cgcaagtggc aacctcccgt gcctctgctc 7740
   acctttaccg cctggcaact ggcggccgga ggacttctgc tcgttccagt agctttagtg 7800
   tttgatccgc caatcccgat gcctacagga accaatgttc tcggcctggc gtggctcggc 7860
   ctgatcggag cgggtttaac ctacttcctt tggttccggg ggatctcgcg actcgaacct 7920
   acagttgttt ccttactggg ctttctcagc cccagatctg gggtcgatca gccggggatg 7980
   catcaggccg acagtcggaa cttcgggtcc ccgacctgta ccattcggtg agcaatggat 8040
   aggggagttg atatcgtcaa cgttcacttc taaagaaata gcgccactca gcttcctcag 8100
   cggctttatc cagcgatttc ctattatgtc ggcatagttc tcaagatcga cagcctgtca 8160
   cggttaagcg agaaatgaat aagaaggctg ataattcgga tctctgcgag ggagatgata 8220
   tttgatcaca ggcagcaacg ctctgtcatc gttacaatca acatgctacc ctccgcgaga 8280
   tcatccgtgt ttcaaacccg gcagcttagt tgccgttctt ccgaatagca tcggtaacat 8340
   gagcaaagtc tgccgcctta caacggctct cccgctgacg ccgtcccgga ctgatgggct 8400
   gcctgtatcg agtggtgatt ttgtgccgag ctgccggtcg gggagctgtt ggctggctgg 8460
   tggcaggata tattgtggtg taaacaaatt gacgcttaga caacttaata acacattgcg 8520
   gacgttttta atgtactggg gtggtttttc ttttcaccag tgagacgggc aacagctgat 8580
   tgcccttcac cgcctggccc tgagagagtt gcagcaagcg gtccacgctg gtttgcccca 8640
   gcaggcgaaa atcctgtttg atggtggttc cgaaatcggc aaaatccctt ataaatcaaa 8700
   agaatagccc gagatagggt tgagtgttgt tccagtttgg aacaagagtc cactattaaa 8760
   gaacgtggac tccaacgtca aagggcgaaa aaccgtctat cagggcgatg gcccactacg 8820
   tgaaccatca cccaaatcaa gttttttggg gtcgaggtgc cgtaaagcac taaatcggaa 8880
   ccctaaaggg agcccccgat ttagagcttg acggggaaag ccggcgaacg tggcgagaaa 8940
   ggaagggaag aaagcgaaag gagcgggcgc cattcaggct gcgcaactgt tgggaagggc 9000
   gatcggtgcg ggcctcttcg ctattacgcc agctggcgaa agggggatgt gctgcaaggc 9060
   gattaagttg ggtaacgcca gggttttccc agtcacgacg ttgtaaaacg acggccagtg 9120
   aattaattcc catcttgaaa gaaatatagt ttaaatattt attgataaaa taacaagtca 9180
   ggtattatag tccaagcaaa aacataaatt tattgatgca agtttaaatt cagaaatatt 9240
   tcaataactg attatatcag ctggtacatt gccgtagatg aaagactgag tgcgatatta 9300
   tgtgtaatac ataaattgat gatatagcta gcttagctca tcgggggatc cgtcgaagct 9360
   agcttgggtc ccgctcagaa gaactcgtca agaaggcgat agaaggcgat gcgctgcgaa 9420
   tcgggagcgg cgataccgta aagcacgagg aagcggtcag cccattcgcc gccaagctct 9480
   tcagcaatat cacgggtagc caacgctatg tcctgatagc ggtccgccac acccagccgg 9540
   ccacagtcga tgaatccaga aaagcggcca ttttccacca tgatattcgg caagcaggca 9600
   tcgccatggg tcacgacgag atcctcgccg tcgggcatgc gcgccttgag cctggcgaac 9660
   agttcggctg gcgcgagccc ctgatgctct tcgtccagat catcctgatc gacaagaccg 9720
   gcttccatcc gagtacgtgc tcgctcgatg cgatgtttcg cttggtggtc gaatgggcag 9780
   gtagccggat caagcgtatg cagccgccgc attgcatcag ccatgatgga tactttctcg 9840
   gcaggagcaa ggtgagatga caggagatcc tgccccggca cttcgcccaa tagcagccag 9900
   tcccttcccg cttcagtgac aacgtcgagc acagctgcgc aaggaacgcc cgtcgtggcc 9960
   agccacgata gccgcgctgc ctcgtcctgc agttcattca gggcaccgga caggtcggtc 10020
   ttgacaaaaa gaaccgggcg cccctgcgct gacagccgga acacggcggc atcagagcag 10080
   ccgattgtct gttgtgccca gtcatagccg aatagcctct ccacccaagc ggccggagaa 10140
   cctgcgtgca atccatcttg ttcaatccaa gctcccatgg gccctcgact agagtcgaga 10200
   tctggattga gagtgaatat gagactctaa ttggataccg aggggaattt atggaacgtc 10260
   agtggagcat ttttgacaag aaatatttgc tagctgatag tgaccttagg cgacttttga 10320
   acgcgcaata atggtttctg acgtatgtgc ttagctcatt aaactccaga aacccgcggc 10380
   tgagtggctc cttcaacgtt gcggttctgt cagttccaaa cgtaaaacgg cttgtcccgc 10440
   gtcatcggcg gggtcataac cgtgactccc ttaattctcc gctcatgatc ttgatcccct 10500
   gcgccatcag atccttggcg gcaagaaagc catccagttt actttgcagg gcttcccaac 10560
   cttaccagag ggcgccccag ctggcaattc cggttcgctt gctgtccata aaaccgccca 10620
   gtctagctat cgccatgtaa gcccactgca agctacctgc tttctctttg cgcttgcgtt 10680
   ttcccttgtc cagatagccc agtagctgac attcatccgg ggtcagcacc gtttctgcgg 10740
   actggctttc tacgtgttcc gcttccttta gcagcccttg cgccctgagt gcttgcggca 10800
   gcgtgaagct tgcatgcctg caggtcgacg gcgcgccgag ctcctcgagc aaatttacac 10860
   attgccacta aacgtctaaa cccttgtaat ttgtttttgt tttactatgt gtgttatgta 10920
   tttgatttgc gataaatttt tatatttggt actaaattta taacaccttt tatgctaacg 10980
   tttgccaaca cttagcaatt tgcaagttga ttaattgatt ctaaattatt tttgtcttct 11040
   aaatacatat actaatcaac tggaaatgta aatatttgct aatatttcta ctataggaga 11100
   attaaagtga gtgaatatgg taccacaagg tttggagatt taattgttgc aatgctgcat 11160
   ggatggcata tacaccaaac attcaataat tcttgaggat aataatggta ccacacaaga 11220
   tttgaggtgc atgaacgtca cgtggacaaa aggtttagta atttttcaag acaacaatgt 11280
   taccacacac aagttttgag gtgcatgcat ggatgccctg tggaaagttt aaaaatattt 11340
   tggaaatgat ttgcatggaa gccatgtgta aaaccatgac atccacttgg aggatgcaat 11400
   aatgaagaaa actacaaatt tacatgcaac tagttatgca tgtagtctat ataatgagga 11460
   ttttgcaata ctttcattca tacacactca ctaagtttta cacgattata atttcttcat 11520
   agccagccca ccgcggtggg cggccgcctg cagtctagaa ggcctcctgc tttaatgaga 11580
   tatgcgagac gcctatgatc gcatgatatt tgctttcaat tctgttgtgc acgttgtaaa 11640
   aaacctgagc atgtgtagct cagatcctta ccgccggttt cggttcattc taatgaatat 11700
   atcacccgtt actatcgtat ttttatgaat aatattctcc gttcaattta ctgattgtcc 11760
   gtcgagcaaa tttacacatt gccactaaac gtctaaaccc ttgtaatttg tttttgtttt 11820
   actatgtgtg ttatgtattt gatttgcgat aaatttttat atttggtact aaatttataa 11880
   caccttttat gctaacgttt gccaacactt agcaatttgc aagttgatta attgattcta 11940
   aattattttt gtcttctaaa tacatatact aatcaactgg aaatgtaaat atttgctaat 12000
   atttctacta taggagaatt aaagtgagtg aatatggtac cacaaggttt ggagatttaa 12060
   ttgttgcaat gctgcatgga tggcatatac accaaacatt caataattct tgaggataat 12120
   aatggtacca cacaagattt gaggtgcatg aacgtcacgt ggacaaaagg tttagtaatt 12180
   tttcaagaca acaatgttac cacacacaag ttttgaggtg catgcatgga tgccctgtgg 12240
   aaagtttaaa aatattttgg aaatgatttg catggaagcc atgtgtaaaa ccatgacatc 12300
   cacttggagg atgcaataat gaagaaaact acaaatttac atgcaactag ttatgcatgt 12360
   agtctatata atgaggattt tgcaatactt tcattcatac acactcacta agttttacac 12420
   gattataatt tcttcatagc cagcggatcc gatatcgggc ccgctagcgt taaccctgct 12480
   ttaatgagat atgcgagacg cctatgatcg catgatattt gctttcaatt ctgttgtgca 12540
   cgttgtaaaa aacctgagca tgtgtagctc agatccttac cgccggtttc ggttcattct 12600
   aatgaatata tcacccgtta ctatcgtatt tttatgaata atattctccg ttcaatttac 12660
   tgattgtccg tcgacgaatt cgagctcggc gcgcctctag aggatcgatg aattcagatc 12720
   ggctgagtgg ctccttcaac gttgcggttc tgtcagttcc aaacgtaaaa cggcttgtcc 12780
   cgcgtcatcg gcgggggtca taacgtgact cccttaattc tccgctcatg atcagattgt 12840
   cgtttcccgc cttcagttta aactatcagt gtttgacagg atatattggc gggtaaacct 12900
   aagagaaaag agcgtttatt agaataatcg gatatttaaa agggcgtgaa aaggtttatc 12960
   cttcgtccat ttgtatgtgc atgccaacca cagggttccc ca 13002
<210> 31
   <211> 13905
   <212> DNA
   <213> artificial sequence
<220>
   <223> pflanzlicher Expressionsvektor mit drei Promotor-Terminator-Expre ssionskassetten
<400> 31
   gatctggcgc cggccagcga gacgagcaag attggccgcc gcccgaaacg atccgacagc      60
   gcgcccagca caggtgcgca ggcaaattgc accaacgcat acagcgccag cagaatgcca     120
   tagtgggcgg tgacgtcgtt cgagtgaacc agatcgcgca ggaggcccgg cagcaccggc     180
   ataatcaggc cgatgccgac agcgtcgagc gcgacagtgc tcagaattac gatcaggggt     240
   atgttgggtt tcacgtctgg cctccggacc agcctccgct ggtccgattg aacgcgcgga     300
   ttctttatca ctgataagtt ggtggacata ttatgtttat cagtgataaa gtgtcaagca     360
   tgacaaagtt gcagccgaat acagtgatcc gtgccgccct ggacctgttg aacgaggtcg     420
   gcgtagacgg tctgacgaca cgcaaactgg cggaacggtt gggggttcag cagccggcgc     480
   tttactggca cttcaggaac aagcgggcgc tgctcgacgc actggccgaa gccatgctgg     540
   cggagaatca tacgcattcg gtgccgagag ccgacgacga ctggcgctca tttctgatcg     600
   ggaatgcccg cagcttcagg caggcgctgc tcgcctaccg cgatggcgcg cgcatccatg     660
   ccggcacgcg accgggcgca ccgcagatgg aaacggccga cgcgcagctt cgcttcctct     720
   gcgaggcggg tttttcggcc ggggacgccg tcaatgcgct gatgacaatc agctacttca     780
   ctgttggggc cgtgcttgag gagcaggccg gcgacagcga tgccggcgag cgcggcggca     840
   ccgttgaaca ggctccgctc tcgccgctgt tgcgggccgc gatagacgcc ttcgacgaag     900
   ccggtccgga cgcagcgttc gagcagggac tcgcggtgat tgtcgatgga ttggcgaaaa    960
   ggaggctcgt tgtcaggaac gttgaaggac cgagaaaggg tgacgattga tcaggaccgc   1020
   tgccggagcg caacccactc actacagcag agccatgtag acaacatccc ctcccccttt   1080
   ccaccgcgtc agacgcccgt agcagcccgc tacgggcttt ttcatgccct gccctagcgt   1140
   ccaagcctca cggccgcgct cggcctctct ggcggccttc tggcgctctt ccgcttcctc   1200
   gctcactgac tcgctgcgct cggtcgttcg gctgcggcga gcggtatcag ctcactcaaa   1260
   ggcggtaata cggttatcca cagaatcagg ggataacgca ggaaagaaca tgtgagcaaa   1320
   aggccagcaa aaggccagga accgtaaaaa ggccgcgttg ctggcgtttt tccataggct   1380
   ccgcccccct gacgagcatc acaaaaatcg acgctcaagt cagaggtggc gaaacccgac   1440
   aggactataa agataccagg cgtttccccc tggaagctcc ctcgtgcgct ctcctgttcc   1500
   gaccctgccg cttaccggat acctgtccgc ctttctccct tcgggaagcg tggcgctttt   1560
   ccgctgcata accctgcttc ggggtcatta tagcgatttt ttcggtatat ccatcctttt   1620
   tcgcacgata tacaggattt tgccaaaggg ttcgtgtaga ctttccttgg tgtatccaac   1680
   ggcgtcagcc gggcaggata ggtgaagtag gcccacccgc gagcgggtgt tccttcttca   1740
   ctgtccctta ttcgcacctg gcggtgctca acgggaatcc tgctctgcga ggctggccgg   1800
   ctaccgccgg cgtaacagat gagggcaagc ggatggctga tgaaaccaag ccaaccagga   1860
   agggcagccc acctatcaag gtgtactgcc ttccagacga acgaagagcg attgaggaaa   1920
   aggcggcggc ggccggcatg agcctgtcgg cctacctgct ggccgtcggc cagggctaca   1980
   aaatcacggg cgtcgtggac tatgagcacg tccgcgagct ggcccgcatc aatggcgacc   2040
   tgggccgcct gggcggcctg ctgaaactct ggctcaccga cgacccgcgc acggcgcggt   2100
   tcggtgatgc cacgatcctc gccctgctgg cgaagatcga agagaagcag gacgagcttg   2160
   gcaaggtcat gatgggcgtg gtccgcccga gggcagagcc atgacttttt tagccgctaa   2220
   aacggccggg gggtgcgcgt gattgccaag cacgtcccca tgcgctccat caagaagagc   2280
   gacttcgcgg agctggtgaa gtacatcacc gacgagcaag gcaagaccga gcgcctttgc   2340
   gacgctcacc gggctggttg ccctcgccgc tgggctggcg gccgtctatg gccctgcaaa   2400
   cgcgccagaa acgccgtcga agccgtgtgc gagacaccgc ggccgccggc gttgtggata   2460
   cctcgcggaa aacttggccc tcactgacag atgaggggcg gacgttgaca cttgaggggc   2520
   cgactcaccc ggcgcggcgt tgacagatga ggggcaggct cgatttcggc cggcgacgtg   2580
   gagctggcca gcctcgcaaa tcggcgaaaa cgcctgattt tacgcgagtt tcccacagat   2640
   gatgtggaca agcctgggga taagtgccct gcggtattga cacttgaggg gcgcgactac   2700
   tgacagatga ggggcgcgat ccttgacact tgaggggcag agtgctgaca gatgaggggc   2760
   gcacctattg acatttgagg ggctgtccac aggcagaaaa tccagcattt gcaagggttt 2820
   ccgcccgttt ttcggccacc gctaacctgt cttttaacct gcttttaaac caatatttat 2880
   aaaccttgtt tttaaccagg gctgcgccct gtgcgcgtga ccgcgcacgc cgaagggggg 2940
   tgccccccct tctcgaaccc tcccggcccg ctaacgcggg cctcccatcc ccccaggggc 3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa 3480
   tactgataag ataatatatc ttttatatag aagatatcgc cgtatgtaag gatttcaggg 3540
   ggcaaggcat aggcagcgcg cttatcaata tatctataga atgggcaaag cataaaaact 3600
   tgcatggact aatgcttgaa acccaggaca ataaccttat agcttgtaaa ttctatcata 3660
   attgggtaat gactccaact tattgatagt gttttatgtt cagataatgc ccgatgactt 3720
   tgtcatgcag ctccaccgat tttgagaacg acagcgactt ccgtcccagc cgtgccaggt 3780
   gctgcctcag attcaggtta tgccgctcaa ttcgctgcgt atatcgcttg ctgattacgt 3840
   gcagctttcc cttcaggcgg gattcataca gcggccagcc atccgtcatc catatcacca 3900
   cgtcaaaggg tgacagcagg ctcataagac gccccagcgt cgccatagtg cgttcaccga 3960
   atacgtgcgc aacaaccgtc ttccggagac tgtcatacgc gtaaaacagc cagcgctggc 4020
   gcgatttagc cccgacatag ccccactgtt cgtccatttc cgcgcagacg atgacgtcac 4080
   tgcccggctg tatgcgcgag gttaccgact gcggcctgag ttttttaagt gacgtaaaat 4140
   cgtgttgagg ccaacgccca taatgcgggc tgttgcccgg catccaacgc cattcatggc 4200
   catatcaatg attttctggt gcgtaccggg ttgagaagcg gtgtaagtga actgcagttg 4260
   ccatgtttta cggcagtgag agcagagata gcgctgatgt ccggcggtgc ttttgccgtt 4320
   acgcaccacc ccgtcagtag ctgaacagga gggacagctg atagacacag aagccactgg 4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg attttttaaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa 5640
   ggcaccaggc gggtcaaatc aggaataagg gcacattgcc ccggcgtgag tcggggcaat 5700
   cccgcaagga gggtgaatga atcggacgtt tgaccggaag gcatacaggc aagaactgat 5760
   cgacgcgggg ttttccgccg aggatgccga aaccatcgca agccgcaccg tcatgcgtgc 5820
   gccccgcgaa accttccagt ccgtcggctc gatggtccag caagctacgg ccaagatcga 5880
   gcgcgacagc gtgcaactgg ctccccctgc cctgcccgcg ccatcggccg ccgtggagcg 5940
   ttcgcgtcgt ctcgaacagg aggcggcagg tttggcgaag tcgatgacca tcgacacgcg 6000
   aggaactatg acgaccaaga agcgaaaaac cgccggcgag gacctggcaa aacaggtcag 6060
   cgaggccaag caggccgcgt tgctgaaaca cacgaagcag cagatcaagg aaatgcagct 6120
   ttccttgttc gatattgcgc cgtggccgga cacgatgcga gcgatgccaa acgacacggc 6180
   ccgctctgcc ctgttcacca cgcgcaacaa gaaaatcccg cgcgaggcgc tgcaaaacaa 6240
   ggtcattttc cacgtcaaca aggacgtgaa gatcacctac accggcgtcg agctgcgggc 6300
   cgacgatgac gaactggtgt ggcagcaggt gttggagtac gcgaagcgca cccctatcgg 6360
   cgagccgatc accttcacgt tctacgagct ttgccaggac ctgggctggt cgatcaatgg 6420
   ccggtattac acgaaggccg aggaatgcct gtcgcgccta caggcgacgg cgatgggctt 6480
   cacgtccgac cgcgttgggc acctggaatc ggtgtcgctg ctgcaccgct tccgcgtcct 6540
   ggaccgtggc aagaaaacgt cccgttgcca ggtcctgatc gacgaggaaa tcgtcgtgct 6600
   gtttgctggc gaccactaca cgaaattcat atgggagaag taccgcaagc tgtcgccgac 6660
   ggcccgacgg atgttcgact atttcagctc gcaccgggag ccgtacccgc tcaagctgga 6720
   aaccttccgc ctcatgtgcg gatcggattc cacccgcgtg aagaagtggc gcgagcaggt 6780
   cggcgaagcc tgcgaagagt tgcgaggcag cggcctggtg gaacacgcct gggtcaatga 6840
   tgacctggtg cattgcaaac gctagggcct tgtggggtca gttccggctg ggggttcagc 6900
   agccagcgct ttactggcat ttcaggaaca agcgggcact gctcgacgca cttgcttcgc 6960
   tcagtatcgc tcgggacgca cggcgcgctc tacgaactgc cgataaacag aggattaaaa 7020
   ttgacaattg tgattaaggc tcagattcga cggcttggag cggccgacgt gcaggatttc 7080
   cgcgagatcc gattgtcggc cctgaagaaa gctccagaga tgttcgggtc cgtttacgag 7140
   cacgaggaga aaaagcccat ggaggcgttc gctgaacggt tgcgagatgc cgtggcattc 7200
   ggcgcctaca tcgacggcga gatcattggg ctgtcggtct tcaaacagga ggacggcccc 7260
   aaggacgctc acaaggcgca tctgtccggc gttttcgtgg agcccgaaca gcgaggccga 7320
   ggggtcgccg gtatgctgct gcgggcgttg ccggcgggtt tattgctcgt gatgatcgtc 7380
   cgacagattc caacgggaat ctggtggatg cgcatcttca tcctcggcgc acttaatatt 7440
   tcgctattct ggagcttgtt gtttatttcg gtctaccgcc tgccgggcgg ggtcgcggcg 7500
   acggtaggcg ctgtgcagcc gctgatggtc gtgttcatct ctgccgctct gctaggtagc 7560
   ccgatacgat tgatggcggt cctgggggct atttgcggaa ctgcgggcgt ggcgctgttg 7620
   gtgttgacac caaacgcagc gctagatcct gtcggcgtcg cagcgggcct ggcgggggcg 7680
   gtttccatgg cgttcggaac cgtgctgacc cgcaagtggc aacctcccgt gcctctgctc 7740
   acctttaccg cctggcaact ggcggccgga ggacttctgc tcgttccagt agctttagtg 7800
   tttgatccgc caatcccgat gcctacagga accaatgttc tcggcctggc gtggctcggc 7860
   ctgatcggag cgggtttaac ctacttcctt tggttccggg ggatctcgcg actcgaacct 7920
   acagttgttt ccttactggg ctttctcagc cccagatctg gggtcgatca gccggggatg 7980
   catcaggccg acagtcggaa cttcgggtcc ccgacctgta ccattcggtg agcaatggat 8040
   aggggagttg atatcgtcaa cgttcacttc taaagaaata gcgccactca gcttcctcag 8100
   cggctttatc cagcgatttc ctattatgtc ggcatagttc tcaagatcga cagcctgtca 8160
   cggttaagcg agaaatgaat aagaaggctg ataattcgga tctctgcgag ggagatgata 8220
   tttgatcaca ggcagcaacg ctctgtcatc gttacaatca acatgctacc ctccgcgaga 8280
   tcatccgtgt ttcaaacccg gcagcttagt tgccgttctt ccgaatagca tcggtaacat 8340
   gagcaaagtc tgccgcctta caacggctct cccgctgacg ccgtcccgga ctgatgggct 8400
   gcctgtatcg agtggtgatt ttgtgccgag ctgccggtcg gggagctgtt ggctggctgg 8460
   tggcaggata tattgtggtg taaacaaatt gacgcttaga caacttaata acacattgcg 8520
   gacgttttta atgtactggg gtggtttttc ttttcaccag tgagacgggc aacagctgat 8580
   tgcccttcac cgcctggccc tgagagagtt gcagcaagcg gtccacgctg gtttgcccca 8640
   gcaggcgaaa atcctgtttg atggtggttc cgaaatcggc aaaatccctt ataaatcaaa 8700
   agaatagccc gagatagggt tgagtgttgt tccagtttgg aacaagagtc cactattaaa 8760
   gaacgtggac tccaacgtca aagggcgaaa aaccgtctat cagggcgatg gcccactacg 8820
   tgaaccatca cccaaatcaa gtttttgggg gtcgaggtgc cgtaaagcac taaatcggaa 8880
   ccctaaaggg agcccccgat ttagagcttg acggggaaag ccggcgaacg tggcgagaaa 8940
   ggaagggaag aaagcgaaag gagcgggcgc cattcaggct gcgcaactgt tgggaagggc 9000
   gatcggtgcg ggcctcttcg ctattacgcc agctggcgaa agggggatgt gctgcaaggc 9060
   gattaagttg ggtaacgcca gggttttccc agtcacgacg ttgtaaaacg acggccagtg 9120
   aattaattcc catcttgaaa gaaatatagt ttaaatattt attgataaaa taacaagtca 9180
   ggtattatag tccaagcaaa aacataaatt tattgatgca agtttaaatt cagaaatatt 9240
   tcaataactg attatatcag ctggtacatt gccgtagatg aaagactgag tgcgatatta 9300
   tgtgtaatac ataaattgat gatatagcta gcttagctca tcgggggatc cgtcgaagct 9360
   agcttgggtc ccgctcagaa gaactcgtca agaaggcgat agaaggcgat gcgctgcgaa 9420
   tcgggagcgg cgataccgta aagcacgagg aagcggtcag cccattcgcc gccaagctct 9480
   tcagcaatat cacgggtagc caacgctatg tcctgatagc ggtccgccac acccagccgg 9540
   ccacagtcga tgaatccaga aaagcggcca ttttccacca tgatattcgg caagcaggca 9600
   tcgccatggg tcacgacgag atcctcgccg tcgggcatgc gcgccttgag cctggcgaac 9660
   agttcggctg gcgcgagccc ctgatgctct tcgtccagat catcctgatc gacaagaccg 9720
   gcttccatcc gagtacgtgc tcgctcgatg cgatgtttcg cttggtggtc gaatgggcag 9780
   gtagccggat caagcgtatg cagccgccgc attgcatcag ccatgatgga tactttctcg 9840
   gcaggagcaa ggtgagatga caggagatcc tgccccggca cttcgcccaa tagcagccag 9900
   tcccttcccg cttcagtgac aacgtcgagc acagctgcgc aaggaacgcc cgtcgtggcc 9960
   agccacgata gccgcgctgc ctcgtcctgc agttcattca gggcaccgga caggtcggtc 10020
   ttgacaaaaa gaaccgggcg cccctgcgct gacagccgga acacggcggc atcagagcag 10080
   ccgattgtct gttgtgccca gtcatagccg aatagcctct ccacccaagc ggccggagaa 10140
   cctgcgtgca atccatcttg ttcaatccaa gctcccatgg gccctcgact agagtcgaga 10200
   tctggattga gagtgaatat gagactctaa ttggataccg aggggaattt atggaacgtc   10260
   agtggagcat ttttgacaag aaatatttgc tagctgatag tgaccttagg cgacttttga   10320
   acgcgcaata atggtttctg acgtatgtgc ttagctcatt aaactccaga aacccgcggc   10380
   tgagtggctc cttcaacgtt gcggttctgt cagttccaaa cgtaaaacgg cttgtcccgc   10440
   gtcatcggcg ggggtcataa cgtgactccc ttaattctcc gctcatgatc ttgatcccct   10500
   gcgccatcag atccttggcg gcaagaaagc catccagttt actttgcagg gcttcccaac   10560
   cttaccagag ggcgccccag ctggcaattc cggttcgctt gctgtccata aaaccgccca   10620
   gtctagctat cgccatgtaa gcccactgca agctacctgc tttctctttg cgcttgcgtt   10680
   ttcccttgtc cagatagccc agtagctgac attcatccgg ggtcagcacc gtttctgcgg   10740
   actggctttc tacgtgttcc gcttccttta gcagcccttg cgccctgagt gcttgcggca   10800
   gcgtgaagct tgcatgcctg caggtcgacg gcgcgccgag ctcctcgagc aaatttacac   10860
   attgccacta aacgtctaaa cccttgtaat ttgtttttgt tttactatgt gtgttatgta   10920
   tttgatttgc gataaatttt tatatttggt actaaattta taacaccttt tatgctaacg   10980
   tttgccaaca cttagcaatt tgcaagttga ttaattgatt ctaaattatt tttgtcttct   11040
   aaatacatat actaatcaac tggaaatgta aatatttgct aatatttcta ctataggaga   11100
   attaaagtga gtgaatatgg taccacaagg tttggagatt taattgttgc aatgctgcat   11160
   ggatggcata tacaccaaac attcaataat tcttgaggat aataatggta ccacacaaga   11220
   tttgaggtgc atgaacgtca cgtggacaaa aggtttagta atttttcaag acaacaatgt   11280
   taccacacac aagttttgag gtgcatgcat ggatgccctg tggaaagttt aaaaatattt   11340
   tggaaatgat ttgcatggaa gccatgtgta aaaccatgac atccacttgg aggatgcaat   11400
   aatgaagaaa actacaaatt tacatgcaac tagttatgca tgtagtctat ataatgagga   11460
   ttttgcaata ctttcattca tacacactca ctaagtttta cacgattata atttcttcat   11520
   agccagccca ccgcggtggg cggccgcctg cagtctagaa ggcctcctgc tttaatgaga   11580
   tatgcgagac gcctatgatc gcatgatatt tgctttcaat tctgttgtgc acgttgtaaa   11640
   aaacctgagc atgtgtagct cagatcctta ccgccggttt cggttcattc taatgaatat   11700
   atcacccgtt actatcgtat ttttatgaat aatattctcc gttcaattta ctgattgtcc   11760
   gtcgagcaaa tttacacatt gccactaaac gtctaaaccc ttgtaatttg tttttgtttt   11820
   actatgtgtg ttatgtattt gatttgcgat aaatttttat atttggtact aaatttataa   11880
   caccttttat gctaacgttt gccaacactt agcaatttgc aagttgatta attgattcta   11940
   aattattttt gtcttctaaa tacatatact aatcaactgg aaatgtaaat atttgctaat   12000
   atttctacta taggagaatt aaagtgagtg aatatggtac cacaaggttt ggagatttaa   12060
   ttgttgcaat gctgcatgga tggcatatac accaaacatt caataattct tgaggataat 12120
   aatggtacca cacaagattt gaggtgcatg aacgtcacgt ggacaaaagg tttagtaatt 12180
   tttcaagaca acaatgttac cacacacaag ttttgaggtg catgcatgga tgccctgtgg 12240
   aaagtttaaa aatattttgg aaatgatttg catggaagcc atgtgtaaaa ccatgacatc 12300
   cacttggagg atgcaataat gaagaaaact acaaatttac atgcaactag ttatgcatgt 12360
   agtctatata atgaggattt tgcaatactt tcattcatac acactcacta agttttacac 12420
   gattataatt tcttcatagc cagcggatcc gatatcgggc ccgctagcgt taaccctgct 12480
   ttaatgagat atgcgagacg cctatgatcg catgatattt gctttcaatt ctgttgtgca 12540
   cgttgtaaaa aacctgagca tgtgtagctc agatccttac cgccggtttc ggttcattct 12600
   aatgaatata tcacccgtta ctatcgtatt tttatgaata atattctccg ttcaatttac 12660
   tgattgtccg tcgagcaaat ttacacattg ccactaaacg tctaaaccct tgtaatttgt 12720
   ttttgtttta ctatgtgtgt tatgtatttg atttgcgata aatttttata tttggtacta 12780
   aatttataac accttttatg ctaacgtttg ccaacactta gcaatttgca agttgattaa 12840
   ttgattctaa attatttttg tcttctaaat acatatacta atcaactgga aatgtaaata 12900
   tttgctaata tttctactat aggagaatta aagtgagtga atatggtacc acaaggtttg 12960
   gagatttaat tgttgcaatg ctgcatggat ggcatataca ccaaacattc aataattctt 13020
   gaggataata atggtaccac acaagatttg aggtgcatga acgtcacgtg gacaaaaggt 13080
   ttagtaattt ttcaagacaa caatgttacc acacacaagt tttgaggtgc atgcatggat 13140
   gccctgtgga aagtttaaaa atattttgga aatgatttgc atggaagcca tgtgtaaaac 13200
   catgacatcc acttggagga tgcaataatg aagaaaacta caaatttaca tgcaactagt 13260
   tatgcatgta gtctatataa tgaggatttt gcaatacttt cattcataca cactcactaa 13320
   gttttacacg attataattt cttcatagcc agcagatctg ccggcatcga tcccgggcca 13380
   tggcctgctt taatgagata tgcgagacgc ctatgatcgc atgatatttg ctttcaattc 13440
   tgttgtgcac gttgtaaaaa acctgagcat gtgtagctca gatccttacc gccggtttcg 13500
   gttcattcta atgaatatat cacccgttac tatcgtattt ttatgaataa tattctccgt 13560
   tcaatttact gattgtccgt cgacgagctc ggcgcgcctc tagaggatcg atgaattcag 13620
   atcggctgag tggctccttc aacgttgcgg ttctgtcagt tccaaacgta aaacggcttg 13680
   tcccgcgtca tcggcggggg tcataacgtg actcccttaa ttctccgctc atgatcagat 13740
   tgtcgtttcc cgccttcagt ttaaactatc agtgtttgac aggatatatt ggcgggtaaa 13800
   cctaagagaa aagagcgttt attagaataa tcggatattt aaaagggcgt gaaaaggttt 13860
   atccttcgtc catttgtatg tgcatgccaa ccacagggtt cccca 13905
<210> 32
   <211> 1443
   <212> DNA
   <213> Phaeodactylum tricornutum
<220>
   <221> CDS
   <222> (9)..(1442)
   <223>
<400> 32
   gatctaaa atg ggc aaa gga ggg gac gct cgg gcc tcg aag ggc tca acg      50
            Met Gly Lys Gly Gly Asp Ala Arg Ala Ser Lys Gly Ser Thr
            1               5                   10
   gcg gct cgc aag atc agt tgg cag gaa gtc aag acc cac gcg tct ccg       98
   Ala Ala Arg Lys Ile Ser Trp Gln Glu Val Lys Thr His Ala Ser Pro
   15                  20                  25                  30
   gag gac gcc tgg atc att cac tcc aat aag gtc tac gac gtg tcc aac      146
   Glu Asp Ala Trp Ile Ile His Ser Asn Lys Val Tyr Asp Val Ser Asn
                   35                  40                  45
   tgg cac gaa cat ccc gga ggc gcc gtc att ttc acg cac gcc ggt gac      194
   Trp His Glu His Pro Gly Gly Ala Val Ile Phe Thr His Ala Gly Asp
               50                  55                  60
   gac atg acg gac att ttc gct gcc ttt cac gca ccc gga tcg cag tcg      242
   Asp Met Thr Asp Ile Phe Ala Ala Phe His Ala Pro Gly Ser Gln Ser
           65                  70                  75
   ctc atg aag aag ttc tac att ggc gaa ttg ctc ccg gaa acc acc ggc      290
   Leu Met Lys Lys Phe Tyr Ile Gly Glu Leu Leu Pro Glu Thr Thr Gly
       80                  85                  90
   aag gag ccg cag caa atc gcc ttt gaa aag ggc tac cgc gat ctg cgc      338
   Lys Glu Pro Gln Gln Ile Ala Phe Glu Lys Gly Tyr Arg Asp Leu Arg
   95                  100                 105                 110
   tcc aaa ctc atc atg atg ggc atg ttc aag tcc aac aag tgg ttc tac      386
   Ser Lys Leu Ile Met Met Gly Met Phe Lys Ser Asn Lys Trp Phe Tyr
                   115                 120                 125
   gtc tac aag tgc ctc agc aac atg gcc att tgg gcc gcc gcc tgt gct      434
   Val Tyr Lys Cys Leu Ser Asn Met Ala Ile Trp Ala Ala Ala Cys Ala
               130                 135                 140
   ctc gtc ttt tac tcg gac cgc ttc tgg gta cac ctg gcc agc gcc gtc      482
   Leu Val Phe Tyr Ser Asp Arg Phe Trp Val His Leu Ala Ser Ala Val
           145                 150                 155
   atg ctg gga aca ttc ttt cag cag tcg gga tgg ttg gca cac gac ttt      530
   Met Leu Gly Thr Phe Phe Gln Gln Ser Gly Trp Leu Ala His Asp Phe
       160                 165                 170
   ctg cac cac cag gtc ttc acc aag cgc aag cac ggg gat ctc gga gga      578
   Leu His His Gln Val Phe Thr Lys Arg Lys His Gly Asp Leu Gly Gly
   175                 180                 185                 190
   ctc ttt tgg ggg aac ctc atg cag ggt tac tcc gta cag tgg tgg aaa      626
   Leu Phe Trp Gly Asn Leu Met Gln Gly Tyr Ser Val Gln Trp Trp Lys
                   195                 200                 205
   aac aag cac aac gga cac cac gcc gtc ccc aac ctc cac tgc tcc tcc      674
   Asn Lys His Asn Gly His His Ala Val Pro Asn Leu His Cys Ser Ser
               210                 215                 220
   gca gtc gcg caa gat ggg gac ccg gac atc gat acc atg ccc ctt ctc      722
   Ala Val Ala Gln Asp Gly Asp Pro Asp Ile Asp Thr Met Pro Leu Leu
       225                 230                 235
   gcc tgg tcc gtc cag caa gcc cag tct tac cgg gaa ctc caa gcc gac      770
   Ala Trp Ser Val Gln Gln Ala Gln Ser Tyr Arg Glu Leu Gln Ala Asp
   240                 245                 250
   gga aag gat tcg ggt ttg gtc aag ttc atg atc cgt aac caa tcc tac      818
   Gly Lys Asp Ser Gly Leu Val Lys Phe Met Ile Arg Asn Gln Ser Tyr
   255                 260                 265                 270
   ttt tac ttt ccc atc ttg ttg ctc gcc cgc ctg tcg tgg ttg aac gag      866
   Phe Tyr Phe Pro Ile Leu Leu Leu Ala Arg Leu Ser Trp Leu Asn Glu
                   275                 280                 285
   tcc ttc aag tgc gcc ttt ggg ctt gga gct gcg tcg gag aac gct gct      914
   Ser Phe Lys Cys Ala Phe Gly Leu Gly Ala Ala Ser Glu Asn Ala Ala
               290                 295                 300
   ctc gaa ctc aag gcc aag ggt ctt cag tac ccc ctt ttg gaa aag gct      962
   Leu Glu Leu Lys Ala Lys Gly Leu Gln Tyr Pro Leu Leu Glu Lys Ala
           305                 310                 315
   ggc atc ctg ctg cac tac gct tgg atg ctt aca gtt tcg tcc ggc ttt      1010
   Gly Ile Leu Leu His Tyr Ala Trp Met Leu Thr Val Ser Ser Gly Phe
       320                 325                 330
   gga cgc ttc tcg ttc gcg tac acc gca ttt tac ttt cta acc gcg acc      1058
   Gly Arg Phe Ser Phe Ala Tyr Thr Ala Phe Tyr Phe Leu Thr Ala Thr
   335                 340                 345                 350
   gcg tcc tgt gga ttc ttg ctc gcc att gtc ttt ggc ctc ggc cac aac      1106
   Ala Ser Cys Gly Phe Leu Leu Ala Ile Val Phe Gly Leu Gly His Asn
                   355                 360                 365
   ggc atg gcc acc tac aat gcc gac gcc cgt ccg gac ttc tgg aag ctc      1154
   Gly Met Ala Thr Tyr Asn Ala Asp Ala Arg Pro Asp Phe Trp Lys Leu
               370                 375                 380
   caa gtc acc acg act cgc aac gtc acg ggc gga cac ggt ttc ccc caa      1202
   Gln Val Thr Thr Thr Arg Asn Val Thr Gly Gly His Gly Phe Pro Gln
           385                 390                 395
   gcc ttt gtc gac tgg ttc tgt ggt ggc ctc cag tac caa gtc gac cac      1250
   Ala Phe Val Asp Trp Phe Cys Gly Gly Leu Gln Tyr Gln Val Asp His
       400                 405                 410
   cac tta ttc ccc agc ctg ccc cga cac aat ctg gcc aag aca cac gca      1298
   His Leu Phe Pro Ser Leu Pro Arg His Asn Leu Ala Lys Thr His Ala
   415                 420                 425                 430
   ctg gtc gaa tcg ttc tgc aag gag tgg ggt gtc cag tac cac gaa gcc      1346
   Leu Val Glu Ser Phe Cys Lys Glu Trp Gly Val Gln Tyr His Glu Ala
                   435                 440                 445
   gac ctt gtg gac ggg acc atg gaa gtc ttg cac cat ttg ggc agc gtg     1394
   Asp Leu Val Asp Gly Thr Met Glu Val Leu His His Leu Gly Ser Val
               450                 455                 460
   gcc ggc gaa ttc gtc gtg gat ttt gta cgc gat gga ccc gcc atg taa a   1443
   Ala Gly Glu Phe Val Val Asp Phe Val Arg Asp Gly Pro Ala Met
           465                 470                 475
<210> 33
   <211> 477
   <212> PRT
   <213> Phaeodactylum tricornutum
<400> 33
   Met Gly Lys Gly Gly Asp Ala Arg Ala Ser Lys Gly Ser Thr Ala Ala
   1               5                   10                  15
   Arg Lys Ile Ser Trp Gln Glu Val Lys Thr His Ala Ser Pro Glu Asp
               20                  25                  30
   Ala Trp Ile Ile His Ser Asn Lys Val Tyr Asp Val Ser Asn Trp His
           35                  40                  45
   Glu His Pro Gly Gly Ala Val Ile Phe Thr His Ala Gly Asp Asp Met
       50                  55                  60
   Thr Asp Ile Phe Ala Ala Phe His Ala Pro Gly Ser Gln Ser Leu Met
   65                  70                  75                  80
   Lys Lys Phe Tyr Ile Gly Glu Leu Leu Pro Glu Thr Thr Gly Lys Glu
                   85                  90                  95
   Pro Gln Gln Ile Ala Phe Glu Lys Gly Tyr Arg Asp Leu Arg Ser Lys
               100                 105                 110
   Leu Ile Met Met Gly Met Phe Lys Ser Asn Lys Trp Phe Tyr Val Tyr
           115                 120                 125
   Lys Cys Leu Ser Asn Met Ala Ile Trp Ala Ala Ala Cys Ala Leu Val
       130                 135                 140
   Phe Tyr Ser Asp Arg Phe Trp Val His Leu Ala Ser Ala Val Met Leu
   145                 150                 155                 160
   Gly Thr Phe Phe Gln Gln Ser Gly Trp Leu Ala His Asp Phe Leu His
                   165                 170                 175
   His Gln Val Phe Thr Lys Arg Lys His Gly Asp Leu Gly Gly Leu Phe
   180 185 190
   Trp Gly Asn Leu Met Gln Gly Tyr Ser Val Gln Trp Trp Lys Asn Lys
   195 200 205
   His Asn Gly His His Ala Val Pro Asn Leu His Cys Ser Ser Ala Val
   210 215 220
   Ala Gln Asp Gly Asp Pro Asp Ile Asp Thr Met Pro Leu Leu Ala Trp
   225 230 235 240
   Ser Val Gln Gln Ala Gln Ser Tyr Arg Glu Leu Gln Ala Asp Gly Lys
   245 250 255
   Asp Ser Gly Leu Val Lys Phe Met Ile Arg Asn Gln Ser Tyr Phe Tyr
   260 265 270
   Phe Pro Ile Leu Leu Leu Ala Arg Leu Ser Trp Leu Asn Glu Ser Phe
   275 280 285
   Lys Cys Ala Phe Gly Leu Gly Ala Ala Ser Glu Asn Ala Ala Leu Glu
   290 295 300
   Leu Lys Ala Lys Gly Leu Gln Tyr Pro Leu Leu Glu Lys Ala Gly Ile
   305 310 315 320
   Leu Leu His Tyr Ala Trp Met Leu Thr Val Ser Ser Gly Phe Gly Arg
   325 330 335
   Phe Ser Phe Ala Tyr Thr Ala Phe Tyr Phe Leu Thr Ala Thr Ala Ser
   340 345 350
   Cys Gly Phe Leu Leu Ala Ile Val Phe Gly Leu Gly His Asn Gly Met
   355 360 365
   Ala Thr Tyr Asn Ala Asp Ala Arg Pro Asp Phe Trp Lys Leu Gln Val
   370 375 380
   Thr Thr Thr Arg Asn Val Thr Gly Gly His Gly Phe Pro Gln Ala Phe
   385 390 395 400
   Val Asp Trp Phe Cys Gly Gly Leu Gln Tyr Gln Val Asp His His Leu
   405 410 415
   Phe Pro Ser Leu Pro Arg His Asn Leu Ala Lys Thr His Ala Leu Val
   420 425 430
   Glu Ser Phe Cys Lys Glu Trp Gly Val Gln Tyr His Glu Ala Asp Leu
   435 440 445
   Val Asp Gly Thr Met Glu Val Leu His His Leu Gly Ser Val Ala Gly
   450 455 460
   Glu Phe Val Val Asp Phe Val Arg Asp Gly Pro Ala Met
   465 470 475
<210> 34
   <211> 17061
   <212> DNA
   <213> Phaeodactylum tricornutum, Physcomitrella patens, Caenorhabditis elegans
<220>
   <221> CDS
   <222> (4554)..(5987)
   <223> I
<220>
   <221> CDS
   <222> (2805)..(3653)
   <223>
<220>
   <221> CDS
   <222> (1026)..(1898)
   <223>
<400> 34
   tggggaaccc tgtggttggc atgcacatac aaatggacga aggataaacc ttttcacgcc 60
   cttttaaata tccgattatt ctaataaacg ctcttttctc ttaggtttac ccgccaatat 120
   atcctgtcaa acactgatag tttaaactga aggcgggaaa cgacaatctg atcatgagcg 180
   gagaattaag ggagtcacgt tatgaccccc gccgatgacg cgggacaagc cgttttacgt 240
   ttggaactga cagaaccgca acgttgaagg agccactcag ccgatctgaa ttcatcgatc 300
   ctctagaggc gcgccgagct cctcgagcaa atttacacat tgccactaaa cgtctaaacc 360
   cttgtaattt gtttttgttt tactatgtgt gttatgtatt tgatttgcga taaattttta 420
   tatttggtac taaatttata acacctttta tgctaacgtt tgccaacact tagcaatttg 480
   caagttgatt aattgattct aaattatttt tgtcttctaa atacatatac taatcaactg 540
   gaaatgtaaa tatttgctaa tatttctact ataggagaat taaagtgagt gaatatggta 600
   ccacaaggtt tggagattta attgttgcaa tgctgcatgg atggcatata caccaaacat 660
   tcaataattc ttgaggataa taatggtacc acacaagatt tgaggtgcat gaacgtcacg 720
   tggacaaaag gtttagtaat ttttcaagac aacaatgtta ccacacacaa gttttgaggt    780
   gcatgcatgg atgccctgtg gaaagtttaa aaatattttg gaaatgattt gcatggaagc    840
   catgtgtaaa accatgacat ccacttggag gatgcaataa tgaagaaaac tacaaattta    900
   catgcaacta gttatgcatg tagtctatat aatgaggatt ttgcaatact ttcattcata    960
   cacactcact aagttttaca cgattataat ttcttcatag ccagcccacc gcggtgggcg   1020
   gccgc atg gag gtc gtg gag aga ttc tac ggt gag ttg gat ggg aag gtc   1070
         Met Glu Val Val Glu Arg Phe Tyr Gly Glu Leu Asp Gly Lys Val
         1               5                   10                  15
   tcg cag ggc gtg aat gca ttg ctg ggt agt ttt ggg gtg gag ttg acg     1118
   Ser Gln Gly Val Asn Ala Leu Leu Gly Ser Phe Gly Val Glu Leu Thr
                   20                  25                  30
   gat acg ccc act acc aaa ggc ttg ccc ctc gtt gac agt ccc aca ccc     1166
   Asp Thr Pro Thr Thr Lys Gly Leu Pro Leu Val Asp Ser Pro Thr Pro
               35                  40                  45
   atc gtc ctc ggt gtt tct gta tac ttg act att gtc att gga ggg ctt     1214
   Ile Val Leu Gly Val Ser Val Tyr Leu Thr Ile Val Ile Gly Gly Leu
           50                  55                  60
   ttg tgg ata aag gcc agg gat ctg aaa ccg cgc gcc tcg gag cca ttt     1262
   Leu Trp Ile Lys Ala Arg Asp Leu Lys Pro Arg Ala Ser Glu Pro Phe
       65                  70                  75
   ttg ctc caa gct ttg gtg ctt gtg cac aac ctg ttc tgt ttt gcg ctc     1310
   Leu Leu Gln Ala Leu Val Leu Val His Asn Leu Phe Cys Phe Ala Leu
   80                  85                  90                  95
   agt ctg tat atg tgc gtg ggc atc gct tat cag gct att acc tgg cgg     1358
   Ser Leu Tyr Met Cys Val Gly Ile Ala Tyr Gln Ala Ile Thr Trp Arg
                   100                 105                 110
   tac tct ctc tgg ggc aat gca tac aat cct aaa cat aaa gag atg gcg     1406
   Tyr Ser Leu Trp Gly Asn Ala Tyr Asn Pro Lys His Lys Glu Met Ala
               115                 120                 125
   att ctg gta tac ttg ttc tac atg tct aag tac gtg gaa ttc atg gat     1454
   Ile Leu Val Tyr Leu Phe Tyr Met Ser Lys Tyr Val Glu Phe Met Asp
           130                 135                 140
   acc gtt atc atg ata ctg aag cgc agc acc agg caa ata agc ttc ctc     1502
   Thr Val Ile Met Ile Leu Lys Arg Ser Thr Arg Gln Ile Ser Phe Leu
       145                 150                 155
   cac gtt tat cat cat tct tca att tcc ctc att tgg tgg gct att gct     1550
   His Val Tyr His His Ser Ser Ile Ser Leu Ile Trp Trp Ala Ile Ala
   160                 165                 170                 175
   cat cac gct cct ggc ggt gaa gca tat tgg tct gcg gct ctg aac tca     1598
   His His Ala Pro Gly Gly Glu Ala Tyr Trp Ser Ala Ala Leu Asn Ser
                   180                 185                 190
   gga gtg cat gtt ctc atg tat gcg tat tac ttc ttg gct gcc tgc ctt     1646
   Gly Val His Val Leu Met Tyr Ala Tyr Tyr Phe Leu Ala Ala Cys Leu
               195                 200                 205
   cga agt agc cca aag tta aaa aat aag tac ctt ttt tgg ggc agg tac      1694
   Arg Ser Ser Pro Lys Leu Lys Asn Lys Tyr Leu Phe Trp Gly Arg Tyr
           210                 215                 220
   ttg aca caa ttc caa atg ttc cag ttt atg ctg aac tta gtg cag gct      1742
   Leu Thr Gln Phe Gln Met Phe Gln Phe Met Leu Asn Leu Val Gln Ala
       225                 230                 235
   tac tac gac atg aaa acg aat gcg cca tat cca caa tgg ctg atc aag      1790
   Tyr Tyr Asp Met Lys Thr Asn Ala Pro Tyr Pro Gln Trp Leu Ile Lys
   240                 245                 250                 255
   att ttg ttc tac tac atg atc tcg ttg ctg ttt ctt ttc ggc aat ttt      1838
   Ile Leu Phe Tyr Tyr Met Ile Ser Leu Leu Phe Leu Phe Gly Asn Phe
                   260                 265                 270
   tac gta caa aaa tac atc aaa ccc tct gac gga aag caa aag gga gct      1886
   Tyr Val Gln Lys Tyr Ile Lys Pro Ser Asp Gly Lys Gln Lys Gly Ala
               275                 280                 285
   aaa act gag tga tctagaaggc ctcctgcttt aatgagatat gcgagacgcc          1938
   Lys Thr Glu
           290
   tatgatcgca tgatatttgc tttcaattct gttgtgcacg ttgtaaaaaa cctgagcatg    1998
   tgtagctcag atccttaccg ccggtttcgg ttcattctaa tgaatatatc acccgttact    2058
   atcgtatttt tatgaataat attctccgtt caatttactg attgtccgtc gagcaaattt    2118
   acacattgcc actaaacgtc taaacccttg taatttgttt ttgttttact atgtgtgtta    2178
   tgtatttgat ttgcgataaa tttttatatt tggtactaaa tttataacac cttttatgct    2238
   aacgtttgcc aacacttagc aatttgcaag ttgattaatt gattctaaat tatttttgtc    2298
   ttctaaatac atatactaat caactggaaa tgtaaatatt tgctaatatt tctactatag    2358
   gagaattaaa gtgagtgaat atggtaccac aaggtttgga gatttaattg ttgcaatgct    2418
   gcatggatgg catatacacc aaacattcaa taattcttga ggataataat ggtaccacac    2478
   aagatttgag gtgcatgaac gtcacgtgga caaaaggttt agtaattttt caagacaaca    2538
   atgttaccac acacaagttt tgaggtgcat gcatggatgc cctgtggaaa gtttaaaaat    2598
   attttggaaa tgatttgcat ggaagccatg tgtaaaacca tgacatccac ttggaggatg    2658
   caataatgaa gaaaactaca aatttacatg caactagtta tgcatgtagt ctatataatg    2718
   aggattttgc aatactttca ttcatacaca ctcactaagt tttacacgat tataatttct    2778
   tcatagccag cggatccgcc cacata atg gag aac ttc tgg tct att gtt gtg     2831
                                Met Glu Asn Phe Trp Ser Ile Val Val
                                                    295
   ttt ttt cta ctc tca att ctc ttc att tta tat aac ata tcg aca gta      2879
   Phe Phe Leu Leu Ser Ile Leu Phe Ile Leu Tyr Asn Ile Ser Thr Val
   300                 305                 310                 315
   tgc cac tac tat atg cgg att tcg ttt tat tac ttc aca att tta ttg      2927
   Cys His Tyr Tyr Met Arg Ile Ser Phe Tyr Tyr Phe Thr Ile Leu Leu
                       320                 325                 330
   cat gga atg gaa gtt tgt gtt aca atg atc cct tct tgg cta aat ggg      2975
   His Gly Met Glu Val Cys Val Thr Met Ile Pro Ser Trp Leu Asn Gly
                   335                 340                 345
   aag ggt gct gat tac gtg ttt cac tcg ttt ttc tat tgg tgt aaa tgg      3023
   Lys Gly Ala Asp Tyr Val Phe His Ser Phe Phe Tyr Trp Cys Lys Trp
               350                 355                 360
   act ggt gtt cat aca aca gtc tat gga tat gaa aaa aca caa gtt gaa      3071
   Thr Gly Val His Thr Thr Val Tyr Gly Tyr Glu Lys Thr Gln Val Glu
       365                 370                 375
   ggt ccg gct gta gtt att tgt aat cat cag agt tct ctc gac att cta      3119
   Gly Pro Ala Val Val Ile Cys Asn His Gln Ser Ser Leu Asp Ile Leu
   380                 385                 390                 395
   tcg atg gca tca atc tgg ccg aag aat tgt gtt gta atg atg aaa cga      3167
   Ser Met Ala Ser Ile Trp Pro Lys Asn Cys Val Val Met Met Lys Arg
                   400                 405                 410
   att ctt gcc tat gtt cca ttc ttc aat ctc gga gcc tac ttt tcc aac      3215
   Ile Leu Ala Tyr Val Pro Phe Phe Asn Leu Gly Ala Tyr Phe Ser Asn
               415                 420                 425
   aca atc ttc atc gat cga tat aac cgt gaa cgt gcg atg gct tca gtt      3263
   Thr Ile Phe Ile Asp Arg Tyr Asn Arg Glu Arg Ala Met Ala Ser Val
           430                 435                 440
   gat tat tgt gca tct gaa atg aag aac aga aat ctt aaa ctt tgg gta      3311
   Asp Tyr Cys Ala Ser Glu Met Lys Asn Arg Asn Leu Lys Leu Trp Val
       445                 450                 455
   ttt ccg gaa gga aca aga aat cgt gaa gga ggg ttc att cca ttc aag      3359
   Phe Pro Glu Gly Thr Arg Asn Arg Glu Gly Gly Phe Ile Pro Phe Lys
   460                 465                 470                 475
   aaa gga gca ttc aat att gca gtt cgt gcg cag att ccc att att cca      3407
   Lys Gly Ala Phe Asn Ile Ala Val Arg Ala Gln Ile Pro Ile Ile Pro
                   480                 485                 490
   gtt gta ttc tca gac tat cgg gat ttc tac tca aag cca ggc cga tat      3455
   Val Val Phe Ser Asp Tyr Arg Asp Phe Tyr Ser Lys Pro Gly Arg Tyr
               495                 500                 505
   ttc aag aat gat gga gaa gtt gtt att cga gtt ctg gat gcg att cca      3503
   Phe Lys Asn Asp Gly Glu Val Val Ile Arg Val Leu Asp Ala Ile Pro
           510                 515                 520
   aca aaa ggg ctc act ctt gat gac gtc agc gag ttg tct gat atg tgt      3551
   Thr Lys Gly Leu Thr Leu Asp Asp Val Ser Glu Leu Ser Asp Met Cys
       525                 530                 535
   cgg gac gtt atg ttg gca gcc tat aag gaa gtt act cta gaa gct cag      3599
   Arg Asp Val Met Leu Ala Ala Tyr Lys Glu Val Thr Leu Glu Ala Gln
   540                 545                 550                 555
   caa cga aat gcg aca cgg cgt gga gaa aca aaa gac ggg aag aaa tct      3647
   Gln Arg Asn Ala Thr Arg Arg Gly Glu Thr Lys Asp Gly Lys Lys Ser
                   560                 565                 570
   gag taa gctagcgtta accctgcttt aatgagatat gcgagacgcc tatgatcgca       3703
   Glu
   tgatatttgc tttcaattct gttgtgcacg ttgtaaaaaa cctgagcatg tgtagctcag   3763
   atccttaccg ccggtttcgg ttcattctaa tgaatatatc acccgttact atcgtatttt   3823
   tatgaataat attctccgtt caatttactg attgtccgtc gagcaaattt acacattgcc   3883
   actaaacgtc taaacccttg taatttgttt ttgttttact atgtgtgtta tgtatttgat   3943
   ttgcgataaa tttttatatt tggtactaaa tttataacac cttttatgct aacgtttgcc   4003
   aacacttagc aatttgcaag ttgattaatt gattctaaat tatttttgtc ttctaaatac   4063
   atatactaat caactggaaa tgtaaatatt tgctaatatt tctactatag gagaattaaa   4123
   gtgagtgaat atggtaccac aaggtttgga gatttaattg ttgcaatgct gcatggatgg   4183
   catatacacc aaacattcaa taattcttga ggataataat ggtaccacac aagatttgag   4243
   gtgcatgaac gtcacgtgga caaaaggttt agtaattttt caagacaaca atgttaccac   4303
   acacaagttt tgaggtgcat gcatggatgc cctgtggaaa gtttaaaaat attttggaaa   4363
   tgatttgcat ggaagccatg tgtaaaacca tgacatccac ttggaggatg caataatgaa   4423
   gaaaactaca aatttacatg caactagtta tgcatgtagt ctatataatg aggattttgc   4483
   aatactttca ttcatacaca ctcactaagt tttacacgat tataatttct tcatagccag   4543
   cagatctaaa atg ggc aaa gga ggg gac gct cgg gcc tcg aag ggc tca      4592
              Met Gly Lys Gly Gly Asp Ala Arg Ala Ser Lys Gly Ser
                      575                 580                 585
   acg gcg gct cgc aag atc agt tgg cag gaa gtc aag acc cac gcg tct     4640
   Thr Ala Ala Arg Lys Ile Ser Trp Gln Glu Val Lys Thr His Ala Ser
                   590                 595                 600
   ccg gag gac gcc tgg atc att cac tcc aat aag gtc tac gac gtg tcc     4688
   Pro Glu Asp Ala Trp Ile Ile His Ser Asn Lys Val Tyr Asp Val Ser
               605                 610                 615
   aac tgg cac gaa cat ccc gga ggc gcc gtc att ttc acg cac gcc ggt     4736
   Asn Trp His Glu His Pro Gly Gly Ala Val Ile Phe Thr His Ala Gly
           620                 625                 630
   gac gac atg acg gac att ttc gct gcc ttt cac gca ccc gga tcg cag     4784
   Asp Asp Met Thr Asp Ile Phe Ala Ala Phe His Ala Pro Gly Ser Gln
       635                 640                 645
   tcg ctc atg aag aag ttc tac att ggc gaa ttg ctc ccg gaa acc acc     4832
   Ser Leu Met Lys Lys Phe Tyr Ile Gly Glu Leu Leu Pro Glu Thr Thr
   650                 655                 660                 665
   ggc aag gag ccg cag caa atc gcc ttt gaa aag ggc tac cgc gat ctg     4880
   Gly Lys Glu Pro Gln Gln Ile Ala Phe Glu Lys Gly Tyr Arg Asp Leu
                   670                 675                 680
   cgc tcc aaa ctc atc atg atg ggc atg ttc aag tcc aac aag tgg ttc     4928
   Arg Ser Lys Leu Ile Met Met Gly Met Phe Lys Ser Asn Lys Trp Phe
                   685                 690                 695
   tac gtc tac aag tgc ctc agc aac atg gcc att tgg gcc gcc gcc tgt      4976
   Tyr Val Tyr Lys Cys Leu Ser Asn Met Ala Ile Trp Ala Ala Ala Cys
           700                 705                 710
   gct ctc gtc ttt tac tcg gac cgc ttc tgg gta cac ctg gcc agc gcc      5024
   Ala Leu Val Phe Tyr Ser Asp Arg Phe Trp Val His Leu Ala Ser Ala
       715                 720                 725
   gtc atg ctg gga aca ttc ttt cag cag tcg gga tgg ttg gca cac gac      5072
   Val Met Leu Gly Thr Phe Phe Gln Gln Ser Gly Trp Leu Ala His Asp
   730                 735                 740                 745
   ttt ctg cac cac cag gtc ttc acc aag cgc aag cac ggg gat ctc gga      5120
   Phe Leu His His Gln Val Phe Thr Lys Arg Lys His Gly Asp Leu Gly
                   750                 755                 760
   gga ctc ttt tgg ggg aac ctc atg cag ggt tac tcc gta cag tgg tgg      5168
   Gly Leu Phe Trp Gly Asn Leu Met Gln Gly Tyr Ser Val Gln Trp Trp
               765                 770                 775
   aaa aac aag cac aac gga cac cac gcc gtc ccc aac ctc cac tgc tcc      5216
   Lys Asn Lys His Asn Gly His His Ala Val Pro Asn Leu His Cys Ser
           780                 785                 790
   tcc gca gtc gcg caa gat ggg gac ccg gac atc gat acc atg ccc ctt      5264
   Ser Ala Val Ala Gln Asp Gly Asp Pro Asp Ile Asp Thr Met Pro Leu
       795                 800                 805
   ctc gcc tgg tcc gtc cag caa gcc cag tct tac cgg gaa ctc caa gcc      5312
   Leu Ala Trp Ser Val Gln Gln Ala Gln Ser Tyr Arg Glu Leu Gln Ala
   810                 815                 820                 825
   gac gga aag gat tcg ggt ttg gtc aag ttc atg atc cgt aac caa tcc      5360
   Asp Gly Lys Asp Ser Gly Leu Val Lys Phe Met Ile Arg Asn Gln Ser
                   830                 835                 840
   tac ttt tac ttt ccc atc ttg ttg ctc gcc cgc ctg tcg tgg ttg aac      5408
   Tyr Phe Tyr Phe Pro Ile Leu Leu Leu Ala Arg Leu Ser Trp Leu Asn
               845                 850                 855
   gag tcc ttc aag tgc gcc ttt ggg ctt gga gct gcg tcg gag aac gct      5456
   Glu Ser Phe Lys Cys Ala Phe Gly Leu Gly Ala Ala Ser Glu Asn Ala
           860                 865                 870
   gct ctc gaa ctc aag gcc aag ggt ctt cag tac ccc ctt ttg gaa aag      5504
   Ala Leu Glu Leu Lys Ala Lys Gly Leu Gln Tyr Pro Leu Leu Glu Lys
       875                 880                 885
   gct ggc atc ctg ctg cac tac gct tgg atg ctt aca gtt tcg tcc ggc      5552
   Ala Gly Ile Leu Leu His Tyr Ala Trp Met Leu Thr Val Ser Ser Gly
   890                 895                 900                 905
   ttt gga cgc ttc tcg ttc gcg tac acc gca ttt tac ttt cta acc gcg      5600
   Phe Gly Arg Phe Ser Phe Ala Tyr Thr Ala Phe Tyr Phe Leu Thr Ala
                   910                 915                 920
   acc gcg tcc tgt gga ttc ttg ctc gcc att gtc ttt ggc ctc ggc cac      5648
   Thr Ala Ser Cys Gly Phe Leu Leu Ala Ile Val Phe Gly Leu Gly His
               925                 930                 935
   aac ggc atg gcc acc tac aat gcc gac gcc cgt ccg gac ttc tgg aag      5696
   Asn Gly Met Ala Thr Tyr Asn Ala Asp Ala Arg Pro Asp Phe Trp Lys
           940                 945                     950
   ctc caa gtc acc acg act cgc aac gtc acg ggc gga cac ggt ttc ccc      5744
   Leu Gln Val Thr Thr Thr Arg Asn Val Thr Gly Gly His Gly Phe Pro
       955                     960                     965
   caa gcc ttt gtc gac tgg ttc tgt ggt ggc ctc cag tac caa gtc gac      5792
   Gln Ala Phe Val Asp Trp Phe Cys Gly Gly Leu Gln Tyr Gln Val Asp
   970                     975                     980                 985
   cac cac tta ttc ccc agc ctg ccc cga cac aat ctg gcc aag aca cac      5840
   His His Leu Phe Pro Ser Leu Pro Arg His Asn Leu Ala Lys Thr His
                   990                     995                     1000
   gca ctg gtc gaa tcg ttc tgc aag gag tgg ggt gtc cag tac cac          5885
   Ala Leu Val Glu Ser Phe Cys Lys Glu Trp Gly Val Gln Tyr His
               1005                    1010                    1015
   gaa gcc gac ctt gtg gac ggg acc atg gaa gtc ttg cac cat ttg          5930
   Glu Ala Asp Leu Val Asp Gly Thr Met Glu Val Leu His His Leu
               1020                    1025                    1030
   ggc agc gtg gcc ggc gaa ttc gtc gtg gat ttt gta cgc gat gga          5975
   Gly Ser Val Ala Gly Glu Phe Val Val Asp Phe Val Arg Asp Gly
               1035                    1040                    1045
   ccc gcc atg taa agatctgccg gcatcgatcc cgggccatgg cctgctttaa          6027
   Pro Ala Met
   tgagatatgc gagacgccta tgatcgcatg atatttgctt tcaattctgt tgtgcacgtt    6087
   gtaaaaaacc tgagcatgtg tagctcagat ccttaccgcc ggtttcggtt cattctaatg    6147
   aatatatcac ccgttactat cgtattttta tgaataatat tctccgttca atttactgat    6207
   tgtccgtcga cgagctcggc gcgccgtcga cctgcaggca tgcaagcttc acgctgccgc    6267
   aagcactcag ggcgcaaggg ctgctaaagg aagcggaaca cgtagaaagc cagtccgcag    6327
   aaacggtgct gaccccggat gaatgtcagc tactgggcta tctggacaag ggaaaacgca    6387
   agcgcaaaga gaaagcaggt agcttgcagt gggcttacat ggcgatagct agactgggcg    6447
   gttttatgga cagcaagcga accggaattg ccagctgggg cgccctctgg taaggttggg    6507
   aagccctgca aagtaaactg gatggctttc ttgccgccaa ggatctgatg gcgcagggga    6567
   tcaagatcat gagcggagaa ttaagggagt cacgttatga cccccgccga tgacgcggga    6627
   caagccgttt tacgtttgga actgacagaa ccgcaacgtt gaaggagcca ctcagccgcg    6687
   ggtttctgga gtttaatgag ctaagcacat acgtcagaaa ccattattgc gcgttcaaaa    6747
   gtcgcctaag gtcactatca gctagcaaat atttcttgtc aaaaatgctc cactgacgtt    6807
   ccataaattc ccctcggtat ccaattagag tctcatattc actctcaatc cagatctcga    6867
   ctctagtcga gggcccatgg gagcttggat tgaacaagat ggattgcacg caggttctcc    6927
   ggccgcttgg gtggagaggc tattcggcta tgactgggca caacagacaa tcggctgctc 6987
   tgatgccgcc gtgttccggc tgtcagcgca ggggcgcccg gttctttttg tcaagaccga 7047
   cctgtccggt gccctgaatg aactgcagga cgaggcagcg cggctatcgt ggctggccac 7107
   gacgggcgtt ccttgcgcag ctgtgctcga cgttgtcact gaagcgggaa gggactggct 7167
   gctattgggc gaagtgccgg ggcaggatct cctgtcatct caccttgctc ctgccgagaa 7227
   agtatccatc atggctgatg caatgcggcg gctgcatacg cttgatccgg ctacctgccc 7287
   attcgaccac caagcgaaac atcgcatcga gcgagcacgt actcggatgg aagccggtct 7347
   tgtcgatcag gatgatctgg acgaagagca tcaggggctc gcgccagccg aactgttcgc 7407
   caggctcaag gcgcgcatgc ccgacggcga ggatctcgtc gtgacccatg gcgatgcctg 7467
   cttgccgaat atcatggtgg aaaatggccg cttttctgga ttcatcgact gtggccggct 7527
   gggtgtggcg gaccgctatc aggacatagc gttggctacc cgtgatattg ctgaagagct 7587
   tggcggcgaa tgggctgacc gcttcctcgt gctttacggt atcgccgctc ccgattcgca 7647
   gcgcatcgcc ttctatcgcc ttcttgacga gttcttctga gcgggaccca agctagcttc 7707
   gacggatccc ccgatgagct aagctagcta tatcatcaat ttatgtatta cacataatat 7767
   cgcactcagt ctttcatcta cggcaatgta ccagctgata taatcagtta ttgaaatatt 7827
   tctgaattta aacttgcatc aataaattta tgtttttgct tggactataa tacctgactt 7887
   gttattttat caataaatat ttaaactata tttctttcaa gatgggaatt aattcactgg 7947
   ccgtcgtttt acaacgtcgt gactgggaaa accctggcgt tacccaactt aatcgccttg 8007
   cagcacatcc ccctttcgcc agctggcgta atagcgaaga ggcccgcacc gatcgccctt 8067
   cccaacagtt gcgcagcctg aatggcgccc gctcctttcg ctttcttccc ttcctttctc 8127
   gccacgttcg ccggctttcc ccgtcaagct ctaaatcggg ggctcccttt agggttccga 8187
   tttagtgctt tacggcacct cgaccccaaa aaacttgatt tgggtgatgg ttcacgtagt 8247
   gggccatcgc cctgatagac ggtttttcgc cctttgacgt tggagtccac gttctttaat 8307
   agtggactct tgttccaaac tggaacaaca ctcaacccta tctcgggcta ttcttttgat 8367
   ttataaggga ttttgccgat ttcggaacca ccatcaaaca ggattttcgc ctgctggggc 8427
   aaaccagcgt ggaccgcttg ctgcaactct ctcagggcca ggcggtgaag ggcaatcagc 8487
   tgttgcccgt ctcactggtg aaaagaaaaa ccaccccagt acattaaaaa cgtccgcaat 8547
   gtgttattaa gttgtctaag cgtcaatttg tttacaccac aatatatcct gccaccagcc 8607
   agccaacagc tccccgaccg gcagctcggc acaaaatcac cactcgatac aggcagccca 8667
   tcagtccggg acggcgtcag cgggagagcc gttgtaaggc ggcagacttt gctcatgtta 8727
   ccgatgctat tcggaagaac ggcaactaag ctgccgggtt tgaaacacgg atgatctcgc 8787
   ggagggtagc atgttgattg taacgatgac agagcgttgc tgcctgtgat caaatatcat 8847
   ctccctcgca gagatccgaa ttatcagcct tcttattcat ttctcgctta accgtgacag 8907
   gctgtcgatc ttgagaacta tgccgacata ataggaaatc gctggataaa gccgctgagg 8967
   aagctgagtg gcgctatttc tttagaagtg aacgttgacg atatcaactc ccctatccat 9027
   tgctcaccga atggtacagg tcggggaccc gaagttccga ctgtcggcct gatgcatccc 9087
   cggctgatcg accccagatc tggggctgag aaagcccagt aaggaaacaa ctgtaggttc 9147
   gagtcgcgag atcccccgga accaaaggaa gtaggttaaa cccgctccga tcaggccgag 9207
   ccacgccagg ccgagaacat tggttcctgt aggcatcggg attggcggat caaacactaa 9267
   agctactgga acgagcagaa gtcctccggc cgccagttgc caggcggtaa aggtgagcag 9327
   aggcacggga ggttgccact tgcgggtcag cacggttccg aacgccatgg aaaccgcccc 9387
   cgccaggccc gctgcgacgc cgacaggatc tagcgctgcg tttggtgtca acaccaacag 9447
   cgccaggccc gcagttccgc aaatagcccc caggaccgcc atcaatcgta tcgggctacc 9507
   tagcagagcg gcagagatga acacgaccat cagcggctgc acagcgccta ccgtcgccgc 9567
   gaccccgccc ggcaggcggt agaccgaaat aaacaacaag ctccagaata gcgaaatatt 9627
   aagtgcgccg aggatgaaga tgcgcatcca ccagattccc gttggaatct gtcggacgat 9687
   catcacgagc aataaacccg ccggcaacgc ccgcagcagc ataccggcga cccctcggcc 9747
   tcgctgttcg ggctccacga aaacgccgga cagatgcgcc ttgtgagcgt ccttggggcc 9807
   gtcctcctgt ttgaagaccg acagcccaat gatctcgccg tcgatgtagg cgccgaatgc 9867
   cacggcatct cgcaaccgtt cagcgaacgc ctccatgggc tttttctcct cgtgctcgta 9927
   aacggacccg aacatctctg gagctttctt cagggccgac aatcggatct cgcggaaatc 9987
   ctgcacgtcg gccgctccaa gccgtcgaat ctgagcctta atcacaattg tcaattttaa 10047
   tcctctgttt atcggcagtt cgtagagcgc gccgtgcgtc ccgagcgata ctgagcgaag 10107
   caagtgcgtc gagcagtgcc cgcttgttcc tgaaatgcca gtaaagcgct ggctgctgaa 10167
   cccccagccg gaactgaccc cacaaggccc tagcgtttgc aatgcaccag gtcatcattg 10227
   acccaggcgt gttccaccag gccgctgcct cgcaactctt cgcaggcttc gccgacctgc 10287
   tcgcgccact tcttcacgcg ggtggaatcc gatccgcaca tgaggcggaa ggtttccagc 10347
   ttgagcgggt acggctcccg gtgcgagctg aaatagtcga acatccgtcg ggccgtcggc 10407
   gacagcttgc ggtacttctc ccatatgaat ttcgtgtagt ggtcgccagc aaacagcacg 10467
   acgatttcct cgtcgatcag gacctggcaa cgggacgttt tcttgccacg gtccaggacg 10527
   cggaagcggt gcagcagcga caccgattcc aggtgcccaa cgcggtcgga cgtgaagccc 10587
   atcgccgtcg cctgtaggcg cgacaggcat tcctcggcct tcgtgtaata ccggccattg 10647
   atcgaccagc ccaggtcctg gcaaagctcg tagaacgtga aggtgatcgg ctcgccgata 10707
   ggggtgcgct tcgcgtactc caacacctgc tgccacacca gttcgtcatc gtcggcccgc 10767
   agctcgacgc cggtgtaggt gatcttcacg tccttgttga cgtggaaaat gaccttgttt 10827
   tgcagcgcct cgcgcgggat tttcttgttg cgcgtggtga acagggcaga gcgggccgtg 10887
   tcgtttggca tcgctcgcat cgtgtccggc cacggcgcaa tatcgaacaa ggaaagctgc 10947
   atttccttga tctgctgctt cgtgtgtttc agcaacgcgg cctgcttggc ctcgctgacc 11007
   tgttttgcca ggtcctcgcc ggcggttttt cgcttcttgg tcgtcatagt tcctcgcgtg 11067
   tcgatggtca tcgacttcgc caaacctgcc gcctcctgtt cgagacgacg cgaacgctcc 11127
   acggcggccg atggcgcggg cagggcaggg ggagccagtt gcacgctgtc gcgctcgatc 11187
   ttggccgtag cttgctggac catcgagccg acggactgga aggtttcgcg gggcgcacgc 11247
   atgacggtgc ggcttgcgat ggtttcggca tcctcggcgg aaaaccccgc gtcgatcagt 11307
   tcttgcctgt atgccttccg gtcaaacgtc cgattcattc accctccttg cgggattgcc 11367
   ccgactcacg ccggggcaat gtgcccttat tcctgatttg acccgcctgg tgccttggtg 11427
   tccagataat ccaccttatc ggcaatgaag tcggtcccgt agaccgtctg gccgtccttc 11487
   tcgtacttgg tattccgaat cttgccctgc acgaatacca gcgacccctt gcccaaatac 11547
   ttgccgtggg cctcggcctg agagccaaaa cacttgatgc ggaagaagtc ggtgcgctcc 11607
   tgcttgtcgc cggcatcgtt gcgccacatc taggtactaa aacaattcat ccagtaaaat 11667
   ataatatttt attttctccc aatcaggctt gatccccagt aagtcaaaaa atagctcgac 11727
   atactgttct tccccgatat cctccctgat cgaccggacg cagaaggcaa tgtcatacca 11787
   cttgtccgcc ctgccgcttc tcccaagatc aataaagcca cttactttgc catctttcac 11847
   aaagatgttg ctgtctccca ggtcgccgtg ggaaaagaca agttcctctt cgggcttttc 11907
   cgtctttaaa aaatcataca gctcgcgcgg atctttaaat ggagtgtctt cttcccagtt 11967
   ttcgcaatcc acatcggcca gatcgttatt cagtaagtaa tccaattcgg ctaagcggct 12027
   gtctaagcta ttcgtatagg gacaatccga tatgtcgatg gagtgaaaga gcctgatgca 12087
   ctccgcatac agctcgataa tcttttcagg gctttgttca tcttcatact cttccgagca 12147
   aaggacgcca tcggcctcac tcatgagcag attgctccag ccatcatgcc gttcaaagtg 12207
   caggaccttt ggaacaggca gctttccttc cagccatagc atcatgtcct tttcccgttc 12267
   cacatcatag gtggtccctt tataccggct gtccgtcatt tttaaatata ggttttcatt 12327
   ttctcccacc agcttatata ccttagcagg agacattcct tccgtatctt ttacgcagcg 12387
   gtatttttcg atcagttttt tcaattccgg tgatattctc attttagcca tttattattt 12447
   ccttcctctt ttctacagta tttaaagata ccccaagaag ctaattataa caagacgaac 12507
   tccaattcac tgttccttgc attctaaaac cttaaatacc agaaaacagc tttttcaaag 12567
   ttgttttcaa agttggcgta taacatagta tcgacggagc cgattttgaa accacaatta 12627
   tgggtgatgc tgccaactta ctgatttagt gtatgatggt gtttttgagg tgctccagtg 12687
   gcttctgtgt ctatcagctg tccctcctgt tcagctactg acggggtggt gcgtaacggc 12747
   aaaagcaccg ccggacatca gcgctatctc tgctctcact gccgtaaaac atggcaactg 12807
   cagttcactt acaccgcttc tcaacccggt acgcaccaga aaatcattga tatggccatg 12867
   aatggcgttg gatgccgggc aacagcccgc attatgggcg ttggcctcaa cacgatttta 12927
   cgtcacttaa aaaactcagg ccgcagtcgg taacctcgcg catacagccg ggcagtgacg 12987
   tcatcgtctg cgcggaaatg gacgaacagt ggggctatgt cggggctaaa tcgcgccagc 13047
   gctggctgtt ttacgcgtat gacagtctcc ggaagacggt tgttgcgcac gtattcggtg 13107
   aacgcactat ggcgacgctg gggcgtctta tgagcctgct gtcacccttt gacgtggtga 13167
   tatggatgac ggatggctgg ccgctgtatg aatcccgcct gaagggaaag ctgcacgtaa 13227
   tcagcaagcg atatacgcag cgaattgagc ggcataacct gaatctgagg cagcacctgg 13287
   cacggctggg acggaagtcg ctgtcgttct caaaatcggt ggagctgcat gacaaagtca 13347
   tcgggcatta tctgaacata aaacactatc aataagttgg agtcattacc caattatgat 13407
   agaatttaca agctataagg ttattgtcct gggtttcaag cattagtcca tgcaagtttt 13467
   tatgctttgc ccattctata gatatattga taagcgcgct gcctatgcct tgccccctga 13527
   aatccttaca tacggcgata tcttctatat aaaagatata ttatcttatc agtattgtca 13587
   atatattcaa ggcaatctgc ctcctcatcc tcttcatcct cttcgtcttg gtagcttttt 13647
   aaatatggcg cttcatagag taattctgta aaggtccaat tctcgttttc atacctcggt 13707
   ataatcttac ctatcacctc aaatggttcg ctgggtttat cgcacccccg aacacgagca 13767
   cggcacccgc gaccactatg ccaagaatgc ccaaggtaaa aattgccggc cccgccatga 13827
   agtccgtgaa tgccccgacg gccgaagtga agggcaggcc gccacccagg ccgccgccct 13887
   cactgcccgg cacctggtcg ctgaatgtcg atgccagcac ctgcggcacg tcaatgcttc 13947
   cgggcgtcgc gctcgggctg atcgcccatc ccgttactgc cccgatcccg gcaatggcaa 14007
   ggactgccag cgctgccatt tttggggtga ggccgttcgc ggccgagggg cgcagcccct 14067
   ggggggatgg gaggcccgcg ttagcgggcc gggagggttc gagaaggggg ggcacccccc 14127
   ttcggcgtgc gcggtcacgc gcacagggcg cagccctggt taaaaacaag gtttataaat 14187
   attggtttaa aagcaggtta aaagacaggt tagcggtggc cgaaaaacgg gcggaaaccc 14247
   ttgcaaatgc tggattttct gcctgtggac agcccctcaa atgtcaatag gtgcgcccct 14307
   catctgtcag cactctgccc ctcaagtgtc aaggatcgcg cccctcatct gtcagtagtc 14367
   gcgcccctca agtgtcaata ccgcagggca cttatcccca ggcttgtcca catcatctgt 14427
   gggaaactcg cgtaaaatca ggcgttttcg ccgatttgcg aggctggcca gctccacgtc 14487
   gccggccgaa atcgagcctg cccctcatct gtcaacgccg cgccgggtga gtcggcccct 14547
   caagtgtcaa cgtccgcccc tcatctgtca gtgagggcca agttttccgc gaggtatcca 14607
   caacgccggc ggccgcggtg tctcgcacac ggcttcgacg gcgtttctgg cgcgtttgca 14667
   gggccataga cggccgccag cccagcggcg agggcaacca gcccggtgag cgtcgcaaag 14727
   gcgctcggtc ttgccttgct cgtcggtgat gtacttcacc agctccgcga agtcgctctt 14787
   cttgatggag cgcatgggga cgtgcttggc aatcacgcgc accccccggc cgttttagcg 14847
   gctaaaaaag tcatggctct gccctcgggc ggaccacgcc catcatgacc ttgccaagct 14907
   cgtcctgctt ctcttcgatc ttcgccagca gggcgaggat cgtggcatca ccgaaccgcg 14967
   ccgtgcgcgg gtcgtcggtg agccagagtt tcagcaggcc gcccaggcgg cccaggtcgc 15027
   cattgatgcg ggccagctcg cggacgtgct catagtccac gacgcccgtg attttgtagc 15087
   cctggccgac ggccagcagg taggccgaca ggctcatgcc ggccgccgcc gccttttcct 15147
   caatcgctct tcgttcgtct ggaaggcagt acaccttgat aggtgggctg cccttcctgg 15207
   ttggcttggt ttcatcagcc atccgcttgc cctcatctgt tacgccggcg gtagccggcc 15267
   agcctcgcag agcaggattc ccgttgagca ccgccaggtg cgaataaggg acagtgaaga 15327
   aggaacaccc gctcgcgggt gggcctactt cacctatcct gcccggctga cgccgttgga 15387
   tacaccaagg aaagtctaca cgaacccttt ggcaaaatcc tgtatatcgt gcgaaaaagg 15447
   atggatatac cgaaaaaatc gctataatga ccccgaagca gggttatgca gcggaaaagc 15507
   gccacgcttc ccgaagggag aaaggcggac aggtatccgg taagcggcag ggtcggaaca 15567
   ggagagcgca cgagggagct tccaggggga aacgcctggt atctttatag tcctgtcggg 15627
   tttcgccacc tctgacttga gcgtcgattt ttgtgatgct cgtcaggggg gcggagccta 15687
   tggaaaaacg ccagcaacgc ggccttttta cggttcctgg ccttttgctg gccttttgct 15747
   cacatgttct ttcctgcgtt atcccctgat tctgtggata accgtattac cgcctttgag 15807
   tgagctgata ccgctcgccg cagccgaacg accgagcgca gcgagtcagt gagcgaggaa 15867
   gcggaagagc gccagaaggc cgccagagag gccgagcgcg gccgtgaggc ttggacgcta 15927
   gggcagggca tgaaaaagcc cgtagcgggc tgctacgggc gtctgacgcg gtggaaaggg 15987
   ggaggggatg ttgtctacat ggctctgctg tagtgagtgg gttgcgctcc ggcagcggtc 16047
   ctgatcaatc gtcacccttt ctcggtcctt caacgttcct gacaacgagc ctccttttcg 16107
   ccaatccatc gacaatcacc gcgagtccct gctcgaacgc tgcgtccgga ccggcttcgt 16167
   cgaaggcgtc tatcgcggcc cgcaacagcg gcgagagcgg agcctgttca acggtgccgc 16227
   cgcgctcgcc ggcatcgctg tcgccggcct gctcctcaag cacggcccca acagtgaagt  16287
   agctgattgt catcagcgca ttgacggcgt ccccggccga aaaacccgcc tcgcagagga  16347
   agcgaagctg cgcgtcggcc gtttccatct gcggtgcgcc cggtcgcgtg ccggcatgga  16407
   tgcgcgcgcc atcgcggtag gcgagcagcg cctgcctgaa gctgcgggca ttcccgatca  16467
   gaaatgagcg ccagtcgtcg tcggctctcg gcaccgaatg cgtatgattc tccgccagca  16527
   tggcttcggc cagtgcgtcg agcagcgccc gcttgttcct gaagtgccag taaagcgccg  16587
   gctgctgaac ccccaaccgt tccgccagtt tgcgtgtcgt cagaccgtct acgccgacct  16647
   cgttcaacag gtccagggcg gcacggatca ctgtattcgg ctgcaacttt gtcatgcttg  16707
   acactttatc actgataaac ataatatgtc caccaactta tcagtgataa agaatccgcg  16767
   cgttcaatcg gaccagcgga ggctggtccg gaggccagac gtgaaaccca acatacccct  16827
   gatcgtaatt ctgagcactg tcgcgctcga cgctgtcggc atcggcctga ttatgccggt  16887
   gctgccgggc ctcctgcgcg atctggttca ctcgaacgac gtcaccgccc actatggcat  16947
   tctgctggcg ctgtatgcgt tggtgcaatt tgcctgcgca cctgtgctgg gcgcgctgtc  17007
   ggatcgtttc gggcggcggc caatcttgct cgtctcgctg gccggcgcca gatc        17061
<210> 35
   <211> 290
   <212> PRT
   <213> Phaeodactylum tricornutum, Physcomitrella patens, Caenorhabditis elegans
<400> 35
   Met Glu Val Val Glu Arg Phe Tyr Gly Glu Leu Asp Gly Lys Val Ser
   1 5 10 15
   Gln Gly Val Asn Ala Leu Leu Gly Ser Phe Gly Val Glu Leu Thr Asp
   20 25 30
   Thr Pro Thr Thr Lys Gly Leu Pro Leu Val Asp Ser Pro Thr Pro Ile
   35 40 45
   Val Leu Gly Val Ser Val Tyr Leu Thr Ile Val Ile Gly Gly Leu Leu
   50 55 60
   Trp Ile Lys Ala Arg Asp Leu Lys Pro Arg Ala Ser Glu Pro Phe Leu
   65 70 75 80
   Leu Gln Ala Leu Val Leu Val His Asn Leu Phe Cys Phe Ala Leu Ser
   85 90 95
   Leu Tyr Met Cys Val Gly Ile Ala Tyr Gln Ala Ile Thr Trp Arg Tyr
   100 105 110
   Ser Leu Trp Gly Asn Ala Tyr Asn Pro Lys His Lys Glu Met Ala Ile
   115 120 125
   Leu Val Tyr Leu Phe Tyr Met Ser Lys Tyr Val Glu Phe Met Asp Thr
   130 135 140
   Val Ile Met Ile Leu Lys Arg Ser Thr Arg Gln Ile Ser Phe Leu His
   145 150 155 160
   Val Tyr His His Ser Ser Ile Ser Leu Ile Trp Trp Ala Ile Ala His
   165 170 175
   His Ala Pro Gly Gly Glu Ala Tyr Trp Ser Ala Ala Leu Asn Ser Gly
   180 185 190
   Val His Val Leu Met Tyr Ala Tyr Tyr Phe Leu Ala Ala Cys Leu Arg
   195 200 205
   Ser Ser Pro Lys Leu Lys Asn Lys Tyr Leu Phe Trp Gly Arg Tyr Leu
   210 215 220
   Thr Gln Phe Gln Met Phe Gln Phe Met Leu Asn Leu Val Gln Ala Tyr
   225 230 235 240
   Tyr Asp Met Lys Thr Asn Ala Pro Tyr Pro Gln Trp Leu Ile Lys Ile
   245 250 255
   Leu Phe Tyr Tyr Met Ile Ser Leu Leu Phe Leu Phe Gly Asn Phe Tyr
   260 265 270
   Val Gln Lys Tyr Ile Lys Pro Ser Asp Gly Lys Gln Lys Gly Ala Lys
   275 280 285
   Thr Glu
   290
<210> 36
   <211> 282
   <212> PRT
   <213> Phaeodactylum tricornutum, Physcomitrella patens, Caenorhabditis elegans
<400> 36
   Met Glu Asn Phe Trp Ser Ile Val Val Phe Phe Leu Leu Ser Ile Leu
   1 5 10 15
   Phe Ile Leu Tyr Asn Ile Ser Thr Val Cys His Tyr Tyr Met Arg Ile
               20                  25                  30
   Ser Phe Tyr Tyr Phe Thr Ile Leu Leu His Gly Met Glu Val Cys Val
           35                  40                  45
   Thr Met Ile Pro Ser Trp Leu Asn Gly Lys Gly Ala Asp Tyr Val Phe
       50                  55                  60
   His Ser Phe Phe Tyr Trp Cys Lys Trp Thr Gly Val His Thr Thr Val
   65                  70                  75                  80
   Tyr Gly Tyr Glu Lys Thr Gln Val Glu Gly Pro Ala Val Val Ile Cys
                   85                  90                  95
   Asn His Gln Ser Ser Leu Asp Ile Leu Ser Met Ala Ser Ile Trp Pro
               100                 105                 110
   Lys Asn Cys Val Val Met Met Lys Arg Ile Leu Ala Tyr Val Pro Phe
           115                 120                 125
   Phe Asn Leu Gly Ala Tyr Phe Ser Asn Thr Ile Phe Ile Asp Arg Tyr
       130                 135                 140
   Asn Arg Glu Arg Ala Met Ala Ser Val Asp Tyr Cys Ala Ser Glu Met
   145                 150                 155                 160
   Lys Asn Arg Asn Leu Lys Leu Trp Val Phe Pro Glu Gly Thr Arg Asn
                   165                 170                 175
   Arg Glu Gly Gly Phe Ile Pro Phe Lys Lys Gly Ala Phe Asn Ile Ala
               180                 185                 190
   Val Arg Ala Gln Ile Pro Ile Ile Pro Val Val Phe Ser Asp Tyr Arg
           195                 200                 205
   Asp Phe Tyr Ser Lys Pro Gly Arg Tyr Phe Lys Asn Asp Gly Glu Val
       210                 215                 220
   Val Ile Arg Val Leu Asp Ala Ile Pro Thr Lys Gly Leu Thr Leu Asp
   225                 230                 235                 240
   Asp Val Ser Glu Leu Ser Asp Met Cys Arg Asp Val Met Leu Ala Ala
                   245                 250                 255
   Tyr Lys Glu Val Thr Leu Glu Ala Gln Gln Arg Asn Ala Thr Arg Arg
   260 265 270
   Gly Glu Thr Lys Asp Gly Lys Lys Ser Glu
   275 280
<210> 37
   <211> 477
   <212> PRT
   <213> Phaeodactylum tricornutum, Physcomitrella patens, Caenorhabditis elegans
<400> 37
   Met Gly Lys Gly Gly Asp Ala Arg Ala Ser Lys Gly Ser Thr Ala Ala
   1               5                   10                  15
   Arg Lys Ile Ser Trp Gln Glu Val Lys Thr His Ala Ser Pro Glu Asp
               20                  25                  30
   Ala Trp Ile Ile His Ser Asn Lys Val Tyr Asp Val Ser Asn Trp His
           35                  40                  45
   Glu His Pro Gly Gly Ala Val Ile Phe Thr His Ala Gly Asp Asp Met
       50                  55                  60
   Thr Asp Ile Phe Ala Ala Phe His Ala Pro Gly Ser Gln Ser Leu Met
   65                  70                  75                  80
   Lys Lys Phe Tyr Ile Gly Glu Leu Leu Pro Glu Thr Thr Gly Lys Glu
                   85                  90                  95
   Pro Gln Gln Ile Ala Phe Glu Lys Gly Tyr Arg Asp Leu Arg Ser Lys
               100                 105                 110
   Leu Ile Met Met Gly Met Phe Lys Ser Asn Lys Trp Phe Tyr Val Tyr
           115                 120                 125
   Lys Cys Leu Ser Asn Met Ala Ile Trp Ala Ala Ala Cys Ala Leu Val
       130                 135                 140
   Phe Tyr Ser Asp Arg Phe Trp Val His Leu Ala Ser Ala Val Met Leu
   145                 150                 155                 160
   Gly Thr Phe Phe Gln Gln Ser Gly Trp Leu Ala His Asp Phe Leu His
                   165                 170                 175
   His Gln Val Phe Thr Lys Arg Lys His Gly Asp Leu Gly Gly Leu Phe
               180                 185                 190
   Trp Gly Asn Leu Met Gln Gly Tyr Ser Val Gln Trp Trp Lys Asn Lys
   195 200 205
   His Asn Gly His His Ala Val Pro Asn Leu His Cys Ser Ser Ala Val
   210 215 220
   Ala Gln Asp Gly Asp Pro Asp Ile Asp Thr Met Pro Leu Leu Ala Trp
   225 230 235 240
   Ser Val Gln Gln Ala Gln Ser Tyr Arg Glu Leu Gln Ala Asp Gly Lys
   245 250 255
   Asp Ser Gly Leu Val Lys Phe Met Ile Arg Asn Gln Ser Tyr Phe Tyr
   260 265 270
   Phe Pro Ile Leu Leu Leu Ala Arg Leu Ser Trp Leu Asn Glu Ser Phe
   275 280 285
   Lys Cys Ala Phe Gly Leu Gly Ala Ala Ser Glu Asn Ala Ala Leu Glu
   290 295 300
   Leu Lys Ala Lys Gly Leu Gln Tyr Pro Leu Leu Glu Lys Ala Gly Ile
   305 310 315 320
   Leu Leu His Tyr Ala Trp Met Leu Thr Val Ser Ser Gly Phe Gly Arg
   325 330 335
   Phe Ser Phe Ala Tyr Thr Ala Phe Tyr Phe Leu Thr Ala Thr Ala Ser
   340 345 350
   Cys Gly Phe Leu Leu Ala Ile Val Phe Gly Leu Gly His Asn Gly Met
   355 360 365
   Ala Thr Tyr Asn Ala Asp Ala Arg Pro Asp Phe Trp Lys Leu Gln Val
   370 375 380
   Thr Thr Thr Arg Asn Val Thr Gly Gly His Gly Phe Pro Gln Ala Phe
   385 390 395 400
   Val Asp Trp Phe Cys Gly Gly Leu Gln Tyr Gln Val Asp His His Leu
   405 410 415
   Phe Pro Ser Leu Pro Arg His Asn Leu Ala Lys Thr His Ala Leu Val
   420 425 430
   Glu Ser Phe Cys Lys Glu Trp Gly Val Gln Tyr His Glu Ala Asp Leu
   435 440 445
   Val Asp Gly Thr Met Glu Val Leu His His Leu Gly Ser Val Ala Gly
   450 455 460
   Glu Phe Val Val Asp Phe Val Arg Asp Gly Pro Ala Met
   465 470 475